# EUROPÄISCHE PATENTANMELDUNG

(11) **EP 0 031 794 A2**
(43) Veröffentlichungstag der Anmeldung: **08.07.1981**
(21) Anmeldenummer: 80810386.5
(22) Anmeldetag: 15.12.1980
(51) Int. Cl.: C07D 501/20, C07D 501/57, C07D 501/59, A61K 31/545, C07D 233/38, C07D 317/28

(54) **Cephalosporinderivative, Verfahren zu ihrer Herstellung und sie enthaltende pharmazeutische Zusammensetzungen**

(30) Priorität: 19.12.1979 CH 11283/79
(71) Anmelder: CIBA-GEIGY AG, 4002 Basel (CH)
(72) Erfinder: Wehrli, Hansuli, Dr., CH-4153 Reinach (CH); Kocsis, Karoly, Dr., CH-4305 Olsberg (CH); Scartazzini, Riccardo, Dr., CH-4059 Basel (CH)

(57) **Zusammenfassung**

Verbindungen der Formel 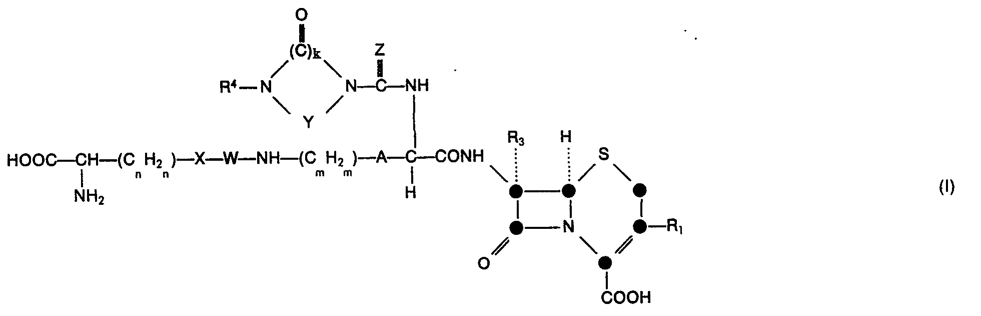 worin der Index n eine ganze Zahl von 1 bis 4, der Index m 0 oder 1, X Sauerstoff, Schwefel oder die Gruppe -NH-, W eine Gruppe -CO-, -CO-NHSO₂- oder -SO₂NH-CO-, oder X-W zusammen eine Gruppe -CO- oder -CO-NHSO₂-, A gegebenenfalls substituiertes Phenylen, Thienylen oder Furylen, Z Sauerstoff oder Schwefel, Y Niederalkylen, der Index k den Wert 1 oder 2, R₄ Wasserstoff, einen gegebenenfalls substituierten niederaliphatischen oder cycloaliphatischen Rest oder Acyl, R₁ Wasserstoff, Niederalkyl, Niederalkoxy, Halogen oder eine Gruppe der Formel . -CH₂-R₂, worin R₂ eine freie, veresterte oder verätherte Hydroxy- oder Mercaptogruppe oder eine quaternäre Ammoniumgruppe darstellt, und R₃ Wasserstoff oder Methoxy bedeuten, worin die Carboxylgruppen gegebenenfalls in physiologisch spaltbarer Form verestert sind, und Salze von solchen Verbindungen mit salzbildenden Gruppen werden erhalten, indem man in einer Verbindung der Formel I, worin mindestens eine der vorhandenen funktionellen Gruppen geschützt ist, die funktionelle(n) Gruppe(n) freisetzt.

Die Verbindungen sind in vitro und in vivo gegen gram-positive und gram-negative Bakterien und Kokken wirksam.

## Beschreibung

Die Erfindung betrifft neue Acylamino-3-cephem-4-carbonsäure-Verbindungen und ihre Salze, Verfahren zur ihrer Herstellung, diese Verbindungen enthaltande pharmazeutische Mittel mit antibiotischer Wirksamkeit und ihre therapeutische Verwendung zur Behandlung von Infektionen, sowie neue Zwischenprodukte und deren Herstellung.

Es sind bereits zahlreiche 7ß-Acylamino-3-cephem-4-carbonsäure-Verbindungen bekannt geworden, die sich durch die Substituenten in 3-Stellung des 3-Cephongerüstes, sowie durch die Acylgruppe an der 7ß-Aminogruppe unterscheiden. Uebersichten über solche Verbindungen, Verfahren zu ihrer Herstellung, sowie such über ihre antibiotischen Aktivitäten wurden beispielsweise von Edwin H. Flynn, Cephalosporins and Penicillina, Academic Press, New York und London. 1972, J. Cs. Jászberény und T.E. Gunda, Progr. Med. Chm., Vol. 12, 1975, S. 395-477, und Peter G. Sammes, Chemical Reviews, 1976, Vol. 76, No. 1, S. 113-155, veröffentlicht.

Des Auftreten von neuen pathogenen Keimen, die gegen die bisher benutzten Antibiotika resistent geworden sind, sowie die bekannten Allergieerscheinungen, wecken das Bedürfnis nach neuen, aktiven Verbindungen, die die genannten Nachteile in geringeren Masse oder überhaupt nicht besitzen.

Der vorliegenden Erfindung liegt die Aufgabe zugrunde, neue 7β-Acylamino-3-cephem-4-carbonsäure-Verbindungen herzustellen, die neuartige Acylgruppen besitzen, welche durch das Vorhandensein einer endständigen α-Aminocarbonsäursgruppierung und einer Azacyclyl(thio)-ureidogruppe charakterisiert sind. Die neuen Verbindungen zeichnen sich durch ein breites Wirküngsspektrum gegenüber normalen und resistenten Keimen, insbesondere auch gegenüber Pseudomonas, aus.

Die als Ausgangsmaterialien zu benutzenden, die neuartigen Acylgruppen enthaltenden entsprechenden Carbonsäuren und deren reaktionsfähige funktionelle Derivate, worin funktionelle Gruppen gegebenenfalls geschützt sind, sowie Verfahren zu ihrer Herstellung, sind ebenfalls Gegenstand der vorliegenden Erfindung.

Die Erfindung betrifft insbesondere Acylamino-3-cephem-4-carbonsäure-Verbindungen der Formel 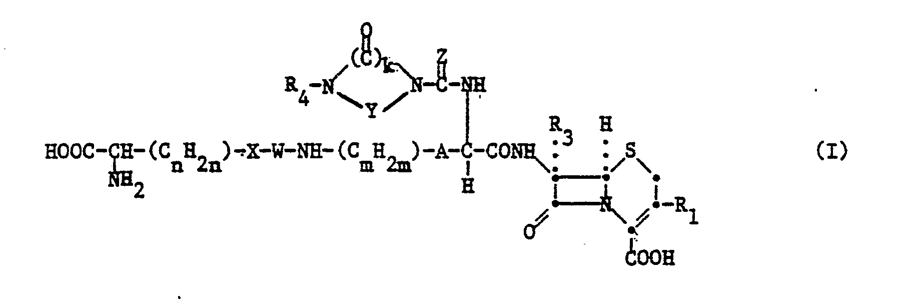 worin der Index n eine ganze Zahl von 1 bis 4, der Index m 0 oder 1, X Sauerstoff, Schwefel oder die Gruppe -NH-, W eine Gruppe -CO-, -CO-NHSO₂ oder -SO₂NH-CO-, oder X-W zusammen eine Gruppe -CO- oder -CO-NHSO₂-, A gegebenenfalls substituiertes Phenylen, Thienylen oder Furylen, Z Sauerstoff oder Schwefel, Y Niederalkylen, der Index k den Wert 1 oder 2, R₄ Wasserstoff, einen gegebenenfalls substituierten niederaliphatischen oder cycloaliphatischen Rest oder Acyl, R₁ Wasserstoff, Niederalkyl, Niederalkoxy, Halogen oder eine Gruppe der Formel -CH₂-R₂, worin R₂ eine freie, veresterte oder verätherte Hydroxy- oder Mercaptogruppe oder eine quaternäre Ammoniumgruppe darstellt, und R₃ Wasserstoff oder Methoxy bedeuten, worin funktionelle Gruppen gegebenenfalls in geschützter Form vorliegen, Salze von solchen Verbindungen mit sauren und/oder basischen Gruppen, Verfahren zur Herstellung dieser Verbindungen, solche Stoffe enthaltende pharmazeutische Mittel und deren therapeutische Verwendung. '

In der vorliegenden Beschreibung der Erfindung bedeutet der Ausdruck "Nieder" in Gruppen wie Niederalkyl, Niederalkylen, Niederalkoxy, Niederalkanoyl und dergleichen, dass die entsprechenden Grup- - pen, sofern nicht ausdrücklich anders definiert, bis zu 7, bevorzugt bis zu 4 C-Atome enthalten.

Eine Gruppe -(CₙH₂ₙ) - ist eine verzweigte oder unverzweigte Alkylenkette, und ist insbesondere Methylen, 1,2-Aethylen, 1,3-Propylen oder 1,4-Butylen, ferner beispielsweise 1,1-Aethylen, 1,1-Propylen, 1,2-Propylen, 1,1-Butylen, oder 1,1-Isobutylen.

Eine gegebenenfalls substituierte Phenylengruppe A ist insbesondere p-, kann aber auch o- oder m-Phenylen sein. Substitutenten der Phenylengruppe sind beispielsweise Niederalkyl, wie Methyl, Hydroxy, Niederalkoxy, wie Methoxy, und/oder Halogen, wie Fluor, Chlor oder Brom.

Eine gegebenenfalls substituierte Thienylengruppe A ist insbesondere 2,5-Thienylen, ferner 2,4- oder 2,3-Thienylen.

Eine gegebenenfalls substituierte Furylengruppe A ist insbesondere 2,5-Furylen, ferner 2,4- oder 2,3-Furylen.

Substitutenten der Thienylen- und Furylengruppe A sind beispielsweise Niederalkyl, wie Methyl, Niederalkoxy, wie Methoxy, und/oder Halogen, wie Fluor, Chlor oder Brom.

Der Niederalkylenrest Y ist gegebenenfalls verzweigt und über einander benachbarte Kohlenstoffatome an die beiden Ring-Stickstoffatome gebunden. Y kann 2-7 Kohlenstoffatome enthalten und beispielsweise ein entsprechend gebundenes Heptylen, z.B. 1.2- oder 2.3-Heptylen, sein, ist aber vorzugsweise ein entsprechender Rest mit 2-4 Kohlenstoffatomen, z.B. 1.2- oder 2.3-Butylen, und insbesondere 1.2₋Aethylen.

Ein niederaliphatischer Rest R₄ ist Niederalkyl, Niederalkenyl oder Niederalkinyl.

Ein Niederalkylrest R₄ ist insbesondere ein Rest mit 1-4 Kohlenstoffatomen, wie Methyl, Isopropyl oder Isobutyl, vor allem Aethyl.

Als Niederalkenyl besitzt R₄ vorzugsweise 2-5 Kohlenstoffatome und die Doppelbindung geht vorzugsweise vom bindenden Kohlenstoffatom (1-Stellung) aus. Beispiele für derartige Reste sind Vinyl, Propenyl und insbesondere 1-Isobutenyl.

Als Niederalkinyl besitzt R, vorzugsweise 2-4 Kohlenstoffatom und ist vor allem Aethinyl und 1-Propinyl.

Ein cycloaliphatischer Rest R₄ ist Cycloalkyl mit 3-8, vorzugsweise 3-6 C-Atomen und ist vor allem Cyclopropyl, Cyclobutyl, Cyclopentyl oder Cyclohexyl.

Substituenten für vorstehend genannte Reste R₄, insbesondere für ein entsprechendes Niederalkyl, sind Hydroxy, Niederalkanoyloxy, Niederalkoxycarbonyloxy, Niederalkoxy, Niederalkylthio, Halogen, insbesondere Chlor oder Brom, gegebenenfalls funktionell abgewandeltes Carboxy und gegebenenfalls niederalkyliertes, wie diniederalkyliertes Amino. Bevorzugte Substituenten sind Hydroxy und Niederalkoxy, die vorzugsweise am bindenden Kohlenstoffatom (also in 1-Stellung) fixiert sind. Beispiele für substituierte Reste dieser Art sind 1-Hydroxyäthyl, Methoxymethyl, 1-Methoxyäthyl, 2-Methoxyäthyl, 4-Methoxybutyl und insbesondere 1-Hydroxyisobutyl.

Ein Acylrest R₄ ist ein von einer Carbonsäure mit bis zu 12 Kohlenstoffatomen, einem Halbester der Kohlensäure oder Thiokohlensäure, einer aromatischen oder niederaliphatischen Sulfonsäure oder einem gegebenenfalls N-substituierten Halbamid der Kohlen-, Thiokohlen-, Iminokohlen- oder Schwefelsäure abgeleiteter Acylrest.

Ein Carbonsäurerest R₄ ist in erster Linie ein aromatischer, wie gegebenenfalls durch Halogen, z.B. Chlor, Niederalkyl, Hydroxy, Niederalkoxy, Diniederalkylamino, z.B. Dimethylamino, Niederalkylsulfonylamino, z.B. Mesylamino, Niederalkanoylamino, z.B. Acetamido, Amino oder Nitro substituierter Naphthoe- oder Benzoesäurerest, ein gegebenenfalls durch Niederalkyl oder Halogen, z.B. Chlor, substituierter heterocyclischer Carbonsäurerest, wie ein entsprechender Pyridin-, Thiophen-, Furan- oder Pyrrol-Carbonsäurerest oder insbesondere ein gegebenenfalls durch Halogen, z.B. Chlor, Niederalkoxy, Niederalkanoyloxy, Cyan oder Phenoxy substituierter niederaliphatischer Carbonsäurerest, wie entsprechendes Niederalkanoyl, z.B. entsprechendes Acetyl. Ein Kohlensäure- oder Thiokohlensäurehalbesterrest R₄ ist z.B.Niederalkoxycarbonyl z.B. Aethoxycarbonyl, Arylniederalkoxycarbonyl, z.B. Benzyloxycarbonyl, Cycloalkoxycarbonyl, z.B. Cyclohexyloxycarbonyl, oder Aryloxycarbonyl, z.B. Phenoxycarbonyl, oder entsprechendes -thiocarbonyl.

Ein von einer aromatischen Sulfonsäure abgeleiteter Acylrest R₄ ist 1- oder 2-Naphthalinsulfonyl oder gegebenenfalls durch Halogen, z.B. Chlor oder Niederalkyl substituiertes Benzolsulfonyl, z.B. Benzolsulfonyl, Chlor-, z.B. o-Chlorbenzolsulfonyl oder Tosyl, z.B. p-Tosyl. Ein von einer niederaliphatischen Sulfonsäure abgeleiteter Acylrest R₄ ist vor allem Niederalkylsulfonyl, z.B. Mesyl.

Ein von einem gegebenenfalls N-substituierten, wie N-niederalkylierten Halbamid der Kohlensäure, Thiokohlensäure, Iminokohlensäure oder Schwefelsäure abgeleiteter Acylrest ist vor allem ein am Stickstoffatom gegebenenfalls durch Niederalkyl, Halogen-, z.B.

Chlorniederalkyl oder Niederalkoxyniederalkyl; z.B. Methoxyäthyl oder Aethoxyäthyl, mono- oder di-substituiertes Carbamoyl, Thiocarbamoyl, Iminocarbamoyl oder Sulfamoyl.

Eine Niederalkylgruppe R enthält 1-4 C-Atome und ist beispielsweise Aethyl, Propyl, Butyl oder insbesondere Methyl.

Eine Niederalkoxygruppe R₁ enthält 1-4 C-Atome und ist beispielsweise Methoxy, Aethoxy, Propoxy oder Butoxy.

R₁ als Halogen ist Fluor, Brom, Jod oder bevorzugt Chlor.

Eine veresterte Hydroxy- oder Mercaptogruppe R₂ ist durch eine niederaliphatische Carbonsäure oder durch eine gegebenenfalls N-substituierte Carbaminsäure verestert.

Mit niederaliphatischen Carbonsäuren veresterte Hydroxygruppen R₂ sind insbesondere Niederalkanoyloxy, insbesondere Acetyloxy, ferner Formyloxy, Propionyloxy, Valeryloxy, Hexanoyloxy, Heptanoyloxy oder Pivaloyloxy.

Mit niederaliphatischen Carbonsäuren veresterte Mercaptogruppen R₂ sind Niederalkanoylthio, wie Acetylthio, Formylthio, Propionylthio, Valeroylthio, Hexanoylthio, Heptanoylthio oder Pivaloylthio.

In einer durch eine gegebenenfalls N-substituierte Carbaminsäure veresterte Hydroxy- oder Mercaptogruppe R₂ sind N-Substituenten gegebenenfalls durch Halogen, z.B. Chlor, oder durch Niederalkanoyloxy, z.B. Acetoxy oder Propionyloxy, substituiertes Niederalkyl, z.B. Methyl, Aethyl, 2-Chloräthyl, oder 2-Acetoxyäthyl. In dieser Art veresterte Hydroxygruppen R₂ sind z.B. Carbamoyloxy, N-Methylcarbamoyloxy, N-Aethylcarbamoyloxy, N-(2-Chloräthyl)-carbamoyloxy oder N-(2-Acetoxyäthyl)-carbamoyloxy. Entsprechende veresterte Mercaptogruppen R₂ sind z.B. Carbamoylthio, N-Methylcarbamoylthio, N-Aethylcarbamoylthio; N-(2-Chloräthyl)-carbamoylthio oder N-(2-Acetoxyäthyl)-carbamoylthio.

Verätherte Hydroxy- und Mercaptogruppen R₂ sind beispielsweise mit einem aliphatischen Kohlenwasserstoffrest veräthert, und sind insbesondere Niederalkoxy, insbesondere mit 1-4 C-Atomen, in erster Linie Methoxy, sowie Aethoxy, n-Propyloxy oder Isopropyloxy, ferner geradkettiges oder verzweigtes Butyloxy, oder Niederalkylthio, vorzugsweise mit 1-4 C-Atomen, in erster Linie Methylthio, sowie Aethylthio, n-Propylthio oder Isopropylthio, ferner geradkettiges oder verzweigtes Butylthio.

Verätherte Mercaptogruppen R₂ sind insbesondere durch einen gegebenenfalls substituierten, über ein Ringkohlenstoffatom an die Mercaptogruppe gebundenen, heterocyclischen Rest mit 1 bis 4 Ringstickstoffatomen und gegebenenfalls einem weiteren Ringheteroatom der Gruppe Sauerstoff und Schwefel veräthert.

Solche heterocyclische Reste sind in erster Linie gegebenenfalls substituierte, z.B. die unten genannten Substituenten enthaltende, monocyclische, fünfgliedrige und sechsgliedrige diaza-, triaza-, tetraza-, thiaza-, thiadiaza-, thiatriaza-, oxaza- oder oxadiazacyclische Reste aromatischen Charakters.

Substituenten solcher Heterocyclylreste sind u.a. Niederalkyl, insbesondere Methyl, sowie Aethyl, n-Propyl, Isopropyl oder geradkettiges oder verzweigtes Butyl, oder durch Hydroxy, verestertes Hydroxy, wie Niederalkanoyloxy, Halogen, wie Chlor, Carboxy, verestertes Carboxy, wie Niederalkoxycarbonyl, Sulfo, amidiertes Sulfo, Amino, Mono- oder Diniederalkylamino, Acylamino, wie Niederalkanoylamino, oder durch substituiertes, wie durch Carboxy oder Halogen substituiertes Niederalkanoylamino substituiertes Niederalkyl, beispielsweise 2-Hydroxyäthyl, 2-Acetoxyäthyl, 2-Chloräthyl, Carboxymethyl, 2-Carboxyäthyl, Aethoxycarbonylmethyl, 2-Aethoxycarbonyl- äthyl, Sulfomethyl, 2-Sulfoäthyl, Sulfamylmethyl, 2-Sulfamyläthyl, 2-Aminoäthyl, 2-Dimethylaminoäthyl oder 2-Acetylaminoäthyl. Weitere Substituenten des heterocyclischen Restes sind Cycloalkyl, z.B. Cyclopentyl oder Cyclohexyl, Aryl, wie gegebenenfalls durch Halogen, z.B. Chlor, oder Nitro substituiertes Phenyl, Arylniederalkyl, z.B. Benzyl, oder Heterocyclyl, wie Furyl, z.B. 2-Furyl, Thienyl, z.B. 2-Thienyl, oder Oxazolyl, z.B. 2- oder 5-Oxazolyl, oder funktionelle Gruppen, wie Halogen, z.B. Fluor, Chlor oder Brom, gegebenenfalls substituiertes Amino, wie gegebenenfalls durch Niederalkyl mono- oder di-substituiertes Amino, z.B. Amino, Methylamino oder Dimethylamino, Acylamino, wie Niederalkanoylamino oder durch Halogen oder Carboxy substituiertes Niederalkanoylamino, wie Acetylamino, 3-Chlorpropionylamino oder 3-Carboxypropionylamino, Nitro, Hydroxy, Niederalkoxy, z.B. Methoxy, Aethoxy, n-Butyloxy oder 2-Aethylhexyloxy, oder gegebenenfalls funktionell abgewandeltes Carboxy, wie Carboxy, verestertes Carboxy, wie Niederalkoxycarbonyl, z.B. Methoxycarbonyl oder = Aethoxycarbonyl, gegebenenfalls substituiertes, wie N-mono- oder N,N-diniederalkyliertes Carbamoyl, z.B. N-Methylcarbamoyl oder N,N-Dimethylcarbamoyl, oder Cyan, sowie Oxo oder Oxido, wobei einer oder mehrere solche Substituenten, die in erster Linie mit Ringkohlenstoffatomen aber auch, insbesondere Niederalkyl und Oxido, mit Ringstickstoffatomen verbunden sind, vorhanden sein können.

Bevorzugte heterocyclisch verätherte Mercaptogruppen R₂, worin der heterocyclische Rest einen entsprechenden monocyclischen, fünfgliedrigen Rest darstellt, sind u.a. Imidazolylthio, z.B. 2-Imidazolylthio, gegebenenfalls durch Niederalkyl und/oder Phenyl substituiertes Triazolylthio, z.B. 1H-1,2,3-Triazol-4-ylthio, 1-Methyl-lH-1,2,3-triazol-4-ylthio, 1H-1,2,4-Triazol-3-ylthio, 5-Methyl-1H-1,2,4-triazol-3-ylthio, 3-Methyl-1-phenyl-1H-1,2,4-triazol-5-ylthio, 4,5-Dimethyl-4H-1,2,4-triazol-3-yithio oder 4-Phenyl-4H-1,2,4-triazol-3-ylthio, insbesondere gegebenenfalls wie angegeben substituiertes Tetrazolylthio, z.B. 1H-Tetrazol-5-ylthio, 1-Methyl-lH-tetrazol-5- ylthio, 1-Carboxymethyl-1H-tetrazol-5-ylthio, 1-(2-Carboxyäthyl)-lH- tetrazol-5₋ylthio, 1-Sulfomethyl-lH-tetrazol-5-ylthio, 1-(2-Sulfoäthyl)-1H-tetrazol-5-ylthio, 1-(2-Dimethylaminoäthyl)-1H-tetrazol-5- ylthio, 1-Phenyl-lH-tetrazol-5-ylthio oder l-(4-Chlorphenyl)-lH- tetrazol-5-ylthio, gegebenenfalls durch Niederalkyl oder Thienyl substituiertes Thiazolylthio oder Isothiazolylthio, z.B. 2-Thiazolylthio, 4-(2-Thienyl)-2-thiäzolylthio, 4,5-Dimethyl-2-thiazolylthio, 3-Isothiazolylthio, 4-Isothiazolylthio oder 5-Isothiazolylthio, insbesondere auch gegebenenfalls wie angegeben substituiertes Thiadiazolylthio, z.B. 1,2,3-Thiadiazol-4-ylthio, 1,2,3-Thiadiazol-5-ylthio, 1,3,4-Thiadiazol-2-ylthio, 2-Methyl-1,3,4,-thiadiazol-5-ylthio, 2-(3-Carboxypropionylamino)-1,3,4,-thiadiazol-5-ylthio, 1,2,4-Thiadiazol-5-ylthio oder 1,2,5-Thiadiazol-3-ylthio, Thiatriazolylthio, z.B. 1,2,3,4-Thiatriazolyl-5-ylthio, gegebenenfalls wie angegeben substituiertes Oxazolylthio oder Isoxazolylthio, z.B. 5-Oxazolylthio, 4-Methyl-5-oxazolylthio, 2-Oxazolylthio, 4,5-Diphenyl-2-oxazolylthio oder 3-Methyl-5-isoxazolylthio, oder gegebenenfalls wie angegeben substituiertes Oxadiazolylthio, z.B. 1,2,4-Oxadiazol-5-ylthio, 2-Methyl-1,3,4-oxadiazol-5-ylthio, 2-Phenyl-1,3,4-oxadiazol-5-ylthio, 5-(4-Nitrophenyl)-1,3,4-oxadiazol-2-ylthio oder 2-(2-Thienyl)-1,3,4,- oxadiazol-5-ylthio.

Bevorzugte heterocyclisch verätherte Mercaptogruppen R₂, worin der heterocyclische Rest einen entsprechenden monocyclischen, sechsgliedrigen Rest oder einen entsprechenden partiell gesättigten Rest darstellt, enthalten insbesondere 1 bis 3 Stickstoffatome und sind z.B. gegebenenfalls durch Halogen substituiertes 1-Oxido-pyridylthio, z.B. 1-Oxido-2-pyridylthio oder 4-Chlor-l-oxido-2-pyridylthio, gegebenenfalls durch Hydroxy substituiertes Pyridazinylthio, z.B. 3-Hydroxy-6-pyridazinylthio, gegebenenfalls durch Niederalkyl, Niederalkoxy oder Halogen substituiertes N-Oxido-pyridazinylthio, z.B. 2-Oxido-6-pyridazinylthio, 3-Chlor-l-oxido-6-pyridazinylthio, 3-Methyl-2- oxido-6-pyridazinylthio, 3-Methoxy-l-oxido-6-pyridazinylthio, 3-Aethoxy-l-oxido-6-pyridazinylthio, 3-n-Butyloxy-1-oxido-6-pyridazinylthio oder 3-(2-Aethylhexyloxy)-1-oxido-6-pyridazinylthio, gegebenenfalls durch Niederalkyl, Amino, Diniederalkylaminooder Carboxy substituiertes 2-0xo-1,2-dihydro-pyrimidinylthio, z.B. 2-Oxo-1,2-dihydro-4-pyrimidinylthio, 5- oder 6-Methyl-2-oxo-1,2-dihydro-4-pyrimidinylthio, 1-Amino- oder 6-Amino-2-oxo-l,2-dihydro-4-pyrimidinylthio, 6-Dimethylamino-2-oxo-1,2-dihydro-4-pyrimidinylthio, 5-Carboxy-2-oxo-1,2-dihydro-4-pyrimidinylthio oder 6-Cärboxy-2-oxo-1,2-dihydro-4-pyrimi- dinylthio, oder gegebenenfalls durch Niederalkyl, z.B. Methyl, Carboxyniederalykl, z.B. Carboxymethyl, oderSulfoniederalkyl, z.B. Sulfomethyl, und Oxo substituiertes nicht-aromatisches Triazinyl, insbesondere gegebenenfalls wie angegeben substituiertes 5,6-Dioxo-tetrahydro-as-triazinyl, z.B. 1- oder 2-Methyl-5,6-dioxo-1,2,5,6-tetrahydro-as-triazin-3-yl, 4-Methyl-5,6-dioxo-1,4,5,6-tetrahydro-as-triazin-3-yl, 1- oder 2-Carboxymethyl-5,6-dioxo-1,2,5,6-tetrahydro-as-triazin-3-yl, 4-Carboxymethyl-5,6-dioxo-l,4,5,6-tetrahydro-as-triazin-3-yl, 1- oder 2-Sulfomethyl-5,6-dioxo-1,2,5,6-tetrahydro-as-triazin-3-yl oder 4-Sulfomethyl-5,6-dioxo-1,4,5,6-tetrahydro-as- triazin-3-yl.

Quaternäre Ammoniumgruppen R₂ sind von tertiären organischen Basen, vorzugsweise von entsprechenden aliphatischen Aminen oder in erster Linie von entsprechenden heterocyclischen Stickstoffbasen abgeleitete, über das Stickstoffatom mit dem Methylkohlenstoffatom verbundene, quaternäre Ammoniumgruppen.

In einer quaternären Ammoniumgruppe R2, die von einer tertiären organischen Base abgeleitet wird, ist das Stickstoffatom an das Methylenkohlenstoffatom gebunden und liegt demgemäss in quaternisierter, positiv geladener Form vor. Quaternäre Ammoniumgruppen sind u.a. Triniederalkylammonium, z.B. Trimethylammonium, Triäthylammonium, Tripropylammonium, z.B. Tributylammonium, insbesondere aber gegebenenfalls substituierte, z.B. Niederalkyl, wie Methyl, Hydroxyniederalkyl, wie Hydroxymethyl, Amino, substituiertes Sulfonamido, wie 4-Aminophenylsulfonamido, Hydroxy, Halogen; wie Fluor, Chlor, Brom oder Jod, Halogenniederalkyl, wie Trifluormethyl, Sulfo, gegebenenfalls funktionell abgewandeltes Carboxy, wie Carboxy, Niederalkoxycarbonyl, z.B. Methoxycarbonyl, Cyan, gegebenenfalls durch Niederalkyl, z.B. Methyl oder Aethyl, oder Hydroxyniederalkyl, z.B. Hydroxymethyl, N-mono- oder N,N-disubstituiertes Carbamoyl, z.B. Carbamoyl, N-Methylcarbamoyl oder N,N-Dimethyl-carbamoyl, gegebenenfalls durch Niederalkyl N-substituiertes Hydrazinocarbonyl, z.B. Hydrazinocarbonyl, Carboxyniederalkyl, wie Carboxymethyl, Niederalkanoyl, wie Acetyl, oder 1-Niederalkyl-pyrrolidinyl, wie 1-Methyl-2-pyrrolidinyl, mono-oder polysubstituierte, monocyclische oder bicyclische azacyclische Ammoniumgruppen aromatischen Charakters, mit 1 oder 2 Ringstickstoff-und gegebenenfalls einem Ringschwefelatom, wie Pyrimidinium, Pyridazinium, Thiazolium, Chinolinium und in erster Linie Pyridinium.

Heterocyclische Ammoniumgruppen R₂ sind in erster Linie gegebenenfalls Niederalkyl, Hydroxyniederalkyl, Amino, substituiertes Sulfonamido, Hydroxy, Halogen, Trifluormethyl, Sulfo, Carboxy, Niederalkoxycarbonyl, Cyan, Niederalkanoyl, 1-Niederalkyl-pyrrolidinyl = oder gegebenenfalls durch Niederalkyl oder Hydroxyniederalkyl N-substituiertes Carbamoyl enthaltendes Pyridinium, z.B. Pyridinium, 2-, 3- oder 4-Methyl-pyridinium, 3,5-Dimethyl-pyridinium, 2,4,6-Trimethyl-pyridinium, 2-, 3- oder 4-Aethyl-pyridinium, 2-, 3- oder 4-Propyl-pyridinium oder insbesondere 4-Hydroxymethyl-pyridinium, ferner 2-Amino- oder 2-Amino-6-methyl-pyridinium, 2-(4-Aminophenyl- sülfonylamido)-pyridinium, 3-Hydroxy-pyridinium, 3-Fluor-, 3-Chlor-, 3-Jod- oder insbesondere 3-Brom-pyridinium, β-Trifluormethyl-pyridinium, 3-Sulfopyridinium, 2-, 3- oder 4-Carboxy- oder 2,3- oder 3,4-Dicarboxy-pyridinium, 4-Methoxycarbonyl-pyridinium, 3- oder 4-Cyanpyridinium, 3-Carboxymethyl-pyridinium; 3- oder 4-Acetyl-pyridinium, 3-(1-Methyl-2-pyrrolidinyl)-pyridinium, und insbesondere 4-Carbamoyl-, 3-Carbamoyl-, 3,4-Dicarbamoyl-, 3- oder 4-N-Methylcarbamoyl, 4-N,N-Dimethylcarbamoyl-, 4-N-Aethylcarbamoyl-, 3-N,N-Diäthylcarbamoyl, 4-N-Propylcarbamoyl-, 4-Isopropylcarbamoyl- und 4-Hydroxymethylcarbamoyl-pyridinium.

Die in Verbindungen der Formel I vorhandenen funktionellen Gruppen, insbesondere Carboxyl- und Amino-, ferner Hydroxy- und Sulfogruppen, sind gegebenenfalls durch Schutzgruppen geschützt, die in der Penicillin-, Cephalosporin- und Peptidchemie verwendet werden.

Solche Schutzgruppen sind leicht, das heisst ohne das unerwünschte Nebenreaktionen stattfinden, beispielsweise solvolytisch, reduktiv, photolytisch oder auch unter physiologischen Bedingungen, ahspaltbar.

Schutzgruppen dieser Art, sowie ihre Abspaltung sind beispielsweise beschrieben in "Protective Groups in Organic Chemistry", Plenum Press, London, New York, 1973, ferner in "The Peptides", Vol. I, Schröder and Lubke, Academic Press, London, New York, 1965, sowie in "Methoden der organischen Chemie", Houben-Weyl, 4.Auflage, Bd. 15/1, Georg Thieme Verlag, Stnttgart, 1974.

So sind Carboxylgruppen, wie die 4-Carboxylgruppe, z.B. üblicherweise in veresterter Form geschützt, wobei solche Estergruppierungen unter schonenden Bedingungen leicht spaltbar sind. In dieser Art geschützte Carboxylgruppen enthalten als veresternde Gruppen in erster Linie in 1-Stellung verzweigte oder in 1- oder 2-Stellung geeignet substituierte Niederalkylgruppen. Bevorzugte, in veresterter form vorliegende Carboxylgruppen sind u.a. tert.-Niederalkoxycarbonyl, z.B. tert.-Butyloxycarbonyl, Arylmethoxycarbonyl, mit einem oder zwei Arylresten, wobei diese gegebenenfalls, z.B. durch Niederalkyl, wie tert.-Niederalkyl, z.B. tert.-Butyl, Niederalkoxy, wie Methoxy, Hydroxy, Halogen, z.B. Chlor, und/oder Nitro, mono- oder polysubstituierte Phenylreste darstellen, wie gegebenenfalls, z.B. wie oben erwähnt, substituiertes Benzyloxycarbonyl, z.B. 4-Nitro-benzyloxycarbonyl oder 4-Methoxy-benzyloxycarbonyl, oder gegebenenfalls, z.B. wie oben erwähnt, substituiertes Diphenylmethoxycarbonyl, z.B. Diphenylmethoxycarbonyl oder Di-(4-methoxyphenyl)-methoxycarbonyl, 1-Niederalkoxyniederalkoxycarbonyl, wie Methoxymethoxycarbonyl, 1-Methoxyäthoxycarbonyl oder 1-Aethoxymethoxycarbonyl, 1-Niederalkylthioniederalkoxycarbonyl, wie 1-Methylthiomethoxycarbonyl oder 1-Aethylthioäthoxycarbonyl, Aroylmethoxycarbonyl, worin die Aroylgruppe gegebenenfalls, z.B. durch Halogen, wie Brom, substituiertes Benzoyl darstellt, z.B. Phenacyloxycarbonyl, 2-Halogenniederalkoxycarbonyl, z.B. 2,2,2-Trichloräthoxycarbonyl, 2-Bromäthoxycarbonyl oder 2-Jodäthoxycarbonyl, oder 2-(trisubstituiertes Silyl)-äthoxycarbonyl, worin die Substituenten unabhängig voneinander je einen gegebenenfalls substituierten, z.B. durch Niederalkyl, Niederalkoxy, Aryl, Halogen und/oder Nitro substituierten, aliphatischen, araliphatischen, cycloaliphatischen oder aromatischen Kohlenwasserstoffrest mit z.B. bis zu 15 Kohlenstoffatomen, wie entsprechendes, gegebenenfalls substituiertes Niederalkyl, Phenylniederalkyl, Cycloalkyl oder Phenyl bedeuten, z.B. 2-Triniederalkylsilyläthoxycarbonyl, wie 2-Trimethylsilyläthoxycarbonyl oder 2-(Di-n-butyl-methyl-silyl)-äthoxycarbonyl, oder 2-Triarylsilylithoxycarbonyl, wie 2-Iriphenylsilyläthoxycarbonyl.

Weitere, in veresterter Form vorliegende geschützte Carboxylgruppen sind entsprechende Silyloxycarbonyl-, insbesondere organische Silyloxycarbonylgruppen, ferner entsprechende Stannyloxycarbonylgrup- pen. In diesen enthält das Silicium- bzw. Zinnatom vorzugsweise Niederalkyl, insbesondere Methyl, ferner Niederalkoxy, z.B. Methoxy, und/ oder Halogen, z.B. Chlor, als Substituenten. Geeignete Silyl- bzw. Stannylschutzgruppen sind in erster Linie Triniederalkylsilyl, insbesondere Trimethylsilyl, ferner Dimethyl-tert.butylsilyl, Niederalkoxy- niederalkyl-halogen-silyl, z.B. Methoxy-methyl-chlor-silyl, oder Diniederalkyl-halogen-silyl, z.B. Dimethyl-chlor-silyl, oder entsprechend substituierte Stannylverbindungen, z.B. Tri-n₋butylstannyl.

Bevorzugte geschützte Carboxylgruppen sind tert.-Niederalkoxycarbonyl, wie tert.-Butyloxycarbonyl, und in erster Linie gegebenenfalls, z.B. wie oben erwähnt, substituiertes Benzyloxycarbonyl, wie 4-Nitro-benzyloxycarbonyl, oder Diphenylmethoxycarbonyl.

Eine unter physiologischen Bedingungen spaltbare, veresterte Carboxylgruppe ist in erster Linie eine Acyloxymethoxycarbonylgruppe, worin Acyl z.B. den Rest einer organischen Carbonsäure, in erster Linie einer gegebenenfalls substituierten Niederalkancarbonsäure bedeutet, oder worin Acyloxymethyl den Rest eines Lactons bildet. Solche Gruppen sind Niederalkanoyloxymethoxycarbonyl, z.B. Acetyloxymethyloxycarbonyl oder Pivaloyloxymethoxycarbonyl, Aminoniederalkanoylmethoxycarbonyl, insbesondere α-Amino-niederalkanoyloxymethoxycarbonyl, z.B. Glycyloxymethoxy-_{.} carbonyl, L-Valyloxymethoxycarbonyl oder L-Leucyloxymethoxycarbonyl, ferner Phthalidyloxycarbonyl, z.B. 2-Phthalidyloxycarbonyl, oder Indanyloxycarbonyl, z.B. 5-Indanyloxycarbonyl.

Eine geschützte Aminogruppe, kann z.B. in Form einer leicht spaltbaren Acylamino-, Arylmethylamino-, verätherten Mercaptoamino-, 2-Acyl-niederalk-1-en-1-yl-amino-, Silyl- oder Stannylaminogruppe oder als Azidogruppe vorliegen.

In einer entsprechenden Acylaminogruppe ist Acyl beispielsweise der Acylrest einer organischen Carbonsäure mit z.B. bis zu 18 Kohlenstoffatomen, insbesondere einer gegebenenfalls, z.B. durch Halogen oder Aryl, substituierten Alkancarbonsäure oder gegebenenfalls, z.B. durch Halogen, Niederalkoxy oder Nitro, substituierten Benzoesäure, oder eines Kohlensäurehalbesters. Solche Acylgruppen sind beispielsweise Niederalkanoyl, wie Formyl, Acetyl, oder Propionyl, Halogen-niederalkanoyl, wie 2-Balogenacetyl, insbesondere 2-Chlor-, 2-Brom-, 2-Jod-, 2,2,2-Trifluor- oder 2,2,2-Trichloracetyl, gegebenenfalls, z.B. durch Halogen, Niederalkoxy oder Nitro substituiertes Benzoyl, z.B. Benzoyl, 4-Chlorbenzoyl, 4-Methoxybenzoyl oder 4-Nitrobenzoyl, oder in 1-Stellung der Niederalkylrestes verzweigtes oder in 1- oder 2-Stellung geeignet substituiertes Niederalkoxycarbonyl, insbesondere tert.-Niederalkoxycarbonyl, z.B. tert.-Butyloxycarbonyl, Arylmethoxycarbonyl mit einem oder zwei Arylresten, die vorzugsweise gegebenenfalls, z.B. durch Niederalkyl, insbesondere tert.-Niederalkyl, wie tert.-Butyl, Niederalkoxy, wie Methoxy, Hydroxy, Halogen, z.B. Chlor, und/oder Nitro, mono-oder polysubstituiertes Phenyl darstellen, wie gegebenenfalls substituiertes Benzyloxycarbonyl, z.B. 4-Nitro-benzyloxycarbonyl, oder substituiertes Diphenylmethoxycarbonyl, z.B. Benzhydryloxycarbonyl oder Di-(4-methoxyphenyl)-methoxyearbonyl, Aroylmethoxycarbonyl, worin die Aroylgruppe vorzugsweise gegebenenfalls,z.B. durch Halogen, wie Brom, substituiertes Benzoyl darstellt, z.B. Phenacyloxycarbonyl, 2-Halogen-niederalkoxycarbonyl, z.B. 2,2,2-Trichloräthoxycarbonyl, 2-Bromäthoxycarbonyl oder 2-Jodäthoxycarbonyl, oder 2-(trisubstituiertes Silyl)-äthoxycarbonyl, worin die Substituenten unabhängig voneinander je einen gegebenenfalls substituierten, z.B. durch Niederalkyl, Niederalkoxy, Aryl, Halogen oder Nitro, substituierten, aliphatischen, araliphatischen, cycloaliphatischen oder aromatischen Kohlenwasserstoffrest mit z.B. bis zu 15 C-Atomen, wie entsprechendes, gegebenenfalls substituiertes Niederalkyl, Phenylniederalkyl, Cycloalkyl oder Phenyl, bedeuten, z.B. 2-Triniederalkylsilyläthoxycarbonyl, wie 2-Triaethylsilyläthoxycarbonyl oder 2-(Di-n-butyl-methyl-silyl)-äthoxycarbonyl, oder 2-Triarylsilyläthoxycarbonyl, wie 2-Triphenylsilyläthoxycarbonyl.

Weitere,als Aminoschutzgruppen in Frage kommende Acylreste sind auch entsprechende Reste organischer Phosphor-, Phosphon-oder Phosphinsäuren, wie Diniederalkylpholphoryl, z.B. Dimethylphosphoryl, Diäthylphosphoryl, Di-n-propylphosphoryl oder Diisopropylphosphoryl, Dicycloalkylphosphoryl, z.B. Dicyclohexylphosphoryl, gegebenenfalls substituiertes Diphenylphosphoryl, z.B. Diphenylphosphoryl, gegebenenfalls, z.B. durch Nitro substituiertes Di-phenylniederalkylphosphoryl, z.B. Dibenzylphosphoryl oder Di-4-nitrobenzyl- phoaphoryl, gegebenenfalls substituiertes Phenyloxy-phenyl-phosphonyl, z.B. Phenyloxy-phenyl-phosphonyl, Diniederalkylphosphinyl, z.B. Diäthylphosphinyl, oder gegebenenfalls substituiertes Diphenylphosphinyl, z.B. Diphenylphosphinyl.

In einer Arylmethylaminogruppe, die eine Mono-, Di- oder insbesondere Triarylmethylamino darstellt, sind die Arylreste insbesondere gegebenenfalls substituierte Phenylreste. Solche Gruppen sind beispielsweise Benzyl-, Diphenylmethyl- und insbesondere Tritylamino.

Eine verätherte Mercaptogruppe in einer mit einem solchen Rest geschützten Aminogruppe ist in erster Linie Arylthio oder Arylniederalkylthio, worin Aryl insbesondere gegebenenfalls, z.B. durch Niederalkyl, wie Methyl oder tert.-Butyl, Niederalkoxy, wie Methoxy, Halogen, wie Chlor, und/oder Nitro substituiertes Phenyl ist. Eine entsprechende Aminoschutzgruppe ist z.B. 4-Nitrophenylthio.

In einem als Aminoschutzgruppe verwendbaren 2-Acyl-niederalk-l-en-1-yl-rest ist Acyl z.B. der entsprechende Rest einer Niederalkancarbonsäure, einer gegebenenfalls, z.B. durch Niederalkyl, wie Methyl oder tert.-Butyl, Niederalkoxy, wie Methoxy, Halogen, wie Chlor, und/ oder Nitro substituierten Benzoesäure, oder insbesondere eines Kohlensäurehalbesters, wie eines Kohlensäure-niederalkylhalbestars. Entsprechende Schutzgruppen sind in erster Linie 1-Niederalkanoyl-prop-1-en-2-yl, z.B. 1-Acetyl-prop-1-en-2-yl, oder 1-Niederalkoxycarbonyl-prop- l-en-2-yl, z.B. l-Aethoxycarbonyl-prop-l-en-2-yl.

Eine Silyl- oder Stannylaminogruppe ist in erster Linie eine - organische Silyl- bzw. Stannylaminogruppe, worin das Silicium- bzw. Zinnatom vorzugsweise Niederalkyl, insbesondere Methyl, ferner Niederalkoxy, z.B. Methoxy, und/oder Halogen, z.B. Chlor, als Substituenten enthält. Entsprechende Silyl- oder Stannylgruppen sind in erster Linie Triniederalkylsilyl, insbesondere Trimethylsilyl, ferner Dimethyl-tert.-butyl-silyl, Niederalkoxy-niederalkyl-halogen-silyl, z.B. Methoxy-methyl-chlor-silyl, oder Diniederalkyl-halogensilyl, z.B. Dimethyl-chlor-silyl, oder entsprechend substituiertes Stannyl, z.B. Tri-n-butylstannyl.

Eine Aminogruppe kann auch in protonierter Form geschützt werden; als entsprechende Anionen kommen in erster Linie diejenigen von starken anorganischen Säuren, wie von Halogenwasserstoffsäuren, z.B. das Chlor- oder Bromanion, oder von organischen Sulfonsäuren, wie p-Toluolsulfonsäure, in Frage.

Bevorzugte Aminoschutzgruppen sind Acylreste von Kohlensäurehalbestern, insbesondere tert.-Butyloxycarbonyl, gegebenenfalls,z.B. wie angegeben, substituiertes Benzyloxycarbonyl, z.B. 4-Nitro-benzyloxycarbonyl, oder Diphenylmethoxycarbonyl, oder 2-Halogen-niederalkoxycarbonyl, wie 2,2,2-Trichloräthoxycarbonyl, ferner Trityl oder Formyl.

Hydroxyschutzgruppen sind z.B. Acylreste, wie gegebenenfalls, z.B. durch Halogen, substituiertes Niederalkanoyl, wie 2,2-Dichloracetyl, oder insbesondere die im Zusammenhang mit einer geschützten Aminogruppe genannten Acylreste von Kohlensäurehalbestern, insbesondere 2,2,2-Trichloräthoxycarbonyl, oder organischen Silyl- oder Stannylreste, ferner leicht abspaltbare veräthernde Gruppen, wie tart.-Niederalkyl, z.B. tert.-Butyl, 2-oxa- oder 2-thia-aliphatische oder -cycloaliphatische Kohlenwasserstoffreste, in erster Linie 1-Niederalkoxy-niederalkyl oder 1-Niederalkylthio-niederalkyl, z.B. Methoxymethyl, 1-Methoxyäthyl, 1-Aethoxy-äthyl, 1-Methylthiomethyl, 1-Methylthio-äthyl oder 1-Aethylthioathyl, oder 2-oxa- oder 2-Thiacycloalkyl mit 5-7 Ringatomen, z.B. 2-Tetrahydrofuryl oder 2-Tetrahydropyranyl oder entsprechende Thiaanaloge, sowie gegebenenfalls substituiertes 1-Phenylniederalkyl, wie gegebenenfalls substituiertes Benzyl oder Diphenylmethyl, wobei als Substituenten der Phenylreste z.B. Halogen, wie Chlor, Niederalkoxy, wie Mathoxy und/oder Nitro in Frage kommen.

Eine geschützte Sulfogruppe ist in erster Linie eine veresterte, wie mit einem aliphatischen, cycloaliphatischen, cycloaliphatisch- aliphatischen,aromatischen oder araliphatischen Alkohol, z.B. einem Niederalkanol, oder mit einem Silyl- oder Stannylrest, wie Triniederalkylsilyl, veresterte Sulfogruppe. In einer Sulfogruppe kann die Hydroxygruppe beispielsweise, wie die Hydroxygruppe in einer veresterten Carboxygruppe veräthert sein.

Salze von erfindungsgemässen Verbindungen sind in erster Linie pharmazeutisch verwendbare, nicht-toxische Salze, wie diejenigen von Verbindungen der Formel I mit sauren Gruppen, z.B. mit einer freien Carboxyl- und Sulfogruppe. Solche Salze sind in erster Linie Metall- oder Ammoniumsalze, wie Alkalimetall- und Erdalkalimetall-, z.B. Natrium-, Kalium-, Magnesium- oder Calciumsalze, sowie Amoniumsalze mit Ammoniak oder geeigneten organischen Aminen, wobei in erster Linie aliphatische, cycloaliphatische, cycloaliphatisch-aliphatische oder araliphatische primäre, sekundäre oder tertiäre Mono-, Di- oder Polyamine, sowie heterocyclische Basen für die Salzbildung in Frage kommen, wie Niederalkylamine, z.B. Triäthylamin, Hydroxyniederalkylamine, z.B. 2-Hydroxyäthylamin, Bis-(2-hydroxyäthyl)-amin oder Tris-(2-hydroxyäthyl)-amin, basische aliphatische Ester von Carbonsäuren, z.B. 4-Aminobenzoesäure-2-diäthylaminoäthyl- ester, Niederalkylenamine, z.B. l-Aethyl-piperidin, Cycloalkylamine, z.B. Dicyclohexylamin, oder Benzylamine, z.B. N,N'-Dibenzyl-äthylendiamin, ferner Basen vom Pyridintyp, z.B. Pyridin, Collidin oder Chinolin. Verbindungen der Formel I mit einer basischen Gruppen können Säureadditionssalze, z.B. mit anorganischen Säuren, wie Salzsäure, Schwefelsäure oder Phosphorsäure, oder mit geeigneten organischen Carbon- oder Sulfonsäuren, z.B. Trifluoressigsäure, sowie mit Aminosäuren, wie Arginin und Lysin, bilden. Bei Anwesenheit von mehreren sauren oder basischen Gruppen können Mono- oder Pölysalze gebildet werden. Verbindungen der Formel I mit einer sauren, z.B. freien Carboxylgruppe, und einer freien basischen, z.B. einer Aminogruppe, können auch in Form von inneren Salzen, d.h. in zwitterionischer Form vorliegen, oder es kann ein Teil des Moleküls als inneres Salz, und ein anderer Teil als normales Salz vorliegen.

Zur Isolierung oder Reinigung können auch pharmazeutisch ungeeignete Salze Verwendung finden. Zur therapeutischen Anwendung gelangen nur die pharmazeutisch verwendbaren, nicht toxischen Salze, die deshalb bevorzugt werden.

Der Acylrest an der 7ß-Aminogruppe enthält zwei Asymmetriezentren. Das Asymmetriezentrum nächst zur 7-Aminogruppe liegt in der R,S-, der S- oder bevorzugt in der R-Konfiguration vor. Das Asymmetriezentrum an der endständigen Aminocarbonsäuregruppierung kann die R-, S- oder R,S-Konfiguration besitzen, wobei ebenfalls die R-Konfiguration bevorzugt ist.

Die Verbindungen der Formel I, worin Carboxylgruppen gegebenenfalls in physiologisch spaltbarer Form verestert sind, und ihre pharmazeutisch verwendbaren, nichttoxischen Salze sind wertvolle, antibiotisch wirksame Substanzen, die insbesondere als antibakterielle Antibiotika verwendet werden können. Beispielsweise sind sie in vitro gegen grampositive und gramnegative Mikroorganismen, wie gegen Kokken, z.B. Staphylococcus, Streptococcus und Neisseria, sowie gegen Haemophilus influenza, in Minimaldosen von etwa 0,01 bis 32 µg/ml, gegen gramnegative Bakterien, wie Enterobakterien, z.B. Escherichia coli, Klebsiella, Salmonella oder Proteus sp., oder Pseudomonas aeruginosa, gegen Haemophilus und Anaerobier in Minimaldosen von etwa 0,001 bis 128 _{f}g/ml wirksam. In vivo, bei subkutaner Applikation an der Maus, sind sie beispielsweise bei grampositiven Infektionen, z.B. durch Staphylococcus aureus, im Dosenbereich von etwa 3 bis 50 mg/Kg, bei gramnegativen Infektionen, z.B. durch Enterobakterien, wie Escherichia coli, Proteus sp. oder Klebsiella, oder Pseudomonas aeruginosa, im Dosenbereich von 2 bis 100 mg/Kg wirksam.

Die neuen Verbindungen können deshalb entsprechend, z.B. in Form von antibiotiach wirksamen Präparaten zur Behandlung von durch grampositive oder insbesondere von durch gramnegative Bakterien und Kokken, insbesondere durch von Escherichia coli verursachten systemischen Infektionen Verwendung finden, wobei die minimale ED₅₀ bei subkutaner Injektion bei etwa 3 mg/Kg liegt.

Verbindungen der Formel I, worin die funktionellen Gruppen geschützt sind werden als Ausgangsmaterialien zur Herstellung von Verbindungen der Formel I verwendet.

Die vorliegende Erfindung betrifft in erster Linie diejenigen Verbindungen der Formel I, worin die Gruppe -(CₙH₂ₙ)- unverzweigt ist und die Indices n, m, und k die angegebenen Bedeutung haben, X Sauerstoff oder die Gruppe -NH-, W eine Gruppe -CO- oder -CO-NHSO₂-, oder X-W zusammen eine Gruppe -CO- oder -CO-NHSO₂- bedeuten, A p-, o-oder m-Phenylen, 2,5-Thienylen oder 2,5-Furylen darstellt,Y 1,2-Aethylen, R₄ Wasserstoff, gegebenenfalls durch Hydroxy, Niederalkanoyloxy, Niederalkoxycarbonyloxy, Phenoxycarbonyloxy, Niederalkoxy, Niederalkylthio, Halogen, gegebenenfalls funktionell abgewandeltes Carboxy, oder gegebenenfalls mono- oder diniederalkyliertes Amino substituiertes Niederalkyl,sowie Niederalkenyl oder Acyl, Z Sauerstoff oder Schwefel, R Wasserstoff, Niederalkyl, Niederalkoxy, Halogen oder eine Gruppe der Formel -CH₂-R₂, worin R₂ Niederalkanoyloxy, Carbamoyloxy, N-Niederalkylcarbamoyloxy, Triazolylthio, Tetrazolylthio, Thiazolylthio, Thiatriazolylthio, Thiadiazolylthio, Oxazolylthio, Oxadiazolylthio, 5,6-Dioxo-tetrahydro-as-triazinylthio oder Pyridinio darstellt, worin die heterocyclischen Ringe gegebenenfalls beispielsweise durch Niederalkyl, N,N-Diniederalkylaminoniederalkyl, Carboxyniederalkyl, Sulfoniederalkyl, Amino, Carboxyniederalkanoylamino oder Carbamoyl substituiert sein können, und R₃ Wasserstoff oder Methoxy bedeuten, pharmazeutisch verwendbare Salze von solchen Verbindungen, sowie die zu ihrer Herstellung verwendbaren Ausgangsstoffe und Zwischenprodukte.

Besonders hervorzuheben sind Verbindungen der Formel I, worin die Gruppe -(CₙH₂ₙ)- unverzweigt ist und die Indices n, m und ₖ die angegebene Bedeutung haben, X Sauerstoff oder die Gruppe -NH- bedeutet, W eine Gruppe -CO- oder -CONHSO₂-, oder X-W zusammen eine Gruppe -CO- oder -CONHSO₂- bedeuten, A p-, o- oder m-Phenylen, oder, wenn m 1 ist, auch 2,5-Thienylen oder 2,5-Furylen darstellt, Y 1,2-Aethylen, R₄ Wasserstoff, gegebenenfalls durch Hydroxy, Niederalkanoyloxy, Niederalkoxycarbonyloxy, Niederalkoxy, Niederalkylthio, Halogen, z.B. Chlor, Carboxy, Amino oder Diniederalkylamino substituiertes Niederalkyl, sowie Niederalkenyl oder gegebenenfalls durch Halogen, z.B. Chlor, Niederalkoxy, Niederalkanoyloxy, Cyan oder Phenoxy substituiertes Niederalkanoyl, gegebenenfalls durch Niederalkyl, Hydroxy, Niederalkoxy oder'Chlor substituiertes Benzoyl, einen gegebenenfalls durch Hydroxy oder Chlor substituierten Pyridin-, Pyrimidin- oder Pyrazincarbonsäurerest, Niederalkoxycarbonyl, gegebenenfalls durch Halogen, z.B. Chlor oder Niederalkyl substituiertes Aryl-, wie Benzol-oder Naphthalinsulfonyl, gegebenenfalls am N-Atom niederalkyliertes Carbamoyl, Thiocarbamoyl, Iminocarbamoyl (=Guanyl) oder Sulfamoyl, Z Sauerstoff, R Wasserstoff, Methyl, Methoxy, Chlor oder eine Gruppe der Formel -CH₂-R₂, worin R Acetoxy, Carbamoyloxy, gegebenenfalls durch Methyl, Carboxymethyl, Sulfomethyl oder Dimethylaminoäthyl substituiertes Tetrazolylthio, z.B. Tetrazol-5-ylthio, 1-Methyl-1H-tetrazol-5-ylthio, 1-Sulfomethyl-1H-tetrazol-5-ylthio, 1-Carboxymethyl- lH-tetrazol-5-ylthio, oder 1-(2-Dimethplaminoäthyl)-1H-tetrazol-5- ylthio, Thiadiazolylthio, insbesondere 2-Methyl-1,3,4-thiadiazol-5- ylthio, durch Methyl substituiertes 5,6-Dioxo-tetrahydro-as-triazinylthio, z.B. 2-Methyl-5,6-dioxo-1,2,5,6-tetrahydro-as-triazin-3-ylthio oder 4-Methyl-5,6-dioxo-l,4,5,6-tetrahydro-as-triazin-3-ylthio, oder Carbamoylpyridinio, insbesondere 4-Carbamoylpyridinio, darstellt, und R₃ Wasserstoff oder Methoxy bedeuten, pharmazeutisch verwendbare Salze von solchen Verbindungen, sowie die zu ihrer Herstellung verwendbaren Ausgangsstoffe und Zwischenprodukte.

Bevorzugt sind Verbindungen der Formel I, worin die Gruppe -(CₙE₂ₙ)-Methylen bedeutet, die Indices m und k die angegebene Bedeutung haben, X Sauerstoff und W eine Gruppe -CO-, A p- oder o-Phenylen, Y 1,2-Aethylen, R₄ Wasserstoff, Niederalkyl, insbesondere Aethyl oder Niederalkylsulfonyl, insbesondere Mesyl, Z Sauerstoff, R Wasserstoff, Methyl, Methoxy, Chlor oder eine Gruppe der Formel -CH₂-R₂, worin R₂ Acetoxy, Carbamoyloxy, gegebenenfalls durch Methyl, Carboxymethyl, Sulfomethyl oder Dimethylaminoäthyl substituiertes Tetrazolylthio, insbesondere 1-Methyl-1H-tetrazol-5-ylthio, 1-Sulfomethyl-lH-tetrazol-5-ylthio, 1-Carboxymethyl-1H-tetrazol-5-ylthio, oder 1-(2-Dimethylaminoäthyl)-1H-tetrazol-5-ylthio, Thiadiazolylthio, insbesondere 2-Methyl-1,3,4-thiadiazol-5-ylthio, durch Methyl substituiertes 5,6-Dioxo-tetrahydro-as-triazinylthio, z.B. 2-Methyl-5,6-dioxo-1,2,5,6-tetrahydro-as-triazin-3-ylthio oder 4-Methyl-5,6-dioxo-1,4,5,6-tetrahydro-as-triazin-3-ylthio, oder Carbamoylpyridinio, insbesondere 4-Carbamoylpyridinio, darstellt, und.R₃ Wasserstoff oder Methoxy bedeuten, pharmazeutisch verwendbare Salze von solchen Verbindungen, sowie die zur ihrer Herstellung verwendbaren Ausgangsstoffe und Zwischenprodukte.

Die Erfindung betrifft insbesondere die in den Beispielen beschriebenen Verbindungen der Formel I, deren pharmazeutisch unbedenklichen Salze, sowie die dort beschriebenen Ausgangsstoffe und Zwischenprodukte.

Die Verbindungen der vorliegenden Erfindung werden nach an sich bekannten Verfahren erhalten.

Verbindungen der Formel I, worin die Carboxylgruppen gegebenenfalls in physiologisch spaltbarer Form verestert sind, sowie ihre Salze, werden hergestellt, indem man in einer der Formel I entsprechenden Ausgangsverbindung, worin mindestens eine der vorhandenen funktionellen Gruppen geschützt sind, die funktionelle(n) Gruppe(n) freisetzt, wenn erwünscht, in einer erhaltenen Verbindung eine Gruppe R₁ in eine andere Gruppe R₁ überführt, und/oder, wenn erwünscht, eine freie Carboxylgruppe in eine physiologisch spaltbare veresterte Carboxylgruppe überführt, und/oder, wenn erwünscht, ein erhaltenes Isomerengemisch in die einzelnen Isomeren auftrennt, und/oder, wenn erwünscht, eine erhaltene Verbindung in ein Salz oder ein erhaltenes Salz in eine freie Verbindung oder in ein anderes Salz überführt.

Abspaltung von Schutzgruppen: In einer erhaltenen Verbindung der Formel I, worin eine oder mehrere funktionelle Gruppe geschützt sind, können diese, z.B. geschützte Carboxyl-, Amino-, Hydroxy- und/oder Sulfogruppen, in an sich bekannter Weise, mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig freigesetzt werden.

So kann man tert.-Niederalkoxycarbonyl oder in 2-Stellung durch eine organische Silylgruppe oder in 1-Stellung durch Niederalkoxy oder Niederalkylthio substituiertes Niederalkoxycarbonyl oder gegebenenfalls substituiertes Diphenylmethoxycarbonyl z.B.durch Behandeln mit einer geeigneten Säure, wie Ameisensäure oder Trifluoressigsäure, gegebenenfalls unter Zugabe einer nucleophilen Verbindung, wie Phenol oder Anisol, in freies Carboxyl überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators,freigesetzt werden. Ferner kann man geeignet substituiertes Benzyloxycarbonyl, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zink, oder Metallsalz, wie einem Chrom-II-salz, z.B. Chrom-II-chlorid, üblicherweise in Gegenwart eines Wasserstoff-abgebenden Mittels, das zusammen mit dem Metall nascierenden Wasserstoff zu erzeugen vermag, wie einer Säure, in erster Linie einer geeigneten Carbonsäure, wie einer gegebenenfalls, z.B. durch Hydroxy, substituierten Niederalkancarbonsäure, z.B. Essigsäure, Ameisensäure, Glycolsäure, Diphenylglycolsäure, Milchsäure, Mandelsäure, 4-Chlor-mandelsäure oder Weinsäure, oder eines Alkohols oder Thiols, wobei man vorzugsweise Wasser zugibt, in freies Carboxyl überführen. Durch Behandeln mit einem reduzierenden Metall oder Metallsalz, wie oben beschrieben, kann man auch 2-Halogea-niederalkoxycarbonyl (gegebenenfalls nach Umwandlung einer 2-Brom-niederalkoxycarbonylgruppe in eine entsprechende 2- _{Jod}-niederalkoxycarbonylgruppe) oder Aroylmethoxycarbonyl in freies Carboxyl umwandeln, wobei Aroylmethoxycarbonyl ebenfalls durch Behandeln mit einem nucleophilen, vorzugsweise salzbildenden Reagens, wie Natriumthiophenolat oder Natriumjodid, gespalten werden kann. Substituiertes 2-Silyläthoxycarbonyl kann auch durch Behandeln mit einem, das Fluoridanion liefernden Salz der Fluorwasserstoffsäure, wie einem Alkalimetallfluorid, z.B. Natrium- oder Kaliumfluorid, in Anwesenheit eines macrocyclischen Polyäthers ("Kronenäther"), oder mit einem Fluorid einer organischen quaternären Base, wie Tetra-niederalkylammoniumfluorid oder Triniederalkyl-aryl-ammoniumfluorid, z.B. Tetraäthylammoniumfluorid oder Tetrabutylammoniumfluorid, in Gegenwart eines aprotischen polaren Lösungsmittels, wie Dimethylsulfoxid oder N,N-Dimethylacetamid, in freies Carboxyl übergeführt werden. Mit einer organischen Silyl- oder Stannylgruppe, wie Triniederalkylsilyl oder -stannyl, z.B. Trimethylsilyl, verestertes Carboxyl kann in üblicherweise solvolytisch, z.B. durch Behandeln mit Wasser, einem Alkohol oder Säure freigesetzt werden.

Eine geschützte Aminogruppe setzt man in an sich bekannter und je nach Art der Schutzgruppen in verschiedenartiger Weise, vorzugsweise mittels Solvolyse oder Reduktion, frei. 2-Halogen-niederalkoxycarbonylamino (gegebenenfalls nach Umwandlung einer 2-Brom-niederalkoxycarbonylaminogruppe in eine 2-Jod-niederalkoxycarbonylaminogruppe), Aroylmethoxycarbonylamino oder 4-Nitrobenzyloxycarbonylamino kann z.B. durch Behandeln mit einem geeigneten chemischen Reduktionsmittel, wie Zink in Gegenwart einer geeigneten Carbonsäure, wie wässriger Essigsäure, gespalten werden. Aroylmethoxycarbonylamino kann auch durch Behandeln mit einem nucleophilen, vorzugsweise salzbildenden Reagens, wie Natriumthiophenolat, und 4-Nitro-benzyloxyearbonylamino auch durch Behandeln mit einem Alkalimetall-, z.B. Natriumdithionit, gespalten werden. Gegebenenfalls substituiertes Diphenylmethoxycarbonylamino, tert.-Niederalkoxycarbonylamino oder 2-trisubstituiertes Silyläthoxycarbonylamino kann durch Behandeln mit einer geeigneten Säure, z.B. Ameisen- oder Trifluoressigsäure, gegebenenfalls substituiertes Benzyloxycarbonylamino z.B. mittels Hydrogenolyse, d.h. durch Behandeln mit Wasserstoff in Gegenwart eines geeigneten Hydrierkatalysators, wie eines Palladiumkatalysators, gegebenenfalls substituiertes Triarylmethylamino, Formylamino oder 2-Acyl-niederalk-l-en-l-yl-amino, z.B. durch Behandeln mit einer Säure, wie Mineralsäure, z.B. Chlorwasserstoffsäure, oder einer organischen Säure, z.B. Ameisen-, Essig- oder Trifluoressigsäure, gegebenenfalls in Gegenwart von Wasser, und eine mit einer organischen Silyl- oder Stannylgruppe geschützte Aminogruppe z.B. mittels Hydrolyse oder Alkoholyse freigesetzt werden. Eine durch 2-Halogenacetyl, z.B. 2-Chloracetyl, geschützte Aminogruppe kann durch Behandeln mit Thioharnstoff in Gegenwart einer Base, oder mit einem Thiolatsalz, wie einem Alkalimetallthiolat, des Thioharnstoffs und anschliessende Solvolyse, wie Alkoholyse oder Hydrolyse, des entstandenen Kondensationsprodukts freigesetzt werden. Eine durch 2-substituiertes Silyläthoxycarbonyl geschützte Aminogruppe kann auch durch Behandeln mit einem Fluoridanionen liefernden Salz der Fluorwasserstoffsäure, wie oben im Zusammenhang mit der Freisetzung einer entsprechend geschützten Carboxylgruppe angegeben, in die freie Aminogruppe übergeführt werden. Eine Phosphor-, Phosphon- oder Phosphin-amidogruppe kann z.B. durch Behandeln mit einer Phosphorhaltigen Säure, wie einer Phosphor-,Phosphon- oder Phosphinsäure, z.B. Orthophosphorsäure oder Polyphosphorsäure, einem sauren Ester, z.B. Monomethyl-, Monoäthyl-, Dimethyl- oder Diäthylphosphat oder Monomethylphosphonsäure, oder einem Anhydrid davon, wie Phosphorpentoxid, in die freie Aminogruppe übergeführt werden.

Ein in Form einer Azidogruppe geschütztes Amino wird z.B. durch Reduktion in freies Amino übergeführt, beispielsweise durch katalytische Hydrierung mit Wasserstoff in Gegenwart eines Hydrier- ^{_} katalysators, wie Platinoxid, Palladium oder Raney-Nickel, oder auch durch Behandeln mit Zink in Gegenwart einer Säure, wie Essigsäure. Die katalytische Hydrierung wird vorzugsweise in einem inerten Lösungsmittel, wie einem halogenierten Kohlenwasserstoff, z.B. Methylenchlorid, oder auch in Wasser oder einem Gemisch von Wasser und einem organischen Lösungsmittel, wie einem Alkohol oder Dioxan, bei etwa 20°C bis 25^{*}C, oder auch unter Kühlen oder Erwärmen, durchgeführt.

Eine durch eine geeignete Acylgruppe, eine organische Silyl-oder Stannylgruppe oder durch gegebenenfalls substituiertes 1-Phenylniederalkyl geschützte Hydroxygruppe wird wie eine entsprechend geschützte Aminogruppe freigesetzt. Eine durch 2,2-Dichloracätyl geschützte Hydroxygruppe wird z.B. durch basische Hydrolyse, während eine durch tert.-Niederalkyl oder durch einen 2-oxa- oder 2-thia-aliphatischen oder -cycloaliphatischen Kohlenwasserstoffrest verätherte Hydroxygruppe durch Acidolyse, z.B. durch Behandeln mit einer Mineralsäure oder einer starken Carbonsäure, z.B. Trifluoressigsäure, freigesetzt.

Eine geschützte, insbesondere veresterte Sulfogruppe wird analog einer geschützten Carboxylgruppe freigesetzt.

Die beschriebenen Spaltungsreaktionen werden unter an sich bekannten Bedingungen durchgeführt, wenn notwendig unter Kühlen oder Erwärmen, in einem geschlossenen Gefäss und/oder in einer Inertgas-, z.B. Stickstoffatmosphäre.

Bevorzugt werden beim Vorhandensein von mehreren geschützten funktionellen Gruppen die Schutzgruppen so gewählt, dass gleichzeitig mehr als eine solche Gruppe abgespalten werden kann, beispielsweise acidolytisch, wie durch Behandeln mit Trifluoressigsäure oder Ameisensäure, oder reduktiv, wie durch Behandeln mit Zink und Essigsäure, oder mit Wasserstoff und einem Hydrierkatalysator, wie einem Palladium Kohle/Katalysator.

Austausch von R₂ gegen anderes R₂ : In einer Verbindung der Formel I, worin eine Aminogruppe, wenn notwendig, geschützt ist, und worin die Carboxylgruppe in 4-Stellung des Cephemringes in freier Form vorliegt, kann man in an sich bekannter Weise eine Gruppe R₁ durch einen anderen Rest R₁ ersetzen oder in einen anderen Rest R₁ umwandeln. So ist es z.B. möglich, in einer Verbindung der Formel I, worin R₁ eine Gruppe der Formel -CH₂-R₂ bedeutet, und R₂ z.B. einen, durch nucleophile Substituenten ersetzbaren Rest darstellt, oder in einem Salz davon, durch Behandeln mit einer entsprechenden Mereaptan- oder mit einer Thiocarbonsäureverbindung einen solchen Rest R₂ durch eine verätherte bzw. veresterte Mercaptogruppe R₂ zu ersetzen. Ein geeigneter, durch eine verätherte Mercaptogruppe ersetzbarer Rest ist beispielsweise eine durch eine niederaliphatische Carbonsäure veresterte Hydroxygruppe. Solche veresterten Hydroxygruppen sind insbesondere Acetyloxy, ferner Formyloxy.

Die Reaktion einer solchen Verbindung mit einer geeigneten Mercaptanverbindung kann unter neutralen oder schwach basischen Bedingungen in Gegenwart von Wasser und gegebenenfalls einem, mit Wasser mischbaren organischen Lösungsmittel durchgeführt werden. Die basischen Bedingungen können beispielsweise durch Zugabe einer anorganischen Base, wie eines Alkalimetall- oder Erdalkalimetallhydroxids, -carbonats oder -hydrogencarbonats, z.B. von Natrium-, Kalium- oder Calciumhydroxid, -carbonat oder -hydrogencarbonat, eingestellt werden. Als organische Lösungsmittel können z.B. mit Wasser mischbare Alkohole, z.B. Niederalkanole, wie Methanol oder Aethanol, Ketone, z.B. Niederalkanone, wie Aceton, Amide, z.B. Niederalkancarbonsäureamide, wie Dimethylformamid, oder Nitrile, z.B. Niederalkansäurenitrile, wie Acetonitril, und ähnliche verwendet werden.

Veresterte Hydroxygruppen R2 in einer Verbindung der Formel I, worin R₁ die Gruppe -CH₂-R₂ bedeutet, wobei R₂ für eine, durch den Acylrest eines gegebenenfalls substituierten Halbamids der Kohlensäure veresterte Hydroxygruppe steht, kann man z.B. einführen, indem man eine entsprechende Verbindung der Formel I, worin R₂ für freies Hydroxy steht (das man z.B. durch Abspaltung des Acetylrestes aus einer Acetyloxygruppe R₂, z.B. durch Hydrolyse in schwach-basischem Medium, wie mit einer wässrigen Natriumhydroxydlösung bei pH 9-10, oder durch Behandeln mit einer geeigneten Esterase, wie einem entsprechenden Enzym aus Rhizobium tritolii, Rhizobium lupinii, Rhizobium japonicum oder Bacillus subtilis, oder einer geeigneten Citrus-Esterase, z.B. aus Orangenschalen, freisetzen kann), mit einem geeigneten Kohlensäurederivat, insbesondere mit einer Isocyanat- oder Carbaminsäureverbindung, wie einem Silylisocyanat, z.B. Silyltetraisocyanat, einem Sulfonylisocyanat, z.B. Chlorsulfonylisocyanat, oder Carbaminsäurehalogenid, z.B. -chlorid (die zu N-unsubstituierten 3-Aminocarbonyloxymethyl-Verbindungen führen), oder dann mit einer N-substituierten Isocyanat- oder mit einer N-mono- oder N,N-disubstituierten Carbaminsäure-Verbindungen, wie einem entsprechenden Carbaminsäurehalogenid, z.B. -chlorid, umsetzt, wobei man üblicherweise in Gegenwart eines Lösungs- oder Verdünnungsmittels und, wenn notwendig, unter Kühlen oder Erwärmen, in einem geschlossenen Gefäss und/oder in einer Inertgas-, z.B. Stickstoffatmosphäre, arbeitet.

Ferner kann man eine Verbindung der Formel I, worin R₁ eine Gruppe -CH₂-R₂ bedeutet, wobei R₂ z.B. den oben definierten, durch nucleophile Substitution ersetzbaren Rest darstellt, mit einer tertiären organischen Base, insbesondere einem gegebenenfalls substituierten Pyridin, unter neutralen oder schwach sauren Bedingungen, bevorzugt bei einem pH-Wert von etwa 6,5, in Gegenwart von Wasser und gegebenenfalls in einem mit Wasser mischbaren organischen Lösungsmittel umsetzen und so zu Verbindungen der Formel I gelangen, worin R₁ den Rest der Formel -CH₂-R₂ darstellt und R₂ für eine quaternäre Ammoniumgruppe steht. Die schwach-sauren Bedingungen können durch Zugabe einer geeigneten organischen oder anorganischen Säure, beispielsweise Essigsäure, Chlorwasserstoffsäure, Phosphorsäure oder auch Schwefelsäure eingestellt werden. Als organische Lösungsmittel können beispielsweise die vorstehend genannten, mit Wasser mischbaren Lösungsmittel verwendet werden. Zur Erhöhung der Ausbeute können der Reaktionsmischung gewisse Salze zugesetzt werden, beispielsweise Alkalimetall-, wie Natrium- und insbesondere Kaliumsalze, von anorganischen Säuren, wie Halogenwasserstoffsäure, z.B. Chlorwasserstoff- und insbesondere Jodwasserstoffsäure, sowie der Thiocyansäure, oder organischen Säuren, wie Niederalkancarbonsäuren, z.B. Essigsäure. Vertreter solcher Salze sind beispielsweise Kaliumjodid und Kaliumthiocyanat. Auch Salze von geeigneten Anionenauatauachera, z.B. flüssige Ionenaustauscher in Salzform, wie z.B. Amberlite LA-1 (flüssige sekundäre Amine mit einem Molekulargewicht von 351-393; Oel-laslich und Wasserunlöslich; mAeq./g = 2,5-2,7, z.B. in Acetatform), mit Säuren, z.B. Essigsäure, können für diesen Zweck verwendet werden.

Quaternäre Ammoniumgruppen R₂ können vorteilhafterweise unter Verwendung eines Zwischenprodukts der Formel I, in welchem R₂ für eine substituierte, insbesondere für eineraromatische substituierte Carbonylthiogruppe und in erster Linie für die Benzoylthiogruppe steht, hergestellt werden. Ein solches Zwischenprodukt, das man z.B. durch Umsetzen einer Verbindung der Formel I, worin R₂ im Rest R₁ eine veresterte Hydroxygruppe, insbesondere eine Niederalkanoyloxy- z.B. Acetyloxygruppe bedeutet, mit einem geeigneten Salz, wie einem Alkalimetall-, z.B. Natriumsalz, einer Thiocarbonsäure, wie einer aromatischen Thiocarbonsäure, z.B. Thiobenzoesäure, erhalten kann, wird mit dem tertiären Amin, insbesondere einer tertiären heterocyclischen Base, wie einem gegegebenenfalls substituierten Pyridin, umgesetzt, wobei man die quaternäre Ammoniumverbindung erhält. Die Reaktion wird üblicherweise in Gegenwart eines geeigneten Entschwefelungsmittels, insbesondere eines Quecksilbersalzes, z.B. Quecksilber-IIperchlorat, und eines geeigneten Lösungs- oder Verdünnungsmittels oder eines Gemisches, wenn notwendig, unter Kühlen oder Erwärmen, in einem geschlossenen Gefäss und/oder in einer Inertgas-, z.B. Stickstoffatomosphäre, durchgeführt.

Veresterung einer freien Carboxylgruppe: Die Ueberführung einer freien Carboxylgruppe in einer erhaltenen Verbindung der Formel I in eine veresterte Carboxylgruppe, die unter physiologischen Bedingungen spaltbar ist, erfolgt nach an sich bekannten Veresterungsmethoden, beispielsweise indem man eine Verbindung der Formel I, worin andere funktionelle Gruppen, wie Amino-, Hydroxy- oder Sulfogruppen, gegebenenfalls in geschützter Form vorliegen, oder ein bezüglich der zu veresternden Carboxylgruppe reaktionsfähiges funktionelles Derivat davon, oder ein Salz davon, mit einem entsprechenden Alkohol oder einem reaktionsfähigen funktionellen Derivat davon, verestert.

Salzbildung: Salze von Verbindungen der Formel I können in an sich bekannter Weise hergestellt werden. So kann man Salze von Verbindungen der Formel I mit sauren Gruppen, z.B. durch Behandeln mit Metallverbindungen, wie Alkalimetallsalzen von geeigneten Carbonsäuren, z.B. dem Natriumsalz der a-Aethylcapronsäure oder Natriumcarbonat, oder mit Ammoniak oder einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer Säure oder einem geeigneten Anionenaustauschreagens. Innere Salze von Verbindungen der Formel I, welche eine frei-Carboxylgruppe enthalten, können z.B. durch Neutralisieren von Salzen, wie Säureadditionssalzen, auf den isoelektrischen Punkt, z.B. mit schwachen Basen, oder durch Behandeln mit flüssigen Ionenaustauschern gebildet werden.

Salze können in üblicher Weise in die freien Verbindungen übergeführt werden, Metall- und AmmoniumSalze z.B. durch Behandeln mit geeigneten Säuren, und Säureadditionssalze z.B. durch Behandeln mit einem geeigneten basischen Mittel..

Das Verfahren umfasst auch diejenigen Ausführungsformen, wonach als Zwischenprodukte anfallende Verbindungen als Ausgangsstoffe verwendet und die restlichen Verfahrensschritte mit diesen durchge- führt werden, oder das Verfahren auf irgendeiner Stufe abgebrochen wird; ferner können Ausgangsstoffe in Form von Derivaten verwendet oder während der Reaktion gebildet werden.

Vorzugsweise werden solche Ausgangsstoffe verwendet und die Reaktionsbedingungen so gewählt, dass man zu den vorstehend als besonders aufgeführten Verbindungen gelangt.

Herstellung geschützter Verbindungen der Formel I: Die Ausgangsverbindungen der Formel I, worin mindestens eine der funktionellen Gruppen in geschützter Form vorliegt, und Verfahren zu ihrer Herstellung sind ebenfalls Gegenstand der vorliegenden Erfindung. Diese Verbindungen und Salze von solchen Verbindungen mit salzbildender Gruppe können in an sich bekannter Weise hergestellt werden, indem man z.B.

### a) in einer Verbindung der Formel -

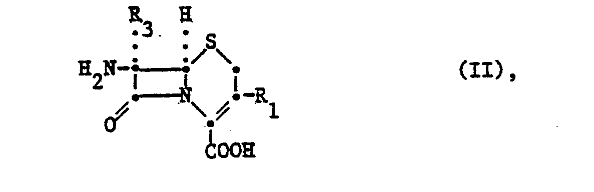

worin die Aminogruppe gegebenenfalls durch eine die Acylierung erlaubende Gruppe substituiert ist, und worin die 4-Carboxylgruppe und gegebenenfalls im Rest R₁ vorhandene weitere funktionelle Gruppen in geschützter Form vorliegen können, die Aminogruppe durch Behandeln mit einer Säure der Formel 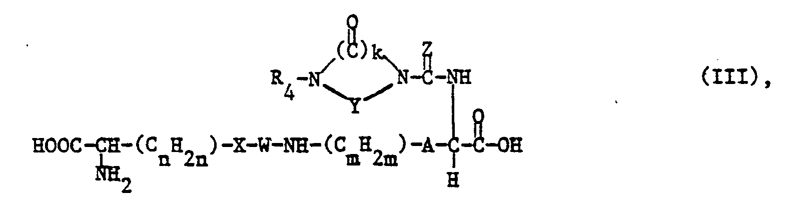 worin die Aminocarbonsäuregruppierung HOOC-CH(NH₂)- und gegebenenfalls in den Gruppierungen A und R₄ vorhandene weitere funktionelle Gruppen in geschützter Form vorliegen, oder mit einem reaktionsfähigen funktionellen Säurederivat oder einem Salz davon acyliert, oder

### b) in einer Verbindung der Formel

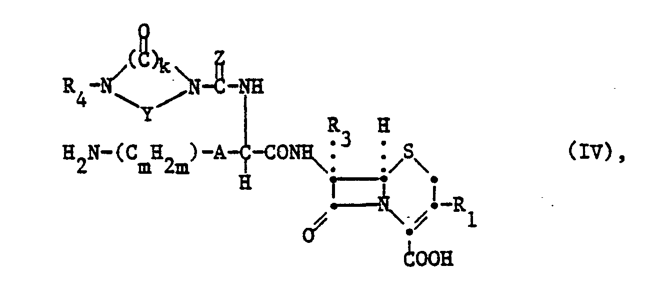

worin die Aminogruppe gegebenenfalls durch eine, die Acylierung erlaubende Gruppe substituiert sein kann, und worin die 4-Carboxylgruppe und gegebenenfalls im R₁ und in den Gruppierungen A und R₄ vorhandene weitere funktionelle Gruppen in geschützter Form vorliegen können, die Aminogruppe durch Behandeln mit einem reaktionsfähigen funktionellen Derivat einer Säure der Formel 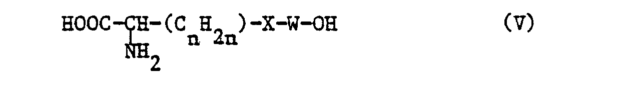 worin die Aminocarbonsäuregruppierung HOOC-CH(NH₂)- in geschützter Form vorliegt, oder wenn X-W zusammen eine Gruppe -CO- bedeuten, auch mit einer entsprechenden freien Säure, oder mit einem Salz davon, acyliert, oder

### c) in einer Verbindung der Formel

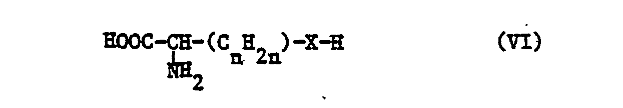

worin X Sauerstoff, Schwefel oder die Gruppe -NH- bedeutet, und worin die Aminocarbonsäuregruppierung HOOC-CH(NH₂)- in geschützter Form vorliegt, die Gruppe -X-H mit einem reaktionsfähigen funktionellen Derivat einer Verbindung der Formel 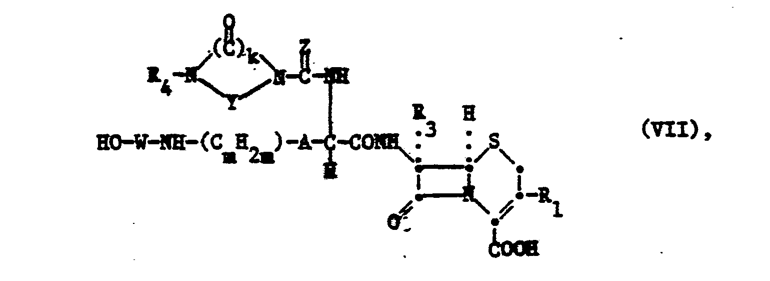 worin die 4-Carboxylgruppe und gegebenenfalls im Rest R₁ und in den Gruppierungen A und R₄ vorhandene funktionelle Gruppen in geschützter From vorliegen können, acyliert, oder

### d) in einer Verbindung der Formel

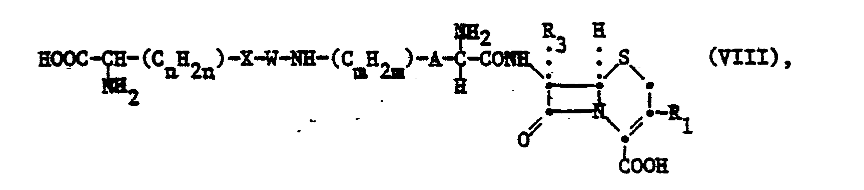

worin die Aminocarbonsäuregruppierung HOOC-CH(NH₂)- in geschützter Form vorliegt, die zu acylierende Aminogruppe gegebenenfalls durch eine, die Acylierung erlaubende Gruppe substituiert ist, und worin die 4-Carboxylgruppe und gegebenenfalls im Rest R und in der Gruppierung A vorhandene weitere funktionelle Gruppen in geschützter Form vorliegen können, die Aminogruppe durch Behandeln mit einem den entsprechenden Acylrest einer Kohlen- oder Thiokohlensäure der Formel 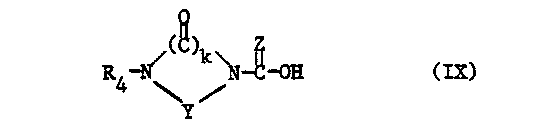 einführenden Acylierungsmittel, worin im Rest R₄ gegebenenfalls vorhandene funktionelle Gruppen in geschützter Form vorliegen können, acyliert, oder

### e) ein reaktionsfähiges Derivat einer Kohlensäure- oder Thiokohlen-

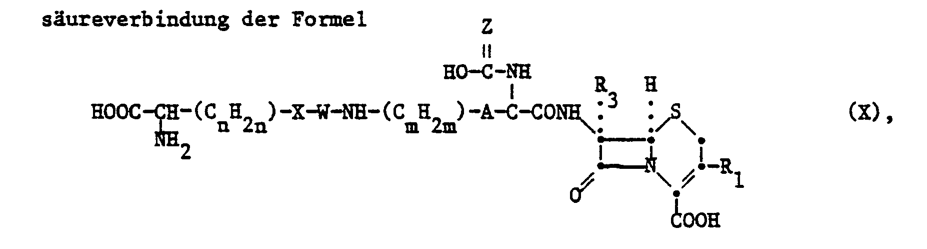

worin die Aminocarbonsäuregruppierung HOOC-CH(NH₂)- in geschützter Form vorliegt, und worin die 4-Carboxylgruppe und gegebenenfalls im Rest R₁ und in der Gruppierung A vorhandene weitere funktionelle Gruppen in geschützter Form vorliegen können, mit einem sekundären Amid der Formel - 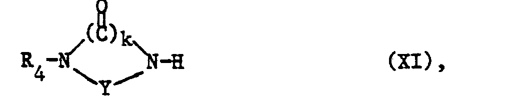 worin im Rest R₄ gegebenenfalls vorhandene funktionelle Gruppen geschützt sein können und die sekundäre Amidogruppe in einer die Acylierung erlaubenden Form vorliegt, reagieren lässt, oder

f) die unter d) oder e) genannte Acylierung in situ vornimmt, indem man eine Aminoverbindung der Formel VIII und eine Amidoverbindung der Formel XI, beide mit der oben angegebenen Bedeutung, gleichzeitig oder nacheinander auf ein bivalentes reaktives Derivat der Kohlen-oder Thiokohlensäure einwirken lässt, oder indem man

### g) eine Verbindung der Formel

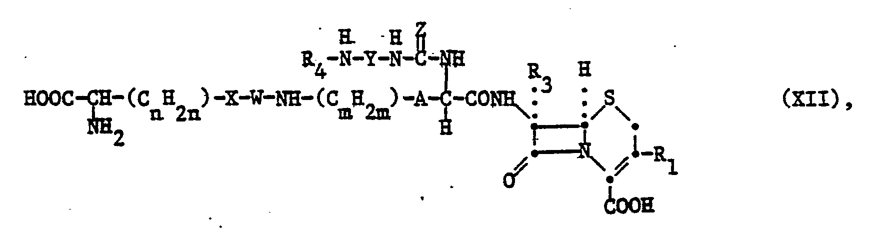

worin die Aminocarbonsäuregruppierung HOOC-CH(NH₂)- in geschützter Form vorliegt, und worin die 4-Carboxylgruppe und gegebenenfalls im Rest R₁ und in der Gruppierung A vorhandene funktionelle Gruppen in geschützter Form vorliegen können, und die später den Piperazinring bildende sekundäre Amino- bzw. Amidogruppen gegebenenfalls durch Gruppen substituiert sind, die die N-Acylierung erlauben, mit einem bivalenten acylierenden Derivat der Kohlensäure oder der Oxalsäure unter Ringschluas diacyliert, oder

### h) eine 2-Cephaverbindung der Formel

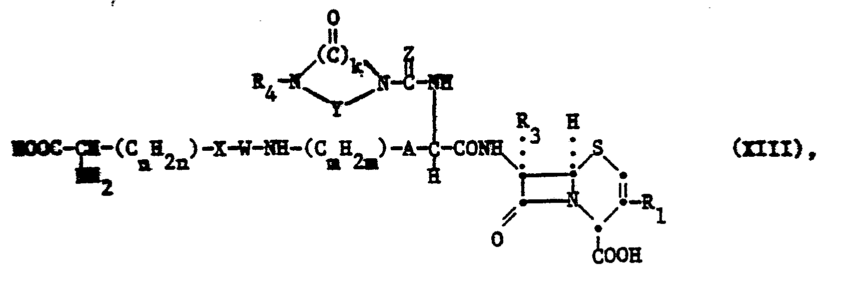

worin funktionelle Gruppen geschützt sind, zur entsprechenden 3-Cephenverbindung der Formel I isomerisiert, oder

### i) in einer Verbindung der Formel

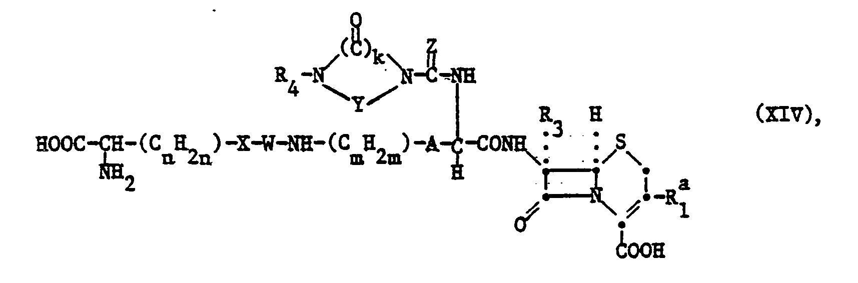

worin 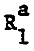 eine freie oder sulfonylierte Hydroxygruppe ist und worin andere funktionelle Gruppen geschützt sind, R^{a}₁ gegen Halogen austauscht, oder

### j) in einer Verbindung der Formel XIV, worin R^{a}₁ eine freie Hydroxygruppe ist und worin andere funktionelle Gruppen geschützt sind, die Hydroxygruppe veräthert, oder

### k) in einer Verbindung der Formel XIV, worin funktionelle Gruppen geschützt sind, und worin R^{a}₁ eine Formylgruppe bedeutet, diese in Gegenwart von Decarbonylierungs-Katalysatoren zu einer Verbindung der Formel I, worin R₁ Wasserstoff bedeutet, decarbonyliert, oder

### 1) in einer Verbindung der Formel XIV, worin R^{a}₁ freies oder verestertes Hydroxy oder ein gegebenenfalls niederalkyliertes oder durch Niederalkylen oder Heteroniederalkylen cyclisch disubstituiertes Amino ist, und worin funktionelle Gruppen geschützt sind, die Gruppe R^{a}₁ durch Wasserstoff ersetzt, und, wenn gewünscht, in einer erhaltenen Verbindung noch nicht geschützte funktionelle Gruppen schützt oder eine Schutzgruppe gegen eine andere austauscht, und/oder, wenn gewünscht, in einem Rest R₁ eine Gruppe R_{Z} gegen eine andere Gruppe R₂ austauscht, und/oder, wenn gewünscht, eine erhaltene Verbindung, worin R₃ Wasserstoff ist, in einer Verbindung überführt, worin R₃ Methoxy ist, und/oder, wenn gewünscht, ein erhaltenes Isomerengemisch in die einzelnen Isomeren auftrennt, und/oder, wenn erwünscht, eine erhaltene Verbindung in ein Salz oder ein erhaltenes Salz in die freie Verbindung oder ein ein anderes Salz überführt.

Verfahren a) bis g) (Acylierungen): Gegebenenfalls vorhandene, die Aminogruppe substituierende und deren Acylierung erlaubende Reste in einem Ausgangsmaterial der Formel II, IV, VIII, XI und XII sind beispielsweise organische Silyl- oder Stannylgruppen, ferner auch Ylidengruppen, die zusammmen mit der Aminogruppe eine Schiff'sche Base bilden. Die genannten organischen Silyl- oder Stannylgruppen sind z.B. die gleichen, die auch mit der 4-Carboxylgruppe am Cephemring eine geschützte Carboxylgruppe zu bilden vermögen. Es sind dies in erster Linie Triniederalkylsilyl, insbesondere Trimethylsilyl. Bei der Silylierung öder Stannylierung einer Carboxylgruppe in einem Ausgangsmaterial der Formel II,IV, VIII, IX oder XII, kann, bei Verwendung eines Ueberschusses des Silylierungs- oder Stannylierungsmittels, die Aminogruppe ebenfalls silyliert oder stannyliert werden. Die genannten Ylidengruppen sind in erster Linie 1-Aryl-niederalkyliden-, insbesondere Arylmethylengruppen, worin Aryl insbesondere für einen carbocyclischen, in erster Linie monocyclischen Arylrest, z.B. für gegebenenfalls, wie durch Niederalkyl, Hydroxy, Niederalkoxy und/oderNitro substituiertes Phenyl steht; solche Arylmethylengruppen sind z.B. Benzyliden, 2-Hydroxybenzyliden oder 4-Nitrobenzyliden, ferner gegebenenfalls, z.B. durch Carboxy substituiertes Oxacycloalkyliden, z.B. 3-Carboxy-2-oxacyclohexyliden.

Die in den Ausgangstoffen der Formeln II bis XIV vorhandenen funktionellen Gruppen können durch die bereits unter den Verbindungen der Formel I genannten Schutzgruppen geschützt sein. Bevorzugt sind alle nicht an den Umsetzungsreatkionen teilnehmenden reaktionsfähigen funktionellen Gruppen, insbesondere aber gegebenenfalls vorhandene Carboxyl-, Sulfo-, Amino-, Hydroxy- und Mercaptogruppen, geschützt.

Den Acylrest einer Carbonsäure einführenden Acylierungsmittel sind beispielsweise die Carbonsäure selbst, wenn diese, wie im Falle einer Säure der Formel III oder V oder der Oxalsäure ausreichend stabil ist, oder reaktionsfähige funktionelle Derivate davon.

Falls gemäss Acylierungsverfahren a), b) oder g) eine freie Säure der Formel III oder V, worin alle funktionellen Gruppen ausser der reagierenden Carboxylgruppe geschützt sind, oder die Oxalsäure zur Acylierung eingesetzt wird, verwendet man üblicherweise geeignete Kondensationsmittel, wie Carbodiimide, beispielsweise N,N'-Diäthyl-, N,N'-Dipropyl-, N,N'-Diisopropyl-, N,N'-Dicyclohexyl- oder N-Aethyl-N^{f-}3-dimethylaminopropyl-carbodiimid, geeignete Carbonylverbindungen, beispielsweise Carbonyldiimidazol, oder Isoxazoliniumsalze, beispielsweise N-Aethyl-5-phenyl-isoxazolinium-3'-sulfonat und N-tert.-Butyl-5-methyl-isoxazoliniumperchlorat, oder eine Acylaminoverbindung, z.B. 2-Aethosy-1-äthoaycarbonyl-1,2-dihydro-chinolin.

Die Kondensationsreaktion wird vorzugsweise in einem wasser- - freien Reaktionsmedium, vorzugsweise in Gegenwart eines Lösungs- oder Verdünnungsmittels, z.B. Methylenchlorid, Dimethylformamid, Acetonitril oder Tetrahydrofuran, wenn erwünscht oder notwendig, unter Kühlen oder Erwärmen, z.B. in einem Temperaturbereich von etwa -40°C bis etwa +100°C, bevorzugt von etwa -20°C bis +50°C, und/oder in einer Inertgasatmosphäre, durchgeführt.

Ein reaktionsfähiges, d.h. Amid-bildendes, bzw. Esterbildendes, funktionelles Derivat einer Säure der Formel III, V, VII, IX oder X, worin alle funktionellen Gruppen ausser der reagierenden Säuregruppe geschützt sind bzw. sein können, oder der Kohlensäure oder Oxalsäure, ist in erster Linie ein Anhydrid einer solchen Säure, inklusive und vorzugsweise ein gemischtes Anhydrid, aber auch ein inneres Anhydrid, d.h. ein entsprechendes Keten oder, in der Säure V, wenn W die Gruppe -S0₂NH-CO- ist, oder wenn X die Gruppe -NH- und W die Gruppe -CO- oder -CO-NHSO₂- ist, oder in der Säure X, ein entsprechendes Isocyanat. Gemischte Anhydride sind z.B. diejenigen mit anorganischen Säuren, wie Halogenwasserstoffsäuren, d.h. die entsprechenden Säurehalogenide, z.B. -chloride oder -bromide, ferner mit Stickstoffwasserstoffsäure, d.h. die entsprechenden Säureazide, mit einer phosphorhaltigen Säure, z.B. Phosphorsäure oder phosphoriger Säure, oder mit einer schwefelhaltigen Säure, z.B. Schwefelsäure, oder mit Cyanwasserstoffsäure. Weitere gemischte Anhydride sind z.B. diejenigen mit organischen Carbonsäuren, wie mit gegebenenfalls, z.B. durch Halogen, wie Fluor oder Chlor, substituierten Niederalkancarbonsäuren, z.B. Pivalinsäure oder Trichloressigsäure, oder mit Halbestern, insbesondere Niederalkylhalbestern der Kohlensäure, wie dem Aethyl- oder Isobutylhalbester der Kohlensäure, oder mit organischen, insbesondere aliphatischen oder aromatischen, Sulfonsäuren, z.B. p-Toluolsulfonsäure.

Weitere, zur Reaktion mit der Amino-, Hydroxy- oder Mercaptogruppe geeignete Säurederivate einer Säure der Formel III, V, VII, XI oder X, worin alle funktionelle Gruppen ausser der reagierenden Carboxylgruppe geschützt sind, bzw. sein können, oder der Kohlensäure oder Oxalsäure, sind aktivierte Ester, wie Ester mit vinylogen Alkoholen (d.h. Enolen), wie vinylogen Niederalkenolen, Cyanmethylester, Trichlormethylester, Iminomethylesterhalogenide, wie Dimethyliminomethylesterchlorid (hergestellt aus der Carbonsäure und Dimethylchlormethylidin-iminiumchlorid der Formel [(CH₃)₂N=CHCl] ⊕CL⊖, das man z.B. aus N,N-Dimethylacetamid und Phosgen erhalten kann) oder Arylester, wie vorzugsweise geeignet, z.B. durch Halogen, wie Chlor, und/oder Nitro, substituierte Phenylester, z.B. 4-Nitrophenyl-, 2,3-oder 2,4-Dinitrophenyl- oder 2,3,4,5,6-Pentachlorphenylester, N-heteroaromatische Ester, wie N-Benztriazol-, z.B. 1-Benztriazolester, oder N-Diacyliminoester, wie N-Succinylimino- oder N-Phthalyliminoester.

Die Acylierung mit einem Säurederivat, wie einem Anhydrid, insbesondere mit einem Säurehalogenid, wird bevorzugt in Anwesenheit eines säurebindenden Mittels, beispielsweise einer organischen Base, wie eines organischen Amins, z.B. eines tertiären Amins, wie Triniederalkylamin, z.B. Trimethylamin, Triäthylamin oder Aethyl-diisopropylamin, oder N,N-Diniederalkyl-anilin, z.B. N,N-Dimethylanilin, oder eines cyclischen tertiären Amins, wie eines N-niederalkylierten Morpholins, wie N-Methylmorpholin, oder einer Base vom Pyridin-Typ, z.B. Pyridin, ferner anorganischen Base, beispielsweise eines Alkalimetall- oder Erdalkalimetallhydroxids, -carbonats oder -hydrogencarbonats, z.B. Natrium-, Kalium- oder Calciumhydroxid, -carbonat oder-hydrogencarbonat, oder eines Oxirans, beispielsweise eines niederen 1,2-Alkylenoxids, wie Aethylenoxid oder Propylenoxid, durchgeführt. Diese Basen spielen vor allem bei der Acylierung von Aminogruppen ein Rolle. Bei der Acylierung von Amidgruppen verwendet man als säurebindende Mittel vorteilhafter einen neutralen Protonenfänger, z.B. einen solchen aus der Gruppe der Oxirane, wie ein niederes 1.2-Alkylenoxid, z.B. Aethylenoxid, oder ein reaktionsfähiges Olefin, wie Niederalkylen,z.B. Propylen, oder ein Molekularsieb, z.B. ein pulver- ° förmiges Typ 4 A-Molekülarsieb und/oder man setzt die (zuvor oder in situ) silylierte oder stannylierte, wie Triniederalkyl-, z.B. Trimethyl-silylierte Amidgruppe um. Bei den genannten Amid-Acylierungsreaktionen setzt man z.B. gemischte Anhydride, insbesondere die entsprechenden Säurechloride, also Säurechloride der Verbindungen der Formel X, sowie die Mono- oder Disäurechloride der Kohlensäure, Thiokohlensäure bzw. Oxalsäure ein.

Die obigen Acylierungen können in einem inerten, gegebenenfalls wasserfreien Lösungsmittel oder Lösungsmittelgemisch vorgenommen werden, beispielsweise in einem Carbonsäureamid, wie einem Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, einem Ester, z.B. Essigsäureäthylester, oder einem Nitril, z.B. Acetonitril, oder Mischungen davon, und, wenn notwendig, bei erniedrigter oder erhöhter Temperatur, z.B. in einem Temperaturbereich von etwa -50°C bis etwa 100°C, bevorzugt von etwa =10°C bis etwa +40°C, und/oder in einer Inertgas-, z.B. Stickstoffatmosphäre. Zur Erhöhung der Löslichkeit der Reagentien, insbesondere der zu acylierenden Amine der Formel III, falls diese als Salze eingesetzt werden, kann dem Lösungsmittel auch Wasser zugesetzt werden. Beispielsweise ist Aceton/Wasser im Verhältnis 0,5:1 bis 3:1 ein besonders gut geeignetes Lösungsmittelgemisch.

In einer acylierenden Säure der Formel III, V oder VII oder in einem Säurederivat davon kann eine geschützte Aminogruppe'auch in ionischer Form vorliegen, d.h. das Ausgangsmaterial der Formel III, V oder VII kann in Form eines Säureadditionssalzes, vorzugsweise mit einer starken anorganischen Säure, wie einer Halogenwasserstoffsäure, z.B. Salzsäure, oder Schwefelsäure verwendet werden.

Ferner kann ein Säurederivat, wenn erwünscht, in situ gebildet werden. So erhält man z.B. ein gemischtes Anhydrid durch Behandeln einer Säure der Formel III, oder einer Säure der Formel V, worin X-W die Gruppe -CO- bedeutet, mit entsprechend geschützten funktionellen Gruppen, oder eines geeigneten Salzes davon, wie eines Ammoniumsalzes, z.B. mit einem organischen Amin, wie 4-Methylmorpholin, oder eines Metall-, z.B. Alkalimetallsalzes, mit einem geeigneten Säurederivat, wie einem entsprechenden Säurehalogenid einer gegebenenfalls substituierten Niederalkancarbonsäure, z.B. Trichloracetylchlorid, oder mit einem Halbester eines Kohlensäurehalogenids, z.B. Chlorameisensäureäthylester oder -isobutylester, und verwendet das so erhältliche gemischte Anhydrid ohne Isolierung.

Ein Säurechlorid einer Säure der Formel V, worin X-W eine Gruppierung -O-CO-, -S-CO- oder -NH-CO- ist und worin die Aminocarbonsäuregruppierung HOOC-CH(NH₂)- in geschützter Form vorliegt, kann, z.B. in situ, gebildet werden, indem man eine Aminocarbonsäure der Formel VI, worin X Sauerstoff, Schwefel oder die Gruppe -NH- bedeutet, und die Gruppierung HOOC-CH(NH₂)- in geschützter Form vorliegt, in Gegenwart eines Salzsäureakzeptors in einem inerten organischen Lösungsmittel oder Lösungsmittelgemisch mit Phosgen behandelt. Die Salzsäureakzeptoren, Lösungsmittel und Reaktionsbedingungen sind die gleichen, die für die Acylietung von Verbindungen der Formel II oder IV genannt wurden; beispielsweise kann die Reaktion in Gegenwart von Pyridin in Methylenchlorid und Toluol bei etwa 0° bis etwa +10°C. stattfinden.

Auf die gleiche Weise kann, z.B. in situ, ein.Säurechlorid einer Säure der Formel VII, worin W die Gruppe -SO₂NH-CO- bedeutet, und worin die 4-Carboxylgruppe und in den Gruppen A, R₄ und R₁ gegebenenfalls vorhandene weitere funktionelle Gruppen geschützt sind, aus einer entsprechend geschützten Verbindung der Formel IV, durch Behandlung mit Chlorsulfonylisocyanat hergestellt werden.

Analog kann ein Säurechlorid einer Säure der Formel IX oder X aus einem sekundären Amid der Formel XI bzw. aus einem Amin der Formel VIII und Phosgen hergestellt werden.

Die entsprechenden reaktionsfähigen Säurederivate der Kohlen- bzw. Thiokohlensäuren der allgemeinen Formel IX bzw. X werden sogar vorzugsweise in situ gebildet, indem man auf die entsprechenden Aminoverbindungen der allgemeinen Formel XI bzw. VIII ein bivalentes reaktives Derivat der Kohlen- bzw. Thiokohlensäure, wie eines der unten genannten, vorzugsweise ein entsprechendes Säurechlorid, wie Phosgen, Thiophosgen oder ein Chlorameisensäurederivat, vorzugsweise in Gegenwart eines der oben genannten neutralen Protonenfänger, einwirken lässt.

Als bevorzugtes, gemäss Acylierungsverfahren d) den Acylrest einer Kohlen- oder Thiokohlensäure der Formel IX einführendes Acylierungsmittel ist daher das entsprechende Carbaminsäure- oder Thiocarbaminsäurehalogenid, insbesondere -chlorid, zu nennen.

Als bevorzugte Kohlen- oder Thiokohlensäureverbindung der Formel X gemäss Acylierungsverfahren e) ist ebenfalls das entsprechende Carbamin- bzw. Thiocarbaminsäurehalogenid, z.B. das entsprechende -chlorid zu nennen, das aber im Gegensatz zu dem entpsrechenden Derivat der allgemeinen Formel IX, als Derivat eines primären Amins leicht unter Abspaltung von Halogenwassertoff bzw. Chlorwasserstoff das entsprechende Isocyanat bzw. Isothiocyanat bilden und als solches mit Amiden der Formel XI spontan unter-Ausbildung der Harnstoffgruppierung in die erfindungsgemässe Verbindung der Formel I übergehen kann. Es ist zweckmässig, auch bei Verfahren d) und e) unpolare inerte Lösungsmittel, wie Chlorniederaliphaten, z.B. Methylenchlorid, Chloroform oder ein gegebenenfalls chloriertes aromatisches Lösungsmittel wie Benzol, Toluol oder Chlorbenzol oder ein inertes ätherisches Lösungsmittel wie Tetrahydrofuran oder Dioxan zu verwenden und die Reaktion bei oder unterhalb Raumtemperatur, also unter Kühlen, durchzuführen. Bivalente reaktive Derivate der Kohlen-, Thiokohlen- oder Oxalsäure, die für die unter f) und g) genannten Acylierungsverfahren Verwendung finden, sind die entsprechenden Säurehalogenide, wie Säurechloride" z.B. Phosgen oder Oxalylchlorid, Orthoester, wie Niederalkylortho- " " ester, z.B. Orthokohlensäuretetramethylester, Imidoester, wie Imidokohlensäureniederalkylester, z.B. Imidokohlensäuredimethylester, Esterhalogenide, wie Halogenameisensäureniederalkylester, z.B. Chlorameisensäuremethylester, Imidhalogenide, wie Imidokohlensäurehalogenide, z.B. Kohlensäureimidchlorid, aktivierten Ester, wie entsprechende Enol- oder Arylester, z.B. Bis-p-nitrophenylcarbonat oder aktivierten Amide, wie entsprechende heterocyclische Bisamide der Kohlensäure, z.B. Carbonyldiimidazol. Bevorzugte Verbindungen dieser Art sind Kohlensäure- oder Thiokohlensäurehalogenide, z.B. Phosgen, Thiophosgen oder Kohlensäuredibromid, Tetramethyl-orthocarbonat, Chlorkohlensäureimidchlorid, Chlorcyan, Chlorameisensäureester, wie der entsprechende Methyl-, Trichlormethyl-, Benzyl- oder Phenylester, Imidokohlensäurediäthylester oder -diphenylester, Carbonyldiimidazol, Carbonylditetrazol, oder Kohlensäure-di-(p-nitrophenyl)-ester, sowie entsprechende Verbindungen der Oxalsäure, z.B. Oxalylchlorid.

Die im Acylierungsverfahren g) verwendeten, acylierend wirkenden bivalenten Derivate der Kohlensäure oder Oxalsäure und ebenso die entsprechenden im Verfahren f) verwendeten bivalenten reaktiven Derivate, können ä priori in doppelt reaktiver Form, oder zunächst in Form eines monoreaktiven Halbderivats der Kohlen- oder Thiokohlensäure (in dem nur ein Säurerest in reaktiver Form vorliegt) eingesetzt und die zweite reaktive Gruppe erst nachträglich gebildet oder aktiviert und (mit einem zweiten Reaktionspartner) zur Reaktion gebracht werden.

Vorzugsweise wird beim Verfahren.g) Phosgen oder Oxalylchlorid, vor allem in einem 2-10 molaren Ueberschuss, in einem unpolaren iaerten Lösungsmittel, wie Methylenchlorid, vorgelegt und darauf die geschützte, an den endständigen Aminogruppen silylierte Verbindung der FormelXII einwirken gelassen. Als zusätzliche Massnahme kann ein zweites säurebindendes Mittel, wie Propylen oder Propylenoxid, verwendet und die Acylierung unter Kühlung z.B. bei oder unterhalb 0°C, vorgenommen werden.

Die Acylierung einer Verbindung der Formel II kann auch durch Verwendung eines geeigneten Derivates der Säure der Formel III in Gegenwart einer geeigneten Acylase erfolgen. Solche Acylasen sind bekannt und können von einer Anzahl von Mikroorganismen gebildet werden, - z.B. durch Acetobacter, wie Acetobacter aurantium, Achromobacter, wie Achromobacter aeris, Aeromonas, wie Aeromonas hydrophila, oder Bacillus, wie Bacillus megaterium 400. In einer solchen enzymatischen Acylierung werden insbesondere Amide, Ester oder Thioester, wie Niederalkyl-, z.B. Methyl- oder Aethylester, der Carbonsäure der Formel III als entsprechende Derivate eingesetzt. Ueblicherweise wird eine solche Acylierung in einem, den entsprechenden Mikroorganismus enthaltenden Nährmedium, in einem Filtrat der Kulturbrühe oder, gegebenenfalls nach Isolierung der Acylase, inklusive nach Adsorption an einen Träger, in einem wässrigen, gegebenenfalls einen Puffer enthaltenden Medium, z.B. in einem Temperaturbereich von etwa +20°C bis etwa +40°C, bevorzugt bei etwa +37°C, durchgeführt.

Verfahren h) (Isomerisierung): In einem 2-Cephem-Ausgangsmaterial der Formel XIII weist die gegebenenfalls geschützte Carboxylgruppe in der 4-Stellung des Cephemringes vorzugsweise die a-Konfiguration auf.

2-Cephem-Verbindungen der Formel XIII können isomerisiertwerden, indem man sie mit einem schwach-basischen Mittel behandelt und die entsprechende 3-Cephem-Verbindung isoliert. Geeignete Isomerisierungsmittel sind z.B. organische, stickstoffhaltige Basen, insbesondere tertiäre heterocyclische Basen aromatischen Charakters, in erster Linie Basen des Pyridin-Typs, wie Pyridin selber, sowie Picoline, Collidine oder Lutidine, ferner Chinolin, tertiäre aromatische Basen, z.B. solche des Anilin-Typs, wie N,N-Diniederalkylaniline, z.B. N,N-Dimethylanilin oder N,N-Diäthylanilin, oder tertiäre aliphatische, azacycloaliphstische oder araliphatische Basen, wie N,N,N-Triniederalkylamine, z.B. N,N,N-Trimethylamin oder N,N-Diisopropyl-N-äthylamin, N-Niederalkyl-azacycloalkane, z.B. N-Methyl-piperidin, oder N-Phenylniederalkyl-N,N-diniederalkyl-amine, z.B. N-Benzyl-N,N-dimethylamin, sowie Gemische von solchen basischen Mitteln, wie das Gemisch einer Base vom Pyridintyp und eines N,N,N-Tri-niederalkylamins, z.B. Pyridin und Triäthylamin. Ferner können auch anorganische oder organische Salze von Basen, insbesondere von mittelstarken bis starken Basen mit schwachen Sauren, wie Alkalimetall-, oder Ammoniumsalze von Niederalkancarbonsäuren, z.B. Natriumacetat, Triäthylammoniumacetat oder N-Methyl-piperidinacetat, sowie andere analoge Basen oder Gemische von solchen basischen Mitteln verwendet werden.

Bei der Isomerisierung von 2-Cephem-Verbindungen der Formel XIII mit basischen Mitteln arbeitet man vorzugsweise in wasserfreiem Medium, in An- oder Abwesenheit eines Lösungsmittels, wie eines gegebenenfalls halogenierten, z.B. chlorierten, aliphatischen, cycloaliphatischen oder aromatischen Kohlenwasserstoffs, oder eines Lösuagsmittelgemisches, wobei als Reaktonsmittel verwendete, unter den Reaktonsbedingungen flüssige Basen gleichzeitig auch als Lösungsmittel dienen können, gegebenenfalls unter Kühlen oder unter Erwärmen, vorzugsweise in einem Temperaturbereich von etwa -30°C bis etwa +100°C, in einer Inertgas-, z.B. Stickstaffatmosphäre, und/oder in einem geschlossenen Gefäss.

So erhältliche 3-Cephem-Verbindungen der Formel I lassen sich in an sich bekannter Weise, z.B. durch Adsorption und/oder Kristallisation, von gegebenenfalls noch vorhandenen 2-Cephem-Ausgangsstoffen abtrennen.

Die Isomerisierung von_2-Cephem-verbindungen der Formel XIII wird vorzugsweise durchgeführt, indem man diese in 1-Stellung oxidiert, wenn erwünscht, gegebenenfalls Isomerengemische der gebildeten 1-Oxide trennt,.und die so erhältlichen 1-Oxide der entsprechenden 3-Cephem- verbindungen, wenn erwünscht, reduziert.

Als geeignete Oxidationsmittel für die Oxidation in 1-Stellung von 2-Cephem-verbindungen der Formel XIII kommen anorganische Persäuren, die eine Reduktionspotential von wenigstens +1,5 Volt aufweisen und aus nicht-metallischen Elementen bestehen, organische Persäuren oder Gemische aus Wasserstoffperoxid und Säuren, insbesondere organische Carbonsäuren, mit einer Diisoziationskonstante von wenigstens 10⁻⁵ in Frage. Geeignete anorganische Persäuren sind Perjod- und Perschwefalsäure. Organische Persäuren sind entsprechende Percarbon- und Persulfonsäuren, die als solche zugesetzt oder durch Verwendung von wenigstens einem Aequivalent Wasserstoffperoxyd und einer Carbonsäure in situ gebildet werden können. Dabei ist es zweckmässig, einen grossen Ueberschuss der Carbonsäure anzuwenden, wenn z.B. Essigsäure als Lösungsmittel verwendet wird. Geeignete Persäuren sind z.B. Perameisensäure, Peressigsäure, Trifluorperessigsäure, Permaleinsäure, Perbenzoesäure, 3-Chlorperbenzoesäure, Monoperphthalsäure oder p-Toluolpersulfonsäure.

Die Oxidation kann ebenfalls unter Verwendung von Wasserstoffperoxid mit katalytischen Mengen einer Säure mit einer Dissoziationskonstante von wenigstens 10⁻⁵ durchgeführt werden, wobei man niedrige Konzentrationen, z.B. 1-2% und weniger, aber auch grössere Mengen der Säure einsetzen kann. Dabei hängt die Wirksamkeit des Gemisches in erster Linie von der Stärke der Säure ab. Geeignete Gemische sind z.B. solche von Wasserstoffperoxid mit Essigsäure, Perchlorsäure oder Trifluoressigsäure.

Die obige Oxidation kann in Gegenwart von geeigneten Katalysatoren durchgeführt werden. So kann z.B. die Oxidation mit Percarbonsäuren durch die Anwesenheit einer Säure mit einer Dissoziationskonstante von wenigstens 10⁻⁵ katalysiert werden, wobei ihre Wirksamkeit von ihrer Stärke abhängt. Als Katalysatoren geeignete Säuren sind z.B. Essigsäure, Perchlorsäure und Trifluoressigsäure. Ueblicherweise verwendet man mindestens äquimolare Mengen des Oxidationsmittels, vorzugsweise einen geringen Ueberschuss von etwa 10% bis etwa 20%, wobei man auch grössere Ueberschüsse, d.h. bis zur 10-fachen Menge des Oxidationsmittels oder darüber, verwenden kann. Die Oxidation wird unter milden Bedingungen, z.B. bei Temperaturen von etwa -50°C bis etwa +100°C, vorzugsweise von etwa -10°C bis etwa +40°C durchgeführt.

Die Reduktion der 1-oxide von 3-Cephem-Verbindungen kann in an sich bekannter Weise durch Behandeln mit einem Reduktionsmittel, wann notwendig, in Anwesenheit eines aktivierenden Mittels, durchgeführt werden. Als Reduktionsmittel kommen beispielsweise in Betracht: Katalytisch aktivierter Wasserstoff, wobei Edelmetallkatalysatoren verwendet werden, welche Palladium, Platin oder Rhodium enthalten, und die man gegebenenfalls zusammen mit einem geeigneten Trägermaterial, wie Kohle oder Bariomsulfat, einsetzt; reduzierende Zinn-, Eisen-, Kupfer- oder Mangankationen, welche in Form von entsprechenden Verbindungen oder Komplexen anorganischer oder organischer Art, z.B. als Zinn-chlorid, -fluorid, -acetat oder -formiat, Eisen-II-chlorid, -sulfat, -oxalat oder -succinat, Kupfer-I-chlorid, -benzoat oder -oxyd, oder Mangan-II-chlorid, -sulfat, -acetat oder -oxyd, oder als Komplexe, z.B. mit Aethylendiamintetraessigsäure oder Nitrolotriessigsäure, verwendet werden; reduzierende Dithionit-, Jod- oder Eisen-II-cyanid-anionen, welche in Form von entsprechenden anorganischen oder organischen Salzen, wie Alkalimetall-, z.B. Natrium- oder Kaliumdithionit, Natrium- oder Kaliumjodid oder -eisen-II-cyanid, oder in Form der entsprechenden Säuren, wie Jodwasserstoffsäure, verwendet werden; reduzierende trivalente anorganische oder organisehe Phosphorverbindungen, wie Phosphine, ferner Ester, Amide und Halogenide der phosphinigen, phosphonigen oder phosphorigen Säure, sowie diesen Phosphorsauerstoffverbindungen entsprechende Phosphor-Schwefelverbindungen, worin organische Reste in erster Linie aliphatische, aromatische oder araliphatische Reste, z.B. gegebenenfalls substituiertes Niederalkyl, Phenyl oder Phenylniederalkyl darstellen , wie z.B. Triphenylphosphin, Tri-n-butylphosphin, Diphenylphosphinigsäuremethylester, Diphenylchlorphosphin, Phenyldichlorphosphin, Benzolphosphonigsäuredimethylester, Butanphosphonigsäuremethylester, Phosphorigsäuretriphenylester, Phosphorigsäuretrimethylester, Phosphortrichlorid, Phosphortribromid, etc.; reduzierende Halogensilanverbindungen, die mindestens ein an das Siliciumatom gebundenes Wasserstoffatom aufweisen, und die ausser Halogen, wie Chlor, Brom oder Jod, auch organische Raste, wie aliphatische oder aromatische Gruppen, z.B. gegebenenfalls substituiertes Niederalkyl oder Phenyl, aufweisen können, wie Chlorsilan, Bromsilan, Di- oder Trichlorsilan, Di- oder Tribromsilan, Diphenylchlorsilan, oder Dimethylchlorsilan, sowie auch Halogensilanverbindungen, worin alle Wasserstoffe durch organische Reste ersetzt sind, wie Triniederalkylhalogensilan, z.B. Trimethylchlorsilan oder Trimethyljodsilan, oder cyclische, Schwefelenthaltende Silane, wie 1,3-Dithia-2,4-disilacyclobutane oder 1,3,5-Trithia-2,4,6-trisila-cyclohexane, deren Siliciumatome durch Kohlenwasserstoffreste, wie insbesondere Niederalkyl substituiert sind, z.B. 2,2,4,4-Tetramethyl-1,3-dithia-2,4-disilacyclobutan oder 2,2,4,4,6,6-Hexamethyl-1,3,5-trithia-2,4,6-trisilacyclohexan, etc.; reduzierende quaternäre Chlormethylen-iminiumsalze, insbesondere -chloride oder -bromide, worin die Iminiumgruppe durch einen bivalenten oder zwei monovalente organische Reste, wie gegebenenfalls substituiertes Niederalkylen bzw. Niederalkyl substituiert ist, wie N-Chlormethylen-N,N-diäthyliminiumchlorid oder N-Chlormethylen-pyrrolidiniumchlorid; oder komplexe Metallhydride, wie Natriumborhydride, in Gegenwart von geeigneten Aktivierungsmitteln, wie Cobalt-II-chlorid, sowie Borandichlorid.

Als aktivierende Mittel, die zusammen mit denjenigen der obgenannten Reduktionsmittel verwendet werden, welche selber nicht Lewissäure-Eigenschaften aufweisen, d.h. in erster Linie zusammen mit den Dithionit-, Jod- oder Eisen-II-cyanid- und den nicht-halogenhaltigen trivalenten Phosphor-Reduktionsmitteln oder bei der katalytischen Reduktion eingesetzt werden, sind insbesondere organische Carbon- und Sulfonsäurehalogenide, ferner Schwefel-, Phosphor-oder Siliciumhalogenide mit gleicher oder grösserer Hydrolysenkonstante zweiter Ordnung als Benzoylchlorid, z.B. Phosgen, Ozalylchlorid, Essigsäurechlorid oder -bromid, Chloressigsäurechlorid; Pivalinsäurechlorid, 4-Methoxybenzoesäurechlorid, 4-Cyanbenzoesäurechlorid, p-Toluolsulfonsäurechlorid, Methansulfonsäurechlorid, Thionylchlorid, Phosphoroxychlorid, Phosphortrichlorid, Phosphortribromid, Phenyldichlorphosphin, Benzolphosphonigsluredichlorid, Dimethylchlorsilan oder Trichlorsilan, ferner geeignete Säureanhydride, wie Trifluoressigsäuresahydrid, oder cyclische Sultone, wie Aethansulton, Propansulton, 1,3-Butansulton oder 1,3-Hexansulton zu erwähnen.

Die Reduktion wird vorzugweise in Gegenwart von Lösungsmitteln oder Cemischen davon durchgeführt, deren Auswahl in erster Linie durch die Löslichkeit der Ausgangsstoffe und die Wahl des Reduktionsmittels bestimmt wird, so z.B. Niederalkancarbonsäuren oder Ester davon, wie Essigsäure und Essigsäureäthylester, bei der katalytischen Reduktion, und z.B. gegebenenfalls substituierte, wie halogenierte oder nitrierte, aliphatische, cycloaliphatische, aromatische oder araliphatische Kohlenwasserstoffe, z.B. Benzol, Methylenchlorid, Chloroform oder Nitromethan, geeignete Säurederivate, wie Niederalkancarbonsäureester oder -nitrile, z.B. Essigsäureäthylester oder Acetonitril, oder Amide von anorganischen oder organischen Säuren, z.B. Dinethylformamid oder Hexamethylphosphoramid, Aether, z.B. Diäthyläther, Tetrahydrofuran oder Dioxan, Ketone, z.B. Aceton, oder Sulfone, insbesondere aliphatische Sulfone, z.B. Dimethylsulfon oder Tetramethylensulfon, etc., zusammen mit den chemischen Reduktionsmitteln, wobei diese Lösungsmittel vorzugsweise kein Wasser enthalten. Dabei arbeitet man gewöhnlicherweise bei Temperaturen von etwa -20°C bis etwa 100°C, wobei bei Verwendung von sehr reaktionsfähigen Aktivierungsmitteln die Reaktion auch bei tieferen Temperaturen durchgeführt werden kann.

### Verfahren i) (Austausch von freiem oder sulfonyliertem Hydroxy gegen Halogen):

Der Austausch der freien Hydroxygruppe R durch Halogen wird auf an sich bekannte Weise, beispielsweise durch Behandeln mit einem halogenierenden, insbesondere chlorierenden Mittel, durchgeführt. Geeignete Halogenierungsmittel zur Einführung von Chlor oder Brom sind beispielsweise Phosphor-Reagentien, wie Di-halogen-triorganyl-phosphorane, Trihalogendiorganyl-phosphorane oder ein Gemisch bestehend aus einem Triorganylphosphin und einem Tetrahalogenmethan. Repräsentative Vertreter der genannten Phosphorane sind Dichlor-triphenyl-, Trichlor-diphenyl oder Dibromtriphenylphosphoran. Repräsentative Vertreter der genannten Phosphine sind Triäthyl-, Tris-(dimethylamino)-und insbesondere Triphenylphosphin. Tetrahalogenmethane sind z.B. Tetrabrom- und insbesondere Tetrachlorkohlenstoff.

Die Reaktion mit den halogenierenden Phosphorreagentien findet auf an sich bekannte Weise in einem inerten, aprotischen, bevorzugt polaren Lösungsmittel, wie einem chlorierten Kohlenwasserstoff, z.B. Methylenchlorid, Chloroform, Tetrachlorkohlenstoff, 1,2-Dichloräthan, einem Nitril, wie Acetonitril oder Benzonitril, oder einem N,N-disubstituierten Carbonsäureamid, wie Dimethylformamid oder N,N-Dimethylacetamid, oder Mischungen davon, je nach Reaktivität des eingesetzten Reagenses unter Kühlen oder Erwärmen, d.h. bei Temperaturen zwischen etwa -60°C bis zur Rückflusstemperatur des verwendeten Lösungsmittel, gegebenenfalls in einer Inertgas-, wie Stickstoffatmosphäre, statt. Bei Verwendung von Triniederalkyl- oder Tris-(diniederalkylamin)-phosphinen und Tetrachlorkohlenstoff oder Tetrabromkohlenstoff ist gewöhnlich Kühlen notwendig, etwa auf -60°C bis -20°C. Beim Halogenieren mit den genannten Phosphoranen kann dem Reaktionsmedium zum Abfangen entstehenden Halogenwasserstoffs eine schwache Base, wie Pyridin oder ein Diniederalkylanilin, wie Dimethylanilin oder ein neutraler Protonenfänger, wie Propylen, zugesetzt werden.

Weitere wichtige Halogenierungsmittel sind entsprechende N,N-diniederalkylierte Halogen-iminiumhalogenid-Verbindungen, insbesondere solche der Formel [(CH₃)₂N=CH-Hal] ⊕Hal⊖, worin Hal Chlor bzw. Brom ist. Die obigen Reagentien werden üblicherweise in situ hergestellt, indem man ein geeignetes N,N-diniederalkyliertes Amid, in erster Linie Dimethylformamid, mit einem Chlorierungs- bzw. Bromierungsmittel, z.B. Phosgen, Carbonyldibromid, Oxalylchlorid, Oxalylbromid, Thionylbromid, Phosphoroxychlorid, Phosphoroxybromid, Phosphorpentachlorid oder:insbesondere mit Phosphortrichlorid, Phosphortribromid oder Thionylchlorid behandelt.

Die obige Reaktion wird üblicherweise in Gegenwart eines Lösungs- oder Verdünnungsmittels durchgeführt, wobei man neben dem normalerweise im Ueberschuss vorliegenden und als Lösungsmittel dienenden Amid, üblicherweise Dimethylformamid, ferner auch Dimethylacetamid, auch Aether z.B. Tetrahydrofuran oder Dioxan, Halogenkohlenwasserstoffe, z.B. Methylenchlorid, oder Sulfoxide, z.B. Dimethylsulfoxid, verwenden kann.

Die Chlorierungs- und Bromierungsmittel werden üblicherweise in Mengen verwendet, die zwei Aequivalenten des 3-Hydroxy-3-cephem-Ausgangsmaterials entsprechen. Die Reaktion kann man z.B. so durchführen, dass man das Chlorierungs- bzw. Bromierungsmittel unter Kühlen zu einer Lösung des 3-Hydroxy-3-cephem-Ausgangsmaterials in Dimethylformamid zugibt, worauf man das Reaktionsgemisch während einigen Stunden bei Raumtemperatur stehen lässt.

Die Chlorierung oder Bromierung kann auch so durchgeführt werden, dass man zunächst das Chlorierungs- bzw. Bromierungsmittel mit dem Amid, insbesondere dem Dimethylformamid, mischt und so das Halogeniminiumhalogenid bildet, worauf man die Lösung des 3-Hydroxy-3-cephem-Ausgangsmaterials im Amid, insbesondere in Dimethylformamid, dem noch ein zusätzliches Lösungsmittel zugegeben werden kann, oder in einem anderen Lösungsmittel, z.B. Tetrahydrofuran, zufügt.

Die Umwandlung der freien Hydroxygruppe R in Fluor kann z.B. durch Behandeln mit einem Reagens der Formel F₃S-Am erfolgen, worin Am eine disubstituierte Aminogruppe darstellt; solche Reagentien sind u.a. von Markovskij et al., Synthesis, Bd. 1973, S. 787 beschrieben worden. Die Gruppe Am steht in erster Linie für Diniederalkylamino, z.B. Dimethylamino, Diäthylamino oder Diisopropylamino, Niederalkylphenyl-amino, z.B. Methylanilino, Niederalkylenamino, z.B. Pyrrolidino oder Piperidino, Oxaniederalkylenamino, z.B. Morpholino, oder gegebenenfalls N-niederalkyliertes Azaniederalkylenamino, z.B. 4-Methylpiperazino.

Die Reaktion wird vorzugsweise in einem geeigneten inerten Lösungsmittel, wie einem gegebenenfalls chlorierten Kohlenwasserstoff, z.B. Cyclopentan, Cyclohexan, Benzol, Toluol, Xylol, Chlorbenzol, Dichlorbenzol, Pentan, Hexan, Chloroform, und vor allem Methylenchlorid oder einem Aether, wie Diäthyläther, Tetrahydrofuran oder vor allem Dioxan, falls notwendig, unter Kühlen oder Erwärmen, z.B. in einem Temperaturbereich von etwa -20°C bis etwa 80°C, vorzugsweise von etwa 0°C bis etwa 30°C und/oder unter einer Inertgasatmosphäre durchgeführt.

Eine sulfonylierte Hydroxygruppe R kann durch Behandeln mit einem anorganischen Fluorid in Gegenwart eines Kronenäthers in Fluor übergeführt werden. Eine sulfonylierte Hydroxygruppe R ist in erster Linie Niederalkylsulfonyloxy, insbesondere Methylsulfonyloxy, kann aber auch Arylsulfonyloxy sein, worin Aryl vorzugsweise gegebenenfalls, z.B. durch Niederalkyl, wie Methyl, Halogen, z.B. Brom, oder Nitro substituiertes Phenyl ist, z.B. 4-Methylphenylsulfonyloxy. Ein anorganisches Fluorid ist in erster Linie ein Metallfluorid, wobei man insbesondere ein Alkalimetallfluorid, z.B. Natriumfluorid, oder ein Schwermetallfluorid, z.B. Silberfluorid verwendet. Die zusammen mit dem anorganischen Fluorid verwendeten Kronenäther sind gegebenenfalls substituierte 18-Kronen-6-äther, wie der Dicyclohexyl-18-kronen-6- äther.

Die Reaktion wird in Gegenwart eines inerten Lösungsmittels, insbesondere eines Nitrils, z.B. Acetonitril oder Propionitril, oder eines Nitroniederalkans, z.B. Nitromethan oder Nitroäthan, unter im wesentlichen wasserfreien Bedingungen und, falls notwendig, unter Kühlen, z.B. in einem Temperaturbereich von etwa -20°C bis etwa 20°C, vorzugsweise bei etwa Raumtemperatur und gegebenenfalls in einer Inertgasatmosphäre durchgeführt. Entsprechende Verfahrensweisen sind beispielsweise in der DE-OS 2.636.962 beschrieben worden.

Verfahren j) (Verätherung der 3-Hydroxygruppe): Die Verätherung der freien Hydroxygruppe Ri wird mit einem den gewünschten Kohlenwasserstoff einführenden konventionellen Verätherungsmittel durchgeführt.

Geeignete Verätherungsmittel sind beispielsweise entsprechende Diazoverbindungen, wie gegebenenfalls substituierte Diazoniederalkane, z.B. Diazomethan, Diazo-n-butan oder Diphenyldiazomethan. Diese Reagentien werden in Gegenwart eines geeigneten inerten Lösungsmittels, wie eines niederaliphatischen, cycloaliphatischen oder aromatischen Kohlenwasserstoffs, wie Hexan, Cyclohexan, Benzol oder Toluol, eines halogenierten niederaliphatischen Kohlenwasserstoffs, z.B. Methylenchlorid, oder eines Aethers, wie eines Diniederalkyläthers, z.B. Diäthyläther, oder eines cyclischen Aethers, z.B. Tetrahydrofuran oder Dioxan, oder eines Lösungsmittelgemisches, und je nach Diazoreagens unter Kühlen, bei Raumtemperatur oder unter leichtem Erwärmen, ferner, wenn notwendig, in einem geschlossenen Gefäss und/oder unter einer Inertgas-, z.B. Stickstoffatmosphäre, zur Anwendung gebracht.

Weitere geeignete Verätherungsmittel sind Ester entsprechender Alkohole, in erster Linie solche mit starken anorganischen oder organischen Säuren, wie Mineralsäuren, z.B. Halogenwasserstoffsäuren, wie Chlorwasserstoff-, Bromwasserstoff- oder Jodwasserstoffsäure, ferner Schwefelsäure oder Halogen-schwefelsäuren, z.B. Fluorsulfonsäure, oder starken organischen Sulfonsäuren, wie gegebenenfalls, z.B. durch Halogen, wie Fluor, substituierten Niederalkansulfonsäuren, oder aromatisehen Sulfonsäuren, wie z.B.₋ gegebenenfalls, z.B. durch Niederalkyl, wie Methyl, Halogen, wie Brom, und/oder Nitro substituierten Benzolsulfonsäuren, z.B. Methansulfon-, Trifluormethansulfon- oder p-Toluolsulfonsäure, z.B. Diniederalkylsulfate, wie Dimethylsulfat, ferner Fluorsulfonsäureniederalkylester, z.B. Fluorsulfonsäuremethylester oder gegebenenfalls Halogen-substituierte Methansulfonsäure-niederalkylester, z.B. Trifluormethansulfonsäuremethylester. Sie werden üblicherweise in Gegenwart eines inerten Lösungsmittels, wie eines gegebenenfalls halogenierten, wie chlorierten aliphatischen, cycloaliphatischen oder aromatischen Kohlenwasserstoffs, z.B. Methylenchlorid, eines Aethers, wie Dioxan oder Tetrahydrofuran, oder eines Gemisches verwendet. Dabei wendet man vorzusgweise geeignete Kondensationsmittel, wie Alkalimetallcarbonate oder -hydrogencarbonate, _ z.B. Natrium- oder Kalimcarbonat oder -hydrogencarbonat (üblicherweise zusammen mit einem Sulfat), oder organische Basen, wie, üblicherweise sterische gehinderte, Triniederalkylamine, z.B. N,N-Diisopropyl-N-äthyl-amin (vorzugsweise zusammen mit Niederalkyl-halogensulfaten oder gegebenenfalls halogensubstituierten Methansulfonsäure-niederalkylestern) an, wobei unter Kuhlen, bei Raumtemperatur oder unter Erwärmen, z.B. bei Temperaturen von etwa -20°C bis etwa 50°C und, wenn notwendig, in einem geschlossenen Gefäss und/oder in einer Inertgas-, z.B. Stickstoffatmosphäre gearbeitet wird.

Durch Phasentransfer-Katalyse (s. E.V. Dehmlow, Angewandte Chemie 5, 187 (1974)) kann die letztgenannte Verätherungsreaktion wesentlich beschleunigt werden. Als Phasentransfer-Katalysatoren können quartäre Phosphoniumsalze und insbesondere quartäre Ammoniumsalze, wie gegebenenfalls substituierte Tetraalyklammoniumhalogenide, z.B. Tetrabutylammoniumchlorid, -bromid oder jodid, oder auch Benzyltriäthylammoniumchlorid in katalytischen oder bis zu äquimolaren Mengen verwendet werden. Als organische Phase kann irgendeines der mit Wasser nicht mischbaren Lösungsmittel dienen, beispielsweise einer der gegebenenfalls halogenierten, wie chlorierten niederaliphatischen, cycloaliphatischen oder aromatischen Kohlenwasserstoffe, wie Tri-oder Tetrachloräthylen, Tetrachloräthan, Tetrachlorkohlenstoff, Chlorbenzol oder auch Toluol oder Xylol. Die als Kondensationsmittel geeigneten Alkalimetallcarbonate oder -hydrogencarbonate, z.B. Kalium-oder Natriumcarbonate oder-hydrogencarbonat, Alkalimetallphosphate, z.B. Kaliumphosphat und Alkalimetallhydroxide, z.B. Natriumhydroxid, können bei basenempfindlichen Verbindungen der Reaktionsmischung titrimetrisch, z.B. mittels eines Titrierautomaten zugesetzt werden, damit der pH-Wert während der Verätherung zwischen etwa 7 und 8.5 bleibt.

Weitere Verätherungsmittel sind entsprechende Acetale, z.B. gem-Niederalkoxyniederalkane, wie 2,2-Dimethoxypropan, die in Gegenwart einer starken organischen Sulfonsäure, wie p-Toluolsulfonsäure, und eines geeigneten Lösungsmittels, wie eines Diniederalkyl- oder Niederalkylensulfoxyds, z.B. Dimethylsulfoxyd, zur Anwendung gelangen, oder Orthoester, z.B. Orthoameisensäure-triniederalkylester, z.B. Orthoameisensäure-triäthylester, die in Gegenwart einer starken organischen Sulfonaäure, wie p-Toluolsulfonsäure, und eines geeigneten Lösungsmittels, wie eines Aethers, z.B. Dioxan, verwendet werden. Wichtige Verätherungsmittel sind auch entsprechende Tri- 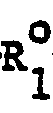 -oxonium- salze (sogenannte Meerweinsalze), worin 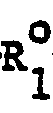 den einzuführenden Rest darstellt, beispielsweise Triniederalkyloxoniümsalze, insbesondere die entsprechenden Salze mit komplexen, fluorhaltigen Säuren, wie die entsprechenden Tetrafluorborate, Hexafluorphosphate, Hexafluorantimonate oder Hexachlorantimonate, beispielsweise Trimethyloxonium-tetrafluorborat. Man verwendet diese Verätherungsmittel vorzugsweise in einem inerten Lösungsmittel, wie einem Aether oder einem halogenierten niederaliphatischen Kohlenwasserstoff, z.B. Tetrahydrofuran oder Methylenchlorid, oder in einem Gemisch.davon, wenn notwendig, in Gegenwart einer Base, wie einer organischen Base, z.B. eines, vorzugsweise sterisch gehinderten, Triniederalkylamins, z.B. N,N-Diisopropyl-N-äthyl-amin, und unter Kühlen, bei Raumtemperatur oder unter leichtem Erwärmen, z.B. bei etwa -20°C bis etwa 50°C, wenn notwendig, in einem geschlossenen Gefäss und/oder in einer Inertgas-, z.B. Stickstoffatmosphäre.

Weitere Verätherungsmittel sind schliesslich entsprechende 1-R -3-Aryltriazenverbindungen, worin R⁰₁ den einzuführenden Rest und Aryl vorzugsweise einen gegebenenfalls substituierten Phenylrest, z.B. Niederalkylphenyl, wie 4-Methylphenyl bedeutet. Charakteristische Beispiele für solche Triazenverbindungen sind 3-Aryl-l-niederalkyltriazene, z.B. 3-(4-Methylphenyl)-l-äthyl-triazen. Diese Reagentien werden üblicherweise in Gegenwart von inerten Lösungsmitteln, wie gegebenenfalls halogenierten niederaliphatischen Kohlenwasserstoffen, Aether oder Gemischen davon, unter Kühlen, bei Raumtemperatur und vorzugsweise bei erhöhter Temperatur, z.B. bei etwa 20°C bis etwa 100°C, verwendet. Entsprechende Verfahrensweisen sind beispielsweise in der DE-OS 2.636.962 beschrieben worden.

Verfahren k) (Decarbonylierung): Die Decarbonylierung der Formylgruppe wird in erster Linie durch Behandeln mit einem Kohlenmonoxydaufnehmenden Schwermetallkomplex durchgeführt. Solche Schwermetallkomplexe sind insbesondere Platinmetall-Komplexe, wobei man unter einem Platinmetall ausser Platin auch Iridium, Rhodium, Palladium und Osmium versteht.

Vorzugsweise verwendet man bis-trisubstituierte Phosphinplatinhalogenide, bis-trisubstituierte Phosphincarbonyliridiumhalogenide tris-trisubstituierte Phosphin-iridiumhalogenide und in erster Linie tris-trisubstituierte Phosphin-rhodiumhalogenide, worin die Substituenten des Phosphins vorzugsweise Niederalkyl, z.B. n-Butyl, und in erster Linie Phenyl darstellen, und die Halogenide in erster Linie Chloride sind. Solche Posphinplatinmetall-Komplexe, welche Kohlenmonoxyd in kovalenter Bindung aufzunehmen vermögen, sind z.B. Bis-triphenylphosphin-platin-II-chlorid [(C₆H₅)₃PtCl₂ oder Bis-triphenyl₋pₕₒₛpₕi_{ncarbon}yₗiᵣi_{d}iᵤₘ-_{II}-chlorid, [(C₆H₅)₃P]₂ Ir(CO)Cl, sowie _{Tris-}triphenylphosphin₋iridium_{-I-}chlorid, [(C₆H₅)₃P]₃Ir _{C}l, in erster Linie aber Tris-triphenyl-phosphin-rhodium-I-chlorid, [(C₆H₅)₃P]₃RhCl.

Wenn erwünscht oder notwendig, kann die Decarbonylierung mit den obgenannten Schwermeₜallkomplexen in Gegenwart von geeigneten Katalysatoren oder Aktivierungsmitteln, z.B. Lewissäuren, wie Bortrifluorid (das man z.B. zusammen mit dem Bis-triphenylphosphin-platinchlorid verwenden kann), ferner eines Perchlorats, wie Alkalimetallperchlorates, z.B. Natriumperchlorat (das man z.B. zusammen mit Bistriphenylphoaphin-carbonyliridiumchlorid verwenden kann), durchgeführt werden.

Vorzugsweise arbeitet man in Gegenwart von inerten Lösungsmitteln, insbesondere von Kohlenwasserstoffen, wie aliphatischen oder cycloaliphatischen, insbesondere aromatischen Kohlenwasserstoffen, z.B. Benzol, Toluol oder Xylol, halogenierten, wie chlorierten Kohlenwasserstoffen, wie entsprechenden niederaliphatischen oder aromatischen Kohlenwasserstoffen, z.B. Methylenchlorid oder Chlorbenzol, Aethern, wie niederaliphatischen, niederaliphatisch-aromatischen, ferner cyclischen Aethern, z.B. Di-n-butyläther, Anisol oder Dioxan, Nitrilen, wie niederaliphatischen Nitrile, z.B. Acetonitril oder Ketonen, wie Niederalkanonen, z.B. Aceton, oder Gemischen von solchen Lösungsmitteln. Dabei wird die Reaktion unter Kühlen, bei Zimmertemperatur oder unter Erwärmen, z.B. bei etwa 10°C bis etwa 150°C, wie bei etwa 40^{*}C bis etwa 120°C, ferner, wenn notwendig, in einem geschlossenen Gefäss und/oder in einer inerten Gasatmosphäre, z.B. unter Stickstoff oder Argon, durchgeführt. Entsprechende Verfahrensweisen sind beispielsweise in der DE-OS. 2.151.567 beschrieben worden.

### Verfahren 1) (Austausch einer freien oder veresterten Hydroxy- oder gegebenenfalls substituierten Aminogruppe gegen Wasserstoff):

Eine gegen Wasserstoff austauschbare veresterte Hydroxygruppe R ist vorzugsweise eine durch eine starke organische oder anorganische Säure, wie eine starke Mineralsäure, z.B. einen Halogenwasserstoff, wie Chlorwasserstoff oder eine organischen Sulfonsäure, wie eine entsprechende niederaliphatische oder aromatische Sulfonsäure, veresterte Hydroxygruppe. So stellt 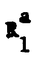 z.B. Halogen, wie Chler, Miederalkylsulfonyloxy, z.B. Methylsulfonyloxy oder Aethylsulfonyloxy oder Arylsulfonyloxy, z.B. p-Toluolsulfonyloxy dar. Ein gegebenenfalls niederalkylierter, durch Niederalkylen oder Heteroniederalkylen cyclisch disubstituierter Aminorest 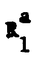 hat die oben für diese Reste angegebene Bedeutung und ist beispielsweise Amino, Methylamino, Dimethylamino, Diäthylamino, Pyrrolidino, Piperidino oder Morpholino.

Der Ersatz der Gruppe 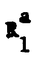 in einer Verbindung der Formel XIV durch Wasserstoff erfolgt entweder in zwei Stufen durch Reduktion der C₃-C₄-Doppelbindung und anschliessende Abspaltung von 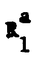 -H oder einstufig, durch unmittelbare hydrierende Abspaltung von 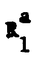 .

Beim zweistufigen Austausch von R gegen Wasserstoff erfolgt die Reduktion der C₃-C₄-Doppelbindung in Ausgangsstoffen der Formel XIV z.B. mittels geeigneter Hydrid-Reduktionsmittel. Geeignete Hydrid-Reduktionsmittel sind in erster Linie komplexe Metallhydride, vorzugsweise entsprechende Borhydride, wie Diboran oder Alkalimetallborhydride, z.B. Natriumborhydrid oder Lithiumborhydrid, ferner Zinkborhydrid, sowie organische Alkalimetallaluminiumhydride, wie Tri-Niederalkoxy-alkalialuminiumhydride, z.B. Tris-(tert.-butyloxy)-lithiumaluminiumhydrid, die man üblicherweise in Gegenwart von Lösungsmitteln, insbesondere von relativ polaren Lösungsmitteln, wie Alkoholen, z.B. Niederalkanolen, wie Methanol oder Aethanol, oder Aethern, wie aliphatischen Aethern, z.B. Glycol- und Polyglycol- äthern, wie Aethylenglycoldimethyläther oder Diäthylenglycoldimethyl- äther, oder cyclischen Aethern, wie Tetrahydrofuran oder Dioxan, oder Lösungsmittelgemischen, auch von wässrigen Lösungsmitteln, anwendet, wobei man bei Temperaturen von etwa -20° bis etwa 80°C, wenn notwendig, in einem geschlossenen Gefäss und/oder in einer Inertgas-, z.B. Stickstoffatmosphäre arbeitet. Falls diese Reduktion in Gegenwart geeigneter Wasser-, Säure- oder Amin-abspaltender Mittel durchführt wird, kann die erwünschte 3-unsubstituierte 3-Cephem-Verbidnung unmittelbar erhalten werden. Es kann aber auch zunächst eine 3- 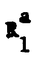 -Cepham-Verbindung entstehen, die entweder isoliert oder in situ weiterverarbeitet werden kann. Diese 3- 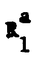 -Cepham-Ausgangsverbindungen lassen sich auch auf anderem Wege, z.B. durch Acylierung einer entsprechenden 7β-Amino-3-R^{a}₁-cepham-Verbindung, erhalten.

In der zweiten Verfahrensstufe erfolgt die Abspaltung der Elemente einer Verbindung der Formel 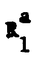 -H, d.h. einer Säure, eines Amins, oder insbesondere von Wasser, aus einer erhaltenen 3-R -Cepham-Verbindung, wobei man diese vorzugsweise mit geeigneten Wasser-, Säure-oder Amin-abspaltenden Mitteln behandelt. Wasser und Amine werden vorzugsweise mittels einer starken organischen Carbon-, wie einer Halogen-niederalkancarbonsiure oder Sulfonsäure, z.B. p-Toluolsulfonsäure oder Methansulfonsäure, abgespalten. Wasser kann insbesondere auch mittels eines geeigneten wasserbindenden Säurederivats, wie eines Anhydrids oder insbesondere Säurehalogenids, wie Säurechlorids, einer vom Phosphor oder Schwefel abgeleiteten Säure, wie einer entsprechenden anorganischen Säure, z.B. mittels Phosphoroxychlorid oder Thionylchlorid oder einer Sulfonsäure, wie einer niederaliphatischen oder aromatischen Sulfonsäure, z.B. mittels Tosylchlorid oder Mesylchlorid, abgespalten werden. Man arbeitet dabei vorzugsweise in Gegenwart einer Base, wie eines tertiären, z.B. Triniederalkylamins oder heterocyclischen Amins, z.B. von Triäthylamin oder Pyridin, oder eines geeigneten Ionenaustauschers, z.B. eines mit Kationen beladenen sulfonierten Polystyrol-Ionenaustauschers.

Feranr kann man zur Wasserabspaltung auch dehydratisierende Carbodiimidverbindungen, z.B. Dicyclohexylcarbodiimid, oder dehydratisierende, über Stickstoffatome disubstituierte Carbonylverbindungen, z.B. Carbonyldiimidazol, verwenden. Dabei verwendet man diese Mittel üblicherweise in Gegenwart eines Lösungsmittels, wie eines gegebenenfalls halogenierten niederaliphatischen oder aromatischen Kohlenwasserstoffs, z.B. Benzol oder Toluol, oder eines Lösungsmittelgemisches, wobei man geeignete saure Mittel, wie Trifluoressigsäure, gleichzeitig auch als Lösungsmittel verwenden kann. Wenn notwendig, verwendet man zusätzlich ein wasser-adsorbierendes Mittel oder einen Wasserabscheider und arbeitet unter Kühlen oder Erwärmen und/oder in einer Inertgas-, z.B. Stickstoffatmosphäre.

Die einstufige hydrierende Abspaltung einer Gruppe 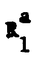 aus einer Verbindung der Formel XIV gelingt am besten, wenn R Halogen, wie Chlor oder eine veresterte Hydroxygruppe, wie eine durch eine Sulfonsäure, wie eine niederaliphatische oder aromatische Sulfonsäure, z.B. Methansulfonsäure oder Toluolsulfonsäure, veresterte Hydroxygruppe ist. In diesem Falle hydriert man vorzugsweise mit Hilfe von metallischen Reduktionsmitteln ("naszierendem Wasserstoff"), z.B. von Aluminium oder insbesondere von Zink, oder von reduzierenden Metallverbindungen, z.B. entsprechenden Metallegierungen, Amalgamen oder Salzen, z.B. Aluminiumamalgam, die man üblicherweise in Gegenwart von schwachen Protonendonatoren, wie Wasser, Alkohol (z.B. Methanol oder Aethanol) oder einer niederaliphatischen Carbonsäure, z.B. Essigsäure oder Ameisensäure anwendet. Zink wendet man beispielsweise in Gegenwart von Eisessig oder Ameisensäure, ein Amalgam z.B. in Gegenwart eines wasserhaltigen inerten organischen Lösungsmittels, wie eines Aethers, an.

Gewisse veresterte Hydroxygruppen 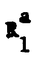 , insbesondere Sulfonyloxy, z.B. Methylsulfonyloxygruppen, lassen sich in Form einer Säure der Formel 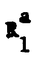 -H auch durch Adsorption, z.B. an Silikagel, Aluminiumoxyd, etc., und Elution (Chromatographie), abspalten.

Bevorzugt wird in einer Verbindung der Formel XIV eine veresterte Hydroxygruppe Ri, wie eine 3-Halogen-, wie 3-Chlor- oder 3-Brom-, eine 3-Toluolsulfonyloxy- oder eine 3-Methansulfonyloxygruppe durch Behandlung mit Zink in Eisessig oder Ameisensäure und eine tertiäre Aminogruppe Ra, wie 3-Morpholino oder 3-Pyrrolidino, durch Behandlung mit Diboran und anschliessend mit Eisessig, durch Wasserstoff ersetzt. Entsprechende Verfahrensweisen sind z.B. in der DE-OS. 2.620.308 beschrieben.

Die Abspaltung von 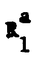 -H wird üblicherweise in Gegenwart eines Lösungsmittels durchgeführt. Bei Anwendung von anorganischen Basen kann man dabei in Wasser, einem wasserlöslichen organischen Lösungsmittel, wie Aceton oder Dioxan oder wässrigen Gemischen davon und bei einem pH von höchstens 9, bei Anwendung von Aminen vorzugsweise auch in einem gegebenenfalls halogenierten, wie chlorierten, niederaliphatischen, cycloaliphatischen oder aromatischen Kohlenwasserstoff, wie Methylenchlorid, einem Niederalkanon, z.B. Aceton, oder Aether, z.B. Tetrahydrofuran oder Dioxan, oder einem entsprechenden Lösungsmittelgemisch inkl. einem wässrigen Gemisch, wenn notwendig unter Kühlen oder Erwärmen und/oder in einer Inertgas-, z.B. Stickstoffatmosphäre, : arbeiten.

In einem erhaltenen Zwischenprodukt der Formel I, worin mindestens eine der funktionellen Gruppen in geschützter Form vorliegt, können auf übliche, an sich bekannte Weise, noch nicht geschützte funktionelle Gruppen geschützt öder vorhandene Schutzgruppen gegen andere Schutzgruppen ausgetauscht werden, z.B. durch Abspalten der vorhandenen Schtuzgruppe und Einführen der gewünschten anderen Schutzgruppe.

In einem erhaltenen Zwischenprodukt der Formel I, worin mindestens eine der funktionellen Gruppen in geschützter Form vorliegt, kann eine im Rest R₁ vorhandene Gruppe R₂ gegen eine andere Gruppe R₂ ausgetauscht werden, wie für diese Reaktion bei den Endprodukten der Formel I angegeben ist.

Einführung von 7α-Methoxy: In einem erhaltenen Zwischenprodukt der Formel I, worin R₃ Wasserstoff ist und alle funktionellen Gruppen in geschützter Form vorliegen, kann die 7a-Methoxygruppe R₃ auf an sich bekannte Weise eingeführt werden, beispielsweise indem man das genannte Zwischenprodukt nacheinander mit einem Anionen-bildenden Mittel, einem N-Halogenierungsmittel und Methanol behandelt.

Ein geeignetes Anion-bildendes Mittel ist in erster Linie eine metallorganische Base, insbesondere eine Alkalimetall-, in erster Linie eine Lithium-organische Base. Solche Verbindungen sind insbesondere entsprechende Alkoholate, wie geeignete Lithium-niederalkanolate, in erster Linie Lithiummethylat, oder entsprechende Metall-Kohlenwasserstoffbasen, wie Lithium-niederalkane und Lithiumphenyl. Die Umsetzung mit der Anion-bildenden metallorganischen Base wird üblicherweise unter Kühlen, z.B. von etwa 0°C bis etwa -80°C, und in Gegenwart eines geeigneten Lösungs- oder Verdünnungsmittel, z.B. eines Aethers, wie Tetrahydrofuran, bei Verwendung von Lithiummethylat auch in Gegenwart von Methanol, und, wenn erwünscht, in einem ge-. _ schlossenen Gefäss und/oder in einer Inertgas-, z.B. Stickstoffatmosphäre, vorgenommen.

Als N-halogenierende Mittel verwendet man üblicherweise ein sterisch gehindertes, organisches Hypohalogenit, insbesondere -chlorit, und in erster Linie ein entsprechendes aliphatisches Hypohalogenit, z.B. -chlorit, wie ein tert.-Niederalkyl-hypohalogenit, z.B. -chlorit. In erster Linie wendet man das tert.-Butylhypochlorit an, das man mit dem nichtisolierten Produkt der Anionisierungsreaktion umsetzt.

Die N-halogenierte Zwischenverbindung wird bei Anwesenheit eines Ueberschusses der Anion-bildenden Base, insbesondere von Lithiummethylat, unter den Reaktionsbedingungen und ohne isoliert zu werden in eine 7-Acyliminocephemverbindung umgewandelt, und diese durch Zugabe von Methanol in eine 7α-Methoxy-cephem-Verbindung übergeführt. Falls notwendig, müssen aus dem N-halogenierten Zwischenprodukt die Elemente der Halogenwasserstoff-, insbesondere der Chlorwasserstoffsäure, abgespalten werden; dies geschieht unter Zugabe einer Halogenwassertoff-abspaltenden Base, wie eines geeigneten Alkalimetall-niederalkanolats, z.B. Lithium-tert.-butylat, wobei diese Reaktion üblicherweise unter den Bedingungen der Anion- und N-Halogenverbindung-bildenden Reaktion stattfindet, wobei man in Gegenwart von Methanol arbeiten und anstelle der Acyliminoverbindung direkt die 7a-Methoxy-cephem-Verbindung erhalten kann. Man geht üblicherweise aus von einer Verbindung der Formel I, worin funktionelle Gruppen in geschützter Form vorliegen, setzt diese mit einem Ueberschuss des Anion-bildenden Mittels, z.B. Lithiummethylat oder Phenyllithium, in Gegenwart von Methanol um, behandelt dann mit dem N-Halogenierungsmittel, z.B. tert.-Butylhypochlorit, und erhält so direkt die gewünschte Verbindung der Formel I, worin funktionelle Gruppen geschützt sind. Man kann auch das Methanol nachträglich zugeben, wobei man die Dehydrohalogenierung und die Zugabe von Methanol bei etwas höheren Temperaturen als die Anion- und N-Halogenverbindung-bildenden Reaktionen, z.B. bei etwa 0°C bis etwa -20^{*}C, wenn notwendig, in einem geschlossenen Gefäss und/oder in einer Inertgas-, z.B. Stickstoffatmosphäre, durchführen kann.

Bei vorstehenden Reaktionen, die unter basischen Bedingungen durchgeführt werden, können 3-Cephemverbindungen, gegebenenfalls teilweise, zu 2-Caphemverbindungen isomerisieren. Eine erhaltene 2-Caphemverbindung oder ein Gemisch aus einer 2- und einer 3-Cephem- verbindung kann in an sich bekannter Weise zur gewünschten 3-Cephem- verbindung isomerisiert werden.

Salzbildung: Salze von Verbindungen der Formel I mit geschützten und mit salzbildenden Gruppen können in an sich bekannter Weise hergestellt werden, z.B. wie für die Salzbildung von Verbindungen der Formel I angegeben.

Salze können in üblicher Weise in die freien Verbinduagen übergeführt werden, Metall- und Ammoniumsalze z.B. durch Behandeln mit geeigneten Säuren, und Säureadditionssalze z.B. durch Behandeln mit einem geeigneten basischen Mittel.

Gemische von Isomeren können in an sich bekannter Weise, z.B. durch fraktionierte Kristallisation, Chromatographie, etc. in die einzelnen Isomeren aufgetrennt werden.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird; ferner können Ausgangsstoffe in Form von Derivaten verwendet oder während der Reaktion gebildet werden.

Vorzugsweise werden solche Ausgangsstoffe verwendet und die Reaktionsbedingungen so gewählt, dass man zu den vorstehend als besonders bevorzugt aufgeführten Verbindungen gelangt.

Ausgangsstoffe: Die im Verfahren zur Herstellung der Verbindungen der vorliegenden Erfindung verwendeten Ausgangsstoffe sind bekannt oder, falls neu, können in an sich bekannter Weise hergestellt werden.

Die Ausgangsverbindungen vom Typ der Formel II, sowie entsprechende Verbindungen mit geschützten funktionellen Gruppen sind bekannt oder können auf an sich bekannte Weise hergestellt werden.

Verbindungen der Formel III, worin die Aminocarbonsäuregruppierung HOOC-CH(NH₂)- und gegebenenfalls in der Gruppierung A und R vorhandene weitere funktionelle Gruppen in geschützter Form vorliegen, und ihre reaktionsfähigen funktionellen Säurederivate sind neu und bilden zusammen mit den Verfahren zu ihrer Herstellung ebenfalls einen Gegenstand der vorliegenden Erfindung.

Die Erfindung betrifft demnach auch Verfahren zur Herstellung von Verbindungen der Formel 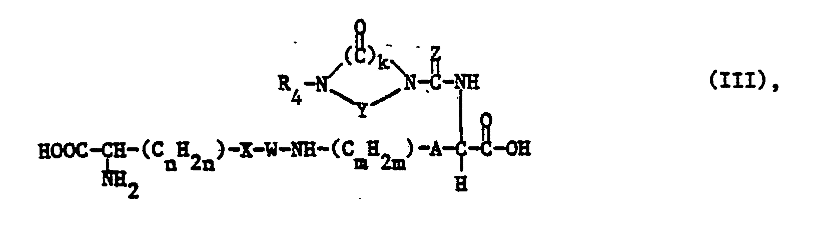 worin die Aminocarbonsäuregruppierung HOOC-CH(NH₂)- und gegebenenfalls in den Gruppierungen A und R₄ vorhandene weitere funktionelle Gruppen in geschützter Form vorliegen, und ihrer reaktionsfähigen funktionellen Säurederivate, dadurch gekennzeichnet, dass man

### a') in einer Verbindung der Formel

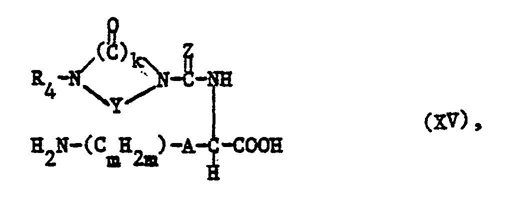

worin die Aminogruppe gegebenenfalls durch eine, die Acylierung erlaubende Gruppe substituiert sein kann, und worin die Carboxylgruppe und gegebenenfalls in den Gruppierungen A und R₄ vorhandene weitere funktionelle Gruppen in geschützter Form vorliegen können, die Aminogruppe durch Behandeln mit einem reaktionsfähigen funktionellen Derivat einer Säure der Formel 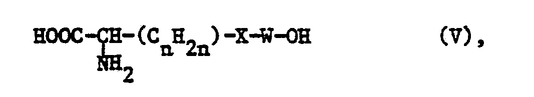 worin die Aminocarbonsäuregruppierung HOOC-CH(NH₂)- in geschützter Form vorliegt, oder wenn X-W zusammen eine Gruppe -CO- bedeuten, auch mit einer entsprechenden freien Säure, oder mit einem Salz davon, acyliert, oder

### b') in einer Verbindung der Formel

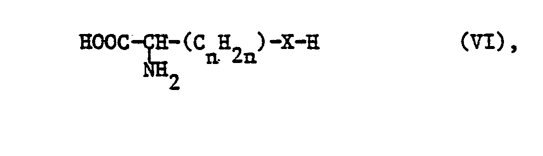

worin X Sauerstoff, Schwefel oder die Gruppe -NH- bedeutet, und worin die Aminocarbonsäuregruppierung HOOC-CH(NH₂)- in geschützter Form vorliegt, die Gruppe -X-H mit einem reaktionsfähigen funktionellen Derivat einer Verbindung der Formel 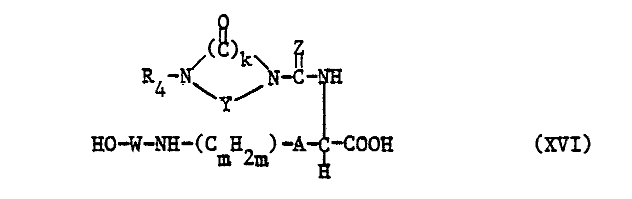 worin die Carboxylgruppe und gegebenenfalls in den Gruppierungen A und R₄ vorhandene funktionelle Gruppen in geschützter Form vorliegen können, acyliert, oder

### c') in einer Verbindung der Formel

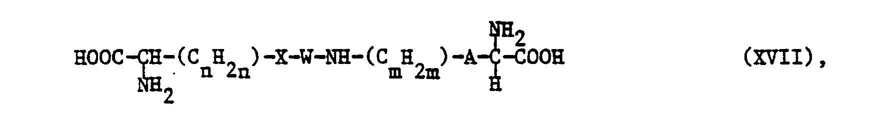

worin die Aminocarbonsäuregruppierung HOOC-CH(NH₂)- in geschützter Form vorliegt, die zu acylierende Aminogruppe gegebenenfalls durch eine, die Acylierung erlaubende Gruppe substituiert ist, und worin die Carboxylgruppe und gegebenenfalls in der Gruppierung A vorhandene weitere funktionelle Gruppen in geschützter Form vorliegen können, die Aminogruppe durch Behandeln mit einem den entsprechenden Acylrest einer Kohlen- oder Thiokohlensäure der Formel 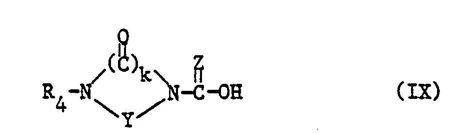 einführenden Acylierungsmittel, worin im Rest R₄ gegebenenfalls vorhandene funktionelle Gruppen in geschützter Form vorliegen können, acyliert, oder

### d') ein reaktionsfähiges Derivat einer Kohlensäure- oder Thiokohlensäureverbindung der Formel

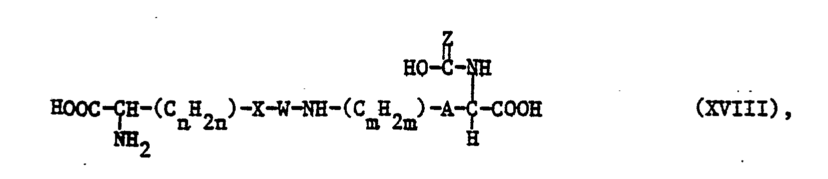

worin die Aminocarbonsäuregruppierung HOOC-CH(NH₂)- in geschützter Form vorliegt; und worin die Carboxylgruppe und gegebenenfalls in der Gruppierung A vorhanden weitere funktionelle Gruppen in geschützter Form vorliegen können, mit einem sekundären Amid der Formel 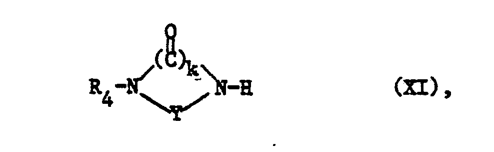 worin im Rest R₄ gegebenenfalls vorhandene funktionelle Gruppen geschützt sein können und die sekundäre Amidogruppe in einer die Acylierung erlaubenden Form vorliegt, reagieren lässt, oder

### e') die unter c') oder d') genannte Acylierung in situ vornimmt, indem man eine Aminoverbindung der Formel XVII und eine Amidoverbindung der Formel XI, beide mit der oben angegebenen Bedeutung, gleichzeitig oder nacheinander auf ein bivalentes reaktives Derivat der Kohlen-oder Thiokohlensäure einwirken lässt, oder indem man

### f') eine Verbindung der Formel

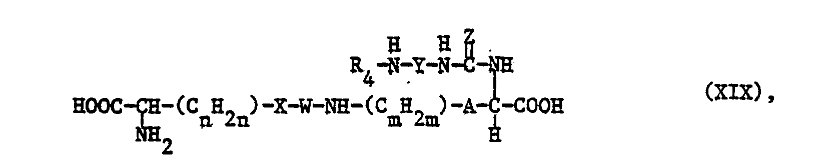

worin die Aminocarbonsäuregruppierung HOOC-CH(NH₂)- in geschützter Form vorliegt, und worin die Carboxylgruppe und gegebenenfalls in der Gruppierung A vorhandene weitere funktionelle Gruppen in geschützter Form vorliegen können, und die später den Piperazinring bildende sekundären Amino- bzw. Amidogruppen gegebenenfalls durch Gruppen substituiert sind, die die N-Acylierung erlauben, mit einem bivalenten acylierenden Derivat der Kohlensäure oder der Oxalsäure unter Ringschluss diacyliert, und gewünschtenfalls in einer erhaltenen Verbindung der Formel III die geschützte Carboxylgruppe in eine freie Carboxylgruppe oder in ein reaktionsfähiges funktionelles Derivat davon überführt.

Die die Acylierung erlaubenden Gruppen, sowie die Schutzgruppen der Aminocarbonsäuregruppierung HOOC-CH(NH₂)- und in den Gruppierungen A und R₄, sind die gleichen wie die unter den Verbindungen der Formeln IV, sowie I erwähnten. Zum intermediären Schutz der Carboxylgruppe in den Verbindungen der Formeln XV, XVI, XVII oder XVIII können an sich ebenfalls die bereits erwähnten Carboxylschutzgruppen verwendet werden, jedoch müssen sich die zum intermediären Schutz in der vorliegenden Acylierung benützten Carboxylschutzgruppen von den übrigen Schutzgruppen, die in den Verbindungen der Formel III notwendigerweise vorhanden bleiben sollen, in der Art ihrer Abspaltung unterscheiden, so dass sie nach den vorliegenden Acylierungsreaktionen selektiv abgespalten werden können. Wenn beispielsweise zum intermediären Schutz der Carboxylgruppe eine hydrogenolytisch abspaltbare Schutzgruppe, wie eine der genannten gegebenenfalls substituierten Benzylgruppen, z.B. die Benzyl- oder p⁼Nitrobenzylgruppe, verwendet wird, dann dürfen die anderen Schutzgruppen hydrogenolytisch nicht spaltbar sein; sie können beispielsweise die genannten, nur acidolytisch abspaltbaren tert.-Niederalkylgruppen, wie tert.Butyl bzw. tert.-Niederalkoxycarbonylgruppen, wie tert.-Butoxycarbonyl, sein.

Die Verfahren a') bis f') werden im übrigen analog ausgeführt wie die Verfahren b) bis g).

In einer erhaltenen Verbindung der Formel III, mit entsprechend geschützten funktionellen Gruppen, kann eine Schutzgruppe, gegebenenfalls selektiv, abgespalten, oder eine, gegebenenfalls bei der Acylierungsreaktion frei gewordene, funktionelle Gruppe geschützt werden.

Reaktionsfähige funktionelle Derivate von Verbindungen der Formel III bezüglich der freien Carboxylgruppe, die im Verfahren a) die 7ß-Amidbildung ermöglichen und worin alle anderen funktionellen Gruppen geschützt sind, d.h. die genannten Anhydride, gemischten Anhydride oder aktivierten Ester, werden auf an sich bekannte Weise hergestellt. Beispielsweise wird ein gemischtes Anhydrid erhalten, indem man eine Verbindung der Formel III, mit geschützten funktionellen Gruppen ausser der zu reagierenden Carboxylgruppe, oder ein geeignetes Salz, z.B. ein Metall-, wie Alkalimetallsalz, oder insbesondere ein Ammoniumsalz, z.B. mit Ammoniak oder einem organischen Amin, wie Triniederalkylamin, z.B. Triäthylamin oder 3-Dimethylaminopropanol, oder einem cyclischen Amin, wie 4-Methylmorpholin, einer solchen Säure der Formel III mit einem geeigneten Halogenierungsmittel, z.B. mit Phosphorpentachlorid, Thionylchlorid oder Oxalylchlorid, oder mit einem Halogenid einer gegebenenfalls substituierten Niederalkancarbonsäure, z.B. Trichloracetylchlorid, oder mit einem Halbester eines Kohltmisäurahalbhaloganids, z.B. Chlorameisensäureäthylester- oder -isobutylester, umsetzt. Die aktivierten Ester werden beispielsweise erhalten, indem man eines der genannten Salze der Säure der Formel III mit einem reaktionsfähigen Derivat eines entsprechenden Alkohols, beispielsweise mit einem Halogenid, wie Chlorid, Bromid oder Jodid, oder mit einem Sulfonylester, z.B. Methansulfonyl- oder p-Toluolsulfonylester, umsetzt, oder indem man die freie Säure mit dem entsprechenden Alkohol in Gegenwart eines geeigneten Kondensationsmittels, wie einer der bei den Acylierungen a), b) oder g) genannten Carbodiimid-oder Carbonylverbindungen, umsetzt. Die Lösungsmittel und Reaktionsbedingungen sind die gleichen, wie für die Acylierungsreaktion angegeben ist.

Verbindungen der Formel IV, worin die Aminogruppe gegebenenfalls durch eine die Acylierung erlaubende Gruppe substituiert sein kann, und worin die 4-Carboxylgruppe und gegebenenfalls im Rest R₁ und in den Gruppierungen A und R₄ vorhandene weitere funktionelle Gruppen in geschützter Form vorliegen können, sind neu. Diese Verbindungen und die Verfahren zu ihrer Herstellung sind ebenfalls Gegenstand der vorliegenden Erfindung.

In den neuen Verbindungen der Formel IV haben die Indices m und k, A, R₁, R₃, Z und R₄ die unter der Formel I genannten Bedeutungen und die bevorzugten Bedeutungen der genannten Symbole entsprechen ebenfalls den unter der Formel I genannten Bevorzugungen. Diese neuen Verbindungen, worin die Carboxylgruppe gegebenenfalls in physio- logisch spaltbarer Form verestert ist, sowie ihre pharmazeutisch verwendbaren Salze, haben ebenfalls antibiotische Wirksamkeit.

Die genannten neuen, antibiotisch wirksamen Verbindungen der Formel IV können als antibakterielle Antibiotika verwendet werden. Beispielsweise sind sie in vitro gegen grampositive und gramnegative Mikroorganismen, wie gegen grampositive und gramnegative Kokken, z.B. Staphylococcus aureus, Streptococcus spp. und Neisseria, in Minimaldosen von etwa 0,01 bis 32 pg/ml, gegen gramnegative Bakterien, wie Enterobakterien, z.B. Escherichia coli, Proteus, Salmonella oder Klebsiella, oder gegen Pseudomonas aeruginosa oder Haemophilus influenzae, in Minimaldosen von etwa 0,05 bis 64 pg/ml wirksam. In vivo, bei subkutaner Applikation an der Maus, sind sie beispielsweise bei grampositiven Infektionen, z.B. durch Staphylococcus aureus, im Dosisbereich von etwa 6 bis 25 mg/Kg, bei gramnegativen Infektionen, z.B. durch Escherichia coli, Proteus spp. oder Pseudomonas aeruginosa, im Dosisbereich von etwa 4 bis 100 mg/Kg wirksam.

Die neuen Verbindungen der Formel IV können deshalb entsprechend, z.B. in Form von antibiotisch wirksamen Präparaten zur Behandlung von durch grampositive oder insbesondere von durch gramnegative Bakterien und Kokken, insbesondere durch von Pseudomonas aeruginosa, verursachten systemischen Infektionen Verwendung finden, wobei die minimale ED₅₀ bei subkutaner Injektion bei etwa 40 mg/Kg liegt.

Verbindungen der Formel IV, worin gegebenenfalls die Aminogruppe und die anderen funktionellen Gruppen wie angegeben substituiert bzw. geschützt sein können, sowie ihre Salze, können hergestellt werden, indem man

### a" in einer Verbindung der Formel

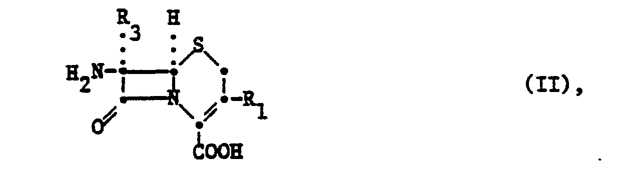

worin die Aminogruppe gegebenenfalls durch eine die Acylierung erlaubende Gruppe substituiert ist, und worin die 4-Carboxylgruppe und gegebenenfalls im Rest R₁ vorhandene weitere funktionalle Gruppen in geschützter Form vorliegen können, die Aminogruppe durch Behandeln mit einer Säure der Formel 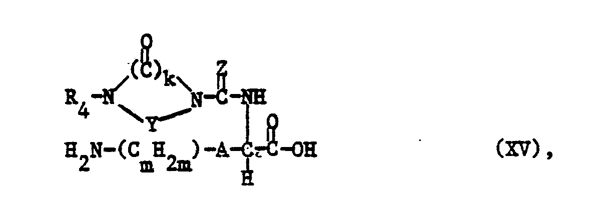 worin die Aminogruppe und gegebenenfalls in den Gruppierungen A und R₄ vorhandene weitere funktionelle Gruppen in geschützter Form vorliegen, oder mit einem reaktionsfähigen funktionellen Säurederivat oder einem Salz davon acyliert, oder

### b") in einer Verbindung der Formel

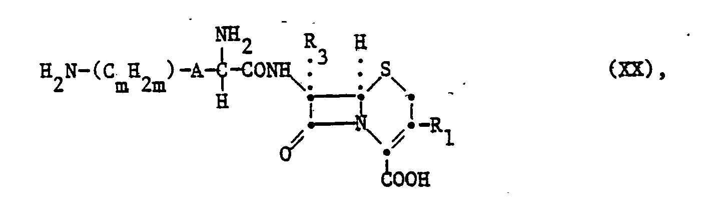

worin die endständige Aminogruppe in geschützter From vorliegt, die zu acylierende Aminogruppe gegebenenfalls durch eine die Acylierung erlaubende Gruppe substituiert ist, und worin die 4-Carboxylgruppe und gegebenenfalls im Rest R₁ und in der Gruppierung A vorhandende weitere funktionelle Gruppen in geschützter Form vorliegen können, die a-Aminogruppe durch Behandeln mit einem den entsprechenden Acylrest einer Kohlen- oder Thiokohlensäure der Formel - 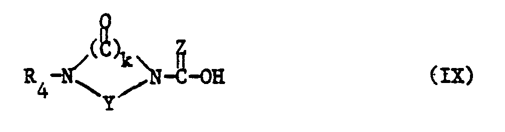 einführenden Acylierungsmittel, worin im Rest R₄ gegebenenfalls vorhandene funktionelle Gruppen in geschützter Form vorliegen können, acyliert, oder

### c") ein reaktionsfähiges Derivat einer Kohlensäure- oder Thiokohlensäureverbindung der Formel

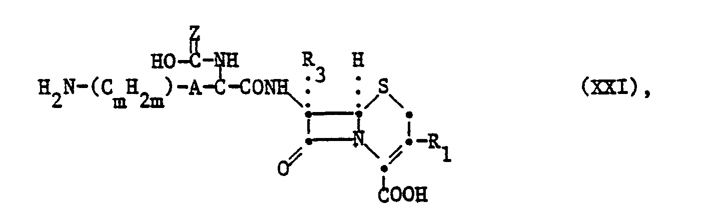

worin die endständige Aminogruppe in geschützter Form vorliegt, und worin die 4-Carboxylgruppe und gegebenenfalls im Rest R₁ und in der Gruppierung A vorhandene weitere funktionelle Gruppen in geschützter Form vorliegen können, mit einem sekundären Amid der Formel 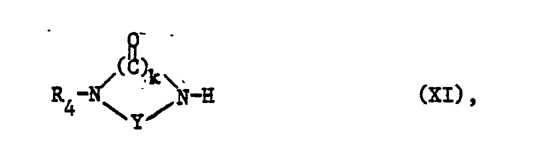 worin im Rest R₄ gegebenenfalls vorhandene funktionelle Gruppen geschützt sein können und die sekundäre Amidogruppe in einer die Acylierung erlaubenden Form vorliegt, reagieren lässt, oder

### d") die unter b") oder c") genannte Acylierung in situ vornimmt, indem man eine Aminoverbindung der Formel XX und eine Amidoverbindung der Formel XI, beide mit der oben angegebenen Bedeutung, gleichzeitig oder nacheinander auf ein bivalentes reaktives Derivat der Kohlen-oder Thiokohlensäure einwirken lässt, oder indem man -

### e") eine Verbindung der Formel

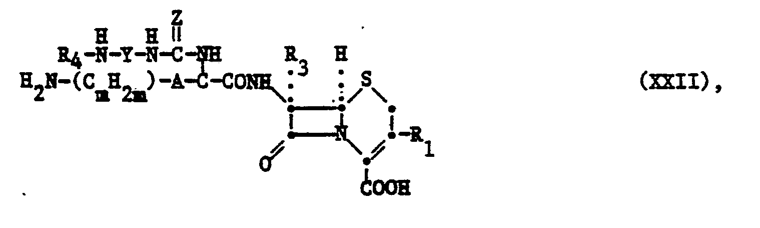

worin die endständige Aminogruppe in geschützter Form vorliegt, und worin die 4-Carboxylgruppe und gegebenenfalls im Rest R₁ und in der Gruppierung A vorhandene weitere funktionelle Gruppen in geschützter Form vorliegen können, und die später den Diazaring bildende sekundäre Amino- bzw. Amidogruppe gegebenenfalls durch Gruppen substituiert sind, die die N-Acylierung erlauben, mit einem bivalenten acylierenden Derivat der Kohlensäure oder der Oxalsäure unter Ringschluss diacyliert, oder

### f") eine 2-Cephemverbindung der Formel

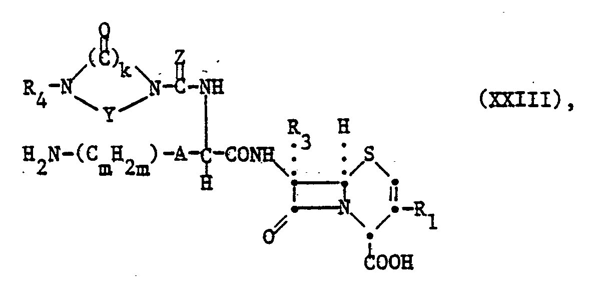

worin funktionelle Gruppen geschützt sind, zur entsprechenden 3-Cephemverbindung der Formel IV isomerisiert, oder

### g") in einer Verbindung der Formel

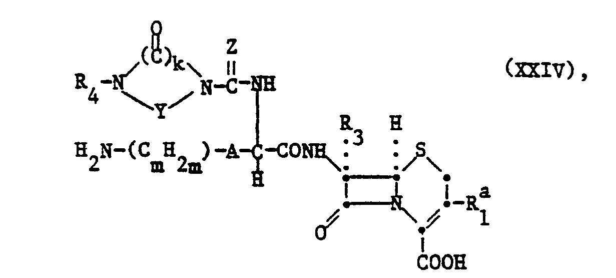

worin 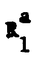 eine freie oder sulfonylierte Hydroxygruppe ist und worin andere funktionelle Gruppen geschützt sind, 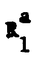 gegen Halogen austauscht, oder

h") in einer Verbindung der Formel XXIV, worin eine freie Hydroxygruppe ist und worin andere funktionelle Gruppen geschützt sind, die Hydroxygruppe veräthert, oder

i") in einer Verbindung der Formel XXIV, worin funktionelle Gruppen geschützt sind, und worin eine Formylgruppe bedeutet, diese in Gegenwart von Decarbonylierungs-Katalysatoren zu einer Verbindung der Formel I, worin R₁ Wasserstoff bedeutet, decarbonyliert, oder

j") in einer Verbindung der Formel XXIV, worin freies oder verestertes Hydroxy oder ein gegebenenfalls niederalkyliertes oder durch

Niederalkylen oder Heteroniederalkylen cyclisch disubstituiertes Amino ist, und worin funktionelle Gruppen geschützt sind, die Gruppe R a durch Wasserstoff ersetzt,
und, wenn erwünscht, in einer erhaltenen Verbindung die Schutzgruppen abspaltet oder noch nicht geschützte funktionelle Gruppen schützt oder eine Schutzgruppe gegen eine andere austauscht, und/oder, wenn gewünscht, in einem Rest R eine Gruppe R₂ gegen eine andere Gruppe R₂ austauscht, und/oder, wenn erwünscht, eine erhaltene Verbindung, worin R₃ Wasserstoff ist, in eine Verbindung überführt, worin R₃ Methoxy ist, und/oder, wenn gewünscht, ein erhaltenes Isomerengemisch in die einzelnen Isomeren auftrennt, und/oder, wenn gewünscht eine erhaltene Verbindung in ein.Salz oder ein erhaltenes Salz in die freie Verbindung oder in ein anderes Salz überführt,

Die in den Ausgangsstoffen der Formeln II, XV, und XX bis XXIV vorhandenen funktionellen Gruppen können durch die bereits unter den Verbindungen der Formel I genannten Schutzgruppen geschützt sein. Bevorzugt sind alle nicht an den Umsetzungsreaktionen teilnehmenden reaktionsfähigen funktionellen Gruppen, insbesondere aber gegebenenfalls vorhandene Carboxyl-, Sulfo-, Amino-, Hydroxy- und Mercaptogruppen, geschützt.

Die in den Verfahren a") bis j") genannten Verfahren, sowie die genannten Nachoperationen können auf analoge Weise durchgeführt werden, wie die Verfahren a) und d) bis 1), bzw. wie die dort angegebenen Nachoperationen. Die Entfernung von Schutzgruppen aus geschützten Verbindungen der Formel IV kann auf die gleiche Weise erfolgen, wie für die Entfernung gleicher Schutzgruppen aus geschützten Verbindungen der Formel I angegeben ist.

Säuren der Formel V, reaktionsfähige funktionelle Derivate davon, die Vorstufen der Formel HOOC-CH(NH₂)-(CₙH₂ₙ) -X-H (VI) und entsprechend geschützte Derivate sind bekannt oder können nach an sich bekannten Methoden, beispielsweise in situ, hergestellt werden.

Reaktionsfähige funktionelle Derivate von Säuren der Formel VII, worin die 4-Carboxylgruppe und gegebenenfalls im Rest R1 und in den Gruppierungen A und R₄ vorhandene funktionelle Gruppen in geschützter Form vorliegen können, werden auf an sich bekannte Weise aus entsprechend geschützten Verbindungen der Formel IV hergestellt.

Verbindungen der Formeln VIII bis XI und entsprechende reaktionsfähige funktionelle und geschützte Derivate davon sind bekannt oder können nach an sich bekannten Methoden hergestellt werden.

Entsprechende reaktionsfähige funktionelle und geschützte Derivate von Verbindungen der Formel X können beispielsweise aus Verbindungen der Formel VIII, worin alle funktionellen Gruppen geschützt sind und die zu acylierende Aminogruppe gegebenenfalls in einer die Acylierung erlaubender Form substituiert ist, hergestellt werden.

Geschützte Verbindungen der Formel XII und ihre reaktionsfähigen Derivate sind neu und sind zusammen mit den Verfahren zu ihrer Herstellung ebenfalls Gegenstand der vorliegenden Erfindung. Sie können aus Verbindungen der Formel VIII oder X durch Umsatz mit einem Diamin der Formel R₄-NH-Y-NH₂, bzw. mit einem reaktionsfähigen Derivat davon der Formel R₄-NH-Y-NH-C(=Z)-OH, gegebenenfalls auch aus einer Verbindung der Formel VIII und einem Diamin der Formel R₄-NH-Y-NH₂ in Gegenwart eines reaktionsfähigen Derivates oder Kohlensäure oder Thiokohlensäure, hergestellt werden.

Die 2-Cephemverbindung der Formel XIII, mit geschützten funktionellen Gruppen, sind neu und zusammen mit den Verfahren zu ihrer Herstellung ebenfalls Gegenstand der vorliegenden Erfindung. Die Verbindungen können aus entsprechenden 3-Cephemverbindungen durch basische Isomerisierung oder nach einem der zur Herstellung von Verbindungen der Formel I geeigneten Verfahren erhalten werden, wobei von entsprechenden 2-Cephemverbindungen ausgegegangen wird oder sofern die Reaktionen unter isomerisierenden, basischen Bedingungen durchgeführt werden.

Verbindungen der Formel XIV, worin Ri die angegebenen Bedeutungen hat und andere funktionelle Gruppen in geschützter Form vorliegen, sind neu und bilden zusammen mit den Verfahren zu ihrer Herstellung ebenfalls einen Gegenstand der vorliegenden Erfindung. Diese Verbindungen können nach einem der zur Herstellung von Verbindungen der Formel I, worin funktionelle Gruppen geschützt sind, beschriebenen Verfahren a) bis h) hergestellt werden, wobei in den Ausgangsverbindungen, z.B. der Formel XXIV, gegebenenfalls in geschützter Form vorliegen muss.

Verbinduagen der Formeln XV, worin die Aminogruppe gegebenenfalls durch eine, die Acylierung erlaubende Gruppe substituiert sein kann, und worin die Carboxylgruppe und gegebenenfalls in den Gruppierungen A und R₄ vorhandene weitere funktionelle Gruppen in geschützter Form vorliegen können, sowie die bezüglich der Carboxylgruppe reaktionsfähigen Derivate davon, sind neu und bilden zusammen mit den Verfahren zu ihrer Herstellung ebenfalls einen Gegenstand der vorliegenden Erfindung.

Diese Verbindungen können beispielsweise hergestellt werden, indem man

### a"') in einer Verbindung der Formel

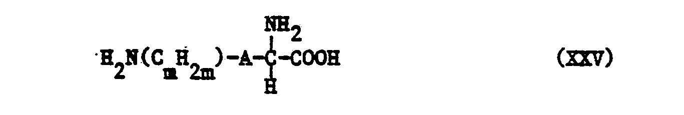

worin die endständige Aminogruppe in geschützter Form vorliegt, die zu acylierende Aminogruppe gegebenenfalls durch eine, die Acylierung erlaubende Gruppe substituiert ist, und worin die Carboxylgruppe und gegebenenfalls in der Gruppierung A vorhandene weitere funktionelle Gruppen in geschützter Form vorliegen können, die a-Aminogruppe durch Behandeln mit einem den entsprechenden Acylrest einer Kohlen-oder Thiokohlensäure der Formel

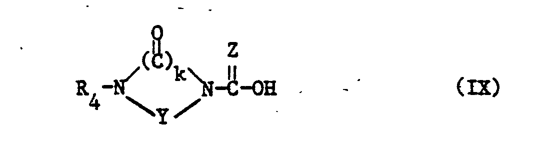

einführenden Acylierungsmittel, worin im Rest R₄ gegebenenfalls vorhandene funktionelle Gruppen in geschützter Form vorliegen können, acyliert, oder

### b"') ein reaktionsfähiges Derivat einer Kohlensäure- oder Thiokohlensäureverbindung der Formel

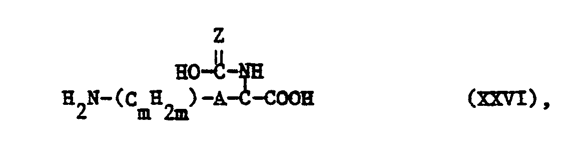

worin die endständige Aminogruppe in geschützter Form vorliegt, und worin die Carboxylgruppe und gegebenenfalls in der Gruppierung A vorhandene weitere funktionelle Gruppen in geschützter Form vorliegen können, mit einem sekundären Amid der Formel 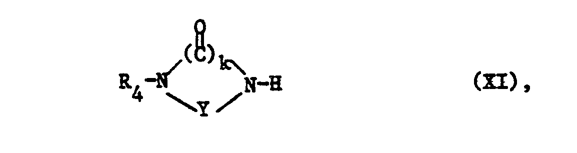 worin im Rest R₄ gegebenenfalls vorhandene funktionelle Gruppen geschützt sein können und die sekundäre Amidogruppe in einer die Acylierung erlaubenden Form vorliegt, reagieren lässt, oder

### c"') die unter a"') oder b"') genannte Acylierung in situ vornimmt, indem man eine Aminoverbindung der Formel XXV und eine Amidoverbindung der Formel XI, beide mit der oben angegebenen Bedeutung, gleichzeitig oder nacheinander auf ein bivalentes reaktives Derivat der Kohlen- oder Thiokohlensäure einwirken lässt, oder indem man

### d"') eine Verbindung der Formel

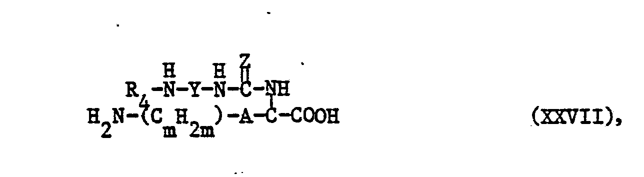

worin die endständige Aminogruppe in geschützter Form vorliegt, und worin die Carboxylgruppe und gegebenenfalls in der Gruppierung A vorhandene weitere funktionelle Gruppen in geschützter Form vorliegen können, und die später den Diazaring bildende sekundäre Amino- bzw. Amidogruppe gegebenenfalls durch Gruppen substituiert sind, die die N-Acylierung erlauben, mit einem bivalenten acylierenden Derivat der Kohlensäure oder der Oxalsäure unter Ringschluss diacyliert, und gewünschtenfalls in einer erhaltenen Verbindung der Formel XIV die ge- : schützte Carboxylgruppe in eine freie Carboxylgruppe oder in ein reaktionsfähiges funktionelles Derivat davon überführt.

Die die Acylierung erlaubenden Gruppen, sowie die Schutzgruppen in den Gruppierungen A und R₄, sind die gleichen wie die unter den Verbindungen der Formeln II oder IV, sowie I erwähnten. Zum intermediären Schutz der Carboxylgruppe in den Verbindungen der Formeln XXV, XXVI und XXVII können an sich ebenfalls die bereits erwähnten Carboxylschutzgruppen verwendet werden, jedoch müssen sich die zum intermediären Schutz in der vorliegenden Acylierung benützten Carboxylschutzgruppen von den übrigen Schutzgruppen, die in den Verbin- dungen der Formel XV notwendigerweise vorhanden bleiben sollen, in der Art ihrer Abspaltung unterscheiden, so dass sie nach den vorliegenden Acylierungsreaktionen selektive abgespalten werden können. Wenn beispielsweise zum intermediären Schutz der Carboxylgruppe eine Hydrogenolytisch abspaltbare Schutzgruppe, wie eine der genannten gegebenenefalls substituierten Benzylgruppen, z.B. die Benzyl- oder p-Nitrobenzylgruppe, verwendet wird, dann dürfen die anderen Schutzgruppen hydrogenolytisch nicht abspaltbar sein; sie können beispielsweise die genannten, nur acidolytische abspaltbaren tert.-Niederalkylgruppen, wie tert.-Bütyl, oder tert.-Niederalkoxycarbonyl, wie tert.-Butoxycarbonyl, sein.

Die Verfahren a"') bis d"') werden im übrigen analog ausgeführt wie die Verfahren d) bis g) oder b") bis e").

In einer erhaltenen Verbindung der Formel XV, mit entsprechend geschützten funktionellen Gruppen, kann eine Schutzgruppe, gegebenenfalls selektiv, abgespalten, oder eine, gegebenenfalls bei der Acylierungsreaktion frei gewordene, funktionelle Gruppe geschützt werden.

Reaktionsfähige funktionelle Derivate von Verbindungen der Formel XV bezüglich der Carboxylgruppe, die im Verfahren a") die 7ß-Amidbildung ermöglichen, und worin alle anderen funktionellen Gruppen geschützt sind, sind die gleichen wie die unter der Formel III genannten Anhydride oder aktivierten Ester und können analog diesen hergestellt werden.

Verbindungen der Formel XVI, worin die Gruppe HO-W- und gegebenenfalls in den Gruppierungen A und R₄ vorhandene funktionelle Gruppen in geschützter Form vorliegen, und worin die Gruppe -COOH gegebenenfalls in reaktionsfähiger Form vorliegt, können aus Verbindungen der Formel XV durch Einführung der Gruppe HO-W-, oder auch analog den Verfahren a"') bis d"') hergestellt werden, wobei in den Ausgangsmaterialien die Aminogruppe durch die geschützte Gruppe HO-W-NH- ersetzt ist.

Verbindungen der Formel XVII und XVIII und ihre geschützten und reaktionsfähigen Derivate sind bekannt oder können auf an sich bekannte Weise hergestellt werden.

Verbindungen der Formel XIX und ihre geschützten und reaktionsfähigen Derivate sind neu. Sie können durch Acylierung von Verbindungen der Formel XVII oder Amidierung von Verbindungen der Formel XVIII hergestellt werden, wobei die Ausgangsverbindungen in geschützter Form eingesetzt werden.

Verbindungen der Formel XX und XXI und ihre geschützten und die Acylierungen erlaubenden Derivate sind bekannt oder können auf an sich bekannte Weise, z.B. durch Acylierung von Verbindungen der Formel II mit dem Acylrest einer Verbindung der Formel XXV oder XXVI, unter entsprechendem Schutz funktioneller Gruppen, hergestellt werden.

Verbindungen der Formel XXII und ihre geschützten und die Acylierungen erlaubenden Derivat sind neu. Sie können auf an sich bekannte Weise hergestellt werden, z.B. indem man ein Diamin der Formel R₄-NH-Y-NH₂, worin die sekundäre Aminogruppe in geschützter Form vorliegt mit einem acylierenden Derivat einer Verbindung der Formel XXI, oder eine Verbindung der Formel XX mit einem acylierenden Derivat einer Verbindung der Formel R₄-NH-Y-NH-C(=Z)-OH behandelt, wobei in den Ausgangsmaterialien nicht an der Acylierungsreaktion teilnehmende Gruppen in geschützter Formund die zu acylierenden Aminogruppen gegebenenfalls in einer die Acylierung erlaubenden Form vorliegen.

Verbindungen der Formel XXIII, worin die Aminogruppe gegebenenfalls durch eine die Acylierung erlaubende Gruppe substituiert sein kann, und worin die 4-Carboxylgruppe und gegebenenfalls im Rest R₁ und in den Gruppierungen A und R₄ vorhandene weitere funktionelle Gruppen in geschützter Form vorliegen können, sind ebenfalls neu. Sie können analog dem Verfahren zur Herstellung von entsprechenden 3-Cepheaverbindungen der Formel IV, z.B. gemäss einem der Verfahren a') bis j") erhalten werden, wenn man entweder von entsprechenden 2-Cephemverbindungen ausgeht oder die Verfahren unter basischen Bedingungen durchführt, oder indem man eine 3-Cephemverbindung der Formel IV isomerisiert.

Verbindungen der Formel XXIV, worin die Aminogruppe gegebenenfalls durch eine die Acylierung erlaubende Gruppe substituiert sein kann, und worin die 4-Carboxylgruppe und gegebenenfalls im Rest R₁ und in den Gruppierungen A und R₄ vorhandene weitere funktionelle Gruppen in geschützter Form vorliegen können, sind neu. Sie können analog dem Verfahren zur Herstellung von Verbindungen der Formel IV, z.B. gemäss einem der Verfahren a") bis f") hergestellt werden, wobei in den Ausgangsmaterialien der Formeln II, XX, XXI, XXII oder XXIII der Rest R durch eine der inerten Gruppen oder durch eine geschützte Hydroxygruppe ersetzt ist.

Verbindungen der Formeln XXV bis XXVII, sowie geschützte und reaktionsfähige Derivate davon, sind bekannt oder auf an sich bekannte Weise herstellbar.

Die pharmakologisch verwendbaren Verbindungen der vorliegenden Erfindung können, z.B. zur Herstellung von pharmazeutischen Präparaten verwendet werden, welche eine wirksame Menge der Aktivsubatanz zusammen oder im Gemisch mit anorganischen oder organischen, festen. oder flüssigen pharmazeutisch verwendbaren Trägerstoffen enthalten, die sich vorzugsweise zur parenteralen Verabreichung eignen.

Vorzugsweise verwendet man die pharmakologisch wirksamen Verbindungen der vorliegenden Erfindung in From von injizierbaren, z.B. intravenös oder subkutan, verabreichbaren Präparaten oder von Infusionslösungen. Solche Lösungen sind vorzugsweise isotonische wässrige Lösungen oder Suspensionen, wobei diese z.B. aus lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparte, die, wenn erwünscht, weitere pharmakologisch wertvolle Stoffe enthalten können, werden in an sich bekannter Weise, z.B. mittels konventioneller Lösungs- oder Lyophilisierungsverfahren, hergestellt und enthalten von 0,1% bis 100%, insbesondere von etwa 1% bis etwa 50%, Lyophilisate bis zu 100% des Aktivstoffes. Je nach Art der Infektion und Zustand des infizierten Organismus verwendet man tägliche Dosen von etwa 0,5 g bis etwa 5 g s.c. zur Behandlung von Warmblütern von etwa 70 kg Gewicht.

Die folgenden Beispiele dienen zur Illustration der Erfindung; Temperaturen werden in Celsiusgraden angegeben.

In den Beispielen werden folgenden Abkürzungen verwendet:
BOC: tert.-Butyloxycarbonyl,
Smp.: Schmelzpunkt
IR.: Infrarotspektrum; die Zahlen geben die Lage der Absorptionsbanden in cm⁻¹ an,
UV.: Ultraviolettspektrum; die Zahlen vor den Klammern geben die Lage der Absorptionsmaxima in nm an; die Zahlen in Klammern geben den ε-Wert des Maximums an.

Beispiel 1: 154 g (R,S)-4-Aminomandelsäure werden in 850 ml 1N wässriger NaOH aufgeschlämmt, wobei bereits ein grosser Teil der Säure in Lösung geht. Mittels Titrator wird mit 2N wässriger NaOH ein pH von 7,5 eingestellt. Nachdem alles Ausgangsmaterial gelöst ist, gibt man 300 ml Di-tert.-butyl-carbonat in 800 ml Dioxan zu und rührt 4 Std. bei Zimmertemperatur wobei das pH bei 7,5 konstant gehalten wird durch Zugabe von 2N wässriger NaCH mittels des Titrators. Darauf nimmt man in Essigester auf, wäscht zweimal mit Wasser, vereinigt die Wasserphasen und stellt sie bei 5° mit konz. wässriger Salzsäure auf pH 3. Anschliessend wird sofort mit Essigester extrahiert, mit gesättigter wässriger NaCl-Lösung neutral gewaschen, über Na₂SO₄ getrocknet und eingedampft. Nach einmaliger Kristallisation des Rohproduktes aus Methanol-Essigester-Hexan resultiere die (R,S)-4-(BOC-Amino)mandelsäure vom Smp. 137-139°C. IR.: 3520, 3420, ₁₇₂₅ (breit), ₁₆₁₅, ₁₅₉₅, 152₀ cm⁻¹ (CH₂Cl₂). UV.: 247 nm (22000) in C₂H₅OH.

Beispiel 2: Zu einer Lösung von 168 g (R,S)-4-(BOC-Amino)-mandelsäure in 500 ml Methanol tropft man bei 5° unter Rühren im Verlaufe von 1 1/2 Std. eine Lösung von 300 g Diphenyldiazomethan in 500 ml CH₂C1₂ und rührt bei der gleichen Temperatur weitere 1 1/2 Std. Die Reaktionsmischung wird im Vakuum bei Zimmertemperatur auf 400 ml Volumen eingeengt und mit 500 ml Essigester versetzt. Man entfärbt die Lösung durch Zutropfen von Essigsäure, verdünnt mit mehr Essigester und wäscht nacheinander mit gesättigten wässrigen Lösungen von NaHCO₃ (bis basisch) und NaCl (bis zum Neutralpunkt). Dann trocknet man über Na₂S0₄ dampft im Vakuum ein und kristallisiert das erhaltene Rohprodukt einmal aus Toluol. Es resultiert der (R,S)-4-(BOC-Amino)-mandelsäure-diphenylmethylester vom Smp. 168°C. IR.: 3500, 3370, 1720, 1620, 1605, 1535 cm⁻¹ (Nujol), UV.: 244 nm (20400) in C₂H₅OH.

Beispiel 3: Zu einer Lösung von 53 g (R,S)-4-(BOC-Amino)-mandelsäure- diphenylmethylester in 350 ml Aceton tropft man bei 0° unter Rühren im Verlaufe von 3 Min. 53 ml einer Lösung von 8N/CrO₃ in 8N wässriger H₂SO₄. Man rührt weitere 10 Min. bei 0° und fügt dann unter Rühren 2 Liter Wasser zu, wobei das Reaktionsprodukt kristallisiert. Die Kristalle werden abgenutscht, mit viel Wasser gewaschen (bis die Waschwasser sich als neutral und farblos erweisen) und am Wasserstrahlvakuum bei 50* bis zur Gewichtskonstanz getrocknet. Das so erhaltene Rohprodukt kristallisiert man einmal aus Aceton-Hexan um, wobei der 2-(4-BOC-Aminophenyl)-glyoxylsäure-diphenylmethylester vom Smp. 126-127°C erhalten wird. IR.: 3400, 1735, 1678, 1602, 1582, 1520, 15₀0 cm⁻¹ (CH₂Cl₂). UV.: 310 nm (24750) in C₂H₅OH.

Beispiel 4: 27 g Hydroxylamin-hydrochlorid werden in einer Reibschale mit 50 g Natriumacetat-trihydrat gut verrieben. Das dabei freigesetzte Hydroxylamin löst man mit 900 ml Aethanol heraus und filtriert die Lösung zur Abtrennung von ungelösten Feststoffen durch eine Glasfilternutsche. Zur erhaltenen Hydroxylaminlösung gibt man 100 g 2-(4-BOC-Aminophenyl)glyoxylsäure-diphenylmethylester und kocht 1 Std. am Rückfluss, wobei sich eine klare Lösung bildet. Darauf engt man auf ca. 500 ml Volumen ein (bis Kristallisationsbeginn), fällt mit 1 1 Wasser, belässt 2 Std. bei 4°C, nutscht ab, wäscht mit Wasser und trocknet die Kristalle am Vakuum bei 50°C bis zur Gewichtskonstanz. Nach einmaliger Umkristallisation des Rohproduktes resultiert der Z-(4-BOC-Aminophenyl)-2-hydroxyiminoessigsäure-diphenylmethylester vom Smp. 185°C (Zers.). IR.: 3330 (Schulter), 3300, 1720, 1685, 1605, 1595, 1538 cm⁻¹ (Nujol). Nach Dünnschichtchromatogramm im System Toluol-Essigester-(1:1) und NMR.-Spektrum liegt ein syn-anti-Gemisch der Oxime vor.

Beispiel 5: 50 g 2-(4-BOC-Aminophenyl)-2-hydroxyimino-essigsäure- diphenylmethylester werden in einer Mischung aus 1660 ml Methanol und 250 ml 2N wässriger NaOH 1 1/2 Std. unter N₂ am Rückfluss gekocht. Die Reaktionsmischung engt man auf 300 ml Volumen ein, verdünnt mit Essigester und wäscht dreimal mit Wasser. Die Wasseranteile werden vereinigt, auf 0°C gekühlt und mit 2N wässriger Salzsäure auf pH 3 gestellt. Man extrahiert mit Essigester, wäscht die organische Phase mit gesättigter wässriger NaCl-Lösung neutral, trocknet über Na₂SO₄ und dampft im Vakuum ein. Der Eindmmpfrückstand wird in 250 ml Wasser versetzt und mit 2N wässriger Natronlauge auf pH 7 gestellt. Anschliessend extrahiert man die wässrige Lösung mit Essigester, - wäscht die organische Phase dreimal mit Wasser, vereinigt die wäss- - rigen Anteile, filtriert sie durch Sand, engt das Filtrat im Vakuum auf ca. 50 ml ein und fällt das Produkt durch Zufügen von 150 m1 Aethanol und 500 ml Aether. Nach dem Abfiltrieren und Trocknen resultiert das 2-(4-BOC-Aminophenyl)-2-hydroxyimino-essigsäure-natriumsalz (syn-anti-Gemisch).

Beispiel 6: 70,45 g 2-(4-BOC-Aminophenyl)-2-hydroxyiminoessigsäure- natriumsalz werden in 2 1 Wasser in Gegenwart von 15 g 10-proz. Pd/C erschöpfend hydriert (Zimmertemperatur, Normaldruck). Man filtriert vom Katalysator ab, wäscht mit Wasser nach und engt das Filtrat am Vakuum auf ein Volumen von 1 Liter ein. Darauf stellt man mit 2N wässriger Salzsäure auf pH 4, belässt 15 Min. bei Zimmertemperatur, filtriert anschliessend das ausgefällte Produkt ab, wäscht es zweimal mit wenig Wasser, sowie einmal mit Aethanol und trocknet es am Wasserstrahlvakuum bei 50°C. Man erhält das (R,S)-2-(4-BOC-Aminophenyl)-glycin vom Smp. 270°C (Zers.). IR.: 3370, 3250-2200 (breit), 1700, 1620, 1595, 1535 (Nujol).

Beispiel 7: 17,5 g (R,S)-2-(4-BOC-Aminophenyl)-glycin werden in 250 ml Tetrahydrofuran aufgeschlämmt, mit 19,3 ml Bis(trimethylsilyl)-acetamid versetzt und 70 Min. unter N₂ bei Zimmertemperatur gerührt, wobei nach 40 Min. nochmals 19,3 ml Bis(trimethylsilyl)-acetamid zugesetzt werden. Anschliessend werden nacheinander 7,3 ml N-Methylmorpholin und 10,72 g 2-Imidazolidon-1-carbonylchlorid zugegeben. Die Reaktionsmischung wird weitere 5 Std. bei Zimmertemperatur unter N₂ gerührt und darauf in Wasser gegossen. Man stellt mit 1N wässriger Salzsäure auf pH 3, extrahiert mit Essigester, wäscht die organische Phase dreimal mit gesättigter wässriger NaCl-Lösung, trocknet sie über Natriumsulfat und dampft im Vakuum ein. Die erhaltene amorphe (R,S)-2-(4-tOC-Aminophenyl)-2-(2-imidazolidon-1-carboxamido)-essigsäure wird ohne Reinigung weiterverarbeitet. IR.: 3430, 3300 damit teilweise überlagert 3500-2200 (breit), 1740-1660 (breit), 1610, 1595, 1520 cm⁻¹ (CH₂C1₂).

Beispiel 8: In zum vorangehenden Beispiel analoger Weise wird aus 15 g (R,S)-2-(4-BOC-Aminophenyl)-glycin und 21,58 g 3-Methansulfonyl-3-imidazoli_{d}on-l-carbonylchlorid die (R,S)-2-(4-BOC-Aminophenyl)-2-(3-methansulfonyl-2-imidazolidon-1-carboaamido)-essigsäure hergestellt, die ohne Reinigung weiterverarbeitet wird.

Beispiel 9: In zu den beiden vorangehenden Beispielen analoger Weise wird aus (R,S)-2-(4-BOC-Aminophenyl)-glycin und 25,3 g 2,3-Dioxo-4- äthylpiperazin-carbonylchlorid die (R,S)-2-(4-BOC-Aminophenyl)-2-(4-äthyl-2,3-dioxo-piperazin-l-carboxamido)-essigsäure hergestellt, die ohne Reinigung weiterverarbeitet wird.

Beispiel 10: 12,7 g (R,S)-2-(4-BOC-Aminophenyl)-2-(2-imidazolidon- l-carboxamido)-essigsäure werden in 100 ml Tetrahydrofuran gelöst, auf -20°C abgekühlt und nacheinander mit 3,85 ml N-Methylmorpholin und 3,6 ml Chlorameisensäure-isobutylester versetzt. Man rührt 3 Std. bei -20°C, senkt darauf die Temperatur auf -40°C ab, fügt 13,5 g 3-[(1-Methyl-1H-tetrazol-5-yl)-thiomethyl]-7ß-amino-3-cephem-4-carbonsäure-diphenylmethylester in fester Form zu, rührt 10 Min. bei -40°C, sowie 2 1/2 Std. bei 0°C und arbeitet darauf wie folgt auf: Die Reaktionsmischung wird in Essigester aufgenommen und nacheinander mit lN wässriger Salzsäure, gesättigter wässriger NaCl-Lösung, gesättigter wässriger NaHCO₃ Lösung und nochmals NaCl-Lösung bis zum Neutralpunkt gewaschen. Man trocknet die organische Phase über Na₂SO₄, dampft sie im Vakuum ein und chromatographiert den Rückstand an 900 g Kieselgel (Eluiermittel: Toluol-Essigester (1:1); Fraktionengrösse 500 ml). Aus den Fraktionen 18-50 erhält man nach Umfällen aus CH₂Cl₂-Diäthyläther₋Hexan den 3-[(1-Methyl-1H-tetrazol-5-yl)-thiomethyl]-7β-[(2R)-(4-BOC-aminophenyl)-2-(2-imidazolidon-1-carboxamido)-acetamido]-3-cephem-4-carboasäure-diphenylmethylester. 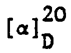 =-108° ± 1° (0,814% in CHC1₃). IR.: 3440, 3300, 1792, 1725, 1705, 1690, 1670, 1620, 1600, 1515 cm⁻¹ (CH₂Cl₂). UV.: 246 (29600, EtOH).

Spätere Fraktionen bestehen aus einem binären Gemisch der obigen Verbindung mit dem entsprechenden 2S-Derivat.

Beispiel 11: 5 g 3-[(1-Methyl-1H-tetrazol-5-yl)-thiomethyl]-7β-[(2R)-2-(4-BOC-aminophenyl)-2-(2-imidazolidon-1-carboxamido)-acetamido]-3-cephem-4-carbonsäure-diphenylmethylester werden mit 2,3 g p-Toluolsulfonsäure-monohydrat in 50 ml Acetonitril 2 1/2 Std. bei Zimmertemperatur gerührt. Nach Zugabe von 500 ml Aether, Absaugen des Niederschlages, Waschen mit 300 ml Aether und Trocknen am Vakuum erhält man das 3-[(1-Methyl-1H-tetrazol-5-yl)-thiomethyl]-7β-[(2R)-2-(4-aminophenyl)-2-(2-imidazolidon-l-carboxamido)-acetamido]-3-cephem-4-carbonsäure-diphenylmethylester-p-toluolsulfonat. 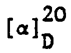 = -37°±1° (0,758% in CH₃0H). IR.: 3420, 3320, 2640, 1785, 1730, 1662, 1540, ₁52₀, ₁470, ₁385 cm⁻¹ (Nujol). UV.: 25₀ (16200, _{E}tOH).

Beispiel 12: 5,01 g 3-[(1-Methyl-1H-tetrazol-5-yl)-thiomethyl]-7β-[(2R)-2-(4-aminophenyl)-2-(2-imidazolidon-1-carboxamido)-acetamido]-3-cephem-4-carbonsäure-diphenylmethylester-p-toluolsulfonat werden in 150 ml Tetrahydrofuran zusammen mit 1,04 ml Pyridin und 4,15 g (2R)-2-BOC-Amino-2-diphenylmethoxycarbonyläthoxy-carbonylchlorid 1 Std. bei 0° gerührt. Man nimmt in Essigester auf, wäscht nacheinander mit 1N wässriger Salzsäure, gesättigter wässriger NaCl-Lösung, gesättigter wässriger NaHCO₃-Lösung und nochmals NaCl-Lösung bis zum Neutralpunkt. Das nach dem Trocknen über Natriumsulfat und dem Eindampfen erhaltene Rohprodukt chromatographiert man an 400 g Kieselgel (Eluiermittel: Toluol-Essigester-(1:1); Fraktionen à 500 ml). Durch Vereinigung der produkthaltigen Fraktionen resultiert der 3-[(1-Methyl-lH- tetrazol-5-yl)-thiomethyl]-7β-{(2R)-2-[4-((2R)-2-BOC-amino-2-diphenyl- methoxycarbonpläthoxy-carboaylamino)-phenyl]-2-(2-imidazolidon-1-carboxamido)-acetamido}-3-cephem-4-carbonsäure-diphenylmethylester. 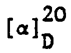 = -19±1° (1,006% in DMSO). IR.: 3420,3330,.1785, 1752,1725, 1703, 1690, 1620, 1605, 1527, 1486 cm⁻¹ (Nujol). UV.: 246 (30100; CH₃OH).

Beispiel 13: 1,2 g 3-[(1-Methyl-1H-tetrazol-5-yl)-thiomethyl]-7β-[(2R)-2-(4-BOC-Aminophenyl)-2-(2-imidazolidon-1-carboxamido)-acetamido]-3-cephem-4-carbonsäure-diphenylmethylester werden in einer Mischung von 3,24 ml CH₂C1₂ und 1,164 ml Anisol bei Zimmertemperatur gelöst. Man gibt 15,36 ml auf 0° gekühlte Trifluoressigsäure zu der Lösung und rührt ohne externe Kühlung während 15 Min. Nach Zugabe von 250 ml Hexan-Aether-(2:1) wird 5 Min. gerührt, der Niederschlag abgenutscht und mit 200 ml Hexan-Aether-(2:1)-Mischung nachgewaschen. Man löst den Filterrückstand in 100 ml Methanol, fügt 100 ml Wasser hinzu, stellt durch Zugabe von 1N wässriger Natronlauge auf pH 7 und extrahiert mit Essigester. Die organische Phase wird dreimal mit Wasser gewaschen. Alle wässrigen Phasen werden vereinigt und im Vakuum auf ca. 10 ml eingeengt.Durch Zugabe von ca. 300 ml Aethanol entsteht eine weisse Fällung, die abgenutscht, zweimal je mit Aethanol und Aether gewaschen und am Vakuum getrocknet wird. Es handelt sich dabei um das 3-[(1-Methyl-1H-tetrazol-5-yl)-thiomethyl]-7β-[(2R)-2-(4-aminophenyl)-2-(2-imidazolidon-1-carboxamido)-acetamido]-3-cephem-4-carbonsäure-natriumsalz. Zersetzung ab 170°C. [a]_{D}²⁰= -17°±1° (0,995% in DMSO). IR.: 3310, 1770, 1725 (breit), 1665, 1612, 1520 cm⁻¹ (Nujol). UV.: 245 (16550; H₂0).

Beispiel 14: 2,2 g 3-[(1-Methyl-1H-tetrazol-5-yl)-thiomethyl]-7β-{(2R)-[4-(2R)-2-BOC-amino-2-diphenylmethoxycarbonyläthoxycarbonyl- amino)-phenyl]-2-(2-imidazolidon-1-carboxamido)-acetamido}3-cephem-4-carbonsäure-diphenylmethylester werden in 3,75 ml CH₂C1₂ und 1,35 ml Anisol mit 17,7 ml Trifluoressigsäure analog umgesetzt und aufgearbeitet wie im Beispiel 13 beschrieben. Man erhält das 3-[(1-Methyl-1H-tetrazol-5-yl)-thiomethyl]-7β-{(2R) -2-[4-((2R)-2-Amino-2-carboxy- äthoxycarbonylamino)-phenyl]-2-(2-imidazolidon-1-carboxamido)-acetamido}-3-cephem-4-carbonsäure-natriumsalz. Zersetzung ab 175°C. [α]_{D}²⁰= -3°±1° (0,775% in H₂O). IR.: 3450 (Schulter), 3300, 1770, 1730, 1655, 1560, 1537 cm⁻¹ (Nujol). UV.: 244 (25750; H₂0).

Beispiel 15: 9,17 g (R,S)-2-(4-BOC-Aminophenyl)-2-(3-methansulfonyl-2-imidazolidon-1-carboxamido)-essigsäure und 8 g 3-[(1-Methyl-lH- tetrazol-5-yl)-thiomethyl]-7ß-amino-3-cephem-4-carbonsäure-diphenylmethylester werden wie im Beispiel 10 beschrieben mit 2,155 ml Chlorameisensäureisobutylester in 2,31 ml N-Methylmorpholin und 100 ml Tetrahydrofuran umgesetzt und aufgearbeitet. Filtration des Rohproduktes an 400 g Kieselgel in Essigester und anschliessendes Umfällen der Eluate aus CH₂C1₂₋Aether-Hexan liefert 3-[(1-Methyl-lH-tetrazol-5-yl)-thiomethyl]-7ß-[(2R,S)-2-(4-BOC-aminophenyl)-2-(3-methansulfonyl-2-imidazolidon-l-carboxamido)-acetamido]-3-cephem-4-carbonsäure- diphenylmethylester (Epimerengemisch). IR.: 3400, 3300, 1785, 1735, 1720, 1705, 1690, 1670, 1605, 1515, 1355, 1165 (CH₂Cl₂). UV.: 248 (18100; EtOH).

Beispiel 16: 7,86 g 3-[(1-Methyl-1H-tetrazol-5-yl)-thiomethyl]-7β-[(2R,S)-2-(4-BOC-aminophenyl)-2-(3-methansulfonyl-2-imidazolidon-l-carboxamido)-acetamido]-3-cephem-4-carbonsäure-diphenylmethylester werden mit 3,2 g p-Toluolsulfonsäure-monohydrat in 70 ml Acetonitril wie im Beispiel 11 beschrieben umgesetzt und aufgearbeitet. Man erhält das 3-[(l-Methyl-lH-tetrazol-5-yl)-thiomethyl]-7ß-[(2R,S)-2-(4-aminophenyl)-2-(3-methansulfonyl-2-imidazolidon-1-carboxamido)-acetamido]-3-cephem-4-carbonsäure-diphenylmethylester-p-toluolsul- fonat. IR.: 3450 (Schulter), 3320, 2620 (Schulter), 1780, 1740, 1705 (Schulter), 1693 (Schulter), 1670, 1630, 1600 (Schulter), 1530, 1512 cm⁻¹ (Nujol). UV.: 250 (16200; Et0H).

Beispiel 17: 7,3 g 3-[(1-Methyl-1H-tetrazol-5-yl)-thiomethyl]-7β-[(2R,S)-2-(4-aminophenyl)-2-(3-methansulfonyl-2-imidazolidon-1-car- boxamido)-acetamido]-3-cephem-4-carbonsäure-diphenylmethylester-p-toluolsulfonat werden mit 6,05 g (2R)-2-BOC-Amino-2-diphenylmethoxy- carbonyläthoxycarbonylchlorid und 1,52 ml Pyridin in 150 ml Tetrahydrofuran wie im Beispiel 12 beschrieben umgesetzt und aufgearbeitet. Das anfallende Rohprodukt chromatographiert man an 400 g Kieselgel (Fraktionengrösse 500 ml). Dabei wird wie folgt eluiert: Fr. 1-24 mit Toluol-Essigester-(2:1)-Gemisch, Fr. 25-31 mit Toluol-Essigester-(1:1)-Gemisch sowie Fr. 32-36 mit Essigester. Aus den Fraktionen 10-16 erhält man nach dem Eindampfen und Umfällen aus CH₂Cl₂-Aether-Heaan den 3-[(1-Methyl-1H-tetrazol-5-yl)-thiamethyl]-7β- {(2R)-2-[4-C(2R)-2-BOC-amino-2-diphenylmethoxycarbonyläthoxycar- bonylamino)-phenyl]-2-(3-methansulfonyl-2-imidazolidon-l-carboxamido)-acetamido}-3-cephem-4-carbonsäure-diphenylmethylester. Zersetzung ab 140°C. [α]_{D}²⁰= -9°±1° (0,860% in DMSO). IR.: 3330, 1790, 1735 (breit), 1680, 1610, 1605, 1530, 1382, 1170 cm⁻¹ (Nujol). UV.: 246 (29200; EtOH).

Aus den Fraktionen 29-32 wird in analoger Weise der 3-[(1-Methyl-1H-tetrazol-5-yl)-thiomethyl]-7β- {(2S)-2-[4-((2R)-2-BOC-amino-2-diphenylmethoxycarbonyläthoxycarbonylamino)-phenyl]-2-(3-methansulfonyl-2-imidazolidon-1-carboxamido)-acetamido}-3-cephem-4-carbonsäure- diphenylmethylester erhalten. Zersetzung ab 150°. 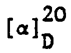 = +16°±1° (0,681% in DMSO). IR.: 3330, 1790, 1738, 1720 (Schulter), 1700 (Schulter), 1683 (Schulter), 1615, 1605, 1533, 1380, 1170 cm⁻¹ (Nujol). UV.: 246 (28500; EtOH).

Beispiel 18: 1,2 g 3-[(1-Methyl-1H-tetrazol-5-yl)-thiomethyl]-7β-[(2R,S)-2-(4-BOC-aminophenyl)-2-(3-methansulfonyl-2-imidazolidon-1-carboxamido)-acetamido]-3-cephem-4-carbonsäure-diphenylmethylester werden in 3,24 ml CH₂Cl₂ und 1,164 ml Anisol mit 15,4 ml Trifluoressigsäure wie im Beispiel 13 beschrieben umgesetzt und aufgearbeitet. Dabei resulitert das 3-[(1-Methyl-1H-tetrazol-5-yl)-thiamethyl]-7β-[(2R,S)-2-(4-aminophenyl)-2-(3-methansulfonyl-2-imidazolidon-1-carboxamido)-acetamido]-3-cephem-4-carbonsäure-natriumsalz (Epimerengemisch). Zersetzung ab 170°. 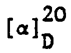 +17°±1° (0,686% in H₂O). IR.: 3380, 1770, 1740, 1680, 1620, 1522, 1383, 1170 (Nujol). UV.: 246 (18300; H₂0).

Beispiel 19: 1,2 g 3-[(1-Methyl-1H-tetrazol-5-yl)-thiomethyl]-7β-{(2R)-2-[4-((2R)-2-BOC-amino-2-diphenylmethoaycarbonyläthoaycarbonyl- amino)-pheayl]-2-(3-methansulfonyl-2-imidazolidon-1-carboxamido)-acetamido}-3-cephem-4-carbonsäure-diphenylmethylester werden gelöst in 2 ml CH₂Cl₂ und 0,74 ml Anisol mit 9,6 ml Trifluoressigsäure analog Beispiel 13 behandelt und aufgearbeitet. Man erhält das 3-[(1-Methyl-1H-tetrazol-5-yl)-thiomethyl]-7ß-(2R)-2-[4-((2R)-2-amino-2-carboxyäthoxycarbonylamino)-phenyl]-2-(3-methansulfonyl- amino-2-imidazolidon-1-carboxamido)-acetamido}-3-cephem-4-carbonsäure-natriumsalz. Zersetzung ab 189°; 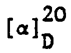 = +19°±1° (0,662% in H₂O). IR.: 3350, 1770, 1740, 1662, 1612, 1536, 1383, 1170 cm⁻¹ (Nujol). UV.: 244 (25200; H₂O).

Beispiel 20: 0,9 g 3-[(1-Methyl-1H-tetrazol-5-yl)-thiomethyl]-7β- {(2S)-2-[4-((2R)-2-BOC-Amino-2-diphenylmethoxycarbonyläthoxycarbonylamino)-phenyl]-2-(3-methansulfonyl-2-imidazolidon-1-carboxamido)-acetamido}-3-cephem -4-carbonsäure-diphenylmethylester werden in 1,5 ml CH₂C1₂ und 0,56 ml Anisol mit 7,2 ml Trifluoressigsäure analog Beispiel 13 behandelt und aufgearbeitet. Man erhält das 3-[(1-Methyl-1H-tetrazol-5-y1)-thiomethy1]-7β-{(2S)-2-[4-((2R)-2-amino-2-carboxyäthoxycarbonyl- amino)-phenyl]-2-(3-methansulfonyl-2-imidazolidon-1-carboxamido)-acetamido}-3-cephem-4-carbonsäure-natriumsalz. Zersetzung ab 184°; 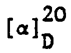 = +59°+1° (0,735% in H₂0); IR.: 3360, 1770, 1738, 1674, 1613, ₁535, 1397, 1382, 1363, 1170 cm⁻¹ (Nujol). UV.: 243 (25700; H₂0).

Beispiel 21: 10 g (R,S)-2-(4-BOC-Aminophenyl)-2-(4-äthyl-2,3-dioxo- piperazin-1-carboxamido)-essigsäure und 9,07 g 3-[(1-Methyl-lH-tetrazol-5-yl)-thiomethyl]-7β-amino-3-cephem-4-carbonsäure-diphenylmethylester werden wie im Beispiel 10 beschrieben umgesetzt (2,46 ml Chlorameisensäureisobutylester; 2,64 ml N-Methylmorpholin und 243 ml Tetrahydrofuran) und aufgearbeitet. Das anfallende Rohprodukt wird an 400 g Kieselgel chromatographiert. Es werden Fraktionen a 500 ml genommen. Eluiert werden die Fraktionen 1-37 mit Toluol-Essigester-(1:1), die späteren Fraktionen mit Essigester. Die Fraktionen 8-13 werden vereinigt, eingedampft und der Eindampfrückstand einmal aus CH₂Cl₂ -Aether-Hexan umgefällt. Man erhält den 3-[(1-Methyl-1H-tetrazol-5-yl)-thiomethyl]-7β-[(2R) -2-(4-BOC-aminophenyl)-2-(4- äthyl-2,3-dioxo-piperazin-l-carboxamido)-acetamido]-3-cephem-4-carbonsäure-diphenylmethylester. 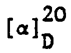 = -95°±1° (0,915% in CHC1₃). IR.: 3410, 3280, 1785, 1715, 1690, 1615, 1592, 1508 cm^{- 1}(CH₂Cl₂). UV.: 248 (31800; EtOH).

Die anschliessenden Fraktionen 14-29 bestehen gemäss Dünnschichtchromatogramm (System: Essigester) aus einem Gemisch der obigen R-Verbindung und dem entsprechenden S-Derivat, das durch nochmalige analoge Chromatographie weiter aufgetrennt werden kann. Aus den abschliessenden Fraktionen 30-44 erhält man nach dem Eindampfen und einmaligem Umfällen aus CH₂Cl₂-Aether-Hexan den 3-[(1-Methyl-1H-tetrazol-5-yl)-thiomethyl]-7β-[(2S)-2-(4-BOC-aminophenyl)-2-(4-äthyl-2,3-dioxo-piperazin-1-carboxamido)-acetamido]-3-cephem-4-carbonsäure-diphenylmethylester. 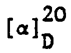 = -116°±1° (0,951% in CHCl₃). IR.: 3400, 3280, 1783, 1712, 1687, 1608, 1590, 1507 cm⁻¹ (CH₂Cl₂); UV.: 248 (32400; EtOH).

Beispiel 22: 3,40 g 3-[(1-Methyl-1H-tetrazol-5-yl)-thiomethyl]-7β-[(2R)-2-(4-BOC-aminophenyl)-2-(4-äthyl-2,3-dioxo-piperazin-1-carbox- amido)-acetamido]-3-cephem-4-carbonsäure-diphenylmethylester werden mit 1,38 g p-Tolualsulfonsäure-monohydrat in 30 ml Acetonitril wie im Beispiel 11 beschrieben umgesetzt und aufgearbeitet. Man erhält das 3-[(1-Methyl-1H-tetrazol-5-yl)-thiomethyl]-7β-[(2R)-2-(4-aminophenyl)-2-(4-äthyl-2,3-dioxo-piperazia-1-carboaamido)-acetamido]-3-cephem-4-carbonsäure-diphenylmethylester-p-toluolsulfonat. 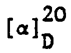 = -32°±1° (0,96% in DMSO). IR.: 3450, 3320, 2625, 1785, 1718, 1683, 1515 cm⁻¹ (Nujol); UV.: 248 (20700); EtOH).

Beispiel 23: 1,58 g 3-[(1-Methyl-1H-tetrazol-5-yl)-thiomethyl]-7β-[(2S)-2-(4-N-BOC-aminophenyl)-2-(4-äthyl-2,3-dioxo-piperazin-l-car- boaamido)-acetamido]-3-cephe-4-carbonsäure-diphenylmethylester werden mit 0,70 g p-Toluolsulfonsäure-monohydrat in 15 ml Acetonitril wie im Beispiel 11 beschrieben umgesetzt und aufgearbeitet. Man erhält das 3-[(1-Methyl-1H-tetrazol-5-yl)-thiomethyl]-7β-[(2S)-2-(4-aminophenyl)-2-(4-äthyl-2,3-dioxo-piperazin-1-carboxamido)-acetamido]-3-cephem-4-carbonsäure-diphenylmethylester-p-toluolsulfonat. IR.: 3450, 3315, 2620, 1782, 1715, 1685, 1515 cm-¹(Najol); UV.: 247 (21000; EtOH).

Beispiel 24: 3,2 g 3-[(1-Methyl-1H-tetrazol-5-yl)-thiomethyl]-7β-[(2R)-2-(4-aminophenyl)-2-(4-äthyl-2,3-dioxo-piperazin-1-carboxamido)-acetamido]-3-cephem-4-carbonsäure-diphenylmethylester-p-toluolsulfonat werden mit 2,65 g (2R)-2-BOC-Amino-2-diphenylmethoxycarbonyl- äthoxycarbonylchlorid und 0,67 ml Pyridin in 65 ml Tetrahydrofuran wie im Beispiel 12 beschrieben umgesetzt und aufgearbeitet. Das anfallende Rohprodukt chromatographiert man an 400 g Kieselgel wobei Fraktionen a 500 ml genommen werden. Die Fraktionen werden mit Toluol-Essigester-(1:1) eluiert. Aus den Fraktionen 7-28 wird nach Umfällen aus CH₂C1₂-Aether-Hexan der 3-[(1-Methyl-1H-tetrazol-5-yl)-thiomethy1]-7β-{(2R)-2-[4-{(2R)-2-BOC-amino-2-dipheny1methoxycarbony1- äthoxycarbonylamino)-pheayl]-2-(4-äthyl-2,3-dioxo-piperazin-1-car- boxamido)-acetamido}-3-cephem-4-carbonsäure-diphenylmethylester. 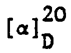 = -69°±+1° (1,00% in CDCl₃). IR.: 3400, 3300,1785,1715 (breit), 1690 (Schulter), 1615, 1598, 1510 cm⁻¹ (CH₂Cl₂). UV.: 246 (33300; EtOH).

Beispiel 25: 1,61 g 3-[(1-Methyl-1H-tetrazol-5-yl)-thiomethyl]-7β-[(2S)-2-(4-aminophenyl)-2-(4-äthyl-2,3-dioxo-piperazin-1-carboxamido)-acetamido]-3-cephem-4-carbonsäure-diphenylmethylester-p-toluolsulfonat werden mit 1,33 g (2R)-2-BOC-Amino-2-diphenylmethoxycarbonyl- äthoaycarbonylchlorid und 0,34 ml Pyridin in 40 ml Tetrahydrofuran wie im Beispiel 12 beschrieben umgesetzt und aufgearbeitet. Das anfallende Rohprodukt chromatographiert man an 250 g Kieselgel wobei Fraktionen ä 250 ml genommen werden. Die Fraktionen werden mit Toluol-Essigester-(1:1) eluiert. Aus den Fraktionen 16-37 wird nach Umfäl aus CH₂C1₂-Aether-Hexan 3-[(1-Methyl-1H-tetrazol-5-yl)-thiomethyl]-7β-{(2S)-2-[4-((2R)-2-BOC-amino-2-diphenylmethoxycarbonyläthoxycar- bonylamino)-pheny1]-2-(4-äthyl-2,3-dioxo-piperazin-1-carboxamido)-acetamido}-3-cephem-4-carbonsäure-diphenylmethylester erhalten. IR.: 3405, 3302, 1782, 1715 (breit), 1690 (Schulter), 1610, 1595, 1510 cm⁻¹ (CH₂Cl₂). UV.: 247 (33000; EtOH).

Beispiel 26: 1,5 g 3-[(1-Methyl-1H-tetrazol-5-yl)-thiomethyl]-7β-[(2R)-2-(4-BOC-aminophenyl)-2-(4-äthyl-2,3-dioxo-piperazin-l-car- boxamido)-acetamido]-3-cephem-4-carbonsäurediphenylmethylester werden in 4,05 ml CH₂Cl₂ und 1,455 ml Anisol gelöst und mit 19,25 ml CF₃COOH wie im Beispiel 13 beschrieben umgesetzt und aufgearbeitet. Man erhält das 3-[(1-Methyl-1H-tetrazol-5-yl)-thiomethyl]-7β-[(2R)-2-(4-aminophenyl)-2-(4-äthyl-2,3-dioxo-piperazin-1-carboxamido)-acetamido]-3-cephem-4-carbonsäure-natriumsalz. Zersetzung ab 200°C. 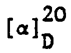 = -6°±1° (1,072% in H₂0). IR.: 3350, 1770, 1712, 1680, 1610, 1520 cm⁻¹ (Nujol). UV.: 245 (22000; H₂0).

Beispiel 27: 1,5 g 3-[(1-Methyl-1H-tetrazol-5-yl)-thiomethyl]-7β-[(2S)-2-(4-BOC-aminopheny1)-2-(4-äthyl-2,3-dioxo-piperazin-1-car- boxamido)-acetamido]-3-cephem-4-carbonsäure-diphenylmethylester werden in 4,05 ml CH₂C1 und 1,455 ml Anisol gelöst und mit 19,25 ml CF₃COOH wie im Beispiel 13 beschrieben umgesetzt und aufgearbeitet. Man erhält das 3-[(1-Methyl-1H-tetrazol-5-yl)-thiamethy1]-7β-[(2S)-2-(4-aminophenyl)-2-(4-äthyl-2,3-dioao-pigerazin-1-carboxamido)-acetamido]-3-cephem-4-carbonsäure-natriumsalz. Zersetzung ab 205°; 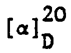 = +32°±1° (0,910Z in H₂O). IR.: 3380, 1770, 1715, 1680, 1612, ₁5₁8 cm⁻¹ (Nujol). UV.: 244 (20800; H₂0).

Beispiel 28: 1,5 g 3-[(1-Methyl-1H-tetrazol-5-yl)-thiomethyl]-7β-(2R)-2-[4-((2R)-2-BOC-amino-2-diphenylmethoxycarbonyläthosycarbonyl- amino)-phenyl]-2-(4-äthyl-2,3-dioxo-piperazin-l-carboxamido)-acet- mmido}-3-cephem-4-carbonsäure-diphenylmethylester werden in 2,5 ml CH₂C1₂ und 0,925 ml Anisol gelöst und mit 12 ml Trifluoressigsäure wie im Beispiel 13 beschrieben umgesetzt und aufgearbeitet. Man erhält das 3-[(1-Methyl-1H-tetrazol-5-yl-thiomethyl]-7β- {(2R)-2-[4-((2R)-2-amino-2-carboxyäthoxycarbonylamino)-phenyl]-2-(4-äthyl-2,3-dioxo-piperazin-1-carboaamido)-acetamido}-3-cephem-4-carbonsäure-natriumsalz. Zersetzung ab 195°; 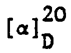 = +7°±1° (0,964% in H₂0); IR.: 3440 (Schulter), 3300, 1770, 1720, 1682, 1615, 1520 (breit) cm⁻¹ (Nujol). UV.: 244 (29700; EtOH).

Beispiel 29: 0,58 g 3-[(1-Methyl-1H-tetrazol-5-yl)-thiomethyl]-7β-{(2S)-2-[4-((2R)-2-BOC-amino-2-diphenylmethoxyäthoxycarbonylamino)-phenyl]-2-(4-äthyl-2,3-dioxo-piperazin-l-carboxamido)-acetamido}-3-cephem-4-carboasäure-diphenylmethylester werden in 1 ml CH₂Cl₂ und 0,2 ml Anisol gelöst und mit 5 ml Trifluoressigsäure wie im Beispiel 13 beschrieben umgesetzt und aufgearbeitet. Man erhält das 3-[(1-Methyl-1H-tetrazol-5-yl-thiomethyl]-7β- {(2S)-[4-((2R)-2-amino-2-car- boxyäthoxycarbonylamino)-phenyl]-2-(4-äthyl-2,3-dioxo-piperazin-1-carboxamido)-acetamido}-3-cephem-4-carbonsäure-natriumsalz. Zersetzung ab 195°; 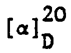 = +35°±1° (0,995% in H₂O); IR.: 3440 (Schulter), 3300, 1770, 1715, 1678, 1610, 1515 (breit) cm⁻¹ (Nujol). UV.: 244 (30000; ^{H}₂⁰).

Beispiel 30: Zu einer Lösung von 0,61 g3-[(1-Methyl-lH-tetrazol-5-yl)-thiomethyl]-7ß-[(2R)-2-(4-aminophenyl)-2-(2-imidazolidon-l-carboxamido)-acetamido]-3-cephem-4-carbonsäure-natriumsalz in 10 ml Wasser gibt man 10 ml Aceton und tropft anschliessend unter Rühren bei Zimmertemperatur im Verlaufe von 40 Min. eine Lösung von 0,43 g (2R)-2-BOC-Amino-2-diphenylmethoxycarbonyläthoxycarboaylchlorid in 10 ml Aceton zu, wobei der pH-Wert der Reaktionslösung bei 7,5 durch Zugabe von 1N wässriger Natronlauge konstant gehalten wird. Nach dem Zutropfen rührt man 10 Min. unter gleichen Bedingungen, extrahiert die Reaktionsmischung mit Essigester und wäscht zweimal mit Wasser nach. Die vereinigten Wasseranteile werden am Vakuum auf ca. 10 ml Volumen eingeengt. Das Produkt wird durch Zugabe von 200 ml Aethanol gefällt, abgenutscht, je zweimal mit Aethanol und Aether gewaschen und bis zur Gewichtskonstanz am Vakuum bei 50° getrocknet. Man erhält das 3-[(1-Methyl-1H-tetrazol-5-y1)-thiomethy1]-7β-{(2R)-2-[4-((2R)-2-BOC-amino-2-diphenylmethoxycarbonyläthoxycarbonylamino)-phenyl]-2-(2-imidazolidon-1-carboxamido)-acetamido}-3-cephem-4-carbonsäure-natriumsalz. UV.: 248 nm (26000; H₂⁰).

Beispiel 31: 2,3 g 3-[(1-Methyl-1H-tetrazol-5-yl)-thiomethyl]-7β-{(2R)-2-[4-((2R)-2-BOC-amino-2-diphenylmethoxycarbonyläthoxycar- bonylamino)-phenyl]-2-(2-imidazolidon-1-carboxamido)-acetamido1-3-cephem-4-carbonsäure-natriumsalz werden in einer Mischung von 3,75 ml CH₂C1₂ und 1,35 ml Anisol bei Zimmertemperatur aufgeschlämmt, mit 17,7 ml auf 0° gekühlter Trifluoressigsäure versetzt und ohne externe Kühlung während 15 Min. (Aufschlämmung geht in Lösung) gerührt. Dann wird wie im Beispiel 13 beschrieben gefällt und aufgearbeitet. Man erhält das 3-[(1-Methyl-1H-tetrazol-5-yl)-thiomethyl]-7β-{(2R)-2-[4-((2R)-2-Amino-2-carboxyäthoxycarbonylamino)-phenyl]-2-(2-imid- azolidon-1-carboxamido)-acetamido}-3-cephem-4-carbonsäure-natriumsalz das mit dem im Beispiel 14 beschriebenen Präparat identisch ist.

Beispiel 32: 0,68 g 3-[(1-Methyl-1H-tetrazol-5-yl)-thiomethyl]-7β-[(2R,S)-2-(4-aminophenyl)-2-(3-methansulfonylamino-2-imidazolidon- l-carboxamido)-acetamido]-3-cephem-4-carbonsäure-natriumsalz werden wie im Beispiel 30 beschrieben umgesetzt. Man erhält das 3-[(1-Methyl-1H-tetrazol-5-yl)-thiomethyl]-7β-{(2R,S)-2-[4-((2R)-2-BOC-Amino-2-di- phenylmethoxycarbonyläthoaycarbonylamino)-phenyl]-2-(3-methansulfonyl-2-imidazolidon-1-carboxamido)-acetamido}-3-acephem-4-carbonsäure-natriumsalz. UV.: 247 (25500; H₂0).

Beispiel 33: 2,3 g 3-[(1-Methyl-1H-tetrazol-5-yl)-thiomethyl]-7ß- {(2R,S)-2-[4-((2R)-2-BOC-amino-2-diphenylmethoxycarbonyläthoxycar- bonylamino)-phenyl]-2-(3-methansulfonyl-2-imidazolidon-1-caxboxamido)-acetamido}-3-cephem-4-carbonsäure-natriumsalz werden wie im Beispiel 31 beschrieben umgesetzt. Man erhält das 3-[(l-Methyl-lH-tetrazol-5-y1)-thiomethy1]-7β-{(2R,S)-2-[4-((2R)-2-amino-2-carboxyäthoxy- carbonylamino)-phenyl]-2-(3-methansulfonyl-2-imidazolidon-1-carbox- amido)-acetamido}-3-cephem-4-carbonsäure-natriumsalz. IR.: 3355, 1770, 1739, 1668, 1612, 1536, 1383, 1363, 1170 cm⁻¹ (Nujol). UV.: 244 (25400; H₂0).

Beispiel 34: 0,66 g 3-[(1-Methyl-1H-tetrazol-5-yl)-thiomethyl]-7β-[(2R)-2-(4-aminophenyl)-2-(4-äthyl-2,3-dioxo-piperazin-l-carboxamido)-acetamido]-3-cephem-4-carbonsäure-natriumsalz werden wie im Beispiel 30 beschrieben umgesetzt. Man erhält das 3-[(1-Methyl-1H-tetrazol-5-yl)-thiomethyl]-7β-{2R)-2-[4-((2R)-2-BOC-amino-2-diphenylmethoxy- carbonyläthoxycarbonylamino)-phenyl]-2-(4-äthyl-2,3-dioxo-piperazin-1-carboxamido)-acetamido}-3-cephem-4-carbonsäure-natriumsalz. UV.: 249 (26500; B₂0).

Beispiel 35: 2,25 g 3-[(1-Methyl-1H-tetrazol-5-yl)-thiomethyl]-7β-(2R)-2-[4-((2R)-2-BOC-amino-2-diphenylmethoxycarbonyläthozycarbonyl- amino)-phenyl}-2-(4-äthyl-2,3-dioxo-piperazin-l-carboxamido)-acetamido}-3-cephem-4-carbonsäure-natriumsalz werden wie im Beispiel 31 beschrieben umgesetzt. Man erhält das 3-[(1-Methyl-1H-tetrazol-5-yl)-thiomethyl]-7β- {(2R)-2-[4((2R)-2-amino-2-carboxyäthoxycarbonyl- amino)-phenyl]-2-(4-äthyl-2,3-diozo-piperazin-1-carboxamido)-acetamido}-3-cephem-4-carbonsäure-natriumsalz, das mit dem gemäss Beispiel 28 erhaltenen Präparat identisch ist.

Beispiel 36: 0,66 g 3-[(1-Nethyl-1R-tetrazol-5-yl)-thiomethyl]-7β-{(2S)-2-[4-aminophenyl]-2-(4-äthyl-2,3-dioxo-piperazin-1-carboxamido)-acetamido}-3-cephem-4-carbonsäurenatriumsalz werden wie im Beispiel 30 beschrieben umgesetzt. Man erhält das 3-[(1-Methyl-1H-tetrazol-5-yl)-thiomethyl]-7β-{(2S)-2-[4-((2R)-2-BOC-amino-2-diphenylmethoxy- carbonyläthoxycarbonylamino)-phenyl]-2-(4-äthyl-2,3-dioxo-piperazin- l-carboxamido)-acetamidol-3-cephem-4-carbonsäure-natriumsalz. UV.: 249 (26500; S₂0).

Beispiel 37: 2,20 g 3-[(1-Methyl-1H-tetrazol-5-yl)-thiomethyl]-7β-{(2S)-2-[4-((2R)-2-BOC-amino-2-diphenylmethoxycarbonyläthoxycarbonyl- amino)-phenyl]-2-(4-äthyl-2,3-dioxo-piperazia-1-carboxamido)-acetamido}-3-cephem-4-carbonsäure-natriumsalz werden wie im Beispiel 31 beschrieben umgesetzt. Man erhält das 3-[(1-Methyl-1H-tetrazol-5-yl)-thiomethyl]-7β-{(2S)-2-[4-((2R)-2-amino-2-carboxyäthoxycarbonyl- amino)-phenyl]-2-(4-äthyl-2,3-dioxo-piperazin-l-carboxamido)-acetamido}-3-cephem-4-carbonsäure-natrimsalz das mit dem gemäss Beispiel 29 erhaltenen Präparat identisch ist.

Beispiel 38: Eine Lösung von 120 g p-Aminomethyl-acetophenon- äthylenketal in 1 Liter Dioxan-Wasser-(l:l) wird mit einer Lösung von 434,5 g Di-tert.-butyl-dicarbonat in 250 ml Dioxan versetzt. Man rührt 2 Std. bei Zimmertemperatur wobei die Reaktionsmischung durch laufende Zugabe von 2NNaOH (Titrator) bei pH 8,5 konstant gehalten wird. Anschliessend wird in Essigester aufgenommen, mit wässriger NaCl-Lösung neutral gewaschen, getrocknet und eingedampft. Das erhaltene Rohprodukt wird an 400 g Kieselgel chromatographiert wobei man aus den Toluol-Eluaten nach Kristallisation aus Hexan das N-BOC-p-Aminomethyl- acetophenon-äthylenketal erhält. Smp. 105-107°C. IR: 3450, 1710, 1500 cm⁻¹ {CH₂Cl₂).

Beispiel 39: 125 g N-BOC-p-Aminomethyl-acetophenon-äthylenketal werden in 250 ml Methanol und 250 ml Essigsäure sowie 50 ml Wasser während 15 Min. auf 65° erwärmt. Man kühlt auf 0°, stelt mit 10N wässriger NaOH auf pH 10, extrahiert mit Essigester und wäscht mit gesättigter wässriger NaCl-Lösung neutral. Das nach dem Trockneu und Eindampfen resultierende Rohprodukt kristallisiert man aus Hexan. Man erhält das N-BOC-p-Aminomethylacetophenon vom Smp. 73-75°. IR.: 3420, 1707, 1680; 1607, 1500 cm (CH₂Cl₂). UV.: 250 nm (15700) in C₂H₅OH.

Beispiel 40: 106 g N-BOC-p-Aminomethyl-acetophenon und 79,7 g Se0₂ werden in 520 ml Pyridin unter N₂ 15 Std. bei 80°C gerührt. Man kühlt ab, filtriert vom ausgefallenen Selen ab, wäscht mit Aceton nach, dampft das Filtrat am Vakuum zur Trockne ein, nimmt den Rückstand in 500 ml Toluol auf und dampft nochmals zur Trockne ein. Darauf suspendiert man den Rückstand in 300 ml Methanol und 500 ml Wasser, stellt mit 2N Salzsäure auf pH 3, extrahiert mit Essigester, wäscht mit gesättigter wässriger NaCl-Lösung neutral, trocknet die organische Phase und dampft sie im Vakuum ein. Das anfallende Rohprodukt wird aus einem Gemisch von 100 ml Essigester, 300 ml Aether und 200 ml Hexan zur Kristallisation gebracht, wobei man die N-BOC-p-Aminomethyl-phenylglyoxylsäure vom Smp. 176-178° (Zersetzung) erhält.

Beispiel 41: 34,1 g Natriumacetat 3H₂O und 17,7 g Hydroxylaminhydrochlorid werden in einer Reibschale zu einem Brei verrieben. Das freie Hydroxylamin wird mit 400 ml Aethanol aus dem Brei herausgelöst. Die Lösung wird durch Filtration von Feststoffen befreit und zusammen mit 41,8 g N-BOC-p-Aminomethyl-phenylglyoxylsäure 2-Std. am Rückfluss gekocht. Man dampft im Vakuum zur Trockene ein, nimmt in Essigester auf und extrahiert zweimal mit je 150 ml gesättigter wässriger NaBCO₃-Lösung. Die vereinigten Bicarbonatlösungen werden auf 0° abgekühlt, mit 2N Salzsäure auf pH 2 gestellt und mit Essigester extrahiert. Nach dem Neutralwaschen der Essigesterphase mit gesättigter NaCl-Lösung, dem Trocknen und Eindampfen resultiert das N-BOC-p-Aminomethylphenylglyoxylsäure-oxim (syn+anti) (Smp. 253°. Zersetzung) das ohne Reinigung weiterverarbeitet wird.

Beispiel 42: 31,8 g N-BOC-p-Aminomethyl-phenyl-glyoxy1säure-oxim (syn+anti) werden in 1 Liter Wasser aufgeschlämmt, durch Zugabe der berechneten Menge 2N-Natronlauge in Lösung gebracht, wobei am Ende der Laugenzugabe ein pH von 7 erreicht wird. Man hydriert die so erhaltene Lösung in Gegenwart von 2 g 10 proz. Pd/C bis zum Ende der Wasserstoffaufnahme. Die Hydrierlösung filtriert man vom Katalysator ab, wäscht mit Wasser nach, engt sie im Vakuum auf ca. 500 ml ein, stellt mit 2N Salzsäure auf pH 4,5 und filtriert das dabei ausgefallene Produkt ab. Man wäscht den Filterrückstand mit wenig Eiswasser und mehrmals mit Hexan und trocknet ihn am Vakuum bei 70°. Man erhält das N-BOC-p-Aminomethyl-(R,S)-phenylglycin vom Smp. 248° (Zersetzung).

Beispiel 43: Aus 18 g N-BOC-p-Aminomethyl-(R,S)-phenylglycin und 28,9 g 1-Chlorcarbonyl-2,3-dioxo-4-äthyl-piperazin wird in Analogie zu Beispiel 9 die (2R,S)-2-[4-(N-BOC-Aminomethyl)-phenyl]-2-(4-äthyl-2,3-dioxo-piperazin-i-carboxamido)-essigsäure dargestellt.

Beispiel 44: 16 g (2R,S)-2-(4-N-BOC-Aminomethylphenyl)-2-(4-äthyl-2,3-dioxo-piperazin-1-carboxamido)-essigsäure und 14,1 g 7ß-Amino-3-[(1-methyl-1H-tetrazol-5-yl)-thiomethyl]-3-cephem-4-carbonsäure- diphenylmethylester werden wie im Beispiel 21 beschrieben umgesetzt und aufgearbeitet. Man erhält den 7ß-[(2R)-2-(4-N-BOC-Aminomethyl- phenyl)-2-(4-äthyl-2,3-dioxo-piperazin-1-cä.rboxamido)-acetamido]-3-[(l-methyl-lH-tetrazol-5-yl)-thiomethyl]-3-cephem-4-carbonsäure-diphenylmethylester . 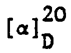 = -84°±1° (0,794% in CHCl₃). IR.: 3480, 1800, 1720 (breit), 1620, 1505 cm⁻¹ (CH₂Cl₂). UV.: 262 nm (12550) in C₂H₅OH.

Als zweites Produkt erhält man den 7ß-[(2S)-2-(4-N-BOC-Amino- methylphenyl)-2-(4-äthyl-2,3-dioxo-piperazin-1-carboxamido)-acetamido]-3-[(1-methyl-1H-tetrazol-5-yl)-thiomethyl]-3-cephem-4-carbonsäure-diphenylmethylester. 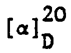 = -85°±1° (0,947%in CKCl₃ )-. IR.: 3420, 1795, 1720, 1700, 1620, 1515 cm⁻¹ (CH₂Cl₂). UV.: 258 nm (12220) in C₂H_{S}OH.

Beispiel 45: 3,3 g 7β-[2-((2R)-4-N-BOC-Aminomethylphenyl)-2-(4-äthyl-2,3-dioxo-piperazin-1-carboxamido)-acetamido]-3-[(1-methyl-1H-tetrazol-5-yl)-thiomethyl]-3-cephem-4-carbonsäure-diphenylmethylester werden mit 1,3 g p-Toluolsulfonsäure-monohydrat wie im Beispiel 22 beschrieben umgesetzt und aufgearbeitet. Man erhält das 7ß-[(2R)-2-(4-Amino- methylphenyl)-2-(4-äthyl-2,3-dioxo-piperazin-1-carboxamido)-acetamido]-3-[(1-methyl-1H-tetrazol-5-yl)-thiomethyl]-3-cephem-4-carbonsäure-diphenylmethylester-p-toluolsulfonat. [a]_{D} = -34°±1° (₀,903 in _{CH30H). IR.: 3500-2500} (breit), ₁₇₉₀, ₁₇₂₂, ₁₆85, 1515 cm⁻¹ (Nujol). UV.: 255 nm (11200) in C₂H₅OH.

Beispiel 46: 3,8 g 7ß-[(2S)-2-(4-N-BOC-Aminomethylphenyl)-2-(4-äthyl-2,3-dioxo-piperazin-1-carboxamido)-acetamido]-3-[(1-methyl-1H-tetrazol-5-yl)-thiomethyl]-3-cephem-4-carbonsäure-diphenylmethylester werden mit 1,5 g p-Toluolsulfonsäure-monohydrat wie im Beispiel 23 beschrieben umgesetzt und aufgearbeitet. Man erhält das 7ß-[(2S)-2-(4-Aminomethylphenyl)-2-(4-äthyl-2,3-dioxo-piperazin-1-carboxamido)-acetamido]-3-[(1-methyl-1H-tetrazol-5-yl)-thiomethyl]-3-cephem-4-carbonsäure-diphenylmethylester-p-toluolsulfonat. 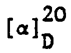 = -10°±1° (1,046 % in CH₃OH). IR.: 3500-2500 breit, 1790, 1718, 1685, 1517 cm⁻¹ (Nujol). UV.: 260 nm (11400) in C₂H₅OH.

Beispiel 47: 3,45 g 7ß-[(2R)-2-(4-Aminomethylphenyl)-2-(4-äthyl-2,3-dioxo-piperazin-1-carboxamido)-acetamido]-3-[E1-methyl-1H-tetrazol-5-yl)-thiomethyl]-3-cephem-4-carbonsäure-diphenylmethylester-p-toluolsulfonat werden wie im Beispiel 24 mit (2R)-2-BOC-Amino-2-diphenylmethoxycarbonyl-äthoxycarbonylchlorid umgesetzt und aufgearbeitet. Man erhält den 7β-[(2R)-2-[4-((2R)-2-BOC-Amino-2-diphenylmethoxy- carbonyläthoxycarbonylaminomethyl)-phenyl]-2-(4-äthyl-2,3-dioxo-piperazin-1-carboxamido)-acetamido}-3-[(1-methyl-1H-tetrazol-5-yl)-thiomethyl]-3-cephem-4-carbonsäure-diphenylmethylester. 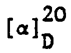 = -61°±1° (0,85%in CHCl₃). IR.: 3400, 3370, 1785, 1710, 1690 (Schulter) 1500 cm⁻¹ (_{CH2C12}). UV.: 256 nm (13600) in C₂H₅OH.

Beispiel 48: 3,9 g 7ß-[(2S)-2-(4-Aminomethylphenyl)-2-(4-äthyl-2,3-dioxo-piperazin-1-carboxamido)-acetamido]-3-[(1-methyl-1H-tetrazol-5-yl)-thiomethyl]-3-cephem-4-carbonsäure-diphenylmethylester-p-toluolsulfonat werden wie im Beispiel 25 umgesetzt..Man erhält den 7ß- {(2S)-2-[4-((2R)-2-BOC-Amino-2-diphenylmethoxycarbonyläthoxycarbonyl- aminomethyl)-phenyl]-2-(4-äthyl-2,3-dioxo-piperazin-1-carboxamido)-acetamido}-3-[(1-methyl-1H-tetrazol-5-yl)-thiomethyl]-3-cephem-4-carbonsäure-diphenylmethylester. 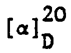 = -40°±1° (0,975% in CHCl₃). IR.: 3400, 3250, 1785, 1710, 1695 (Schulter), 1610, 1500 cm⁻¹ (CH₂Cl₂). UV.: 258 nm (13100) in C₂H_{S}OH.

Beispiel 49: 1,0 g 7β-{(2R)-2-[4-((2R)-2-BOC-Amino-2-diphenylmethoxy- carbonyläthoxycarbonylaminomethyl)-phenyl]-2-(4-äthyl-2,3-dioxo-piperazin-1-carboxamido)-acetamido}-3-[1-methyl-1H-tetrazol-5-yl)-thiomethyl]-3-cephem-4-carbonsäure-diphenylmethylester werden wie im Beispiel 28 beschrieben umgesetzt. Man erhält das 7β-{(2R)-2-[4-((2R)-Amino-2-carboxyäthoxycarbonylaminomethyl)-phenyl]-2-(4-äthyl-2,3-dioxo- piperazin-1-carboxamido)-acetamido}-3-[(1-methyl-1H-tetrazol-5-yl)-thiomethyl]-3-cephem-4-carbonsäure-natriumsalz. 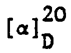 = +6°±1° (0,805 % in H₂0). IR.: 3500-2500 (breit), 1775, 1715, 1680, 1615, 1515 cm⁻¹ (Nujol). UV.: 245 nm (26200) in H₂O.

Beispiel 50: 1,0 g 7β-{(2S)-2-[4-((2R)-BOC-Amino-2-diphenylmethoxy- carbonyläthoxycarbonylaminomethyl)-phenyl]-2-(4-äthyl-2,3-dioxo-piperazin-1-carboxamido)-acetamido]-3-[(1-methyl-1H-tetrazol-5-yl)-thiomethyl]-3-cephem-4-carbonsäure-diphenylmethylester werden wie im Beispiel 29 beschrieben umgesetzt. Man erhält das 7β-{(2S)-2-[4-((2R)-2-Amino-2-carboxyäthoxycarbonylaminomethyl)-phenyl]-2-(4-äthyl-2,3-dioxo-piperazin-1-carboxamido)-acetamido}-3-[(1-methyl-1H-tetrazol-5-yl)-thiomethyl]-3-cephem-4-carbonsäure-natriumsalz. 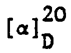 = +50°±1° (0,817 % in H₂0). IR.: 3500-2500 breit, 1775, 1713, 1682, 1614, 1512 cm⁻¹ (Nujol). U_{V}.: 245 nm (26200) in H₂O.

Beispiel 51: 13,7 g (2R,S)-2-(4-BOC-Aminophenyl)-(4-äthyl-2,3-dioxo- piperazin-1-carboxamido)-essigsäure und 11,04 g 7ß-Amino-3-carbamoyl- oxymethyl-3-cephem-4-carbonsäure-diphenylmethylester werden wie im Beispiel 21 beschrieben umgesetzt, aufgearbeitet und chromatographiert. Dabei resultiert nach Kristallisation aus Aceton-Hexan der 7ß-[(2R)-2-(4-BOC-Aminophenyl)-2-(4-äthyl-2,3-dioxo-piperazin-1-carboxamido)-acetamido]-3-carbamoyloxymethyl-3-cephem-4-carbonsäure-diphenylmethylester. [α]²⁰_{D}= 8°±1° (0,873% in CHCl₃). IR: 3500, 3395, 3260, 1786, 1720, 1693, 1615, 1590, 1518 cm⁻¹ (CH₂Cl₂). UV: 248 nm (32200) in C₂H₅OH.

Als zweites Produkt erhält man nach Umkristallisation aus Aceton-Hexan den 7ß-[(2S)-2-(4-BOC-Aminophenyl)-2-(4-äthyl-2,3-dioxo- piperazin-1-carboxamido)-acetamido]-3-carbamoyloxymethyl-3-cephem-4-carbonsäure-diphenylmethylester 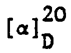 = -61°±1° (0, 823% in CHCl₃). IR: 3480, 3395, 3260, 1782, 1725, 1690, 1615, 1595, 1517 cm⁻¹ (CH₂Cl₂). _{UV}: 248 nm (32600) in C₂H₅OH.

Beispiel 52: 5,5 g 7β-[(2R)-2-(4-BOC-Aminophenyl)-2-(4-äthyl-2,3-dioxo-piperazin-1-carboxamido)-acetamido]-3-carbamoyloxymethyl-3-cephem-4-carbonsäure-diphenylmethylester werden in 200 ml Acetonitril mit 2,44 g p-Toluolsulfonsäure-monohydrat wie im Beispiel 22 beschrieben umgesetzt. Dabei erhält man 7ß-[(2R)-2-(4-Aminophenyl)-2-(4-äthyl-2,3-dioxo-piperazin-1-carboxamido)-acetamido]-3-carbamoyloxymethyl-3-cephem-4-carbonsäure-diphenylmethylester-p-toluolsulfonat. 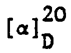 = +11°±1° (1,0987 inMeOH); IR: 3500-2500 breit mit Schultern bei 3300 und 2620, 1780, 1710, 1680, 1610, 1510 cm⁻¹ (Nujol). UV: 250 nm (19600) in CH₃CH.

Beispiel 53: 3,3 g 7β-[(2S)-2-(4-BOC-Aminophenyl)-2-(4-äthyl-2,3-dioxo-piperazin-l-carboxamido)-acetamido]-3-carbamoyloxymethyl-3-cephem-4-carbonsäure-diphenylmethylester werden in 100 ml Acetonitril mit 1,46 g p-Toluolsulfonsäure-monohydrat wie im Beispiel 22 beschrieben umgesetzt. Dabei erhält man das 7ß-[(2S)-2-(4-Aminophenyl)-2-(4- äthyl-2,3-dioxo-piperazin-1-carboxamido)-acetamido]-3-carbamoyloxy- methyl-3-cephem-4-carbonsäure-diphenylmethylester-p-toluolsulfonat. 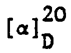 = +27°±1° (0,954% in MeOH); IR: 3500-2500 breit mit Schultern bei 3300 und 2620, 1781, 1712, 1679, 1610, 1513 cm⁻¹ (Nujol); UV: 250 nm (19700) in CH₃0H.

Beispiel 54: 5,0 g 7β-[(2R)-2-(4-Aminophenyl)-2-(4-äthyl-2,3-dioxo- piperazin-1-carboxamido)-acetamido]-3-carbamoyloxymethyl-3-cephem-4-carbonsäure-diphenylmethylester-p-toluolsulfonat werden wie im Beispiel 24 mit (2R)-2-BOC-Amino-2-diphenylmethoxycarbonyläthoxy- carbonylchlorid umgesetzt. Dabei resultiert der 7β-{(2R)-2-[4-((2R)-2-BOC-Amino-2-diphenylmethoxycarbonyläthoxycarbonylamino)-phenyl]-2-(4-äthyl-2,3-dioxo-piperazin-1-carboxamido)-acetamido}-3-carbamoyl- oxymethyl-3-cephem-4-carbonsäure-diphenylmethylester. 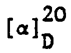 = +8°±1° (0,895% in CHCl₃). IR.: 3610, 3500, 3320, 1790, 1720, 1690, 1605, 1525 cm⁻¹ (CH₂Cl).

Beispiel 55: 3,3 g 7ß-[(2S)-2-(4-Aminophenyl)-2-(äthyl-2,3-dioxo- piperazin-1-carboxamido)-acetamido]-3-carbamoyloxymethyl-3-cephem-4-carbonsäure-diphenylmethylester-p-toluolsulfonat werden wie im Beispiel 24 mit (2R)-2-BOC-Amino-2-diphenylmethoxycarbonyläthoxy- carbonylchlorid umgesetzt. Dabei resultiert der 7β-{(2S)-2-[4-((2R)-2-BOC-Amino-2-diphenylmethoxycarbonyläthoxycarbonylamino)-phenyl]-2-(4-äthyl-2,3-dioxo-piperazin-1-carboxamido)-acetamido}-3-carbamoyloxymethyl-3-cephem-4-carbonsäure-diphenylmethylester. 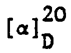 = 37°±1° (0,865% in CHCl₃ ). IR.: 3610, 3500, 3320, 1789, 1718, 1689, 1603, 1523 cm (CH₂Cl₂).

Beispiel 56: 2,25 g 7β-{(2R)-2-[4-((2R)-2-BOC-Amino-2-diphenylmethoxy- carbonyläthoxycarbonylamino)-phenyl]-2-(4-äthyl-2,3-dioxo-piperazin-1-carboxamido)-acetamido}-3-carbamoyloxymethyl-3-cephem-4-carbonsäure- diphenylmethylester werden wie im Beispiel 28 beschrieben umgesetzt. Man erhält das 7β-{(2R)-2-[4-((2R)-2-Amino-2-carboxyäthoxycarbonyl- amino)-phenyl]-2-(4-äthyl-2,3-dioxo-piperazin-1-carboxamido)-acetamido}-3-carbamoyloxymethyl-3-cephem-4-carbonsäure-natriumsalz. Zersetzung ab 205°. 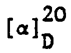 = +42°±1° (1,027% in H₂0). IR.: 3600-2500 (breit), 1772, 1686, 1619, 1520 cm⁻¹ (Nujol).

Beispiel 57: 0,9 g 7β-[(2S)-2-[4-((2R)-2-BOC-Amino-2-diphenylmethoxy- carbonyläthoxycarbonylamino)-phenyi]-2-(4-äthyl-2,3-dioxo-piperazin-1-carboxamido)-acetamido}-3-carbamoyloxymethyl-3-cephem-4-carbonsäure- diphenylmethylester werden wie im Beispiel 28 beschrieben umgesetzt. Man erhält das 7β-{(2S)-2-[4-((2R)-2-Amino-2-carboxäthoxycarbonylamino)-phenyl]-2-(4-äthyl-2,3-dioxo-piperazin-1-carboxamido)-acetämido}-3-carbamoyloxymethyl-3-cephem-4-carbonsäure-natriumsalz. Zersetzung ab ca. 240°. 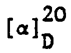 = +64°±1° (0,524% in H₂O). IR.: 3600-2500 (breit), 1770, 1717, 1685, 1617, 1521 cm⁻¹ (Nujol).

Beispiel 58: 5,2 g 7ß-[(2R)-2-(4-BOC-Aminophenyl)-2-(4-äthyl-2,3-dioxo-piperazin-1-carboxamido)-acetamido]-3-cephem-4-carbonsäure- diphenylmethylester werden mit 2,01 g p-Toluolsulfonsäuremonohydrat in 100 ml Acetonitril wie im Beispiel 22 beschrieben umgesetzt. Man erhält das 7β-[(2R)-2-(4-Aminophenyl)-2-(4-äthyl-2,3-dioxo-piperazin-1-carboxamido)-acetamido]-3-cephem-4-carbonsäure-diphenylmethylester-p-toluolsulfonat. IR.: 3500-2500 breit, 1779, 1712, 1681, 1609, 1510 cm⁻¹ (Nujol). UV.: 248 nm ( 21200) in C₂H₅OH.

Beispiel 59: 4,9 g 7ß-[(2R)-2-(4-Aminophenyl)-2-(4-äthyl-2,3-dioxo- piperazin-l-carboxamido)-acetamido]-3-cephem-4-carbonsäure-diphenylmethylester-p-toluolsulfonat werden wie im Beispiel 24 umgesetzt. Man erhält den 7β-{(2R)-2-[4-((2R)-2-BOC-Amino-2-diphenylmethoxy- carbonyläthoxycarbonylamino)-phenyl]-2-(4-äthyl-2,3-dioxo-piperazin- l-carboxamido)-acetamidol-3-cephem-4-carbonsäure-diphenylmethylester. IR.: 3400, 3310, 1787, 1714 (breit), 1691 (Schulter), 1615, 1599, 1511 cm⁻¹ (CH₂Cl₂). UV.: 247 um (32800) in C₂H₅CH.

Beispiel 60: 2,09 g 7β-{(2R)-2-[4-((2R)-2-BOC-Amino-2-diphenylmethoxy- carbonyläthoxycarbonylamino)-phenyl]-2-(4-äthyl-2,3-dioxo-piperazin-1-carboxamido)-acetamido}-3-cephem-4-carbonsäure-diphenylmethylester werden wie im Beispiel 28 beschrieben umgesetzt. Man erhält das 7β-{(2R)-2-[4-((2R)-Amino-2-carboxyäthoxycarbonylamino)-phenyl]-2-(4-äthyl-2,3-dioxo-piperazin-1-carboxamido)-acetamido}-3-cephem-4-carbonsäure-natriumsalz. IR.: 3380, 1771, 1716, 1681, 1613, 1519 cm⁻¹ (Nujol). UV.: 244 nm (19800) in H₂0.

Beispiel 61: 15 g (R,S)-2-(4-BOC-Aminophenyl)-2-(4-äthyl-2,3-dioxo- piperazin-1-carboxamido)-essigsäure und 12,1 g 3-Acetoxymethyl-7ß- amino-3-cephem-4-carbonsäure-diphenylmethylester werden wie im Beispiel 10 beschrieben umgesetzt (3,7 ml Chlorameisensäure- isobutylester; 3,96 ml N-Methylmorpholin, 300 ml Tetrahydrofuran) und aufgearbeitet. Das anfallende Rohprodukt wird an 900 g Kieselgel chromatographiert (Frakionen à 1 Liter).Eluiert werden die Fraktionen 1-12 mit Toluol-Essigester (7:3), die Fraktionen 13 - 18 mit Toluol-Essigester-(3:2), die Fraktionen 19 - 43 mit Toluol-Essigester-(1:1), die späteren Fraktionen mit Essigester. Die Fraktionen 23-27 werden vereinigt, eingedampft und der Eindampfrückstand einmal aus CH₂C1₂-Aether-Hexan umgefällt. Man erhält den 3-Acetoxymethyl-7β-[(2R)-2-(4-BOC-aminophenyl)-2-(4-äthyl-2,3-dioxo-piperazin-1- carboxamido)-acetamido]-3-cephem-4-carbonsäure-diphenylmethylester. [α]_{D}²⁰ = +8°±1°(0,92 % in CHCl₃), IR: 3380, 3270, 1785, 1718, 1690, 1612, 1595, 1515 cm⁻¹ (CH₂Cl₂). UV.: 248 (31800; C₂H₅OH).

Die anschliessenden Fraktionen 28 - 35 bestehen aus einem Gemisch der obigen R-Verbindung und dem entsprechenden S-Derivat, das durch nochmalige analoge Chromatographie weiter aufgetrennt werden kann. Aus den abschliessenden Fraktionen 36 - 48 erhält man nach dem Eindampfen und einmaligem Umfällen aus CH₂Cl₂-Aether-Hexan den 3-Acetoxymethyl-7β-[(2S)-2-(4-BOC-aminophenyl)-2-(4-äthyl-2,3-dioxo- piperazin-1-carboxamido)-acetamido]-3-cephem-4-carbonsäure-diphenylmethylester, 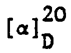 = +22° ± 1° (0,65 in CHC1₃), IR.; 3380, 3270, 1785, 1720, 1692, 1615, 1600, 1515 cm⁻¹ (CH2C12). UV.: 248 (32600; C₂H₅OH).

Beispiel 62: 4,815 g 3-Acetoxymethyl-7ß-[(2R)-2-(4-BOC-aminophenyl)-2-(4-äthyl-2,3-dioxo-piperazin-1-carboxamido)-acetamido]-3-cephem-4-carbonsäurediphenylmethylester werden mit 2,2 g p-Toluolsulfonsäure- monohydrat in 40 ml Acetonitril wie im Beispiel 11 beschrieben umgesetzt und aufgearbeitet. Man erhält das 3-Acetoxymethyl-7ß-[(2R)-2-(4-aminophenyl)-2-(4-äthyl-2,3-dioxopiperazin-1-carboxamido)-acetamido]-3-cephem-4-carbonsäure-diphenylmethylester-p-toluolsulfonat. 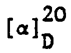 = +15° + 1° (0,94 % in CH OH). IR.: 3480 (Schulter); 3330, 2690, 1790, 1725, 1703, 1691, 1621, 1519 cm⁻¹ (Nujol). UV.: 250 (22000; C₂H₅OH).

Beispiel 63: 3,883 g 3-Acetoxymethyl-7β-[(2S)-2-(4-BOC-aminophenyl)-2-(4-äthyl-2,3-dioxo-piperazin-1-carboxamido)-acetamido]-3-cephem-4-carbonsäurediphenylmethylester werden mit 1,75 g p-Toluolsulfonsäure- monohydrat in 30 ml Acetonitril wie im Beispiel 11 beschrieben umgesetzt und aufgearbeitet. Man erhält das 3-Acetoxymethyl-7ß-[(2S)-2-(4-aminophenyl)-2-(4-äthyl-2,3-dioxo-piperazin-1-carboxamido)-acetamido]-3-cephem-4-carbonsäurediphenylmethylester-p-toluolsulfonat, 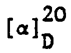 = +32° ± 1° (0,90 % in CH OH). IR.: 3480 (Schulter), 3335, 2650, 1795, 1727, 1685, 1622, 1518 cm⁻¹ (Nujol). UV.: 250 (22000; C₂H₅OH).

Beispiel 64: 4,56 g 3-Acetoxymethyl-7β-[(2R)-2-(4-aminophenyl)-2-(4-äthyl-2,3-dioxo-piperazin-1-carboxamido)-acetamido]-3-cephem-4-carbonsäurediphenylmethylester-p-toluolsulfonat werden mit 3,8 g (2R)-2-BOC-Amino-2-diphenylmethoxycarbonyläthoxycarbonylchlorid und 0,95 ml Pyridin in 135 ml Tetrahydrofuran wie im Beispiel 12 beschrieben umgesetzt und aufgearbeitet. Das anfallende Rohprodukt chromatographiert man an 400 g Kieselgel (Fraktionen à 500 ml) wobei die Fraktionen 1-12 mit Toluol-Essigester-(3:2), die spätem Fraktionen mit Toluol-Essigester-(1:1) eluiert werden. Aus den Fraktionen 9-23 wird nach Umfällen aus CH₂Cl₂-Aether-Hexan der 3-Acetoxymethyl-7β-{(2R)-2-[4-((2R)-2-BOC-amino-2-diphenylmethoxycarbonyl-äthoxycarbonylamino)-phenyl]-2-(4-äthyl-2,3-dioxo-piperazin-1-carboxamido)-acetamido}-3-cephem-4-carbonsäure-diphenylmethylester erhalten. 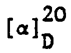 = +8° + 1° (0,82 % in CHC1₃). IR.: 3570, 3400, 1795, 1750 - 1700 (breit), 1620, 1607, 1510 cm⁻¹ (CH₂Cl₂). UV.: 246 (32100; C₂H₅OH).

Beispiel 65: 3,63. g 3-Acetoxymethyl-7ß-[(2S)-2-(4-aminophenyl)-2-(4-äthyl-2,3-dioxo-piperazin-1-carboxamido)-acetamido]-3-cephem-4-carbonsäurediphenylmethylester-p-toluolsulfonat werden mit 3,03 g (2R)-2-BOC-Amino-2-diphenylmethoxycarbonyläthoxycarbonylchlorid und 0,76 ml Pyridin in 100 ml Tetrahydrofuran wie im Beispiel 12 beschrieben umgesetzt und aufgearbeitet. Das Rohprodukt chromatographiert man an 400 g Kieselgel (Fraktionen ä 500 ml) wobei die Fraktionen 1-12 mit Toluol-Essigester-(1:1), die Fraktionen 13-19 mit Toluol-Essigester-(2:3) und die späteren Fraktionen mit Essigester eluiert werden. Aus den Fraktionen 14 - 25 wird nach Umfällen aus CH₂Cl₂-Aether-Hexan 3-Acetoxymethyl-7β-{(2S)-2-[4-((2)R)-2-BOC-amino-2-diphenylmethoxy-carbonyläthoxycarbonylamino)-phenyl]-2-(4-äthyl-2,3-dioxo-piperazin-1-carboxamido)-acetamido}-3-cephem-4-carbonsäure- diphenylmethylester erhalten. 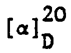 = 19° ± 1° (0,96 % in CHCl₃), IR.: 3570, 3400, 1795, 1750 - 1700 (breit), 1620, 1608, 1510 cm⁻¹(CH₂Cl₂). UV.: 247 (32120; C₂H₅OH).

Beispiel 66: 3,46 g 3-Acetoxymethyl-7β-{(2R)-2-[4-((2R)-2-BOC-amino-2-diphenylmethoxycarbonyläthoxycarbonylamino)-phenyl]-2-(4-äthyl-2,3-dioxo-piperazin-1-carboxamido)-acetamido}-3-cephem-4-carbonsäure-diphenylmethylester werden in 5,9 ml CH₂Cl₂ und 2,15 ml Anisol gelöst und mit 28,5 ml Trifluoressigsäure wie im Beispiel 13 beschrieben umgesetzt und aufgearbeitet. Man erhält das 3-Acetoxymethyl-7β-{(2R)-2-[4-((2R)-2-amino-2-carboxyäthoxycarbonylamino)-phenyl]-2-(4-äthyl-2,3-dioxo-piperazin-1-carboxamido)-acetamido}-3-cephem-4-carbonsäure-natriumsalz.. Zersetzung ab 175°, 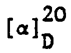 = +58° + 1° (0,82 % in H₂0). IR.: 3340 (breit), 1778, 1730, 1687, 1615, 1540 (breit) cm⁻¹ (Nujol). UV.: 243 (26250; H₂0).

Beispiel 67: 2,27 g 3-Acetoxymethyl-7β-{(2S)-2-[4-((2R)-2-BOC-amino-2-diphenylmethoxycarbonyläthoxycarbonylamino)-phenyl]-2-(4-äthyl-2,3-dioxo-piperazin-1-carboxamido)-acetamido}-3-cephem-4-carbonsäure-diphenylmethylester werden in 3,88 ml CH₂Cl₂ und 1,41 ml Anisol gelöst und mit 18,7 ml Trifluoressigsäure wie im Beispiel 13 beschrieben umgesetzt und aufgearbeitet. Man erhält das 3-Acetoxymethyl-7β-{(2S)-2-[4-((2R)-2-amino-2-carboxyäthoxycarbonylamino)-phenyl]-2-(4-äthyl-2,3-dioxo- piperazin-1-carboxamido)-acetamido}-3-cephem-4-carbonsäure-natriumsalz. Zersetzung ab 175°. 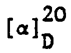 = + 85° + 1° (0,88 % in H₂0). IR.: 3300 (breit), 1770, 1720, 1680, 1612, 1530 (breit) cm⁻¹ (Nujol). _{U}V.: 243 (27900; H₂0).

Beispiel 68: 1,9 g 3-Acetoxymethyl-7β-{(2R)-2-[4-((2R)-2-amino-2-carboxyäthoxycarbonylamino)-phenyl]-2-(4-äthyl-2,3-dioxo-piperazin-1-carboxamido)-acetamido}-3-cephem-4-carbonsäure-natriumsalz werden mit 0,453 g Isonicotinamid und 4,104 g Natriumjodid sowie 0,447 g Trichloressigsäure in 2,73 ml H₂0 während 1 1/2 Std. bei 70° gerührt. Dann kühlt man ab und trägt in 380 ml Aceton ein. Dabei entsteht eine Fällung, die abfiltriert, mit Aceton und Aether gewaschen und anschliessend am Wasserstrahlvakuum bei 50° getrocknet wird. Darauf löst man in 100 ml H₂0 und extrahiert die Wasserphase mit 150 ml Ionentauscher LA₁ (Acetat-Form, flüssig). Die abgetrennte H₂0-Phase wird anschliessend lx mit Hexan und 2x mit Essigester nachextrahiert und schliesslich am Rotavap auf 20 ml Volumen eingeengt. Dann wird das Produkt durch Zugabe von Alkohol gefällt abgenutscht und getrocknet. Zur weiteren Reinigung wird nochmals aus Wasser-Alkohol umgefällt. Man erhält so das 3-(4-Carbamoylpyridinio-methyl)-7ß- {(2R)-2-[4-((2R)-amino-2-carboxyäthoxycarbonylamino)-phenyl]-2-(4-äthyl-2,3-dioxo-piperazin-1-carboxamido)-acetamido}-3-cephem-4-carboxylat. Zersetzung ab 170°. IR.: 3320 (breit), 1778, 1719, 1684, 1619, 1530 (breit) cm⁻¹ (Nujol). UV.: 241 (31800; H₂0).

Beispiel 69: 2,3 g 3-Acetoxymethyl-7β-{(2R)-2-[4-((2R)-2-amino-2-carboxyäthoxycarbonylamino)-phenyl]-2-(4-äthyl-2,3-dioxo-piperazin-1-carboxamido)-acetamido}-3-cephem-4-carbonsäure-natriumsalz und 1,16 g 1-(2-Dimethylaminoäthyl)-5-mercapto-lH-tetrazol werden in 12 ml zweifach destilliertem und entgastem Wasser gelöst. Dann stellt man mit 1N wässriger NaOH auf pH 6,2 und rührt anschliessend unter N₂-Atmosphäre 4 Std bei 65°. Darauf wird abgekühlt, mit 1N HC1 auf pH 5 gestellt und in 600 ml Aethanol eingetragen. Die dabei resultierende Fällung wird abgenutscht, mit Alkohol und Aether gewaschen und getrocknet. Das Rohprodukt trennt man anschliessend an einer Säule aus 100 g silyliertem Kieselgel (Opty-Up C 12; ANTEC AG, CH-4431 Bennwil) wobei Fraktionen à 40 ml genommen werden. Die Fraktionen 1 - 13 eluierte man mit H₂0-Acetonitril-(97:3), die Fraktionen 14 - 20 mit H₂0-Acetonitril-(93:7), die Fraktionen 21-28 mit H₂0-Acetonitril-(9:1) und die späteren Fraktionen mit H₂0-Acetonitril-(4:1). Aus den Fraktionen 33-35 wird nach dem Eindampfen und Umfällen aus H₂0-Aethanol die 3-[1-(2-dimethylaminoäthyl)-1 H-tetrazol-5-yl-thiomethyl]-7β-{(2R)-2-[4-((2R)-2-amino-2-carboxyäthoxy- carbonylamino)-phenyl]-2-(4-äthyl-2,3-dioxo-piperazin-l-carboxamido)₋ acetamido]-3-cephem-4-carbonsäure erhalten. Zersetzung ab 165°. 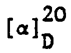 = +34° ± 1° (0,67 % in H₂O), IR.: 3400 (Schulter), 3280 (breit), 1770, 1720, 1680, 1612, 1515 cm⁻¹ (Nujol). UV.: 244 (30800; H₂0).

Beispiel 70: 2 g 3-Acetoxymethyl-7β-{(2R)-2-[4-((2R)-2-amino-2-carboxyäthoxycarbonylamino)-phenyl]-2-(4-äthyl-2,3-dioxo-piperazin-1-carboxamido)-acetamido}-3-cephem-4-carbonsäure-natriumsalz und 0,93 g 1-Carboxymethyl-5-mercapto-1H-tetrazol werden in 10 ml zweifach destilliertem entgastem Wasser wie im Beispiel 69 beschrieben umgesetzt, aufgearbeitet und chromatographisch gereinigt. In Abweichung zum Beispiel 69 werden Fraktionen 1 - 14 mit H₂0 und die späteren Fraktionen mit H₂0-Acetonitril-(97:3) eluiert.

Aus den Fraktionen 12 - 18 wird nach Eindampfen, Lösen des Eindampfrückstandes in 10 ml Wasser und Liophilisation das 3-(1-Carboxymethyl-1H-tetrazol-5-yl-thiomethyl)-7β-{(2R)-2-[4-((2R)-2―amino―2―carboxyäthoxycarbonylamino)-phenyl]-2-(4-äthyl-2,3-dioxo-piperazin-1-carboxamido)-acetamido}-3-cephem-4-carbonsäure-dinatriumsalz erhalten. Zersetzung ab 180°. UV.: 244 (30400; H₂0), NMR.: u.a. 1,30 (t, J=7, NCH₂CH₃); 5,23 (d, J=5, H-C(6)); 5,50 (s,N(H COONa); 5,75 (s, ArCHCONH); 5,92 (d, J=S, H-C(7)); 7,58 (s, 4 arom. H) (CDOOD).

Beispiel 71: 3,3 g 3-Acetoxymethyl-7β-{(2R)-2-[4-((2R)-2-BOC-amino-2-diphenylmethoxycarbonyl-äthoxycarbonylamino)-phenyl]-2-(4-äthyl-2,3-dioxo-piperazin-1-carboxamido)-acetamido}-3-cephem-4-carbonsäure- diphenylmethylester werden in 330 ml abs. Tetrahydrofuran auf -75° abgekühlt. Dann läst man unter Rühren während 30 Min trockenen Stickstoff durch die Lösung. Anschliessend tropft man bei gleicher Temperatur unter Rühren im Verlaufe von 1 Min. in N₂-Atmosphäre eine auf -75° vorgekühlte Lösung von 0,081 g Lithium in 15 ml abs. Methanol zu, rührt 2 Min. nach und gibt 0,2 ml 6-Butylhypochlorit zu. Dann rührt man bei -75° während 33 Min. wobei nach 3 bzw. 18 Min. nochmals 0,2 bzw. 0,1 ml t-Butylhypochlorit zugefügt werden. Anschliessend tropft man nacheinander 3,5 ml Eisessig und 1,5 g Natriumthiosulfat in 2 ml Wasser zu und lässt langsam auf Zimmertemperatur erwärmen. Darauf wird im Vakuum auf ca. 20 ml eingeengt, in Essigester aufgenommen, mit wässrigen Lösungen von NaHC0₃ und NaCl gewaschen, über Na₂S0₄ getrocknet und eingedampft. Das resultierende Rohprodukt chromatographiert man an 400 g Kieselgel (Fraktionen à 500 ml). Dabei werden die Fraktionen 1 - 12 mit Toluol-Essigester-(3:2), die späteren Fraktionen mit Toluol-Essigester-(1:1) eluiert. Aus den Fraktionen 21 - 36 erhält man nach Umfällen aus CH₂C1₂-Aether-Hexan den 3-Acetoxymethyl-7α-methoxy-7β-{(2R)-2-[4-((2R)-2-BOC-amino-2-diphenylmethoxycarbonyl-äthoxycarbonylamino)-phenyl]-2-(4-äthyl-2,3-dioxopiperazin-1-carboxamido)-acetamido}-3-cephem-4-carbonsäure- diphenylmethylester, IR.: 3380, 3250, 1780, 1740 (Schulter), 1715, 1695 (Schulter), 1610, 1595, 1495 cm⁻¹ (CH₂Cl₂).

Beispiel 72: 2,0 g 3-Acetoxymethyl-7α-methoxy-7β-{(2R)-2-[4-((2R)-2-BOC-amino-2-diphenylmethoxycarbonyl-äthoxycarbonylamino)-phenyl]-2-(4-äthyl-2,3-dioxopiperazin-1-carboxamido)-acetamido}-3-cephem-4-carbonsäure-diphenylmethylester werden in 5 ml CH₂C1₂ und 1,3 ml Anisol gelöst und mit 17 ml Trifluoressigsäure wie im Beispiel 13 beschrieben umgesetzt und aufgearbeitet. Man erhält das 3-Acetoxymethyl-7α-methoxy-7β-{(2R)-2-[4-((2R)-2-amino-2-carboxyäthoxycarbonyl- amino)-phenyl]-2-(4-äthyl-'2,3-dioxopiperazin-1-carboxamido)-acetamido}-3-cephem-4-carbonsäure-natriumsalz. Zersetzung ab 185°. 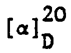 = +62° + 1° (0,69 in H20). IR.: 3320 (breit), 1772, 1730, 1688, 1620, 1520 (breit) cm⁻¹ (Nujol). UV.: 241 (23300; H₂0).

Beispiel 73: 0,8 g 3-Acetoxymethyl-7α-methoxy-7β-{(2R)-2-[4-((2R)-2-amino-2-carboxyäthoxycarbonylamino)-phenyl]-2-(4-äthyl-2,3-dioxo- piperazin-l-carboxamido)-acetamidof-3-cephem-4-carbonsäure-natriumsalz und 0,286 g l-Methyl-5-mercapto-lH-tetrazol-natriumsalz werden in 4 ml zweifach destilliertem und entgastem Wasser gelöst. Dann stellt man mit 1N Salzsäure auf pH 6,2. Darauf wird wie im Beispiel 69 beschrieben umgesetzt (Reaktionsdauer 5 Std.), aufgearbeitet und chromatographiert. In Abweichung zum Beispiel 69 werden Fraktionen ä 20 ml mit H₂0-Acetonitril-(95:5) eluiert. Aus den Fraktionen 8 - 20 erhält man nach dem Eindampfen und Umfällen aus H₂0-Aethanol das 3-[(1-Methyl-1H-tetrazol-5-yl)-thiomethyl]-7α-methoxy-7β-{(2R)-2-[4-((2R)-2-amino-2-carboxyäthoxycarbonylamino)-phenyl]-2-(4-äthyl-2,3-dioxo-piperazin-1-carboxamido)-acetamido}-3-cephem-4-carbonsäurenatriumsalz. Zersetzung ab 195°. 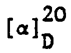 = +31° ± 1° (0,69 in H₂0) IR.: 3450 (Schulter), 3320 (breit), 1775, 1717, 1685, 1618, 1517 cm⁻¹ (Nujol). UV.: 242 (25900; H20).

Beispiel 74: 4,6 g 3-Acetoxymethyl-7ß-[(2R)-2-(4-BOC-aminophenyl)-2-(4-äthyl-2,3-dioxo-piperazin-1-carboxamido)-acetamido]-3-cephem-4-carbonsäure-diphenylmethylester werden in 450 ml Tetrahydrofuran wie im Beispiel 71 beschrieben methoxyliert (0,16 g Lithium in 30 ml Methanol, 0,9 ml t-Butylhypochlorit aufgeteilt in zwei Portionen zu 0,35 und eine dritte Portion zu 0,2 ml). Das anfallende Rohprodukt chromatographiert man an 400 g Kieselgel (Fraktionen à 500 ml). Die Fraktionen 1 - 12 werden mit Toluol-Essigester-(3:2), die späteren Fraktionen mit Toluol-Essigester (1:1) eluiert. Aus den Fraktionen 13 - 18 erhält man nach Kristallisation aus CH₂C1₂-Aether den 3-Acetoxymethyl-7a-methoxy-7ß-[(2R)-2-(4-BOC-aminophenyl)-2-(4-äthyl-2,3-dioxo-piperazin-1-carboxamido)-acetamido]-3-cephem-4-carbonsäure-diphenylmethylester. Zersetzung bei 162°. 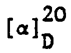 = +34° + 1° (1,00 % in C₂H₅OH). IR.: 3370, 3250, 1780, 1717, 1690, 1612, 1597, 1500 cm⁻¹ (CH₂Cl₂). UV.: 245 (29300; CH₃0H).

Beispiel 75: 2,7 g 3-Acetoxymethyl-7a-methoxy-7ß-[(2R)-2-(4-BOC- aminophenyl)-2(4-äthyl-2,3-dioxo-piperazin-1-carboxamido)-acetamido]-3-cephem-4-carbonsäure-diphenylmethylester werden in 4,9 ml CH₂Cl₂ und 1,7 ml Anisol gelöst mit 20 ml Trifluoressigsäure wie im Beispiel 13 beschrieben umgesetzt und aufgearbeitet. Dabei erhält man das 3-Acetoxymethyl-7a-methoxy-7ß-[(2R)-2-(4-aminophenyl)-2-(4-äthyl-2,3-dioxo-piperazin-l-carboxamido)-acetamido]-3-cephem-4-carbonsäure-natriumsalz. Zersetzung ab 195°. IR.: 3400 (breit), 1773, 1718, 1690, 1618, 1515 cm⁻¹ (Nujol). UV.: 239 (16800; H₂0).

Beispiel 76: 1,0 g 3-Acetoxymethyl-7a-methoxy-7ß-[(2R)-2-(4-aminophenyl)-2-(4-äthyl-2,3-dioxo-piperazin-1-carboxamido)-acetamido]-3-cephem-4-carbonsäure-natriumsalz werden in 25 ml Acetonitril-Wasser (1:1) gelöst und auf 0° abgekühlt. Dann tropft man bei 0° unter Rühren im Verlaufe von 30 Min. eine Lösung von 0,77 g (2R)-2-BOC-Amino-2-diphenylmethoxycarbonyl-äthoxycarbonylchlorid in 10 ml Acetonitril zu wobei das pH der Reaktionslösung durch laufende Zugabe von 0,1 N NaOH (Titrator) bei 7 konstant gehalten wird. Anschliessend wird unter den gleichen Bedingungen 30 Min. nachgerührt. Dann stellt man mit 2N Salzsäure auf pH 3, extrahiert mit Essigester, wäscht mit wässriger NaC1-Lösung neutral, trocknet über Na₂S0₄ und dampft in Vakuum ein.Das anfallende Rohprodukt löst man in 15 ml Methanol und kühlt auf 0° ab. Dann tropft man unter Rühren eine Lösung von 1 g Diphenyldiazomethan in 15 ml CH₂Cl₂ zu (ca. 15 Min.) und rührt anschliessend 3 Stunden bei Zimmertemperatur.

Die Reaktionsmischung wird zur Zerstörung des Ueberschusses an Diphenyldiazomethan mit Essigsäure versetzt, in Essigester aufgenommen,mitwäss- rigen Lösungen von NaHCO₃ und NaCl (bis zum Neutralpunkt) gewaschen, über Na₂SO₄ getrocknet und eingedampft. Das Rohprodukt wird an 200 g Kieselgel auf einer Stufensäule chromatographiert (Fraktionen à 25 ml). Dabei werden die Fraktionen 1-70 mit Toluol-Essigester-(4:1), die spätem Fraktionen mit Toluol-Essigester-(l:l) eluiert. Aus den Fraktionen 160 - 220 erhält man dabei den 3-Acetoxymethyl-7a-methoxy-7β-{(2R)-2-[4-((2R)-2-BOC-amino-2-diphenylmethoxycarbonyl-äthoxy- carbonylamino)-phenyl]-2-(4-äthyl-2,3-dioxo-piperazin-1-carboxamido)-acetamido}-3-cephem-4-carbonsäure-diphenylmethylester der sich im direkten Vergleich (IR., NMR., DS) mit dem im Beispiel 71 beschriebenen Material als identisch erwies.

Beispiel 77: 9,2 g (R,S)-2-(4-BOC-Aminophenyl)-2-(2-imidazolidon-l-carboxamido)-essigsäure und 9,7 g 3-Acetoxymethyl-7ß-amino-3-cephem-4-carbonsäure-diphenylmethylester werden wie im Beispiel 10 beschrieben umgesetzt (3,0 ml Chlorameisensäureisobutylester; 3,1 ml N-Methylmorpholin, 250 ml Tetrahydrofuran) und aufgearbeitet. Das anfallende Rohprodukt wird aus Essigester-Aether umgefällt. Es resultiert der 3-Acetoxymethyl-7ß-[(2R,S)-(4-BOC-aminophenyl)-2-(2-imidazolidon-1-carboxamido)-acetamido]-3-cephem-4-carbonsäure- diphenylmethylester. IR.: 3340, 3300, 1790, 1725, 1704, 1692, 1670, 1620, 1600, 1515 cm⁻¹ (CH₂C1₂). UV.: 246 (29100, C₂H₅0H).

Beispiel 78: 15,2 g 3-Acetoxymethyl-7ß-[(2R,S)-(4-BOC-aminophenyl)-2-(2-imidazolidon-l-carboxamido)-acetamido]-3-cephem-4-carbonsäure- diphenylmethylester werden mit 7,2 g p-Toluolsulfonsäure-monohydrat in 250 ml Acetonitril wie im Beispiel 11 beschrieben umgesetzt und aufgearbeitet. Man erhält das 3-Acetoxymethyl-7ß-[(2R,S)-2-(4-aminophenyl)-2-(2-imidazolidon-1-carboxamido)-acetamido]-3-cephem-4-carbonsäure-diphenylmethylester-p-toluolsulfonat. IR.: 3410, 3330, 2640, 1785, 1728, 1665, 1540 cm⁻¹ (Nujol). UV.: 250 (16600, C₂H₅OH).

Beispiel 79: 15 g 3-Acetoxymethyl-7ß-[(2R,S)-2-(4-aminophenyl)-2-(2-imidazolidon-l-carboxamido)-acetamido]-3-cephem-4-carbonsäure- diphenylmethylester-p-toluolsulfonat werden in 200 ml Tetrahydrofuran zusammen mit 2,02 ml Pyridin und 9,9 g (2R)-2-BOC-Amino-2-diphenyl- methoxycarbonyläthoxy-carbonylchlorid 1 Std. bei 0° gerührt und wie im Beispiel 12 beschrieben aufgearbeitet. Das Rohprodukt chromatographiert man an 400 g Kieselgel wobei Fraktionen a 500 ml genommen werden. Die Fraktionen 1-12 eluiert man mit Toluol-Essigester-(2:1), alle späteren Fraktionen mit Toluol-Essigester (1:1). Aus den Fraktionen 19 - 22 erhält man nach dem Umfällen aus CH₂C1₂-Diäthyläther-Hexan den 3-Acetoxymethyl-7β-{(2R)-2-[4-((2R)-2-BOC-amino-2-diphenylmethoxycarbonyl-äthoxycarbonylamino)-phenyl]-2-(2-imidazolidon-1-carboxamido}-acetamido}-3-cephem-4-carbonsäure- diphenylmethylester. IR.: 3400, 3270, 1785, 1725, 1700 (Schulter) 1770, 1600, 1520 cm⁻¹ (CH₂Cl₂). UV.: 245 (31240, C₂H₅OH).

Die Fraktionen 23 - 27 bestehen aus einem binären Gemisch der obigen Verbindung mit dem entsprechenden 2S-Derivat.

Aus den Fraktionen 28-36 erhält man nach dem Umfällen aus CH₂Cl -Diäthyläther-Hexan den 3-Acetoxymethyl-7β-{(2S)-2-[4-((2R)-2-BOC-amino-2-diphenylmethoxycarbonyläthoxycarbonylamino)-phenyl]-2-(2-imidazolidon-1-carboxamido)-acetamido}-3-cephem-4-carbonsäure- diphenylmethylester. IR.: 3400, 3275, 1790, 1725, 1700 (Schulter), 1772, 1605, 1525 cm⁻¹(Nujol). UV.: 244 (31000, C₂H₅OH).

Beispiel 80: 1,92 g 3-Acetoxymethyl-7β-{(2R)-2-[4-((2R)-2-BOC-amino-2-diphenylmethoxycarbonyl-äthoxycarbonylamino)-phenyl]-2-(2-imidazolidon-1-carboxamido)-acetamido}-3-cephem-4-carbonsäure- diphenylmethylester werden in 190 ml Tetrahydrofuran wie im Beispiel 71 beschrieben methoxyliert (0,049 g Lithium in 8 ml Methanol, 0,34 ml t-Butyl-hypochlorit aufgeteilt in zwei Portionen zu 0,12 und eine dritte Portion zu 0,1 ml). Das anfallende Rohprodukt chromatographiert man an 200 g Kieselgel an einer Stufensäule (Fraktionen zu 20 ml). Die Fraktionen 1-110 eluiert man mit Toluol-Essigester-(4:1), alle späteren Fraktionen mit Toluol-Essigester-(1:1). Aus den Fraktionen 251-340 erhält man nach Umfällen aus CH₂C1₂₋Diäthyläther-Hexan den 3-Acetoxymethyl-7a-methoxy-7β-{(2R)-2-[4-((2R)-2-BOC-amino-2-diphenylmethoxycarbonyl-äthoxycarbonylamino)-phenyl]-2-(2-imidazolidon-1-carboxamido)-acetamido}-3-cephem-4-carbonsäure-diphenylmethylester. IR.: 3400, 3280, 1781, 1725 (breit) mit Schultern bei 1695 und 1685, ₁₆₁₀ (Schulter) ₁₆₀₀, 15₀₅ (breit) cm⁻¹ (CH₂Cl₂). UV: 246 (27900, C₂H₅OH).

Beispiel 81: 2,2 g 3-Acetoxymethyl-7β-{(2S)-2-[4-((2R)-2-BOC-amino-2-diphenylmethoxycarbonyl-äthoxycarbonylamino)-phenyl]-2-(2-imida- zolidon-1-carboxamido)-acetamido}-3-cephem-4-carbonsäure-diphenylmethylester werden in 220 ml Tetrahydrofuran wie im Beispiel 71 beschrieben methoxyliert (0,057 g Lithium in 10 ml Methanol, 0,36 ml t-Butylhypochlorit aufgeteilt in 2 Portionen zu 0,13 und eine dritte Portion zu 0,1 ml). Das anfallende Rohprodukt chromatographiert man 350 g Kieselgel (Fraktionen à 500 ml). Dabei werden die Fraktionen 1-12 mit Toluol-Essigester-(3:2), die Fraktionen 13-36 mit Toluol-Essigester-(1:1) und alle späteren Fraktionen mit Toluol-Essigester-(1:2) eluiert. Aus den Fraktionen 13 - 44 erhält man nach Umfällen aus CH₂C1₂-Diäthyläther-Hexan den 3-Acetoxymethyl-7α-methoxy-7β-{(2S)-2-[4-((2R)-2-BOC-amino-2-diphenylmethoxycarbonyl-äthoxy- carbonylamino)-phenyl]-2-(2-imidazolidon-1-carboxamido)acetamido}-3-cephem-4-carbonsäure-diphenylmethylester. 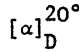 = +58° + 1° (0,842 Z in C₂H₅OH). IR.: 3400, 3275, 1780, 1722 (breit), 1695 (Schulter), 1680 Schulter, 1610 (Schulter), 1600, 1500 (breit) cm (CH₂Cl₂). UV.: 246 (28900; C₂H₅0H).

Beispiel 82: 0,55 g 3-Acetoxymethyl-7α-methoxy-7β-{(2R)-2-[4-((2R)-2-BOC-amino-2-diphenylmethoxycarbonyl-äthoxycarbonylamino)-phenyl-2-(2-imidazolidon-1-carboxamido)-acetamido}-3-cephem-4-carbonsäure-diphenylmethylester werden in lml CH₂C1₂ und 0,4 ml Anisol gelöst und mit 4 ml Trifluoressigsäure wie im Beispiel 13 besehrieben, umgesetzt und aufgearbeitet. Man erhält das 3-Acetoxymethyl-7α-methoxy-7β-{(2R)-2-[4-((2R)-2-amino-2-carboxy-äthoxycarbonyl- amino)-phenyl]-2-(2-imidazolidon-1-carboxamido -acetamido} -3-cephem-4-carbonsäure-natriumsalze.Zersetzung ab 245°. 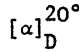 =+73°± 1° (0,848 % in H₂0) IR.: 3300 (breit), 1770, 1730, 1650, 1620, 1535 cm (Nujol), UV.: 243 (21600; H₂0).

Beispiel 83: 1,4 g 3-Acetoxymethyl-7α-methoxy-7β-{(2S)-2-[4-((2R)-2-BOC-amino-2-diphenylmethoxycarbonyl-äthoxycarbonylamino)-phenyl]-2-(2-imidazolidon-1-carboxamide)-acetamido}-3-cephem-4-carbonsäure- diphenylmethylester werden in 3 ml CH₂C1₂ und 1 ml Anisol gelöst und mit 12 ml Trifluoressigsäure wie im Beispiel 13 beschrieben umgesetzt - und aufgearbeitet. Man erhält das 3-Acetoxymethyl-7a-methoxy-7ß- {(2S)-2-[4-((2R)-2-amino-2-carboxy-äthoxycarbonylamino)-phenyl]-2-(2-imidazolidon-1-carboxamido)-acetamido} -3-cephem-4-carbonsäure- natriumsalz. Zersetzung ab 230°. 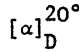 = +110° + 1° (0,715 % in H₂O). IR.: 3300, 1772, 1735, 1658, 1623, 1540 cm⁻¹ (Nujol). UV.: 244 (26400; H₂0).

Beispiel 84: 2 g 3-Acetoxymethyl-7α-methoxy-7β- {(2R)-2-[4-((2R)-2-amino-2-carboxyäthoxycarbonylamino)-phenyl]-2-(4-äthyl-2,3-dioxo-piperazin-1-carboxamido)-acetamido}-3-cephem-4-carbonsäure-natriumsalz werden in 10 ml Wasser mit 1 g 1-Carboxymethyl-5-mercapto-1H-tetrazol wie im Beispiel 70 beschrieben umgesetzt, aufgearbeitet und chromatographisch gereinigt. Man erhält das 3-[(1-Carboxymethyl-1H-tetrazol-5-yl)-thiomethyl]-7α-methoxy-7β-{(2R)-2-[4-((2R)-2-amino-2-carboxyäthoxycarbonyl- amino)-phenyl]-2-(4-äthyl-2,3-dioxo-piperazin-1-carboxamido)-acetamido}-3-cephem-4-carbonsäure-dinatriumsalz. IR.: 3430 (Schulter), 3300, 1770, 1721, 1680, 1615, 1515 (breit) cm⁻¹ (Nujol); UV.: 244 (29700; H₂0).

Beispiel 85: 2,3 g 3-Acetoxymethyl-7α-methoxy-7β-{2R)-2-[4-((2R)-2-amino-2-carboxyäthoxycarbonylamino)-phenyl]-2-(4-äthyl-2,3-dioxo- piperazin-1-carboxamido)-acetamido}-3-cephem-4-carbonsäure-natriumsalz werden in 10 ml Wasser mit 1,1 g 1-Dimethylaminoäthyl-5-mercapto-1H-tetrazol wie im Beispiel 69 beschrieben umgesetzt, aufgearbeitet und chromatographiert. Man erhält die 3-[(1-Dimethylaminoäthyl-lH-tetrazol-5-yl)-thiomethyl]-7α-methoxy-7β-{(2R)-2-[4-((2R)-2-amino-2-carboxy- äthoxycarbonylamino)-phenyl]-2-(4-äthyl-2,3-dioxo-piperazin-1-carboxamido)-acetamido}-3-cephem-4-carbonsäurenatriumsalz. IR.: 3440 (Schulter), 3300, 1768, 1720, 1682, 1617, 1520 (breit) cm (Nujol); UV.: 244 (30 000; C₂H₅0H).

Beispiel 86: Zu einer Lösung von 10 g 3-Amino-acetophenon in 10 ml HCOOH gibt man bei 80° eine auf 80° vorgewärmte Lösung von 5 g HCOONa in 6 ml HCOOH. Nach langsamem Abkühlenlassen wird das Gemisch über Nacht bei Raumtemperatur stehengelassen, dann bei 0° mit 44,5 ml Acetanhydrid versetzt und 4 Std. bei 0° gerührt. Darauf werden 14,4 ml H₂0 zugetropft und im Vakuum eingedampft. Dann nimmt man in Essigester auf, wäscht nacheinander mit gesättigten wässrigen Lösungen von NaHCO₃ und NaCl, trocknet über Na₂S0₄ und dampft im Vakuum ein. Man erhält das N-Formyl-3-amino-acetophenon, das ohne Reinigung weiterverarbeitet wird.
Smp. 96-7°. IR.: 3370, 3300 (Schulter), 2870, 1700, 1680, 1590 cm⁻¹ (CH₂^{C1}₂)_{.}

Beispiel 87: Zu einer siedenden Lösung von 33,8 g LiA1H₄ in 1360 ml THF tropft man im Verlaufe von 2 Std. eine Lösung von 33,8 g N-Formyl-3-amino-acetophenon in 680 ml THF und kocht anschliessend über Nacht am Rückfluss. Dann kühlt man auf 0° ab, zersetzt den Hydridüberschuss durch vorsichtige Zugabe von gesättigter wässriger (NH₄)₂SO₄-Lösung, filtriert durch Celite, wäscht mit CH₂C1₂ nach und dampft das Filtrat im Vakuum ein. Dabei wird m-(N-Methylamino)-1-phenyläthanol erhalten, das roh weiterverarbeitet wird.

Beispiel 88: 34,8 g m-(N-Methylamino)-1-phenyläthanol werden in 600 ml Dioxan-Wasser-(1:1) bei pH 10 (pH konstant gehalten durch Zugabe von 2N NaOH mittels Titrator) mit 52,5 g (BOC)₂0 umgesetzt (Zimmertemperatur, 16 Std.). Dann nimmt man in Essigester auf, wäscht mit gesättigter wässriger NaCl-Lösung neutral, trocknet über Na₂S0₄ und dampft im Vakuum ein. Das anfallende Rohprodukt chromatographiert man an 1,6 g Kieselgel. Aus den Toluol-Essigester-(4:1)-Fraktionen wird m-(N-BOC-N-Methylamino)-1-phenyläthanol erhalten, das direkt weiterverarbeitet wird.
IR.: 3620, 3550, 3400, 1700 (Schulter), 1685, 1605, 1595 (Schulter) cm (CH₂Cl₂).

Beispiel 89: 47,6 g m-(4-BOC-N-Methylamino)-1-phenyläthanol werden bei 0° in 240 ml Aceton während 5 Min. mit 52,7 ml 8N CrO₃ in 8N wässriger H₂SO₄ oxydiert. Dann wird der Cr0₃-Ueberschuss mit Methanol zersetzt, in Essigester aufgenommen, gewaschen mit gesättigten wässrigen Lösungen von NaHCO₃ und NaCl, getrocknet und im Vakuum eingedampft. Das anfallende Rohprodukt chromatographiert man an 750 g Kieselgel. Dabei wird mit Toluol-Essigester-(3:2) N-BOC-3-Methylamino-acetophenon erhalten.
IR.: 1705 (Schulter), 1680, 1600 (Schulter) 1585 cm⁻¹ (CH₂Cl₂).

Beispiel 90: 10 g 3-Amino-acetophenon werden wie im Beispiel 78 beschrieben mit 48,4 g (BOC)₂0 umgesetzt, aufgearbeitet und chromatographiert. Dabei fällt das N-BOC-3-Amino-acetophenon an.
IR.: 3420, 1705, 1680, 1600, 1586 cm⁻¹ (CH₂Cl₂).

Beispiel 91: 45,7 g N-BOC-3-Methylamino-acetophenon werden mit 34 g SeO₂ 17 Std. bei 80° gerührt. Dann filtriert man vom ausgefallenen Se ab, dampft im Vakuum ein, löst in 100 ml Methanol, gibt 100 ml Wasser zu und stellt mit 2N HCl auf pH-2. Darauf extrahiert man mit Essigester, wäscht mit gesättigter NaCl-Lösung neutral, trocknet über Na₂SO₄ und dampft im Vakuum ein. Nach Kristallisation aus Essigester-Methanol resultiert die 2-(3-BOC-Methylaminophenyl)-glyoxylsäure.
Smp. 115⁰ (Zers.). IR.: 3350, 3300-2300 (breit), 1700 (Schulter), 1685, ₁6₀0, 1583 cm⁻¹ (CH₂Cl₂).

Beispiel 92: 15,2 g N-BOC-3-Amino-acetophenon werden mit 12,3 g SeO₂ wie im Beispiel 91 beschrieben umgesetzt und aufgearbeitet. Dabei erhält man die 2-(3-BOC-Aminophenyl)-glyoxylsäure.
IR.: 3350-2300 (breit), 1700, 1685, 1605, 1580 cm⁻¹ (CH₂C1₂).

Beispiel 93: 32,2 g 2-(3-BOC-Methylaminophenyl)-glyoxylsäurewerden mit 13,8 g Hydroxylamin-hydrochlorid wie in Beispiel 4 beschrieben umgesetzt. Dabei resultiert nach Kristallisation aus CH₂Cl₂ CH₃OH-Aether-Hexan die 2-(3-BOC-Methylaminophenyl)-2-hydroxyiminoessigsäure. Smp.135° (Zers.). IR.: 3620, 3600-2300 (breit), 1688, 1600, 1590, (Schulter) cm⁻¹ (CH₂C1₂).

Beispiel 94: 11,7 g 2-(3-BOC-Aminophenyl)-glyoxylsäure werden mit 5 g Hydroxylaminhydrochlorid wie im Beispiel 4 beschrieben umgesetzt. Dabei wird die 2-(3-BOC-aminophenyl)-2-hydroxyiminoessigsäure erhalten. IR.: 3610, 3550-2306 (breit), 1690, 1605, 1595 cm⁻¹ (CH₂C1₂).

Beispiel 95: 25,4 g 2-(3-BOC-Methylaminophenyl)-2-hydroxyimino-essigsäure werden in 300 ml Wasser suspendiert und durch Zugabe von 2N NaOH in Lösung gebracht (pH 7). Dann wird in Gegenwart von 8,6 g 10-% Pd/C erschöpfend hydriert. Darauf filtriert man vom Katalysator ab, stellt mit 2N HCl auf pH 4, engt im Vakuum ein und fällt durch Zugabe von Isopropanol. Dabei resultiert das (R,S)-2-(3-BOC-Methyl- aminophenyl)-glycin, das ohne Reinigung weiter verarbeitet wird. Smp. 135° (Zers.). IR.: 3330-2200, 1705, 1622, 1597, 1536 cm⁻¹ (Nujol).

Beispeil 96: 3,9 g 2-(3-BOC-Aminophenyl)-2-hydroxyiminoessigsäure werden in 150 ml H₂0 in das Na-Salz überführt und erschöpfend hydriert (2 g 10-% Pd/C) wie im Beispiel 95 beschrieben. Dabei resultiert das (R,S)-2-(3-BOC-Aminophenyl)-glycin.
IR.: 3365, 3250-2200, 1700, 1620, 1595, 1535 cm⁻¹ (Nujol).

Beispiel 97: In zum Beispiel 7 analoger Weise wird aus 18,8 g (R,S)-2-(3-BOC-Methylaminophenyl)-glycin und 31,3 g 2,3-Dioxo-4-äthylpiperazin-1-carbonylchlorid die (R,S)-2-(3-BOC-Methylaminophenyl)-2-(4-äthyl-2,3-dioxo-piperazin-1-carboxamido)-essigsäure hergestellt.

Beispiel 98: In zum Beispiel 7 analoger Weise wird aus 2,64 g (R,S)-2-(3-BOC-Aminophenyl)-glycin und 4,46 g 2,3-Dioxo-4-äthylpiperazin-1-carbonylchlorid die (R,S)-2-(3-BOC-Aminophenyl)-2-(4-äthyl-2,3-dioxo- piperazin-l-carboxamido)-essigsäure hergestellt.

Beispiel 99: 6,1 g (R,S)-2-(3-BOC-Methylaminophenyl)-2-(4-äthyl-2,3-dioxo-piperazin-l-carboxamido)-essigsäure und 6,0 g 3-[(1-Methyl-1H-tetrazol-5-yl)-thiomethyl]-7ß-amino-3-cephem-4-carbonsäure-diphenyl- methylester werden wie im Beispiel 10 beschrieben umgesetzt, aufgearbeitet und chromatographiert. Eluiert werden die Fraktionen 1-26 mit Toluol-Essigester-(1:1), die späteren Fraktionen mit Essigester (je 500 ml). Die Fraktionen 16-32 werden vereinigt, eingedampft und der Eindampfrückstand einmal aus CH₂C1₂-Aether-Hexan umgefällt. Man erhält den 3-[(1-Methyl-1H-tetrazo]-5-yl)-thiomethyl]-7β-[(2 R,S)-2-(3-BOC-methylaminophenyl)-2-(4-äthyl-2,3-dioxo-piperazin-l-carboxami- do)-acetamido]-3-cephem-4-carbonsäure-diphenylmethylester. 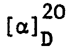 =85° + 1° (0,96 % in CHC1₃). IR.: 3360, 3250, 1785, 1705, 1685, 1602 cm⁻¹(CH₂Cl₂).

Beispiel 100: 3,05 g (R,S)-2-(3-BOC-Aminophenyl)-2-(4-äthyl-2,3-dioxo- piperazin-l-carboxamido)-essigsäure und 3 g3-[(1-Methyl-1H-tetrazol-5-yl)-thiomethyl]-7ß-amino-3-cephem-4-carbonsäure-diphenyl-methylester werden wie im Beispiel 99 beschrieben umgesetzt, aufgearbeitet und chromatographiert. Dabei erhält man nach Umfällen der entsprechenden Eluate den 3-[(1-Methyl-lH-tetrazol-5-yl)-thiomethyl]-7ß-[(2 R,S)-2-(3-BOC-aminophenyl)-2-(4-äthyl-2,3-dioxopiperazin-1-carbox- amido)-acetamido]-3-cephem-4-carbonsäure-diphenylmethylester.
I_{R}.: 3360, 3240, 1780, 1705, 1680, 1600 cm⁻¹ (CH₂Cl₂).

Beispiel 101: 7,5 g 3-[(1-methyl-1H-tetrazol-5-yl-thiomethyl]-7β-[(2 R, S)-2-(3-BOC-methylaminophenyl)-2-(4-äthyl-2,3-dioxo-piperazin-1-carboxamido)-acetamido]-3-cephem-4-carbonsäure-diphenylmethylester werden mit 3,1 g p-Toluolsulfonsäure-monohydrat in 80 ml Acetonitril wie im Beispiel 11 beschrieben umgesetzt und aufgearbeitet. Man erhält das 3-[(1-Methyl-1H-terazol-5-yl)-thiomethyl]-7β-[(2 R,S)-2-(3-methyl- aminophenyl)-2-(4-äthyl-2,3-dioxopiperazin-1-carboxamido)-acetamido]-3-cephem-4-carbonsäure-diphenylmethylester-p-toluolsulfonat. 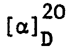 =-24°± 1° (1,00 % in CH₃OH). IR.: 3450 (Schulter), 3320, 1780, 1715, 1685, 1602 cm⁻¹ (Nujol); UV.: 252 (21000; C₂H₅OH).

Beispiel 102: 3,75 g 3-[(1-Methyl-1H-tetrazol-5-yl-thiomathyl]-7β-[(2 R,S)-2-(3-BOC-aminophenyl)-2-(4-äthyl-2,3-dioxo-piperazin-1-carbox- amido)-acetamido]-3-cephem-4-carbonsäure-diphenylmethylester werden mit 1,5 g p-Toluolsulfonsäure-monohydrat in 37 ml Acetonitril wie im Beispiel 11 beschrieben umgesetzt und aufgearbeitet. Man erhält das 3-[(1-Methyl-1H-tetrazol-5-yl)-thiomethyl]-7ß-[(2 R,S)-2-(3-aminophenyl)-2-(4-äthyl-2,3-dioxopiperazin-1-carboxamido)-acetamido]-3-cephem-4-carbonsäure-diphenylmethylester-p-toluolsulfonat.
IR.: 3455 (Schulter), 3320, 1785, 1715, 1690, 1600 cm⁻¹ (Nujol);
_{U}V.: 252 (22 000; C₂H₅0^{H}).

Beipiel 103: 7,1 3-[(1-Methyl-1H-tetrazol-5-yl)-thiomethyl]-7β-[(2 R,S)-2-(3-methylaminophenyl)-2-(4-äthyl-2,3-dioxo-piperazin-1-carboxamido)-acetamido]-3-cephem-4-carbonsäure-diphenylmethylester-p-toluolsulfonat werden mit 5,4 g (2R)-2-BOC-Amino-2-diphenylmethoxy- carbonyläthoxycarbonylchlorid und 1,16 ml Pyridin in 150 ml Tetrahydrofuran wie im Beispiel 12 beschrieben umgesetzt und aufgearbeitet. Das anfallende Rohprodukt chromatographiert man an 600 g Kieselgel wobei Fraktionen à 500 ml genommen werden. Die Fraktionen werden mit Toluol-Essigester-(1:1) eluiert. Aus den Fraktionen 19-33 wird nach Umfällen aus CH₂Cl₂ Aether-Hexan der 3-[(1-Methyl-1H-tetrazol-5-yl)-thiomethyl]-7β-{(2 R,S)-2-[3-((2 R)-2-BOC-amino-2-diphenylmethoxycarbonyl- äthoxycarbonylmethylamino)-phenyl]-2-(4-äthyl-2,3-dioxo-piperazin-1-carboxamido)-acetamido}-3-cephem-4-carbonsäure-diphenylmethylester erhalten. 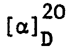
= - 49° + 1° (0,88 % in CHC1₃). IR.: 3370, 3250, 1782, 1720-1680 (breit), 1605 cm⁻¹ (CH₂Cl₂).

Beispiel 104: 7 g 3-[(1-Methyl-1H-tetrazol-5-yl)-thiomethyl]-7β [(2 R,S)-2-(3-aminophenyl)-2-(4-äthyl-2,3-dioxopiperazin-1-carboxamido)-acetamido]-3-cephem-4-carbonsäure-diphenylmethylester-p-toluol-sulfonat werden mit 5,4 g (2R)-2-BOC-Amino-2-diphenylmethoxycarbonyläthoxycarbonyl- chlorid wie in den Beispielen 12 und 103 beschrieben umgesetzt, aufgearbeitet, chromatographiert und ausgefällt. Dabei erhält man den 3-[(1-Methyl-1H-tetrazol-5-yl)-thiomethyl]-7ß- {(2 R,S)-2-[3-((2 R)-2-BOC-amino-2-diphenylmethoxycarbonyläthoxycarbonylamino)-phenyl]-2-(4-äthyl-2,3-dioxo-piperazin-1-carboxamido)-acetamido}-3-cephem-4- carbonsäure-diphenylmethylester.
IR.: 3375, 3225, 1784, 1720-1680 (breit), 1605 cm⁻¹ (CH₂Cl₂).

Beispiel 105: 5,88 g 3-[(1-Methyl-1H-tetrazol-5-yl)-thiomethyl]-7β-{(2 R,S)-2-[3-((2 R)-2-BOC-amino-2-diphenylmethoxycarbonyläthoxy- carbonylmethylamino)-phenyl]-2-(4-äthyl-2,3-dioxo-piperazin-1-carboxamido)-acetamido}-3-cephem-4-carbonsäure-diphenylmethylester werden in 9,3 ml CH₂C1₂ und 3,4 ml Anisol gelöst und mit 44,5 ml CF₃COOH wie im Beispiel 13 beschrieben umgesetzt und aufgearbeitet. Man erhält das 3-[(1-Methyl-1H-tetrazol-5-yl)-thiomethyl]-7ß- {(2 R,S)-2-[3-((2R)-2-amino-2-carboxyäthoxycarbonylmethylamino)-phenyl]-2-(4-äthyl-2,3-dioxopiperazin-1-carboxamido)-acetamido} -3-cepₕem-4-carbonsäure-natriumsalz 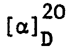 = + 18° + 1° (0,86% in H₂0).
IR.: 3320 (breit), 1770, 1720-1680 (breit), 1620 cm⁻¹ (Nujol).

Beispiel 106: 2,9 g 3-[(1-Methyl-1H-tetrazol-5-yl)-thiomethyl]-7β-{(2 R,S)-2-[3-((2R)-2-BOC-amino-2-diphenylmethoxycarbonyläthoxy- carbonylamino)-phenyl]-2-(4-äthyl-2,3-dioxo-piperazin-1-carboxamido)-acetamido}-3-cephem-4-carbonsäure-diphenylmethylester werden in 4,5 ml CH₂Cl₂ und 1,7 ml Anisol gelöst und mit 22 ml CF₃COOH wie im Beispiel 13 beschrieben umgesetzt und aufgearbeitet. Man erhält das 3-[(1-Methyl-1H-tetrazol-5-yl)-thiomethyl]-7ß- {(2R,S)-2-[3-((2R)-2-amino-2-carboxyäthoxycarbonylamino)-phenyl]-2-(4-äthyl-2,3-dioxo- piperazin-1-carboxamideo)-acetamido}-3-cephem-4-carbonsäure-natriumsalz.
IR.: 3310 (breit), 1772, 1720-1680 (breit), 1620 cm⁻¹ (Nujol).

Analog den vorangehenden Beispielen können, ausgehend von entsprechenden Ausgangsmaterialien und Zwischenprodukten die folgenden Verbindungen, bevorzugt in Form ihrer Natriumsalze, hergestellt werden:

Beispiel 107: 7β-{(2R)-2-[4-((2R)-2-Amino-2-carboxyäthoxycarbonylamino) phenyl]-2-(2-imidazolidon-1-carboxamide)acetamido}-3-acetoxymethyl-3-cephem-4-carbonsäure-natriumsalz,
IR.: 3440, 3300, 1772, 1730-1710 (breit), 1655, 1560, 1537, 1250 cm⁻¹ (Nujol); UV.: 244 (26200) in H₂0.

Beispiel 108: 7β-{(2R)-2-[4-((2R)-2-Amino-2-carboxyäthoxycarbonylamino)-phenyl]-2-(2-imidazolidon-1-carboxamido)acetamido}-3-carbamoyloxy- methyl-3-cephem-4-carbonsäure-natriumsalz,
IR,: 3450-3280 (breit), 1769, 1725, 1660, 1535 cm⁻¹ (Nujol); UV.: 243 (21200) in H₂0.

Beispiel 109: 7β-{(2R)-2-[4-((2R)-2-Amino-2-carboxyäthoxycarbonylamino)-phenyl]-2-(2-imidazolidon-1-carboxamido)acetamido}-3-(4-carbamoyl- pyridinomethyl)-3-cephem-4-carboxylat,
IR.: 3400, 3300, 1770, 1729, 1654, 1560, 1535 cm⁻¹ (Nujol); UV: 244 (21000) in H₂0.

Beispiel 110:7β- {(2R)-2-[4-((2R)-2-Amino-2-carboxyäthoxycarbonylamino)-phenyl]-2-(2-imidazolidon-1-carboxamido)acetamido}-3-(4-methyl-1,2,4-triazo1-3-yl-thiomethyl)-3-cephem-4-carbonsäure-natriumsalz,
IR.: 3445 (Schulter), 3300, 1768, 1728, 1655, 1561, 1538 cm⁻¹ (Nujol); UV.: 244 (19800) in H20.

Beispiel 111: 7β-{(2R)-2-[4-((2R)-2-Amino-2-carboxyäthoxycarbonylamino) phenyl]-2-(2-imidazolidon-1-carboxamido)acetamido}-3-(1-carboxymethyl-1H-tetrazol-5-yl-thiomethyl)-3-cephem-4-carbonsäure-dinatriumsalz,
IR.: 3450, 3300, 1770, 1729, 1656, 1560, 1535 cm⁻¹ (Nujol); UV.: 243 (19720) in H₂0.

Beispiel 112: 7β-{(2R)-2-[4-((2R)-2-Amino-2-carboxyäthoxycarbonylamino) phenyl]-2-(2-imidazolidon-1-carboxamido)acetamido}-3-(1-sulfomethyl-1H-tetrazol-5-yl-thiomethyl)-3-cephem-4-carbonsäure-dinatriumsalz,
IR.: 3455, 3305, 1775, 1730, 1660, 1560, 1538 cm⁻¹ (Nujol); UV.: 244 (20150) in H₂0.

Beispiel 113: 7β-{(2R)-2-[4-((2R)-2-Amino-2-carboxyäthoxycarbonylamino) phenyl]-2-(2-imidazolidon-1-carboxamido)acetamido}-3-(2-methyl-5,6-dioxo 1,4,5,6-tetrahydro-as-triazin-3-yl-thiomethyl)-3-cephem-4-carbonsäuredinatriumsalz,
IR.: 3470-3280(breit),1765, 1730, 1655, 1560, 1535 cm⁻¹ (Nujol) UV.: 242 (24200).

Beispiel 114: 7β-{(2R)-2-[4-((2R)-2-Amino-2-carboxyäthoxycarbonylamino) phenyl]-2-(imidazolidon-1-carboxamido)acetamido}-3-(4-methyl-5,6-dioxo-1,4,5,6-tetrahydro-as-triazin-3-yl-thiomethyl)-3-cephem-4-carbonsäure-dinatriumsalz,
IR.: 3460-3290 (breit), 1766, 1732, 1657, 1561, 1537 cm (_{N}ujol); UV.: 242 (22900) in H₂0.

Beispiel 115: 7β-{(2R)-2-[4-(( 2R)-2-Amino-2-carboxyäthoxycarbonylamino) phenyl]-2-(2-imidazolidon-1-carboxamido)acetamido}-3-(5-methyl-1,3,4-thiadiazol-2-yl-thiomethyl)-3-cephem-4-carbonsäure-natriumsalz,
IR.: 3445 (Schulter), 3300, 1770, 1730, 1655, 1560, 1537 cm⁻¹ (Nujol); UV.: 244 (24700) in H₂O.

Beispiel 116 : 7β-{(2R)-2-[4-((2R)-2-Amino-2-carboxyäthoxycarbonylamino) phenyl]-2-(2-imidazolidon-1-carboxamido)acetamido}-3-(3H-1,2,3-triazol-4-yl-thiomethyl)-3-cephem-4-carbonsäure-natriumsalz,
IR.: 3450 (Schülter), 3310, 1771, 1732, 1657, 1561, 1537 cm⁻¹ UV.: 244 (23300).

Beispiel 117: 7β-{(2R)-2-[4-((2R)-2-Amino-2-carboxyäthoxycarbonylamino) phenyl]-2-(2-imidazolidon-1-carboxamido)acetamido}-3-[1-(2-dimethylaminoäthyl)-1H-tetrazol-5-yl-thiomethyl]-3-cephem-4-carbonsäure-natriumsalz,
IR.: 3450-3290 (breit), 1770, 1730, 1655, 1560, 1535 cm⁻¹ ( UV: 242 (20900) in H₂0.

Beispiel 118: 7ß- {(2R)-2-[4-((2R)-2-Amino-2-carboxyäthoxycarbonylamino) phenyl]-2-(3-methansulfonyl-2-imidazolidon-1-carboxamido)acetamido}-3-acetoxymethyl-3-cephem-4-carbonsäure-natriumsalz,
IR.: 3350, 1770, 1740-1720 (breit), 1662, 1612, 1536, 1383, 1250, 1170 cm⁻¹ (Nujol); UV.: 244 (26000) in H₂0.

Beispiel 119: 7ß- {(2R)-2-[4-((2R)-2-Amino-2-carboxyäthoxycarbonyiamino) phenyl]-2-(3-methansulfonyl-2-imidazolidon-1-carboxamido)acetamido}-3-carbamoyloxymethyl-3-cephem-4-carbonsäure-natriumsalz,
. IR.: 3350-3300 (breit), 1768, 1738, 1661, 1611, 1535, 1383, 1170 cm⁻¹ (Nujol); UV.: 243 (24900) in H₂0.

Beispiel 120: 7β-{(2R)-2-[4-((2R)-2-Amino-2-carboxyäthoxycarbonylamino) phenyl]-2-(3-methansulfonyl-2-imidazolidon-1-carboxamido)acetamido}-3-(4-carbamoylpyridiniomethyl)-3-cephem-4-carboxylat,
IR.: 3350-3290 (breit) 1769, 1740, 1660, 1612, 1534, 1383, 1170 cm⁻¹ (Nujol); UV.: 244 (21110) in H₂0.

Beispiel 121: 7β-{(2R)-2-[4-((2R)-2-Amino-2-carboxyäthoxycarbonylamino) phenyl]-2-(3-methansulfonyl-2-imidazolidon-1-carboxamido)acetamido}-3-(4-methyl-1,2,4-triazol-3-yl-thiomethyl)-3-cephem-4-carbonsäure-natriumsalz,
IR.: 3350, 1771, 1742, 1663, 1613, 1538, 1385, 1174 cm⁻¹ (Nujol); UV.: 244 (25900) in H₂O.

Beispiel 122: 7β-{(2R)-2-[4-((2R)-2-Amino-2-carboxyäthoxycarbonylamino) phenyl]-2-(3-methansulfonyl-2-imidazolidon-1-carboxamido)acetamido}-3-(5-methyl-1,3,4-thiadiazol-2-yl-thiomethyl)-3-cephem-4-carbonsäure-natriumsalz,
IR.: 3349, 1769, 1739, 1660, 1610, 1534, 1381, ₁₁₆₉ cm⁻¹ (Nujo1); _{UV}.: 244 (25700) in H₂O.

Beispiel 123: 7β-{(2R)-2-[4-((2R)-2-Amino-2-carboxyäthoxycarbonylamino) phenyl]-2-(3-methansulfonyl-2-imidazolidon-1-carboxamido)acetamido}-3-(3H-1,2,3-triazol-4-yl-thiomethyl)-3-cephem-4-carbonsäure-natriumsalz,
IR.: 3350, 1770, 1741, 1661, 1611, 1536, 1383, 1171 cm⁻¹ (Nujol); UV.: 244 (25600) in H20.

Beispiel 124: 7β-{(2R)-2-[4-((2R)-2-Amino-2-carboxyäthoxycarbonyl- amino)phenyl]-2-(3-methansulfonyl-2-imidazolidon-1-carboxamido) acetamido}-3-[1-(2-dimethylaminoäthyl)-1H-tetrazol-5-yl-thiomethyl]-3-cephem-4-carbonsäure-natriumsalz,
- IR.: 3340 (breit), 1771, 1739, 1660, 1610, 1536, 1382, 1170 cm⁻¹ (Nujol); UV.: 244 (23200) in H₂0.

Beispiel 125: 7β-{(2R)-2-[4-((2R)-2-Amino-2-carboxyäthoxycarbonylamino) phenyl]-2-(3-methansulfonyl-2-imidazolidon-1-carboxamido)acetamido}-3-(1-carboxymethyl-1H-tetrazol-5-yl-thiomethyl)-3-cephem-4-carbonsäure-dinatriumsalz,
IR.: 3350 (breit), 1770, 1741, 1663, 1613, 1537, 1385, 1172 cm⁻¹ (Nujol); UV.: 243 (22900) in H₂0.

Beispiel 126:7β-{(2R)-2-[4-((2R)-2-Amino-2-carboxyäthoxycarbonylamino) phenyl]-2-(3-methansulfonyl-2-imidazolidon-1-carboxamido)acetamido}-. 3-(1-sulfomethyl-1H-tetrazol-5-yl-thiomethyl)-3-cephem--4-carbonsäure-dinatriumsalz,
IR.: 3350, 1770, 1740, 1662, 1610, 1535, 1382, 1170 cm⁻¹ (_{N}ujol); UV.: 244 (24400) in H₂0.

Beispiel 127: 7β-{(2R)-2-[4-((2R)-2-Amino-2-carboxyäthoxycarbonylamio) phenyl]-2-(3-methansulfonyl-2-imidazolidon-1-carboxamido)acetamido}-3-(2-methyl-5,6-dioxo-1,4,5,6-tetrahydro-as-triazin-3-yl-thiomethyl)-3-cephem-4-carbonsäure-dinatriumsalz,
IR.:3340 (breit), 1770, 1740, 1660, 1610, 1535, 1381, 117₀ cm⁻¹ (Nujol); UV.: 244 (27200) in H₂0.

Beispiel 128: 7β-{(2R)-2-[4-((2R)-2-Amino-2-carboxyäthoxycarbonylamino) phenyl]-2-(3-methansulfonyl-2-imidazolidon-1-carboxamido)acetamido}-3-(4-methyl-5,6-dioxo-1,4,5,6-tetrahydro-as-triazin-3-yl-thiomethyl)-3-cephem-4-carbonsäure-dinatriumsalz,
IR.: 3360 (breit), 1775, 1745, 1665, 1615, 1540, 1385, 1174 cm⁻¹ (Nujol); UV.: 244 (26900) in H₂0.

Beispiel 129: 7β-{(2R)-2-[4-((2R)-2-Amino-2-carboxyäthoxycarbonylamino) phenyl]-2-(4-äthyl-2,3-dioxo-piperazin-1-carboxamido)acetamido}-3-(4-methyl-1,2,4-triazol-3-yl-thiomethyl)-3-cephem-4-carbonsäure-natriumsalz,
IR.: 3440 (Schulter), 3300, 1770, 1720, 1682, 1615, 1520 cm⁻¹ (Nujol); UV.: 244 (29100) in H₂0.

Beispiel 130: 7β-{(2R)-2-[4-((2R)-2-Amino-2-carboxyäthoxycarbonylamino) phenyl]-2-(4-äthyl-2,3-dioxo-piperazin-1-carboxamido)acetamido)-3-(5-methyl-1,3,4-thiadiazol-2-yl-thiomethyl)-3-cephem-4-carbansäure- natriumsalz,
IR.: 3440, 3310, 1772, 1721, 1685, 1617, 1522 cm⁻¹ (Nujol); UV.: 244 (28900) in H₂0.

Beispiel 131: 7β-{(2R)-2-[4-((2R)-2-Amino-2-carboxyäthoxycarbonylamino) phenyl]-2-(4-äthyl-2,3-dioxo-piperazin-1-carboxamido)acetamido}-3-(3H-1,2,3-triazol-4-yl-thiomethyl)-3-cephem-4-carbonsäure-natriumsalz,
IR.: 3445, 3312, 1775, 1723, 1686, 1619, 1525 cm⁻¹ (Nujol); UV.: 244 (29500) in H₂0.

Beispiel 132: 7β-{(2R)-2-[4-((2R)-2-Amino-2-carboxyäthoxycarbonylamino) phenyl]-2-(4-äthyl-2,3-dioxo-piperazin-1-carboxamido)acetamido}-3-(1-sulfomethyl-1H-tetrazol-5-yl-thiomethyl)-3-cephem-4-carbonsäure-dinatriumsalz,
IR.: 3450-3300 (breit), 1771, 1722, 1684, 1617, 1521 cm⁻¹, (_{Nujol}); UV.: 244 (26500) in H₂0.

Beispiel 133: 7β-{(2R)-2-[4-((2R)-2-Amino-2-carboxyäthoxycarbonylamino) phenyl]-2-(4-äthyl-2,3-dioxo-piperazin-1-carboxamido)acetamido}-3- (2-methyl-5,6-dioxo-1,4,5,6-tetrahydro-as-triazin-3-yl-thiomethyl)-3-cephem-4-carbonsäure-diaatriumsalz,
IR.: 3450-3300 (breit) 1770, 1719, 1680, 1613, 1520 cm⁻¹ (Nujol); UV.: 244 (32100) in H20.

Beispiel 134: 7β-{(2R)-2-(4-((2R)-2-Amino-2-carboxyäthoxycarbonylamino) phenyl]-2-[4-äthyl-2,3-dioxo-piperazin-1-carboxamido)acetamido]-3-(4-methyl-5,6-dioxo-1,4,5,6-tetrahydro-as-triazin-3-yl-thiomethyl)-3 -cephem-4-carbonsäure-dinatriumsalz,
IR: 3450-3300 (breit) 1770, 1720, 1681, 1614, 1521 cm (Nujol); UV.: 244 (31900) in H₂0.

Beispiel 135: Trockenampullen oder Vials, enthaltend 0,5 g {7β- (2R)-2-[4-((2R)-2-Amino-2-carboxyäthoxycarbonylamino)-phenyl]-2-(4-äthyl-2,3- dioso-piperazin-1-carboxamido)-acetamido}-3-[(1-methyl-1H-tetrazol-5-yl)-thiomethyl]-3-cephem-4-carbonsäure-natriumsalz werden wie folgt hergestellt:
7β-{(2R)-2-(4-((2R)-2-Amino-2-carboxyäthoxycarbonylamino)-phenyl]-2-(4-äthyl-2,3-dioxo-piperazin-1-carboxamido)-acetamido}-3-[(1-methyl-1H-tetrazol-5-yl)-thiomethyl]-3-cephem-4-carbonsäure-natriumsalz 0,5 g Mannit 0,05 g

Eine sterile wässrige Lösung von 7β-{(2R)-2-[4-((2R)-2-Amino-2-carboxy- äthoxycarbonylamino)-phenyl]-2-(4-äthyl-2,3-dioxo-piperazin-l-carbox- amido)-acetamido}-3-[(1-methyl-1H-tetrazol-5-yl)-thiomethyl]-3-cephem-4-carbonsäure-natriumsalz und des Mannits wird unter aseptischen Bedingungen in 5 ml-Ampullen oder 5 ml-Vials verschlossen und geprüft.

Beispiel 136: Trockenampullen oder Vials, enthaltend 0,5 g 7ß-[(2R)-2-(4-Aminophenyl)-2-(4-äthyl-2,3-dioxo-piperazin-1-carboxamido)-acetamido]-3-[(l-methyl-lH-tetrazol-5-yl)-thiomethyl]-3-cephem-4-carbon- säurenatriumsalz werden wie folgt hergestellt:
Zusammensetzung (für 1 Ampulle oder Vial) 7ß-[(2R)-2-(4-Aminophenyl)-2-(4-äthyl-2,3-dioxo-piperazin-1-carboxamido)-acetamido]-3-[(1-methyl-1H-tetrazol-5-yl)-thiomethyl]-3-cephem-4-carbonsäurenatriumsalz 0,5 g Mannit 0,05 g

Eine sterile wassrige Lösung von 7β-[(2R)-2-(4-Aminophenyl)-2-(4-äthyl-2,3-dioxo-piperazin-1-carboxamido)-acetamido]-3-[(1-methyl-1H-tetrazol-5-yl)-thiomethyl]-3-cephem-4-carbonsäurenatriumsalz und des Mannits wird unter aseptischen Bedingungen in 5 ml-Ampullen oder 5 ml-Vials verschlossen und geprüft.

## Patentansprüche

1. Verbindungen der Formel 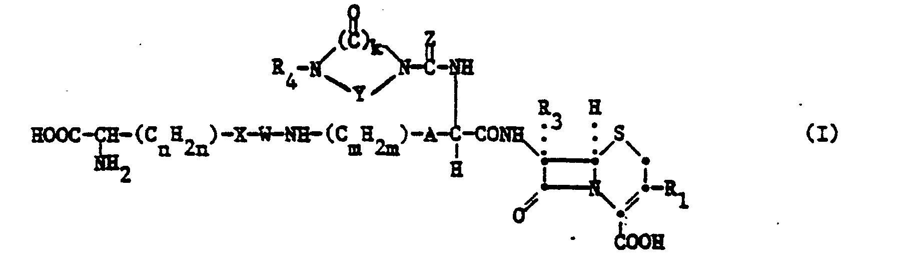 worin der Index n eine ganze Zahl von 1 bis 4, der Index m 0 oder 1, X Sauerstoff, Schwefel oder die Gruppe -NH-, W eine Gruppe -CO-, -CO-NHSO₂- oder -SO₂NH-CO-, oder X-W zusammen eine Gruppe -CO- oder -CO-NHSO₂-, A gegebenenfalls substituiertes Phenylen, Thienylen oder Furylen, Z Sauerstoff oder Schwefel, Y Niederalkylen, der Index k den Wert 1 oder 2, R4 Wasserstoff, einen gegebenenfalls substituierten niederaliphatischen oder cycloaliphatischen Rest oder Acyl, R₁ Wasserstoff, Niederalkyl, Niederalkoxy, Halogen oder eine Gruppe der Formel -CH₂-R₂, worin R₂ eine freie, veresterte oder verätherte Hydroxy- oder Mercaptogruppe oder eine quaternäre Ammoniumgruppe darstellt, und R₃ Wasserstoff oder Methoxy bedeuten, worin die Carboxylgruppen gegebenenfalls in physiologisch spaltbarer Form verestert sind, und Salze von solchen Verbindungen mit salzbildendenden Gruppen.

2. Verbindungen der Formel I, nach Patentanspruch 1, worin die Gruppe -(CₙH₂ₙ)- unverzweigt ist und die Indices n, m, und k die angegebenen Bedeutungen haben , X Sauerstoff oder die Gruppe -NH-, W eine Gruppe -CO- oder -CO-NHSO₂-, oder X-W zusammen eine Gruppe -CO- oder -CO-NHSO₂- bedeuten, A p-, o- oder m-Phenylen, 2,5-Thienylen oder 2,5-Furylen darstellt, Y 1,2-Aethylen, R₄ Wasserstoff, gegebenenfalls durch Hydroxy. Niederalkanoyloxy, Niederalkoxycarbonyloxy, Phenoxycarbonyloxy, Niederalkoxy, Niederalkylthio, Halogen, gegebenenfalls funktionell abgewandeltes Carboxy, oder gegebenenfalls mono- oder diniederalkyliertes Amino substituiertes Niederalkyl sovie Niederalkenyl oder Acyl, Z Sauerstoff oder Schwefel, R₁ Wasserstoff, Niederalkyl, Niederalkoxy, Halogen oder eine Gruppe der Formel -CH₂-R₂, worin R₂ Niederalkanoyloxy, Carbamoyloxy, N-Niederalkylcarbamoyloxy, Triazolylthio, Tetrazolylthio, Thiazolylthio, Thiatriazolylthio, Thiadiazolylthio, Oxazolylthio, Oxadiazolylthio, 5,6-Dioxo-tetrahydro-as-triazinylthio oder Pyridinio darstellt, worin die heterocyclischen Ringe gegebenenfalls beispielsweise durch Niederalkyl, N,N-Diniederalkylaminoniederalkyl, Carboxyniederalkyl, Sulfoniederalkyl, Amino, Carboxyniederalkanoylamino oder Carbamoyl substituiert sein können, und R₃ Wassesrstoff oder Methoxy bedeuten, und pharmazeutisch verwendbare Salze von solchen Verbindungen.

3. Verbindungen der Formel I, nach Patentanspruch 1, worin die Gruppe -(CₙH₂ₙ)- unverzweigt ist und die Indices n, m und k die angegebene Bedeutung haben, X Sauerstoff oder die Gruppe -NH- bedeutet, W eine Gruppe -CO- oder -CONHSO₂-, oder X-W zusammen eine Gruppe -CO- oder -CONHSO₂- bedeuten, A p-, o- oder m-Phenylen, oder, wenn m 1 ist, auch 2,5-Thienylen oder 2,5-Furylen darstellt, Y 1,2-Aethylen, R₄ Wasserstoff, gegebenenfalls durch Hydroxy, Niederalkanoyloxy, Niederalkoxycarbonyloxy, Niederalkoxy, Niederalkylthio, Halogen, z.B. Chlor, Carboxy, Amino oder Diniederalkylamino substituiertes Niederalkyl, sowie Niederalkenyl oder gegebenenfalls durch Halogen, z.B. Chlor, Niederalkoxy, Niederalkanoyloxy, Cyan oder Phenoxy substituiertes Niederalkanoyl, gegebenenfalls durch Niederalkyl, Hydroxy, Niederalkoxy oder Chlor substituiertes Benzoyl, einen gegebenenfalls durch Hydroxy oder Chlor substituierten Pyridin-, Pyrimidin- oder Pyrazincarbonsäurerest, Niederalkoxycarbonyl, gegebenenfalls durch Halogen, z.B. Chlor oder Niederalkyl substituiertes Aryl-, wie Benzol-oder Naphthalinsulfanyl, gegebenenfalls am N-Atom niederalkyliertes Carbamoyl, Thiocarbamoyl, Iminocarbamoyl (-Guanyl) oder Sulfamoyl, Z Sauerstoff, R₁ Wasserstoff, Methyl, Methoxy, Chlor oder eine Gruppe der Formel -CH₂-R₂, worin R₂ Acetoxy, Carbamoyloxy, gegebenenfalls durch Methyl, Carboxymethyl, Sulfomethyl oder Dimethylaminoäthyl substituiertes Tetrazolylthio, z.B. Tetiazol-5-ylthio, 1-Methyl-1H-tetrazol-5-ylthio, 1-Sulfomethyl-1H-tetrazol-5-ylthio, 1-Carboxymethyl- lH-tetrazol-5-ylthio, oder 1-(2-Dimethylaminoäthyl)-1H-tetrazol-5- ylthio, Thiadiazolylthio, insbesondere 2-Methyl-1,3,4-thiadiazol-5- ylthio, durch Methyl substituiertes 5,6-Dioxo-tetrahydro-as-triazinylthio, z.B. 2-Methyl-5,6-dioxo-1,2,5,6-tetrahydro-as-triazin-3-ylthio oder 4-Methyl-5,6-dioxo-1,4,5,6-tetrahydro-as-triazin-3-ylthio, oder Carbamoylpyridinio, insbesondere 4-Carbamoylpyridinio, darstellt, und R₃ Wasserstoff oder Methoxy bedeuten, und pharmazeutisch verwendbare Salze von solchen Verbindungen.

4. Verbindungen der Formel I, nach Patentanspruch 1, worin die Gruppe -(CₙH₂ₙ)-Methylen bedeutet, die Indices m und k die angegebene Bedeutung haben, X Sauerstoff und W eine Gruppe -CO-, A p- oder o-Phenylen, Y 1,2-Aethylen, R₄ Wasserstoff, Nieddralkyl, insbesondere Aethyl, - oder Niederalkylsulfonyl, insbesondere Mesyl, Z Sauerstoff, R₁ Wasserstoff, Methyl, Methoxy, Chlor oder eine Gruppe der Formel -CH₂-R₂, worin R₂ Acetoxy, Carbamoyloxy, gegebenenfalls durch Methyl, Carboxymethyl, Sulfomethyl oder Dimethylaminoäthyl substituiertes Tetrazolylthio, insbesondere 1-Methyl-1H-tetrazol-5-ylthio, 1-Sulfomethyl-1H-tetrazol-5-ylthio, 1-Carboxymethyl-1H-tetrazol-5-ylthio, oder 1-(2-Dimethylaminoäthyl)-1H-tetrazol-5-ylthio, Thiadiazolylthio, insbesondere 2-Methyl-1,3,4-thiadiazol-5-ylthio, durch Methyl substituiertes 5,6-Dioxo-tetrahydro-as-triazinylthio, z.B. 2-Methyl-5,6-dioxo-1,2,5,6-tetrahydro-as-triazin-3-ylthio oder 4-Methyl-5,6-dioxo-1,4,5,6-tetrahydro-as-triazin-3-ylthio, oder Carbamoylpyridinio, insbesondere 4-Carbamoylpyridinio, darstellt, und R₃ Wasserstoff oder Methoxy bedeuten, und pharmazeutisch verwendbare Salze von solchen Verbindungen.

5. 3-[(1-Methyl-1H-tetrazol-5-yl)-thiomethyl]-7β-(2R)-2-[4-((2R)-2-Amino-2-carboxy-äthoxycarbonylamino)-phenyl]-2-(2-imidazolidon-1-carboxamido)-acetamido}-3-cephem-4-carbonsäure und pharmazeutisch annehmbare Salze davon, nach Patentanspruch 1.

6. 3-[ (1-Methyl-1H-tetrazol-5-yl)-thiomethyl]-7β-{(2R)-2-[4-((2R) - 2-amino-2-carboxyäthoxycarbonylamino)-phenyl]-2-(3-methansulfonyl- amino-2-imidazolidon-1-carboxamido)-acetamido}-3-cephem-4-carbonsäure und pharmazeutisch annehmbare Salze davon, nach Patentanspruch 1.

7. 3-[(1-Methyl-1H-tetrazol-5-yl)-thiomethyl]-7β-{(2S)-2-[4-((2R)-2-mino-2-carboxyäthoxycarbonylamino)-phenyl]-2-(3-methansulfonyl-2-imidazolidon-1-carboxamido)-acetamido}-3-cephem-4-carbonsäure und pharmazeutisch annehmbare Salze davon, nach Patentanspruch 1.

8. 3-[(1-Methyl-1H-tetrazol-5-yl-thiomethyl]-7β-{(2R)-2-[4-((2R)-2-amino-2-carboxyäthoxycarbonylamino)-phenyl]-2-(4-äthyl-2,3-dioxo- piperazin-1-carboxamido)-acetamido}-3-cephem-4-carbonsäure und pharmazeutisch annehmbare Salze davon, nach Patentanspruch 1.

9. 3-[(1-Methyl-1H-tetrazol-5-yl-thiomethyl]-7β-{(2S)-[4-((2R)-2-amino-2-carboxyäthoxycarbonylamino)-phenyl]-2-(4-äthyl-2,3-dioxo- piperazin-1-carboxamido)-acetamido}-3-cephem-4-carbonsäure und pharmazeutisch annehmbare Salze davon, nach Patentanspruch 1.

10. 3-[(1-Methyl-1H-tetrazol-5-yl)-thiomethyl]-7β-{(2R,S)-2-[4-((2R)-2-amino-2-carboxyäthoxycarbonylamino)-phenyl]-2-(3-methansulfonyl-2-imidazolidon-1-carboxamido)-acetamido}-3-cephem-4-carbonsäure und pharmazeutisch annehmbare Salze davon, nach Patentanspruch 1.

11. 7β-{(2R)-2-[4-((2R)-Amino-2-carboxyäthyloxycarbonylaminomethyl)-phenyl]-2-(4-äthyl-2,3-dioxopiperazin-1-carboxamido)-acetamido}-3-[(1-methyl-1H-tetrazol-5-yl)-thiomethyl]-3-cephem-4-carbonsäure und pharmazeutisch annehmbare Salze davon, nach Patentan spruch 1.

12. 7β-{(2S)-2-(4-((2R)-2-Amino-2-carboxyäthoxycarbonylaminomethyl)-phenyl]-2-(4-äthyl-2,3-dioxo-piperazin-1-carboxamido)-acetamido}-3-[(1-methyl-1H-tetrazol-5-yl)-thiomethyl]-3-caphem-4-carbonsäure und pharmazeutisch annehmbare Salze davon, nach Patentanspruch 1.

13. 7β-{(2R)-2-[4-((2R)-2-Amino-2-carboxyäthoxycarbonylamino)-phenyl]-2-(4-äthyl-2,3-dioxo-piperazin-1-carboxamido)-acetamido}-3-carbamoyloxymethyl-3-cephem-4-carbonsäre und pharmazeutisch annehmbare Salze davon, nach Patentanspruch 1.

14. 7β-{(2S)-2-[4-((2R)-2-Amino-2-carboxyäthoxycarbonylamino)-phenyl]-2-(4-äthyl-2,3-dioxo-piperazin-1-carboxamido)-acetamido}-3-carbamoyloxymethyl-3-cephem-4-carbonsäure und pharmazeutisch annehmbare Salze davon, nach Patentanspruch 1.

15. 7β-{(2R)-2-[4-((2R)-2-Amino-2-carboxyäthoxycarbonylamino)- phenyl -2-(4-äthyl-2,3-dioxo-piperazin-1-carboxy-amido)-acetamido}-3-[1-(2-dimethylaminoäthyl)-1H-tetrazol-5-ylthiomethyl]-3-cephem-4-carbonsäure und pharmazeutisch annehmbare Salze davon, nach Patentanspruch 1.

16. Dia Natriumsalze der Vezbindungen gemäss einem der Patentansprüche 1-15.

17. Pharmazeutisch verwendbare Präparate enthaltend Verbindungen der Formel I gemäss Patentanspruch 1 oder pharmazeutsich verwendbare Salze von solchem Verbindungen mit salzbildenden Gruppen.

18. Verwendung von Verbindungen der Formel I gemäss Patentanspruch 1 und pharmazeutisch verwendbaren Salzen von solchen Verbindungen mit salzbildenden Gruppen zur Herstellung von pharmazeutischen Präparaten.

19. Verbindungen der Formel I gemäss Patentanspruch 1 und pharmazeutisch verwendbare Salze von solchen Verbindungen mit salzbildenden Gruppen zur Bekämpfung von bakteriellen Infektionen.

20. Verfahren zur Herstellung von Verbindungen der Formel I gemäss Patentanspruch 1 und Salzen von solchen Verbindungen mit salzbildenden Gruppen, dadurch gekennzeichnet, dass man in einer der Formel I entsprechenden Ausgangsverbindung, worin mindestens eine der vorhandenen funktionellen Gruppen geschützt ist, die funktionelle(n) Gruppe(n) freisetzt, wenn erwünscht, in einer erhaltenen Verbindung eine Gruppe R₁ in eine andere Gruppe R₁ überfuhrt, und/oder, wenn erwünscht, eine freie Carboxylgruppe in eine physiologisch spaltbare veresterte Carboxylgruppe überführt, und/oder, wenn erwünscht, ein erhaltenes Isomerengemisch in die einzelnen Isomeren auftrennt, und/oder, wenn erwünscht, eine erhaltene Verbindung in ein Salz oder ein erhaltenes Salz in eine freie Verbindung oder in ein anderes Salz überführt.

21. Verfahren nach Patentanspruch 20, dadurch gekennzeichnet, dass man geschützte funktionelle Gruppen durch Solvolyse oder Reduktion freisetzt.

22. Verbindungen der Formel I, nach Patentanspruch 1, worin mindestens eine der funktionellen Gruppen in geschützter Form vorliegt.

23. Verfahren zur Herstellung von Verbindungen der Formel I gemäss Patentanspruch 1, worin mindestens eine der funktionellen Gruppen in geschützter Form vorliegt, dadurch gekennzeichnet, dass man
a) in einer Verbindung der Formel 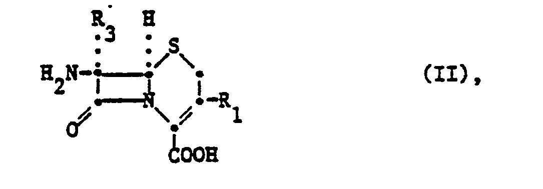 worin die Aminogruppe gegebenenfalls durch eine die Acylierung erlaubende Gruppe substituiert ist, und worin die 4-Carboxylgruppe und gegebenenfalls im Rest R₁ vorhandene weitere funktionelle Gruppen in gesctützter Form vorliegen können, die Aminogruppe durch Behandeln mit einer Säure der Formel 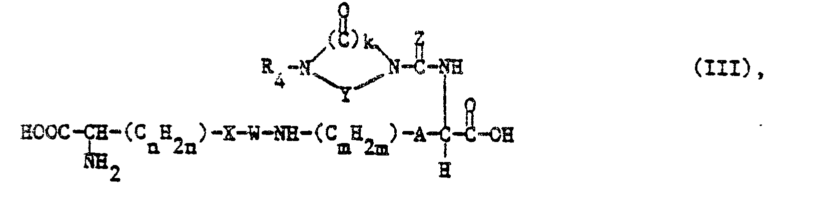 worin die Aminocarbonsäuregruppierung HOOC-CH(NH₂)- und gegebenenfalls in dan Gruppierungen A und R₄ vorhandene weitere funktionelle Gruppen in geschützter Form vorliegen, oder mit einem reaktionsfähigen funktionellen Säurederivat oder einem Salz davon acyliert, oder
b) in einer Verbindung der Formel 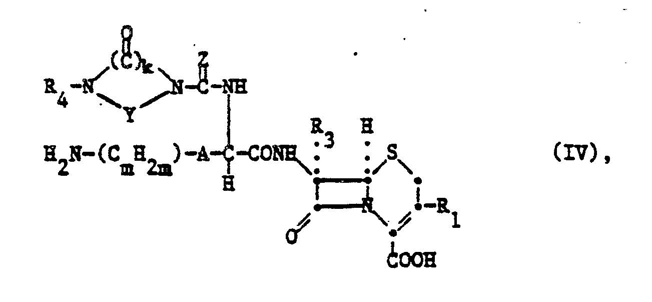 worin die Aminogruppe gegebenenfalls durch eine, die Acylierung erlaubende Gruppe substituiert sein kann, und worin die 4-Carboxylgruppe und gegebenenfalls im R₁ und in den Gruppierungen A und R₄ vorhandene weitere funktionelle Gruppen in geschützter Form vorliegen können, die Aminogruppe durch Behandeln mit einem reaktionsfähigen funktionellen Derivat einer Säure der Formel 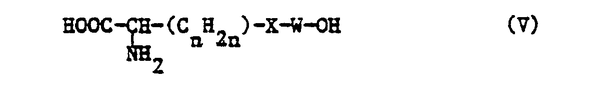 worin die Aminocarbonsäuregruppierung HOOC-CH(NH₂)- in geschützter Form vorliegt, oder wenn X-W zusammen eine Gruppe -CO- bedeuten, auch mit einer entsprechenden freien Säure, oder mit einem Salz davon, acyliert, oder
c) in einer Verbindung der Formel 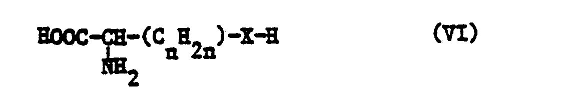 worin X Sauerstoff, Schwefel oder die Gruppe -NH- bedeutet, und worin die Aminocarbonsäuregruppierung HOOC-CH(NH₂)- in geschützter Form vorliegt, die Gruppe -X-H mit einem reaktionsfähigen funktionellen Derivat einer Verbindung der Formel 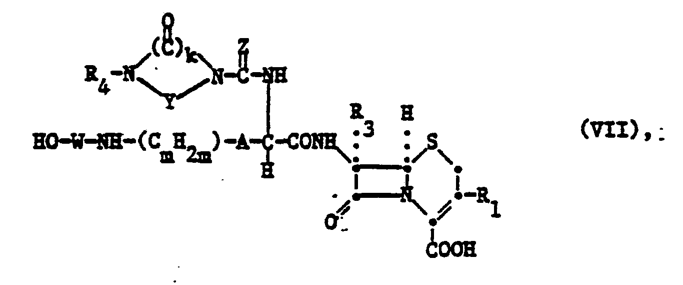 worin die 4-Carboxylgruppe und gegebenenfalls im Rest R₁ und in den Gruppierungen A und R₄ vorhandene funktionelle Gruppen in geschützter Form vorliegen können, acyliert, oder
d) in einer Verbindung der Formel 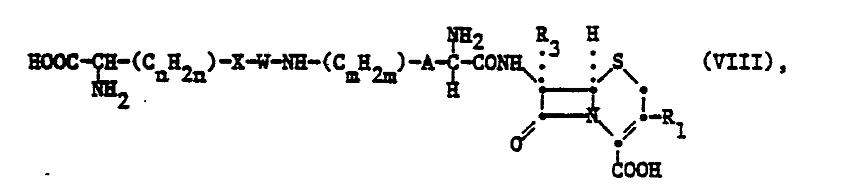 worin die Aminocarbonsäuregruppierung HOOC-CH(NH₂)- in geschützter Form vorliegt, die zu acylierende Aminogruppe gegebenenfalls durch eine, die Acylierung erlaubende Gruppe substituiert ist, und worin die 4-Carboxylgruppe und gegebenenfalls im Rest R₁ und in der Gruppierung A vorhandene weitere funktionelle Gruppen in geschützter Form vorliegen können, die Aminogruppe durch Behandeln mit einem den entsprechenden Acylrest einer Kohlen- oder Thiokohlensäure der Formel 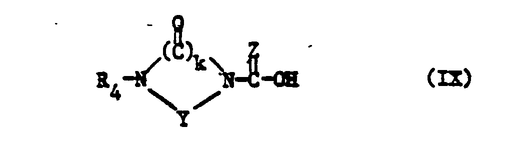 einführenden Acylierungsmittel, worin im Rest R₄ gegebenenfalls vorhandene funktionelle Gruppen in geschützter Form vorliegen können, acyliert, oder
e) ein reaktionsfähiges Derivat einer Kohlensäure- oder Thiokohlensäureverbindung der Formel 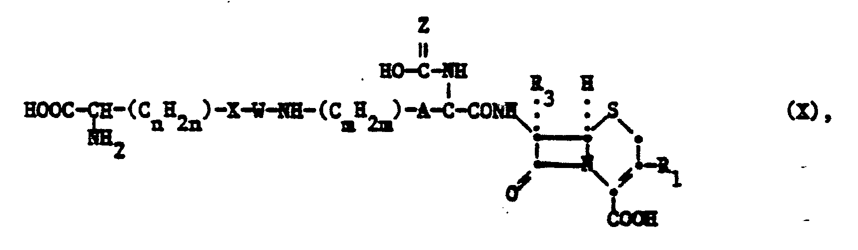 worin die Aminocarbonsäuregruppierung HOOC-CH(NH₂)- in geschützter Form vorliegt, und worin die 4-Carboxylgruppe und gegebenenfalls im Rest R₁ und in der Gruppierung A vorhandene weitere funktionelle Gruppen in geschützter Form vorliegen können, mit einem sekundären Amid der Formel 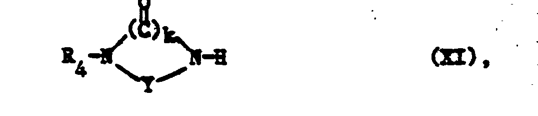 worin im Rest R₄ gegebenenfalls vorhandene funktionelle Gruppen geschützt sein können und die sekundäre Amidogruppe in einer die Acylierung erlaubenden Form vorliegt, reagieren lässt, oder
f) die unter d) oder e) genannte Acylierung in situ vornimmt, indem man eine Aminoverbindung der Formel VIII und eine Amidoverbindung der Formel XI, beide mit der oben angegebenen Bedeutung, gleichzeitig oder nacheinander auf ein bivalentes reaktives Derivat der Kohlen-oder Thiokohlensäure einwirken lässt, oder indem man
g) eine Verbindung der Formel 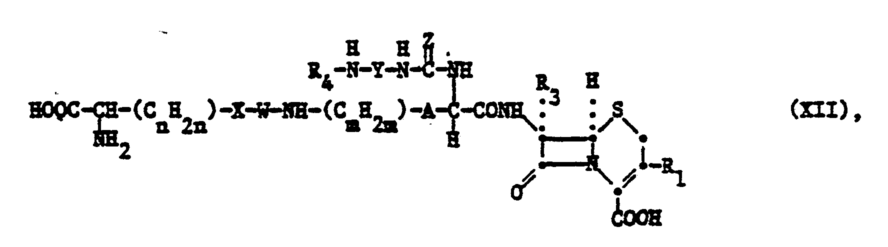 worin die Aminocarbonsäuregruppierung HOOC-CH(NH₂)- in geschützter Form vorliegt, und worin die 4-carboxylgruppe und gegebenenfalls im Rest R₁ und in der Gruppierung A vorhandene funktionelle Gruppen in geschützter form vorliegen können, und die später den Piperazinring bildende sekundäre Amino- bzw. Amidogruppen gegebenenfalls durch Gruppen substituiert sind, die die N-Acylierung erlauben, mit einem bivalenten acylierenden Derivat der Kohlensäure oder der Oxalsäure unter Ringschluss diacyliert, oder
h) eine 2-Cephemverbindung der Formel 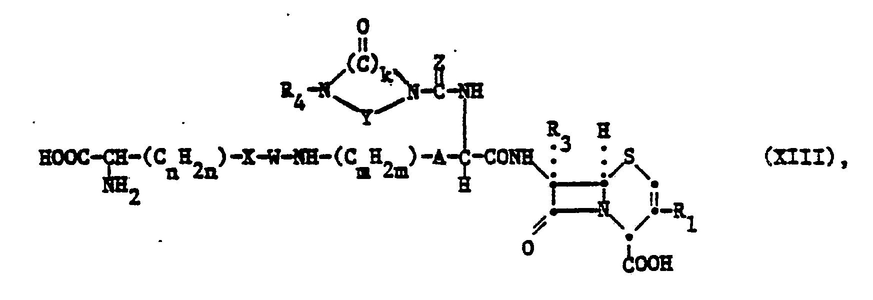 worin funktionelle Gruppen geschützt sind, zur entsprechenden 3-Cephemverbindung der Formel I isomerisiert, oder
i) in einer Verbindung der Formel 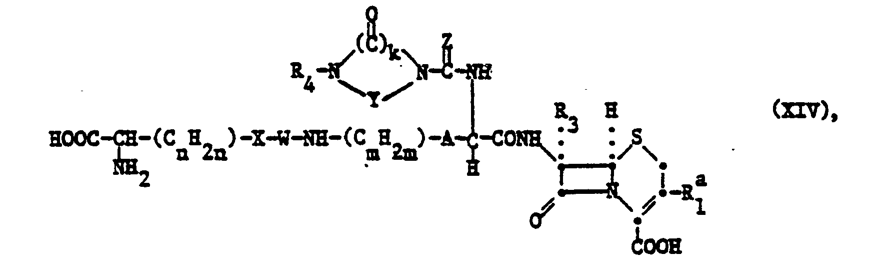 worin 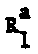 eine freie oder sulfonylierte Hydroxygruppe ist und worin andere funktionelle Gruppen geschützt sind, 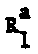 gegen Halogen austauscht, oder
j) in einer Verbindung der Formel XIV, worin 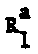 eine freie Hydroxygruppe ist und worin andere funktionelle Gruppen geschützt sind, die Hydroxygruppe veräthert, oder
k) in einer Verbindung der Formel XIV, worin funktionelle Gruppen geschützt sind, und worin 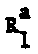 eine Formylgruppe bedeutet, diese in Gegenwert vom Decarbonylierungs-Katalysatoren zu einer Verbindung der Formel I, worin R₁ Wasserstoff bedeutet, decarbonyliert, oder
1) in einer Verbindung der Formel XIV, worin 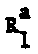 freies oder verestertes Hydroxy oder ein gegebenenfalls niederalkyliertes oder durch Niederalkylen oder Heteroniederalkylen cyclisch disubstituiertes Amino ist, und worin funktionelle Gruppen geschützt sind, die Gruppe 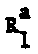 durch Wasserstoff ersetzt, und, wenn gewünscht, in einer erhaltenen Verbindung noch nicht geschützte funktionelle Gruppen schützt oder eine Schutzgruppe gegen eine andere austauscht, und/oder, wenn gewünscht, in einem Rest R₁ eine Gruppe R₂ gegen eine andere Gruppe R₂ austauscht, und/oder, wenn gewünscht, eine erhaltene Verbindung, worin R₃ Wasserstoff ist, in einer Verbindung überführt, worin R₃ Methoxy ist, und/oder, wenn gewünscht, ein erhaltenes Isomerengemisch in die einzelnen Isomeren auftrennt, und/oder, wenn eswünscht, eine erhaltene Verbindung in ein Salz oder ein erhaltenes Salz in die freie Verbindung oder ein ein anderes Salz überführt.

24. Verbindungen der Formel 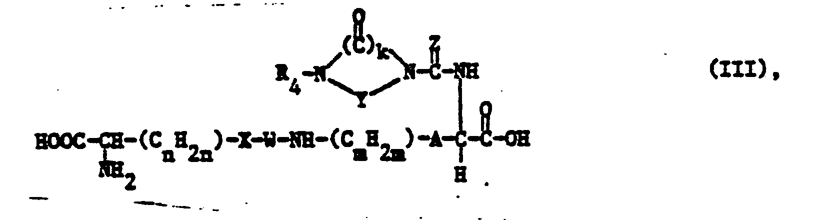 worin der Index n eine ganze Zahl von 1 bis 4, der Index m 0 oder 1, X Sauerstoff, Schwefel oder die Gruppe -NH-, W eine Gruppe -CO-, -CO-NHSO₂- oder -SO₂NH-CO-, oder X-W zusammen eine Gruppe -CO- oder -CO-NHSO₂-, A gegebenenfalls substituiertes Phenylen, Thienylen oder Furylen, Z Sauerstoff oder Schwefel, Y Niederalkylen, der Index k den Wert 1 oder 2, R₄ Wasserstoff, einen gegebenenfalls substituierten niederaliphatischen oder cycloaliphatischen Rest oder Acyl, und worin die Aminocarbonsäuregruppierung HOOC-CH(NH₂)- und gegebenenfalls in den Gruppierungen A und R₄ vorhandene weitere funktionelle Gruppen in geschützter Form vorliegen, und ihrer reaktionsfähigen funktionellen Säurederivate.

25. Verfahren zur Herstellung von Verbindungen der Formel III und ihrer geschützten und reaktionsfähigen funktionellen Derivate nach Patentanspruch 28, dadurch gekennzeichnet, dass man
a') in einer Verbindung der Formel 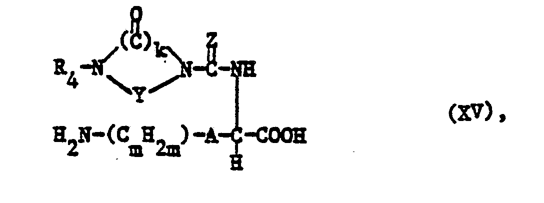 worin die Aminogruppe gegebenenfalls durch eine, die Aeylierung erlaubende Gruppe substituiert sein kann, und.worin die Carboxylgruppe und gegebenenfalls in den Gruppierungen A und R₄ vorhandene weitere funktionelle Gruppen in geschützter Form vorliegen können, die Aminogruppe durch Behandeln mit einem reaktionsfähigen funktionellen Derivat einer Säure der Formel 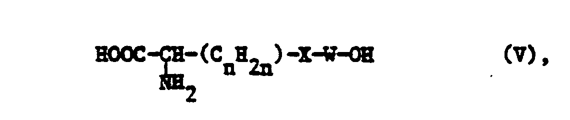 worin die Aminocarbonsäuregruppierung HOOC-CH(NH₂)- in geschützter Form vorliegt, oder wenn X-W zusammen eine Gruppe -CO- bedeuten, auch mit einer entsprechenden freien Säure, oder mit einen Salz davon, acyliert, oder
b') in einer Verbindung der Formel 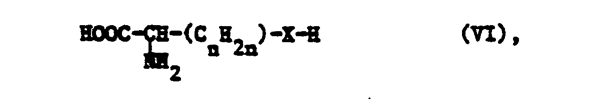 worin X Sausrstoff, Schwefel oder die Gruppe -NH- bedeutet, und werin die Aminocarbonsäuregruppierung HOOC-CH(NH₂)- in geshützter Form vorliegt, die Gruppe -X-H mit einem reaktionsfähigen funktionellen Derivat einer Verbindung der Formel 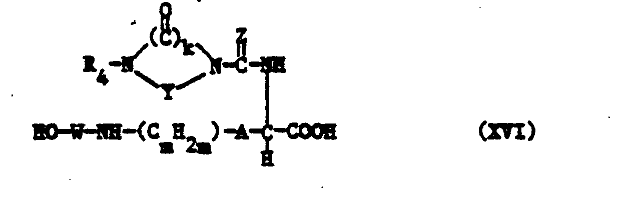 worin die Carboxylgruppe und gegebenenfalls in den Gruppierungen A und R₄ vorhandene funktionelle Gruppen in geschützter Form vorliegen können, acyliert, oder
c') in einer Verbindung der Formel 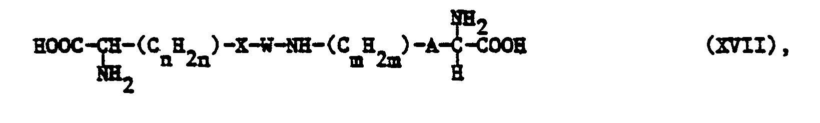 worin die Aminocarbonsäuregruppierung HOOC-CH(NH₂)- in geschützter Form vorliegt, die zu acylierende Aminogruppe gegebenenfalls durch eine, die Acylierung erlaubende Gruppe substituiert ist, und worin die Carboxylgruppe und gegebenenfalls in der Gruppierung A vorhandene weitere funktionelle Gruppen in geschützter Form vorliegen können, die Aminogruppe durch Behandeln mit einem den entsprechenden Acylrest einer Kohlen- oder Thiokohlensäure der Formel 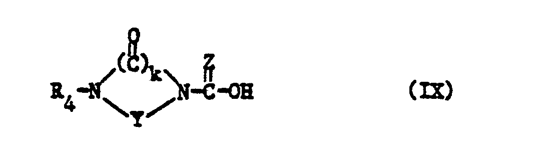 einführenden Aeylierungsmittel, worin im Rest R₄ gegebenenfalls vorhandene funktionelle Gruppen in geschützter Form vorliegen können, acyliert, oder
d') ein reaktionsfähiges Derivat einer Kohlensäure- oder Thiokohlensäureverbindung der Formel 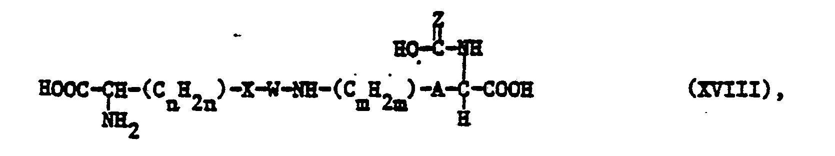 worin die Aminocarbonsäuregruppierung HOOC-CH(NH₂)- in geschützter Form vorliegt, und worin die Carboxylgruppe und gegebenenfalls in der Gruppierung A vorhanden weitere funktionelle Gruppen in geschützter Form vorliegen können, mit einem sekundären Amid der Formel 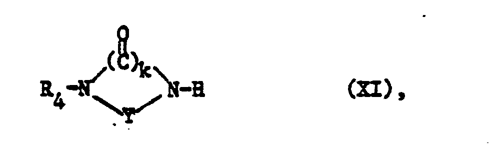 worin im Rest R₄ gegebenenfalls vorhandene funktionelle Gruppen geschützt sein können und die sekundäre Amidogruppe in einer die Acylierung erlaubenden Form vorliegt, reagieren lässt, oder e') die unter c') oder d') genannte Acylierung in situ vornimmt, indem man eine Aminoverbindung der Formel XVII und eine Amidoverbindung der Formel XI, beide mit der oben angegebenen Bedeutung, gleichzeitig oder nacheinander auf ein bivalentes reaktives Derivat der Kohlen-oder Thiokohlensäure einwirken lässt, oder indem man
f') eine Verbindung der Formel 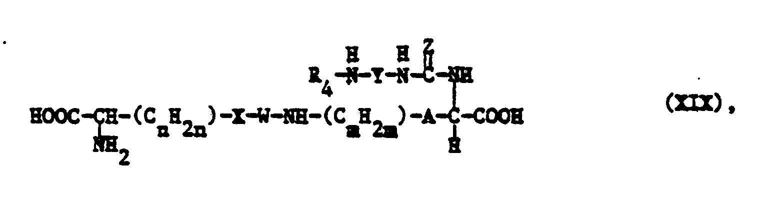 worin die Aminocarbensäuregruppierung HOOC-CH(NH₂)- in geschützter Form vorliegt, und worin die Carboxylgruppe und gegebenenfalls in der Gruppierung A vorhandene weitere funktionelle Gruppen in geschützter form vorliegen können, und die später den Piperazinring bildende sekundären Amino- bzw. Amidogruppen gegebenenfalls durch Gruppen substituiert sind, die die N-Acylierung erlauben, mit einem bivalenten acylierenden Derivat der kohlensllure oder der Oxalsäure unter Ringschluss discyliert, und gewanachtanfalls in einer erhaltenen Verbindung der Formel III die geschützte Carboxylgruppe in eine freie Carboxylgruppe oder in ein resktionsfähiges funktionelles Derivat davon überführt.

26. Verbindungen der Formel 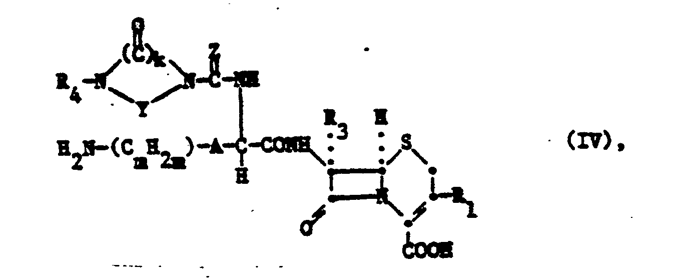 worin der Index m 0 oder 1, X Sauerstoff, Schwefel oder die Gruppe -NH-, W eine Gruppe -CO-, -CO-NHSO₂- oder -SO₂NH-CO-, oder X-W 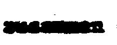 eine Gruppe -CO- oder -CO-NHSO₂-, A gegebenenfalls substituiertes Phenylen, Thienylen oder Furylen, Z. Sausrstoff oder Schwefel, Y Niederalkylen, der Index k den Wart 1 oder 2, R₄ Wasserstoff, einen gegenenenfalls substituierten niederaliphatischen oder cycloaliphatischen Rest oder Acyl, R₁ Wasserstoff, Niederalkyl, Niederalkoxy, Halogen oder eine Gruppe der Formel -CH₂-R₂, worin R₂ eine freie, veresterte oder verätherte Hydraxy- oder Mercaptogruppe oder eine quaternäre Ammoniumgruppe darstellt, und R₃ Wasserstoff oder Methoxy bedeuten, worin die Carboxylgruppen gegebenenfalls in physiologisch spaltbarer Form verestert sind, und Salze von solchen Verbindungen mit salzbildenden Gruppen.

27. Pharmazeutische Präparate entheltend Verbindungen der Formel IV gemäss Patentanspruch 26 oder pharmazeutisch verwendbare Salze davon.

28. Verbindungen der Formel IV gemäss Patentanspruch 26 und pharmazeutisch verwendbare Salze davon zur Bekämpfung von bakteriellen Infektionen.

2₉. Verfahren zur Herstellung von Verbindungen der Formel IV nach Patentanspruch 26, worin gegenenenfalls die Aminogruppe und die anderen funktionellen Gruppen substituiert bzw. geschützt sein können, sowie ihrer Salze, dadurch gekennzeichnet, dass man
a") in einer Verbindung der Formel 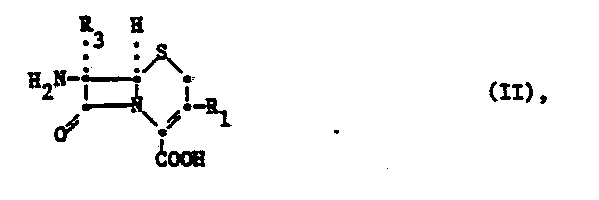 worin die Aminogruppe gegebenenfalls durch eine die Acylierung erlaubende Gruppe substituiert ist, und worin die 4-Carboxylgruppe und gegebenenfalls im Rest R₁ vorhandene weitere funktionelle Gruppen in geschützter Form vorliegen können, die Aminögruppe durch Behandeln mit einer Säure der Formel 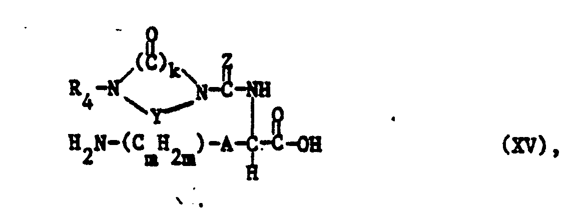 worin die Aminogruppe und gegebenenfells in den Gruppierungen A und R₄ vorhandene weitere funktionelle Gruppen in geschützter Form vorliegen, oder mit einem reaktionsfähigen funktionellen Säurederivat oder einem Salz davon acyliert, oder
b") in einer Verbindung der Formel 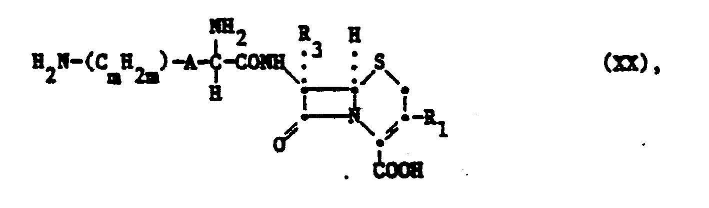 worin die endständige Aminogruppe in geschützter Form vorliegt, die zu acylierende Aminogruppe gegebenenfalls durch eine die Acylierung erlaubende Gruppe substituiert ist, und worin die 4-Carboxylgruppe und gegebenenfalls im Rest R₁ und in der Gruppierung A vorhandende weitere funktionelle Gruppen in geschützter Form vorliegen können, die α-Aminogruppe durch Behandeln mit einem den entsprechenden Acylrest einer Kohlen- oder Thiokohlensäure der Formel 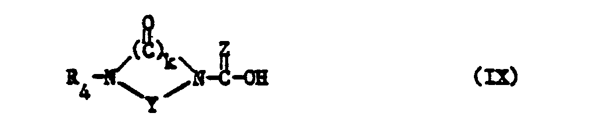 einführenden Acylierungsmittel, worin im Rest R₄ gegebenenfalls vorhandene funktionelle Gruppen in geschützter Form vorliegen können, acyliert, oder
c") ein reaktionsfähiges Derivat einer Kohlensäure- oder Thiokohlensäureverbindung der Formel 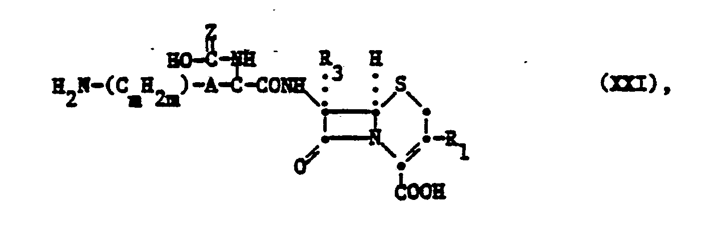 worin die endständige Aminogruppe in geschützter Form vorliegt, und worin die 4-Carboxylgruppe und gegebenenfalls im Rest R₁ und in der Gruppierung A vorhandene weitere funktionelle Gruppen in geschützter Form vorliegen können, mit einem sekuadären Amid der Formel 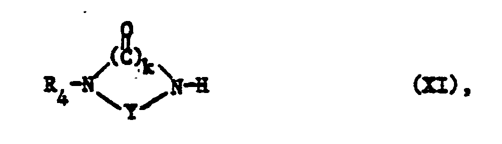 worin im Rest R₄ gegebenenfalls vorhendene funktionelle Gruppen geschützt sein können und die sekundäre Amidogruppe in einer die Acyliarung erlaubenden Form vorliegt, reagieren lässt, oder
d") die unter b") oder c") genannte Acylierung in situ vornimmt, indem man eine Aminoverbindung der Formel XX und eine Amidoverbindung der Formel XI, beide mit der oben angegebenen Bedeutung, gleichzeitig oder nacheinander auf ein bivalentes reaktives Derivat der Kohlen-oder Thiokohlensäure einwirken lässt, oder indem man
e") eine Verbindung der Formel 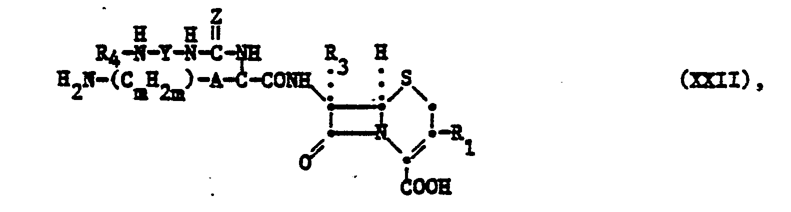 worin die endständige Aminogruppe in geschützter Form vorliegt, und worin die 4-Carboxylgruppe und gegebenenfalls im Rest R₁ und in der Gruppierung A vorhandene weitere funktionelle Gruppen in geschützter Form vorliegen können, und die später den Diazaring bildende sekundäre Amino- bzw. Amidogruppe gegebenenfalls durch Gruppen substituiert sind, die die N-Acylierung erlauben, mit einm bivalenten acylierenden Derivat der Kohlensäure oder der Oxalsäure unter Ringschluss diacyliert, oder
f") eine 2-Cephemverbindung der Formel 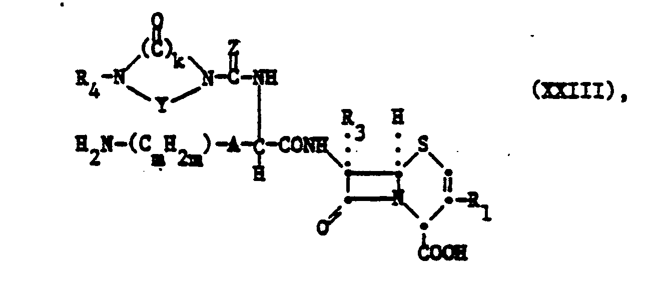 worin funktionelle Gruppen geschützt sind, zur entsprechenden 3-Ca- phemverbindung der Formel IV isomerisiert, oder
g") in einer Verbindung der Formel 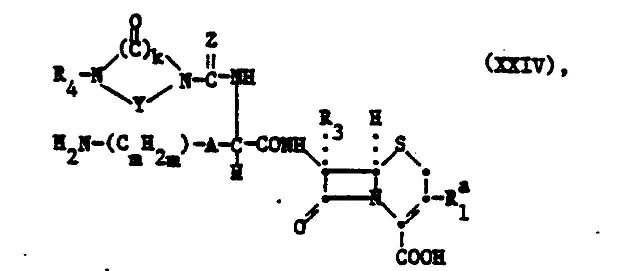 worin 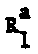 eine freie oder sulfonylierte Hydroxygruppe ist und worin andere funktionelle Gruppen geschützt sind, 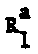 gegen Halogen austauscht, oder
h") in einer Verbindung der Formel XXIV, worin 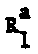 eine freie Hydroxygruppe ist und worin andere funktionelle Gruppen geschützt sind, die Hydroxygruppe veräthert, oder
i") in einer Verbindung der Formel XXIV, worin funktionelle Gruppen geschützt sind, und worin 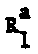 eine Formylgruppe bedeutet, diese in Gegenwart von Decarbonylierungs-katalysatoren zu einer Verbindung der Formel I, worin R₁ Wasserstoff bedeutet, decarbonyliert, oder
j") in einer Verbindung der Formel XXIV, worin 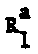 freies oder verestertes Hydroxy oder ein gegebenenfalls niederalkyliertes oder durch Niederalkylen oder Heteroniederalkylen cyclisch disubstituiertes Amino ist, und worin funktionelle Gruppen geschützt sind, die Gruppe 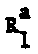 durch Wasserstoff ersetzt,
und, wenn erwünscht, in einer erhaltenen Verbindung die Schutzgruppen abspaltet oder noch nicht geschützte funktionelle Gruppen schützt oder eine Schutzgruppe gegen eine andere austauscht, und/oder, wenn gewänscht, in einem Rest R₁ eine Gruppe R₂ gegen eine andere Gruppe R₂ austauscht, und/oder, wenn erwünscht, eine erhaltene Verbindung, worin R₃ Wasserstoff ist, in eine Verbindung überführt, worin R₃ Methoxy ist, und/oder, wenn gewünscht, ein erhaltenes Isomerengemisch in die einzelnen Isomeren auftrennt, und/oder, wenn gewüascht eine erhaltene Verbindung in ein Salz oder ein erhaltenes Salz in die freie Verbindung oder in ein anderes Salz überführt.

30. Verbindungen der Formel 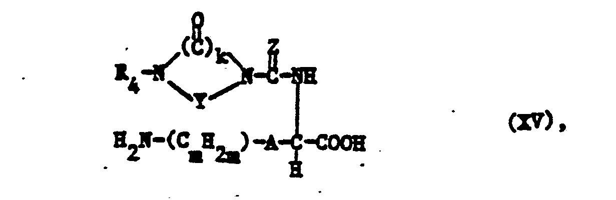 worin der Index m 0 oder 1, A gegebenenfalls substituiertes Phenylen, Thienylen oder Furylen, Z Sauerstoff oder Schwefel, Y Niederalkylen, der Index k den Wert 1 oder 2, R₄ Wasserstoff, einen gegebenenfalls substituierten niederaliphatischen oder cycloaliphatischen Rest oder Acyl bedeuten, worin die Aminogruppe gegebenenfalls durch eine, die Acylierung erlaubende Gruppe substituiert sein kann, und worin die Carboxylgruppe und gegebenenfalls in den Gruppierungen A und R₄ vorhandene weitere funktionelle Gruppen in geschützter Form vorliegen können, sowie die bezüglich der Carboxylgruppe reaktionsfähigen Derivate davon.

31. Verfahren zur Herstellung von Verbindungen der Formel XV gemäss Patentanspruch 30, und ihren geschützten und reaktionsfähigen Derivaten, dadurch gekennzeichnet, dass man
a"') in einer Verbindung der Formel 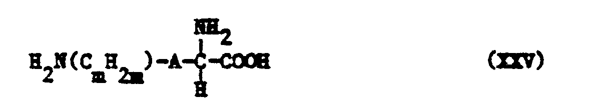 worin die endständige Aminogruppe in geschützter Form vorliegt, die zu acylierende Aminogruppe gegebenenfalls durch eine, die Acylierung erlaubende Gruppe substituiert ist, und worin die Carboxyigruppe und gegebenenfalls in der Gruppierung A vorhandene weitere funktionelle Gruppen in geschützter Form vorliegen können, die α-Aminogruppe durch Behandeln mit einem den entsprechenden Acylrest einer koblen-oder Thiokohlensäure der Formel 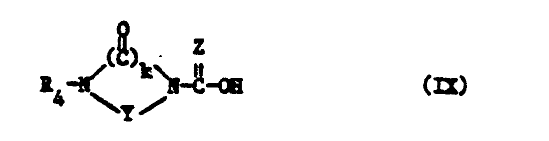 einführenden Acylierungsmittel, worin im Rest R₄ gegebebenfalls vorhandene funktionelle Gruppen in geschützter Form vorliegen können, acyliert, oder
b"') ein reaktionsfähiges Derivat einer Kohlensäure- oder Thiokohlensäureverbindung der Formel 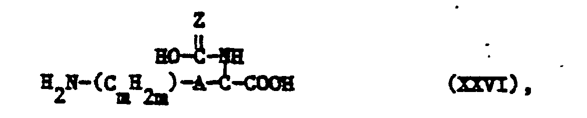 worin die emdständige Aminogruppe in geschützter Form vorliegt, und worin die Carbosylgruppe und gegebenenfalls in der Gruppierung A vorhandene weitere funktionelle Gruppen in geschützter Form vorliegen können, mit einem sekundären Amid der Formel 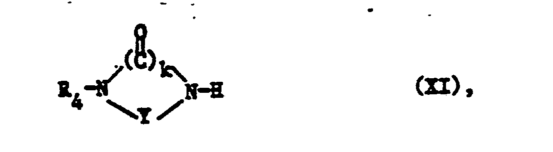 worin im Rest R₄ gegebenenfalls vorhendene funktionelle Gruppen geschützt sein können und die sekundäre Amidogruppe in einer die Acylierung erlaubenden Form vorliegt, resgieren lässt, oder
c"') die unter a"') oder b"') genannte Acylierung in situ vornommt, index man eine Aminoverbindung der Forme 1 XXV und eine Amidoverbindung der Formel ZI, beide mit der oben angegebenen Bedeutung, gleichzeitig oder nacheinder auf ein bivalentes reaktives Derivat der Kohlen- oder Thiokohlensäure einwirken lässt, oder indem man
d"') eine Verbindung der Formel 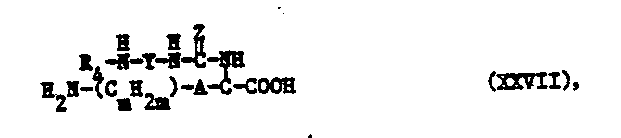 worin die eadständige Aminogruppe in geschützter Form vorliegt, und worin die Carboxylgruppe und gegebenenfalls in der Gruppierung A vorhendene weitere funktionelle Gruppen in geschützter Form vorliegen können, und die später den Diazaring bildende sekundäre Amino- bzw. Amidogruppe gegebenenfalls durch Gruppen substituiert sind, die die N-Acylierung erlauben, mit einem bivalenten acylierenden Derivat der Kohlensäure oder der Oxelsäure unter Ringschluss diacyliert, und gewünschtenfalls in einer erheltenen Verbindung der Formel XIV die geschützte Cerboxylgruppe in eine freie Cerboxylgruppe oder in ein reektionsfähiges funktionelles Derivat davon überführt.

## Patentansprüche (Patentansprüche für folgende(n) Vertragsstaat(en) : Oesterreich)

1. Verfahren zur Herstellung von Verbindungen der Formel 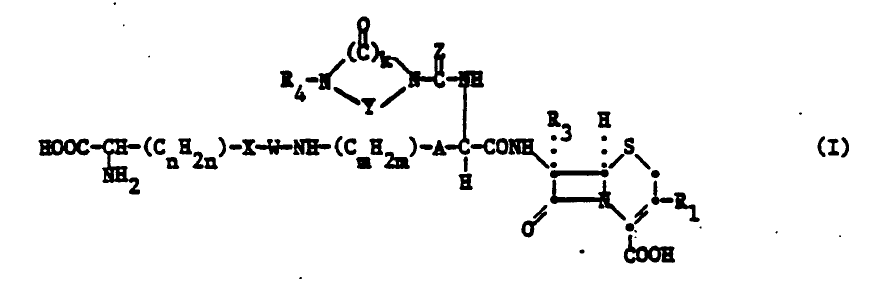 worin der Index n eine ganze Zahl von 1 bis 4, der Index m 0 oder 1, X Sauerstoff, Schwefel oder die Gruppe -NH-, W eine Gruppe -CO-, -CO-NHSO₂- oder -SO₂NH-CO-, oder X-W zusammen eine Gruppe -CO- oder -CO-NHSO₂-, A gegebenenfalls substituiertes Phenylen, Thienylen oder Furylen, Z Sausrstoff oder Schwefel, Y Niederalkylen, der Index k den Wert 1 oder 2, R₄ Wasserstoff, einen gegebenenfalls substituierten niederaliphatischen oder cycloaliphatischen Rest oder Acyl, R₁ Wasserstoff, Niederalkyl, Niederalkoxy, Halogen oder eine Gruppe der Formel -CH₂-R₂, worin R₂ eine freie, veresterte oder verätherte Hydroxy- oder Mercaptogruppe oder eine quatarnäre Ammoniungruppe derstellt, und R₃ Wasserstoff oder Methoxy bedeuten, worin die Carboxylgruppen gegenenenfalls in physiologisch spaltbarer Form verestert sind, und Salzen von solchen Verbindungen mit salzbildenden Gruppen, dadurch gekennzeichnet, dass man in einer der Formel I entsprechenden Ausgangsverbindung, worin mindestens eine der vorhandenen funktionellen Gruppen geschützt ist, die funktionelle(n) Gruppe(n) freisetzt, wenn erwünscht, in einer erhsltenen Verbindung eine Groppe R₁ in eine andere Gruppe R₁ überführt, und/oder, wenn erwünscht, eine freie Carboxylgruppe in eine physiologisch spaltbare veresterte Carborulgruppe überführt, und/oder, wenn erwünscht, ein erhsltanes Iso- marangewisch in die einzelnen Isomeren suftrennt, und/oder, wenn erwünscht, eine erhsltene Verbindung in ein Salz oder ein erhaltenes salz in eine freie Verbindung oder in ein anderes Salz überführt.

2. Verfahren nach Patentanspruch 1, dadurch gekennzeichnet, dass man geschützte funktionelle Gruppen durch Solvolyse oder Reduktion freisetzt.

3. Verfahren nach Patentanspruch 1 oder 2, dadurch gekennzeichnet, dass man von einer entsprechenden geschützten Verbindung ausgehend, eine Verbindung der Formel I oder ein pharmazeutisch verwendbares Salz davon herstellt, worin die
Grupge -(CₙH₂ₙ)- unverzweigt ist und die Indices n, m, und k die angegebenen Bedeutungen haben , X Sauerstoff oder die Gruppe -NH-, W eine Gruppe -CO- oder -CO-NHSO₂-, oder X-V zusammen eine Gruppe -CO- oder -CO-NHSPO₂- bedeuten, A p-, o- oder m-Phenylen, 2,5-Thienylen oder 2,5-Furylen darstellt, Y 1,2-Aethylen, R₄ Wassarstoff, gegebenenfalls durch Hydroxy. Niederalkanoyloxy, Niederalkoxycarbonyloxy, Phenoxycarbonyloxy, Niederalkoxy, Niederalkylthio, Halogen, gegebenenfalls funktionell abgewandeltes Carboxy, oder gegebenenfalls mono- oder diniederalkylierts Amino substituiertes Niederalkyl sowie Niederalkenyl oder Acyl, Z Sauerstoff oder Schwefel, R₁ Wasserstoff, Niederalkyl, Niederalkoxy, Halogen oder eine Gruppe der Formel -CH₂-R₂, worin R₂ Niederalkanoyloxy, Carbamoyloxy, N-Niederalkylcarbamoyloxy, Triazolylthio, Tetrazolylthio, Thiazolylthio, Thiatriazolylthio, Thiadiazolylthio, Oxazolylthio, Oxadiazolylthio, 5,6-Dioxo-tetrahydro-as-triazinylthio oder darstellt, worin die heterocyclischen Ringe gegebenenfalls beispielsweise durch Niederalkyl, N,N-Diniederalkylaminoniederalkyl, Carboxyniederalkyl, Sulfoniederalkyl, Amino, Carboxyniederalkanoylamino oder Carbamoyl substituiert sein können, und R₃ Wasserstoff oder Methoxy bedeuten.

4. Verfahren nach Patentanspruch 1 oder 2, dadurch gekennzeichnet, dass man von einer entsprechenden geschützten Verbindung ausgehend, eine Verbindung der Formel I oder ein pharmazeutisch verwendbares Salz davon herstellt, worin die die Gruppe -(CₙH₂ₙ)- unversweigt ist und die Indices n, m und k die angegebene Bedeutung heben, X Sauerstoff oder die Gruppe -NH- bedeutet, W eine Gruppe -CO- oder -CONHSO₂-, oder X-W zusammen eine Gruppe -CO- oder -CONHSO₂- bedeuten, A p-, o- oder m-Phnylen, oder, wenn m 1 ist, auch 2,5-Thienylen oder 2,5-Furylen darstellt, Y 1,2-Acthylen, R₄ Wasserstoff, gegebenenfalls durch Hydroxy, Niederalkanoyloxy, Niederalkoxycarbonyloxy, Niederalkoxy, Niederalkylthio, Halogen, z.B. Chlor, Carboxy, Amino oder Diniederelkylamino substituiertes Niederalkyl, sowie Niederalkenyl oder gegebenenfalls durch Halogen, z.B. Chlor, Niederalkoxy, Niederalkanoyloxy, Cyan oder Phenoxy substituiertes Niederalkanoyl, gegebenenfalls durch Niederalkyl, Hydroxy, Niederalkoxy oder Chlor substituiertes Benzoyl, einen gegebenenfalls durch Hydroxy oder Chlor substituierten Pyridin-, Pyrimidin- oder Pyrazincarbonsäurerest, Niederalkoxycarbonyl, gegebenenfalls durch Halogen, z.B. Chlor oder Niederalkyl substituiertes Aryl-, wie Benzol-oder Naphthalinsulfonyl, gegebenenfalls m N-Atom niederalkyliertes Carbamoyl, Thiocarbamoyl, Iminocarbamoyl (-Guanyl) oder Sulfamoyl, Z Sauerstoff, R₁ Wasserstoff, Methyl, Methoxy, Chlor oder eine Gruppe der Forme1 -CH₂-R₂, worin R₂ Acetoxy, Cerbamoyloxy, gegebenenfalls durch Methyl, Cerboxymethyl, Sulfomethyl oder Dimethylaminoethyl substituiertes Tetrazolylthio, z.B. Tetrezol-5-ylthio, 1-Methyl-1H-tetrazol-5-ylthio, 1-Sulfomethyl-1H-tetrazol-5-ylthio, 1-Carboxymethy-1H-tetraxol-5-ylthio, oder 1-(2-Dimethylaminoethyl)-1H-tetrazol-5- ylthio, Thiadiazolylthio, insbesondere 2-Methyl-1,3,4-thiadiazol-5- ylthio, durch Methyl substituiertes 5,6-Dioxo-tetrahydro-as-triazinylthio, z.B. 2-Methyl-5,6-dioxo-1,2,3,6-tetrehydro-as-triazin-3-ylthio oder 4-Methyl-5,6-dioxo-1,4,5,6-tetrahydro-as-triazin-3-ylthio, oder Cerbamoylpyridinio, insbesondere 4-Carbamoylpyridinio, darstellt, und R₃ Wasserstoff oder Methoxy bedeuten.

5. Verfahren nach Patentanspruch 1 oder 2, dadurch gekennzeichnet, dass man von einer entsprechenden geschützten Verbindung ausgehend, eine Verbindung der Formel I oder ein pharmazeutisch verwendbares Salz davon herstellt, worin die Gruppe -(CₙH₂ₙ)-Methylen bedeutet, die Indices = und k die angegebene Bedeutung haben, X Sauerstoff und W eine Gruppe -CO-, A p- oder o-Phenylen, Y 1,2-Aethylen, R₄ Wasserstoff, Niederalkyl, insbesondere Aethyl, oder Niederalkylsulfonyl, insbesondere Mesyl, Z Sauerstoff, R₁ Wasserstoff, Methyl, Methoxy, Chlor oder eine Gruppe der Formel -CH₂-R₂, worin R₂ Acetoxy, Carbamoyloxy, gegebenenfalls durch Methyl, Carboxymethyl, Sulfomethyl oder Dimethylaminoäthyl substituiertes Tetrazolylthio, insbsondere 1-Methyl-1H-tetrazol-5-ylthio, 1-Sulfomethyl-1H-tetrazol-5-ylthio, 1-Carboxymethyl-1H-tetrazol-5-ylthio, oder 1-(2-Dimethylaminoäthyl)-1H-tetrazol-5-ylthio, Thiadiasolylthio, insbesondere 2-Methyl-1,3,4-thiadiazol-5-ylthio, durch Methyl substituiertes 5,6-Dioxo-tetrahydro-as-trizinylthio, z.B. 2-Methyl-5,6-dioxo-1,2,5,6-tetrahydro-as-triazin-3-ylthio oder 4-Methyl-5,6-dioxo-1,4,5,6-tetrahydro-as-triazin-3-ylthio, oder Carbamoylpyridinio, insbesondere 4-Carbamoylpyridinio, darstellt, und R₃ Wasserstoff oder Methoxy bedeuten.

6. Verfahren nach Patentanspruch 1 oder 2, dadurch gekennzeichnet, dass man von einer entsprechenden geschützten Verbindung ausgehend die 3-[(1-Methyl-1H-tetrazol-5-yl)-thiomethyl]-7β-{(2R)-2-[4-((2R)-2-Amino-2-carboxy-äthoxycarbonylamino)-phenyl]-2-(2-imidazolidon-1-carboxamido)-acetamido}-3-cephem-4-earbonsäure oder ein pharmazeutisch annehnbares Salz davon herstellt.

7. Verfahren nach Patentanspruch 1 oder 2, dadurch gekennzeichnet, dass man von einer entsprechenden geschützten Verbindung ausgehend die 3-[(1-Methyl-1H-tetrazol-5-yl)-thiomethyl[-7β-{(2R)-2-[4-((2R)-2-amino-2-carboxyäthoxycarbonylamino)-phenyl]-2-(3-methansulfonyl-amino-2-imidazolidon-1-carboxamido)-acetamido)-3-cephem-4-carbonsäure oder ein pharmazeutisch annehmbares Salz davon herstellt.

8. Verfahren nach Patentanspruch 1 oder 2, dadurch gekennzeichnet, dass man von einer entsprechenden geschützten Verbindung ausgehend, die 3-[(1-Methyl-1H-tetrazol-5-yl)-thiomethyl]-7β-[(2S)-2-[4-((2R)-2-amino-2-carboxyäthoxycarbonylamino)-phenyl]-2-(3-methansulfonyl-2-imidazolidon-1-carboxamido)-acetamido]-3-cephem-4-carbonsäure oder ein pharmazeutisch annehmbares Salz davon herstellt.

9. Verfahren nach Patentanspruch 1 oder 2, dadurch gekennzeichnet, dass man von einer entsprechenden geschützten Verbindung ausgehend, die 3-[(1-Methyl-1H-tetrazol-5-yl)-thiomethyl]-7β-{(2R)-[4-((2R)-2-amino-2-carboxyäthoxyearbonylamino)-phenyl]-2-(4-äthyl-2,3-dioxo- piperazin-1-carboxamido)-acetamido}-3-cephem-4-carbonsäure oder ein pharmazeutisch annehmbares Salz davon herstellt.

10. Verfahren nach Patentanspruch 1 oder 2, dadurch gekennzeichnet, dass man von einer entsprechenden geschützten Verbindung ausgehend, die 3-[(1-Methyl-1H-tetrazol-5-yl)-thiomethyl]-7β-{(2S)-[4-((2R)-2-amino-2-carboxyäthoaycarbonylamino)-phenyl]-2-(4-äthyl-2,3-dioxo- piperazin-1-carboxamido)-acetamido}-3-cephem-4-carbonsäure oder ein pharmazeutisch annehmbares Salz davon herstellt.

11. Verfahren nach Patentanspruch 1 oder 2, dadurch gekennzeichnet, dass man von einer entsprechenden geschützten Verbindung ausgehend, die 3-[(1-Methyl-1H-tetrazol-5-yl)-thiomethyl]-7β-{(2R,S)-2-[4-((2R)-2-amino-2-carboxyäthoaycarbonylamino)-phenyl]-2-(3-methansulfonyl-2-imidazolidon-1-carboxamido)-acetamido]-3-cephem-4-carbonsäure oder ein pharmazeutisch annehmbares Salz davon herstellt.

12. Verfahren nach Patentanspruch 1 oder 2, dadurch gekennzeichnet, dass man von einer entsprechenden geschützten Verbindung ausgehend, die 7β-{(2R)-2-[4-((2R)-Amino-2-carboxyäthyloxycarbonylamino- methyl)-phenyl]-2-(4-äthyl-2,3-dioxopiperazin-1-carboxamido)-acetamido]-3-[(1-methyl-1H-tetrazol-5-yl)-thiomethyl]-3-cephem-4-carbonsäure oder ein pharmazeutisch annehmbares Salz davon herstellt.

13. Verfahren nach Patentanspruch 1 oder 2, dadurch gekennzeichnet, dass man von einer entsprechenden geschützten Verbindung ausgehend, die 7β-{(2S)-2-[4-((2R)-2-Amino-2-carboxyäthoxycarbonylamino- methyl)-phenyl]-2-(4-äthyl-2,3-dioxo-piperazin-l-carboxamido)-aeet- amido}-3-[(1-methyl-1H-tetrazol-5-yl)-thiomethyl]-3-cephem-4-carbonsäure oder ein pharmazeutisch annehmbares Salz davon herstellt.

14. Verfahren nach Patentanspruch 1 oder 2, dadurch gekennzeichnet, dass man von einer entsprechenden geschützten Verbindung ausgehend, die 7β-{(2R)-2-[4-((2R)-2-Amino-2-carboxyäthoxycarbonylamino)-phenyl]-2-(4-äthyl-2,3-dioxo-piperazin-1-carboxamido)-acetamido}-3-carbamoyloxymethyl-3-cephem-4-carbonsäure oder ein pharmazeutisch annehmbares Salz davon herstellt.

15. Verfahren nach Patentanspruch 1 oder 2, dadurch gekennzeichnet, dass man von einer entsprechenden geschützten Verbindung ausgehend, die 7β-{(2S)-2-[4-((2R)-2-Amino-2-carboxyäthoxycarbonylamino)-phenyl]-2-(4-äthyl-2,3-dioxo-piperazin-1-carboxamido)-acetamido}-3-carbamoyloxymethyl-3-cephem-4-earbonsäure oder ein pharmazeutisch annehmbares Salz davon herstellt.

16. Verfahren nach Patentanspruch 1 oder 2, dadurch gekennzeichnet, dass man von einer entsprechenden geschützten Verbindung ausgehend, die 7β-{(2R)-2-[4-((2R)-2-Amino-2-carboxyäthoxycarbonylamino)-phenyl]-2-(4-äthyl-2,3-diozo-piperazin-1-carboxamido)-acetamido}-3-[1-(2-dimethylaminoäthyl)-1B-tetrazol-2-ylthiomethyl]-3-cephem-4-carbonsäure oder ein pharmazeutsich annehmbares Salz davon herstellt.

17. Verfahren nach Patentanspruch 1 oder 2, dadurch gekennzeichnet, dass man von einer entsprechenden geschützten Verbindung ausgehend, ein Natriumsalz einer der Verbindungen gemäss einem Patentanspruch 1-16 herstellt.

18. Verfahren zur Herstellung eines pharmazeutischen Präparates, dadurch gekennzeichnet, dass man eine gemäss einem der Patentansprüche 1 bis 17 erhaltene Verbindung mit einem pharmazeutischen Trägermaterial verarbeitet.

^{19.} Verfahren zur Herstellung von Verbindungen der Formel I gemäss Patentanspruch 1, worin mindestens eine der funktionellen Gruppen in geschützter Form vorliegt, dadurch gekennzeichnet, dass man
a) in einer Verbindung der Formel 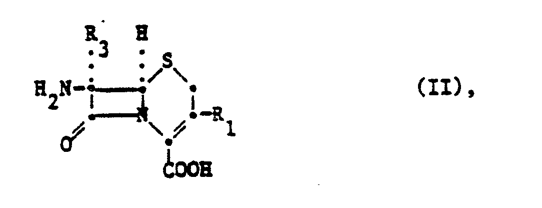 worin die Aminogruppe gegebenenfalls durch eine die Acylierung erlaubende Gruppe substituiert ist, und worin die 4-Carboxylgruppe und gegebenemfalls im Rest R₁ vorhandene weitere funktionelle Gruppen in geschützter Form vorliegen können, die Aminogruppe durch Behandeln mit einer Säure der Formel 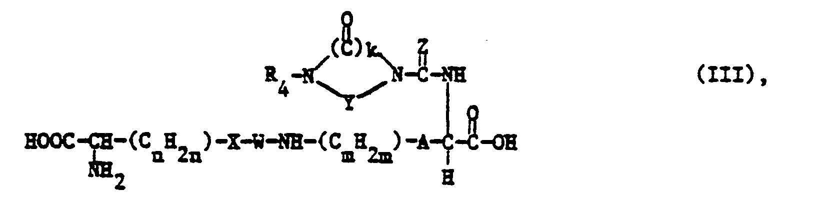 worin die Aminocarbonsäuregruppierung HOOC-CH(NH₂)- und gegebenenfalls in den Gruppierungen A und R₄ vorhandene weitere funktionelle Gruppen in geschützter Form vorliegen, oder mit einem reaktionsfähigen funktionellen Säurederivat oder einem Salz davon acyliert, oder
b) in einer Verbindung der Formel 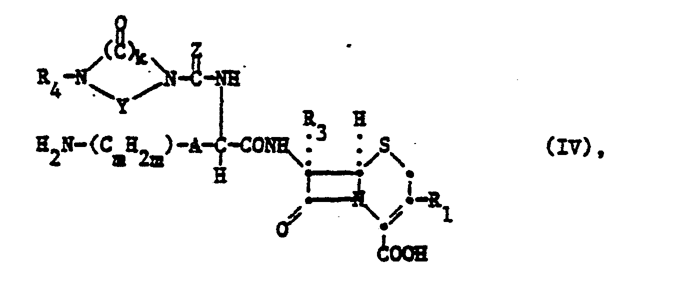 worin die Aminogruppe gagebenenfalls durch eine, die Acylierung er-Izubende Gruppe substituiert sein kann, und worin die 4-Carboxylgruppe und gegebenenfalls im R₁ und in den Gruppierngen A und R₄ vorhandene weitere funktionelle Gruppen in geschützter Form vorliegen können, die Aminogruppe durch Behandeln mit einem reaktionsfähigen funktionellen Derivat einer Säure der Formel 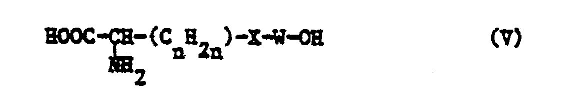 worin die Aminocarbonsäuregruppierung HOOC-CH(NH₂)- in geschützter Form vorliegt, oder wenn X-W zusammen eine Gruppe -CO- bedeuten, auch mit einer entsprechenden freien Säure, oder mit einem Salz davon, acyliert, oder
c) in einer-Verbindung der Formel 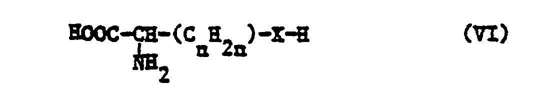 worin X Sauerstoff, Schwefel oder die Gruppe -NH- bedeutet, und worin die Aminocarbonsäuregruppierung HOOC-CH(NH₂)- in geschützter Form vorliegt, die Gruppe -X-H mit einem reaktionsfähigen funktionellen Derivat einer Verbindung der Formel 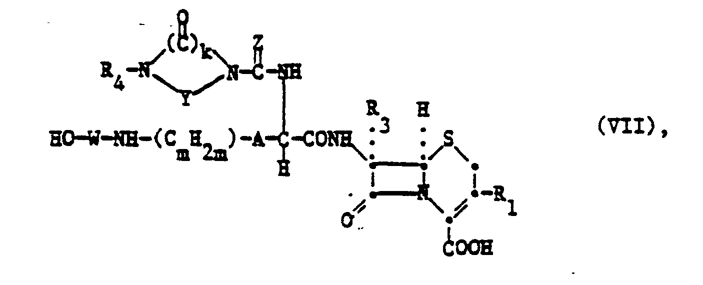 worin die 4-Carboxylgruppe und gegebenenfalls im Rest R und in den Gruppierungen A und R₄ vorhandene funktionelle Gruppen in geschützter Form vorliegen können, acyliert, oder
d) in einer Verbindung der Formel 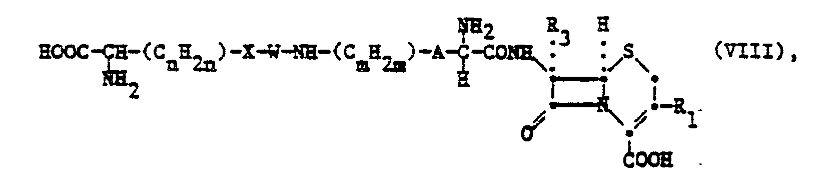 worin die Aminocarbousäuregruppierung HOOC-CH(NH₂)- in geschützter Form vorliegt, die zu acylierende Aminogruppe gegebenenfalls durch eine, die Acylierung erlaubende Gruppe substituiert ist, und worin die 4-Carboxylgruppe und gegebenenfalls im Rest R₁und in der Gruppierung A vorhandene weitere funktionelle Gruppen in geschützter Form vorliegen können, die Aminogruppe durch Behandeln mit einem den entsprechenden Acylrest einer Kohlen- oder Thiokohlensäure der Formel 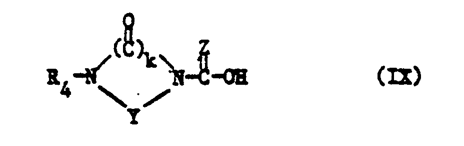 einführenden Acylierungsmittel, worin im Rest R₄ gegebenenfalls vorhandene funktionelle Gruppen in geschützter Form vorliegen können, acyliert, oder
e) ein reaktionsfähiges Derivat einer Kohlensäure- oder Thiokohlensäureverbindung der Formel 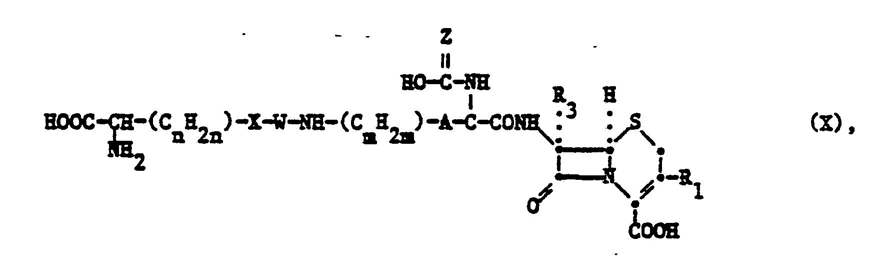 worin die Aminocarbonsäuregruppierung HOOC-CH(NH₂)- in geschützter Form vorliegt, und worin die 4-Carboxylgruppe und gegebenenfalls im Rest R₁ und in der Gruppierung A vorhandene weitere funktionelle Gruppen in geschützter Form vorliegen können, mit einem sekundären Amid der Fomel 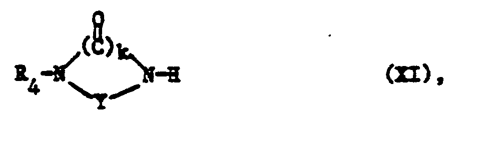 worin im Rest R4 gegebenenfalls vorhandene funktionelle Gruppen geschützt sein können und die sekundäre Amidogruppe in einer die Acylierung erlaubenden Form vorliegt, reagieren lässt, oder
f) die unter d) oder e) genannte Acylierung in situ vornimmt, indem man eine Aminoverbindung der Formel VIII und eine Amidoverbindung der Formel XI, beide mit der oben engegebenen Bedeutung, gleichzeitig oder nacheinander auf ein bivalentes reaktives Derivat der Kohlen-oder Thiokohlensäure einwirken lässt, oder indem man
g) eine Verbindung der Formel 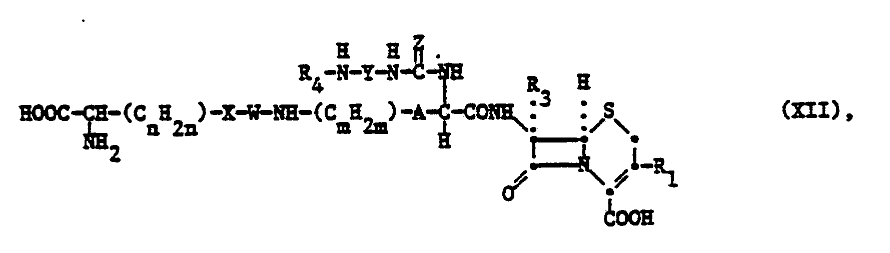 worin die Aminocarbonsäuregruppierung HOOC-CH(NH₂)- in geschützter Form vorliegt, und worin die 4-Carboxylgruppe und gegebenenfalls im Rest R₁ und in der Gruppierung A vorhandene funktionelle Gruppen in geschützter Form vorliegen können, und die später den Piperazinring bildende sekundäre Amino- bzw. Amidogruppen gegebenenfalls durch Gruppen substituiert sind, die die N-Acylierung erlauben, mit einem bivalenten acylierenden Derivat der Kohlensäure oder der Oxalsäure unter Ringschluss diacyliert, oder
h) eine 2-Cephemverbindung der Formal 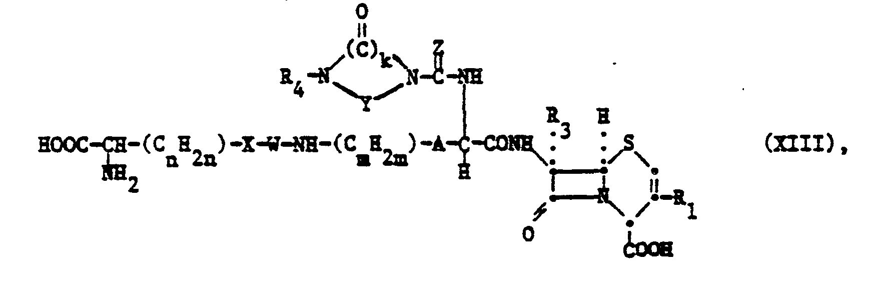 worin funktionelle Gruppen geschützt sind, zur entsprechenden 3-Caphemverbindung der Formel I isomerisiert, oder
i) in einer Verbindung der Formel 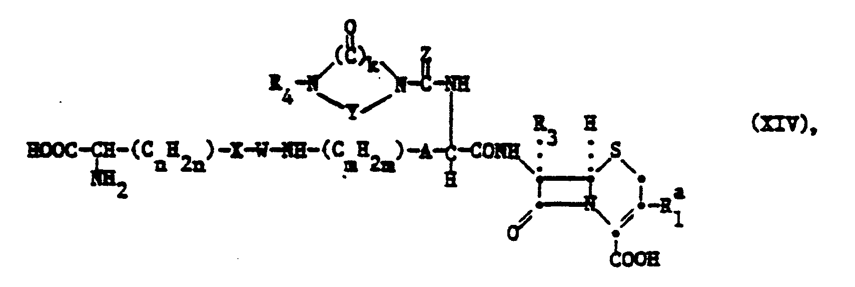 worin R^{a}₁ eine freie oder sulfonylierte Hydroxygruppe ist und worin andere funktionelle Gruppen geschützt sind, R^{a}₁ gegen Halogen austauscht, oder
j) in einer Verbindung der Formel XIV, worin R^{a}₁ eine freie Hydroxygruppe ist und worin andere funktionelle Gruppen geschützt sind, die Hydroxygruppe veräthert, oder k) in einer Verbindung der Formel XIV, worin funktionelle Gruppen geschützt sind, und worin R^{a}₁ eine Formylgruppe bedeutet, diese in Gegenwart von Decarbonylierungs-Katalysatoren zu einer Verbindung der Formel I, worin R₁ Wasserstoff bedeutet, decarbonyliert, oder
1) in einer Verbindung der Formel XIV, worin R^{a}₁ freies oder verestertes Hydroxy oder ein gegebenenfalls niederalkyliertes oder durch Niederalkylen oder Heteroniederalkylen cyclisch disubstituiertes Aaino ist, und worin funktionelle Gruppen geschützt sind, die Gruppe R^{a}₁ durch Wasserstoff ersetzt, und, wenn gewünscht, in einer erhaltenen Verbindung noch nicht geschützte funktionelle Gruppen schützt oder eine Schutzgruppe gegen eine andere austauscht, und/oder, wenn gewünscht, in einem Rest R₁ eine Gruppe R₂ gegen eine andere Gruppe R₂ austauscht, und/oder, wenn gewünscht, eine erhaltene Verbindung, worin R₃ Wasserstoff ist, in einer Verbindung überführt, worin R₃ Methoxy ist, und/oder, wenn gewünscht, ein erhaltenes Isomerengemisch in die einzelnen Isomeren auftrennt, und/oder, wenn erwünscht, eine erhaltene Verbindung in ein Salz oder ein erhaltenes Salz in die freie Verbindung oder ein ein anderes Salz überführt.

2₀. Verfahren zur Herstellung von Verbindungen der Formel III und ihrer geschützten und reaktionsfähigen funktionellen Derivate nach Patentanspruch 19, dadurch gekenaseichnet, dass man
a') in einer Verbindung der Formel 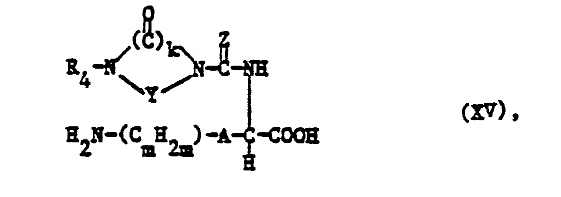 worin die Aminogruppe gegebenenfalls durch eine, die Acylierung erlaubende Gruppe substituiert sein kann, und worin die Carboxylgruppe und gegebenenfalls in den Gruppierungen A und R₄ vorhandene weitere funktionelle Gruppen in geschützter Form vorliegen können, die Aminogruppe durch Behandeln mit einem reaktionsfähigen funktionellen Derivat einer Säure der Formel 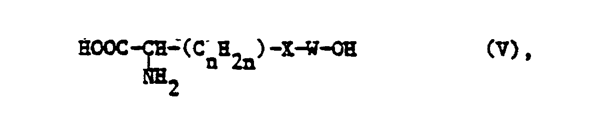 worin die Aminocarbonsäuregruppierung HOOC-CH(NH₂)- in geschützter Form vorliegt, oder wenn X-W zusammen eine Gruppe -CO- bedeuten, auch mit einer entsprechenden freien Säure, oder mit einem Salz davon, acyliert, oder
b') in einer Verbindung der Formel 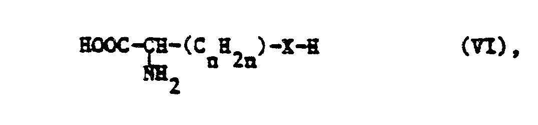 worin X Sauerstoff, Schwefel oder die Gruppe -NH- bedeutet, und worin die Aminocarbonsäuregruppierung HOOC-CH(NH₂)- in geschützter Form vorliegt, die Gruppe -X-H mit einem reaktionsfähigen funktionallen Derivat einer Verbindung der Formel 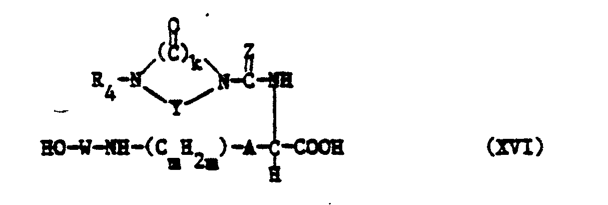 worin die Cerboxylgruppe und gegebenenfalls in den Gruppierungen A und R₄ vorhandene funktionelle Gruppen in geschützter Form vorliegen können, acyliert, oder
c') in einer Verbindung der Formel 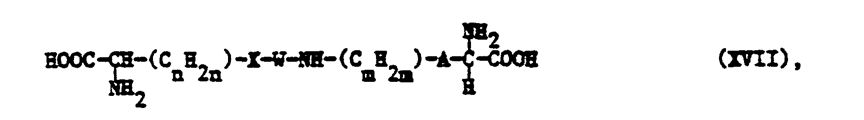 worin die Aminocarbonsäuregruppierung HOOC-CH(NH₂)- in geschützter Form vorliegt, die zu acylierende Aminogruppe gegebenenfalls durch eine, die Acylierung erlaubende Gruppe substituiert ist, und worin die Carboxylgruppe und gegebenenfalls in der Gruppierung A vorhandene weitere funktionelle Gruppen in geschützter Form vorliegen können, die Aminogruppe durch Behandeln mit einem den entsprechenden Acylrest einer Kohlen- oder Thiokohlensäure der Formel 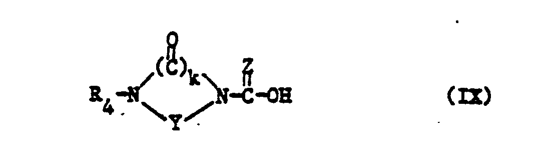 einführenden Acylierungsmittel, worin im Rast R₄ gegebenenfalls vorhandene funktionelle Gruppen in geschützter Form vorliegen können, acyliert, oder
d') ein reaktionsfähiges Derivat einer Kohlensäure- oder Thiokohlensäureverbindung der Formel 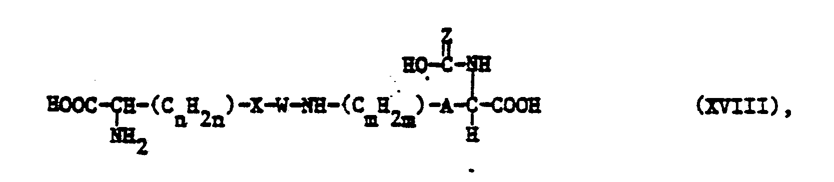 worin die Aminocarbonsäuregruppierung HOOC-CH(NH₂)- in geschützter Form vorliegt, und worin die Carboxylgruppe und gegebenenfalls in der Gruppierung A vorhanden weitere funktionelle Gruppen in geschützter Form vorliegen können, mit einem sekundären Amid der Formel 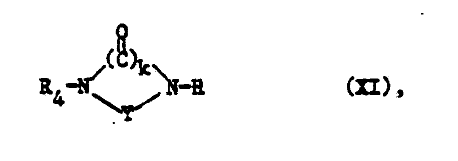 worin im Rest R₄ gegebenenfalls vorhandene funktionelle Gruppen geschützt sein können und die sekundäre Amidogruppe in einer die Acylierung erlaubenden Form vorliegt, reagieren lässt, oder
e') die unter c') oder d') genannte Acylierung in situ vornimmt, indem man eine Aminoverbindung der Formel XVII und eine Amidoverbindung der Formel XI, beide mit der oben angegebenen Bedeutung, gleichzeitig oder nacheinander auf ein bivalentes reaktives Derivat der Kohlen-oder Thiokohlensäure einwirken lässt, oder indem man
f) eine Verbindung der Formel 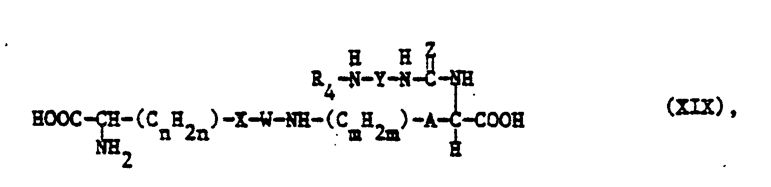 worin die Aminocarbonsäuregruppierung HOOC-CH(NH₂)- in geschützter Form vorliegt, und worin die Carboxylgruppe und gegebenenfalls in der Gruppierung A vorhandene weitere funktionelle Gruppen in geschützter Form vorliegen können, und die später den Piperazinring bildende sekundären Amino- bzw. Amidogruppen gegebenenfalls durch Gruppen substituiert sind, die die N-Acylierung erlauben, mit einem bivalenten acylierenden Derivat der Kohlensäure oder der Oxalsäure unter Ringschluss diacyliert, und gewünxchtenfalls in einer erhaltenen Verbindung der Formel III die geschützte Carboxylgruppe in eine freie Carboxylgruppe oder in ein reaktionsfähiges funktionelles Derivat davon überführt.

21. Verfahren zur Herstellung von Verbindungen der Formel 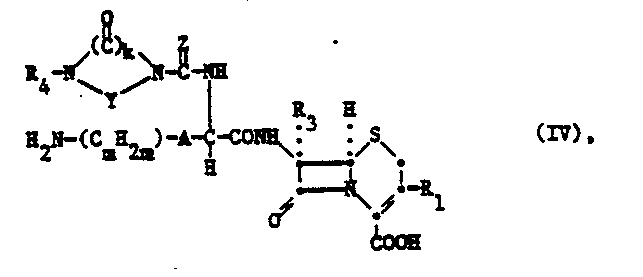 worin der Index m 0 oder 1, X Sauerstoff, Schwefel oder die Gruppe -NH-, W eine Gruppe -CO-, -CO-NHSO₂- oder -SO₂NH-CO-, oder X-W zusammen eine Gruppe -CO-.oder -CO-NHSO₂-, A gegebenenfalls substituiertes Phenylen, Thienylen oder Furylen, Z.Sauerstoff oder Schwefel, Y Niederalkylen, der Index k den Wert 1 oder 2, R₄ Wasserstoff, einen gegebenenfalls substituierten niederaliphatischen oder cycloaliphatischen Rest oder Acyl, R₁ Wesserstoff, Niederalkyl, Niederalkoxy, Halogen oder eine Gruppe der Formel -CH₂-R₂, worin R₂ eine freie, veresterte oder verätherte Hydroxy- oder Mercaptogruppe oder eine quaternäre Ammoniumgruppe darstellt, und R₃ Wasserstoff oder Methoxy bedeuten, worin die Carboxylgruppen gegebenenfalls in physiologisch spaltbarer Form verestert sind, und Salzen von solchen Verbindungen mit salzbildenden Gruppen, dadurch gekennzeichnet, dass man
a") in einer Verbindung der Formel 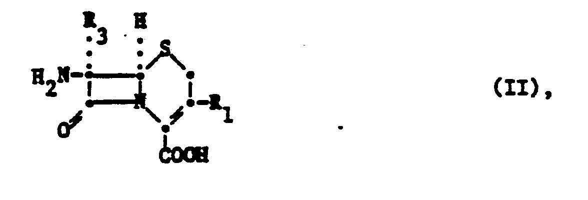 worin die Aminogruppe gegebenenfalls durch eine die Acylierung erlaubende Gruppe substituiert ist, und worin die 4-Carboxylgruppe und gegebenenfalls im Rest R₁ vorhandene weitere funktionelle Gruppen in geschützter Form vorliegen können, die Aminogruppe durch Behandeln mit einer Säure der Formel 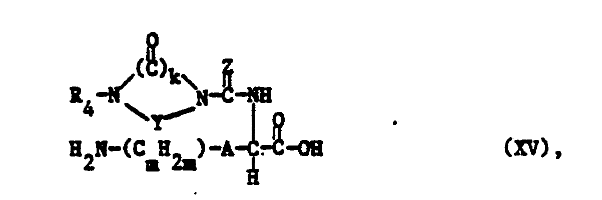 worin die Aminogruppe und gegebenenfalls in den Gruppierungen A und R₄ vorhandene weitere funktionelle Gruppen in geschützter Form vorliegen, oder mit einem reaktionsfähigen funktionellen Säurederivat oder einem Salz davon acyliert, oder
b") in einer Verbindung der Formel 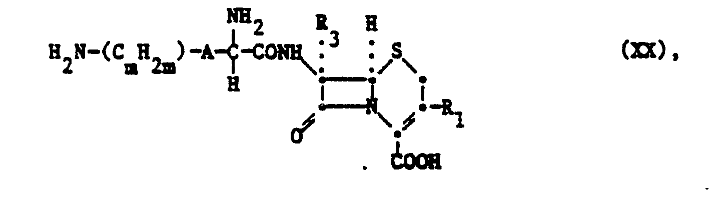 worin die endständige Aminogruppe in geschützter Form vorliegt, die zu acylierende Aminogruppe gegabenaafalls durch eine die Acylierung erlaubende Gruppe substituiert ist, und worin die 4-Carboxylgruppe und gegebenenfalls im Rest R₁ und in der Gruppierung A vorhandende weitere funktionelle Gruppen in geschützter Form vorliegen können, die α-Aminogruppe durch Behandeln mit einem den entsprechenden Acylrest einer Kohlen- oder Thiokohlensäure der Formel 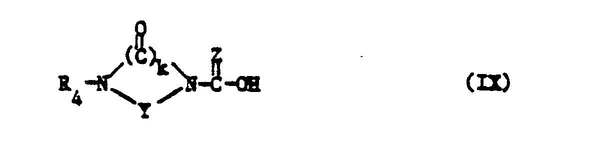 einführenden Acylierungsmittel, worin im Rest R₄ gegebenenfalls vorhandene funktionelle Gruppen in geschützter Form vorliegen können, acyliert, oder
c") ein reaktionsfähiges Derivat einer Kohlensäure- oder Thiokohlensäureverbindung der Formel 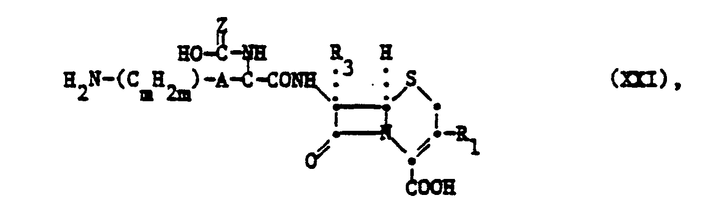 worin die endständige Aminogruppe in geschützter Form vorliegt, und worin die 4-Carboxylgruppe und gegebenenfalls im Rest R₁ und in der Gruppierung A vorhandene weitere funktionelle Gruppen in geschützter Form vorliegen können, mit einem sekundären Amid der Formel 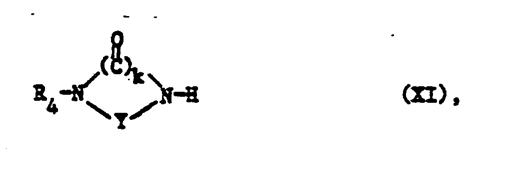 worin im Rest R₄ gegebenenfalls vorhandene funktionelle Gruppen geschützt sein können und die sekundäre Amidogruppe in einer die Acylierung erlaubenden Form vorliegt, reagieren lässt, oder
d") die unter b") oder c") genannte Acylierung in situ vornimt, indem man eine Aminoverbindung der Formel XX und eine Amidoverbindung der Formel XI, beide mit der oben angegebenen Bedeutung, gleichzeitig oder nacheinander auf ein bivalentes reaktives Derivat der Kohlen-oder Thiokohlensäure einwirken lässt, oder indem man
e") eine Verbindung der Formel 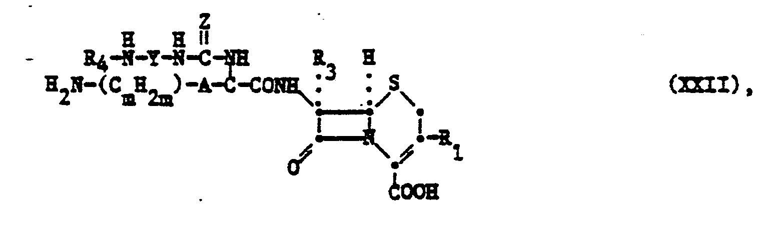 worin die endständige Aminogruppe in geschützter Form vorliegt, und worin die 4-Carboxylgruppe und gegebenenfalls im Rest R₁ und in der Gruppierung A vorhandene weitere funktionelle Gruppen in geschützter Form vorliegen können, und die später den Diazaring bildende sekundäre Amino- bzw. Amidogruppe gegebenenfalls durch Gruppen substituiert sind, die die N-Acylierung erlauben, mit einem bivalenten acylierenden Derivat der Kohlensäure oder der Oxalsäure unter Ringschluss diacyliert, oder
f") eine 2-Cephemverbindung der Formel 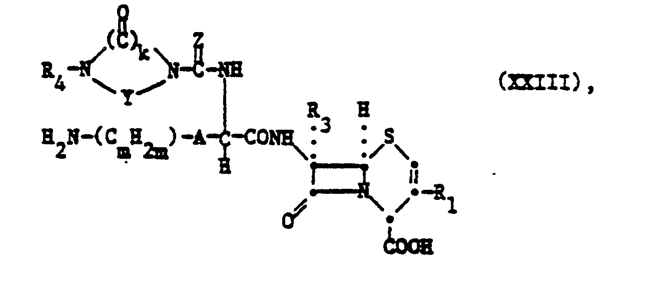 worin funktionelle Gruppen geschützt sind, zur entsprechenden 3-Caphemverbindung der Formel IV isomerisiert, oder
g") in einer Verbindung der Formel 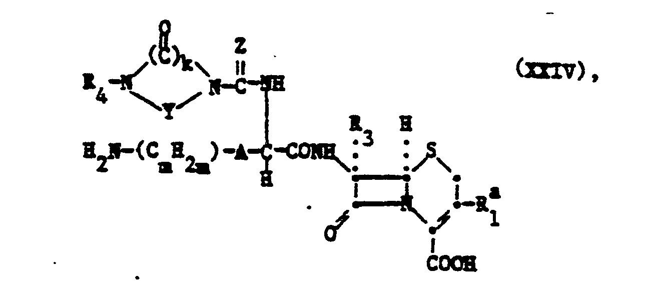 worin 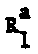 eine freie oder sulfonylierte Hydroxygruppe ist und worin andere funktionelle Gruppen geschützt sind, 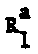 gegen Halogen austauscht, oder
h") in einer Verbindung der Formel XXIV, worin 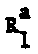 eine freie Hydroxygruppe ist und worin andere funktionelle Gruppen geschützt sind, die Hydroxygruppe veräthert, oder
i") in einer Verbindung der Formel XXIV, worin funktionelle Gruppen geschützt sind, und worin 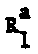 eine Formylgruppe bedeutet, diese in Gegenwart von Decarbonylierungs-Katalysatoren zu einer Verbindung der Formel I, worin R₁ Wasserstoff bedeutet, decarbonyliert, oder j") in einer Verbindung der Formel XXIV, worin 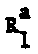 freies oder vereatertes Hydroxy oder ein gegebenenfalls niederalkyliertes oder durch Niederalkylen oder Heteroniederalkylen cyclisch disubstituiertes Amino ist, und worin funktionelle Gruppen geschützt sind, die Gruppe 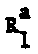 durch Wasserstoff ersetzt,
und, wenn erwünscht, in einer erhaltenen Verbindung die Schutzgruppen abspaltet oder noch nicht geschützte funktionelle Gruppen schützt oder eine Schutzgruppe gegen eine andere austauscht, und/oder, wenn gewünscht, in einem Rest R₁ eine Gruppe R₂ gegen eine andere Gruppe R₂ austauscht, und/oder, wenn erwünscht, eine erhaltene Verbindung, worin R₃ Wasserstoff ist, in eine Verbindung überführt, worin R₃ Methoxy ist, und/oder, wenn gewünscht, ein erhaltenen Isomerengemisch in die einzelnen Isomeren auftrennt, und/oder, wenn gewünscht eine erhaltene Verbindung in ein Salz oder ein erhaltenes Salz in die freie Verbindung oder in ein anderes Salz überfuhrt.

22. Verfahren zur Herstellung eines pharmazeutischen Präparates, dadurch gekennzeichnet, dass man eine gemäss Patentanspruch 21 erhaltene Verbindung mit einem pahrmazeutischen Trägermaterial verarbeitet.

23. Verfahren zur Herstellung von Verbindungen der Formel XV gmiLss Patentanspruch 19, und ihren geschützten und reaktionsfähigen Derivaten, dadurch gekennzeichnet, dass man
a"') in einer Verbindung der Formel 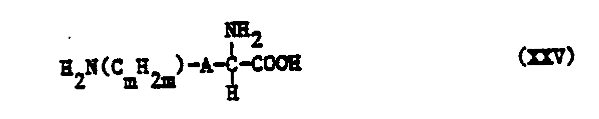 worin die endständige Aminogruppe in geschützter Form vorliegt, die zu acylierende Aminogruppe gegebenenfalls durch eine, die Acylierung erlaubende Gruppe substituiert ist, und worin die Carboxylgruppe und gegebenenfalls in der Gruppierung A vorhandene weitere funktionelle Gruppen in geschützter Form vorliegen können, die α-Aminogruppe durch Behandeln mit einem den entsprechenden Acylrest einer Kohlen-oder Thiokohlensäure der Formel 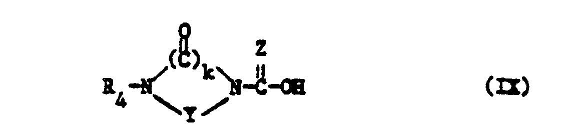 einführenden Acylierungsmittel, worin im Rest R₄ gegebenenfalls vorhandene funktionelle Gruppen in geschützter Form vorliegen können, acyliert, oder
b"') ein reaktionsfähiges Derivat einer Kohlensäure- oder Thiokohlensäureverbinding der Formel 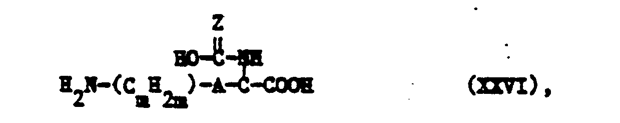 worin die endständige Aminogruppe in geschützter Form vorliegt, und worin die Carboxylgruppe und gegebenenfalls in der Gruppierung A vorhandene weitere funktionelle Gruppen in geschützter Form vorliegen können, mit einem sekundären Amid der Fossel 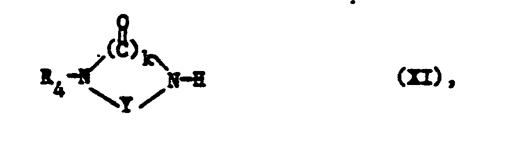 worin im Rest R₄ gegebenenfalls vorhandene funktionelle Gruppen geschützt sein können und die sekundäre Amidogruppe in einer die Acylierung erlaubenden Form vorliegt, reagieren lässt, oder
c"') die unter a"') oder b"') genannte Acylierung in situ vornimmt, indem man eine Aminoverbindung der Formel XXV und eine Amidoverbindung der Formel XI, beide mit der oben angegebenen Bedeutung, gleichzeitig oder nacheinander auf ein bivalentes reaktives Derivat der Kohlen- oder Thiokohlensäure einwirken lässt, oder indem man
d"') eine Verbindung der Förmel 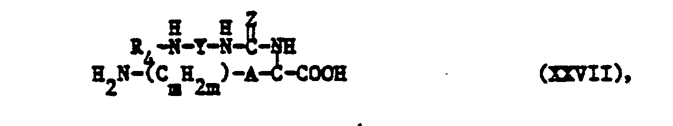 worin die endständige Aminogruppe in geschützter Form vorliegt, und worin die Carboxylgruppe und gegebenenfalls in der Gruppierung A vorhandene weitere funktionelle Gruppen in geschützter Form vorliegen können, und die später den Diazaring bildende sekundäre Amino- bzw. Amidogruppe gegebenenfalls durch Gruppen substituiert sind, die die N-Acylierung erlauben, mit einem bivalente acylierenden Derivat der Kohlensäure oder der Oxalsäure unter Ringschluss diacyliert, und gewünschtenfalls in einer erhaltenen Verbindung der Formel XIV die geschützte Carboxylgruppe in eine freie Carboxylgruppe oder in ein reaktionsfähiges funktionelles Derivat davon überführt.
